# EUROPEAN PATENT APPLICATION

(11) **EP 4 768 582 A2**
(43) Date of publication of application: **01.07.2026**
(21) Application number: 24799914.7
(22) Date of filing: 19.08.2024
(51) Int. Cl.: C12N 15/113, A61K 31/713, A61K 47/54, A61P 9/12

(54) **SIRNA TARGETING AGT GENE EXPRESSION AND CONJUGATE AND USE THEREOF**

(30) Priority: 22.08.2023 CN 202311056610; 07.02.2024 CN 202410174415; 07.06.2024 CN 202410742179
(71) Applicant: Leaderna Therapeutics, Ltd., Chengdu, Sichuan 610219 (CN)
(72) Inventor: LIU, Zhaogui, Chengdu, Sichuan 610219 (CN); WAN, Jinqiao, Chengdu, Sichuan 610219 (CN); WANG, Zhao, Chengdu, Sichuan 610219 (CN); ZHOU, Jiehua, Chengdu, Sichuan 610219 (CN)
(74) Representative: Zanoli, Enrico
(86) International application number: PCT/CN2024/113157
(87) International publication number: WO 2024/227458

(57) **Abstract**

Provided in the present invention are an siRNA that inhibits AGT gene expression and a conjugate thereof. The siRNA contains a sense strand and an antisense strand. The siRNA, siRNA conjugate and pharmaceutical composition provided in the present invention have good stability, an excellent inhibitory activity against the AGT gene, and satisfactory cytotoxicity and immunostimulatory activities.

## Description

### Technical Field

The present invention relates to a siRNA that inhibits the expression of angiotensinogen (AGT) gene, and conjugate and pharmaceutical composition thereof, as well as use thereof in the prevention and/or treatment of abnormal blood pressure-related diseases.

### Background Art

Angiotensinogen (AGT) is an important component of the renin-angiotensin system (RAS) in the human body, and the entire RAS plays a key role in the regulation of blood pressure in animals. AGT is synthesized and secreted in the liver, and then converted into angiotensin I by the enzyme renin (REN), which is then converted into angiotensin II by the angiotensin-converting enzyme (ACE). Most angiotensin II can bind to type I angiotensin II receptors, leading to vasoconstriction and raised blood pressure. This molecule also stimulates the production of the hormone aldosterone, which triggers the renal absorption of Na⁺ ions and water, thereby increasing blood volume and further raising blood pressure.

Hypertension is a serious disease that significantly increases the risk of heart, brain, kidney, and other diseases. According to data released by the World Health Organization, an estimated 1.28 billion adults aged 30-79 worldwide suffer from hypertension. Only 42% of hypertensive patients are diagnosed and treated, and only 21% have their blood pressure effectively controlled. Hypertension is a leading cause of premature death worldwide. Furthermore, according to a report in The Lancet (2019; 394: 1145-58), 2.54 million people died from hypertension in our country in 2017, ranking first among all risk factors for death.

Currently, first-line drugs used clinically to treat hypertension are divided into five main classes: angiotensinase inhibitors, angiotensin II receptor blockers, β-adrenergic receptor blockers, dihydropyridine calcium channel blockers, and diuretics. These five classes of antihypertensive drugs have completely different mechanisms of action. Depending on the patient's age, degree of hypertension, and risk of related clinical complications, most patients will use one of these drugs for treatment. If one method is ineffective, two or even three methods may be used in combination. For high-risk patients, two or even three treatment methods are often used in combination. If blood pressure cannot be effectively controlled by these three methods, it is called refractory hypertension, a common and challenging problem in hypertension treatment. Developing effective, safe, stable, and long-acting antihypertensive drugs has significant clinical value.

The present application provides a small interfering RNA (siRNA) formulation targeting AGT, which specifically binds to AGT mRNA, disrupting the normal translation template function of AGT mRNA and thus preventing the translation of AGT protein. This inhibits the RAS pathway at its source and can be used to treat/prevent diseases related to the RAS pathway, such as hypertension, including refractory and uncontrolled hypertension.

Compared to traditional drugs, siRNA has poor stability and is easily degraded by nucleases when administered systemically. Furthermore, it is necessary to explore ways to further improve its activity while avoiding off-target effects, immune stimulation, cytotoxicity, and other side effects. Therefore, developing more candidate siRNAs that are stable in the blood, possess good in vivo biological activity, have low cytotoxicity, and can effectively inhibit AGT gene expression for a long time is an urgent problem to be solved. At the same time, there is both the necessity for clinical research and the commercial viability of developing drugs using these candidate siRNAs that inhibit AGT gene expression to effectively prevent and/or treat hypertension-related diseases.

### Contents of the Invention

The present invention provides an siRNA for inhibiting AGT gene expression, the siRNA comprising a sense strand and an antisense strand; wherein the antisense strand comprises at least 17 consecutive nucleotides differing by no more than 4 nucleotides from any one of the nucleotide sequences as set forth in SEQ ID NO: 102 to SEQ ID NO: 192, SEQ ID NO: 201 to SEQ ID NO: 207, SEQ ID NO: 209 to SEQ ID NO: 220, SEQ ID NO: 222 to SEQ ID NO: 223, SEQ ID NO: 225 to SEQ ID NO: 233, SEQ ID NO: 235 to SEQ ID NO: 250, SEQ ID NO: 252 to SEQ ID NO: 291, SEQ ID NO: 293 to SEQ ID NO: 341, SEQ ID NO: 343 to SEQ ID NO: 346, the antisense strand is 17-30 nucleotides in length; and the sense strand is 17-30 nucleotides in length and is at least partially complementary to the antisense strand.

The term "at least partially complementary" means that the two sequences can be completely complementary, or have no more than 5, 4, 3, or 2 mismatched base pairs in total, while retaining the ability to hybridize under relevant conditions.

In some embodiments of the present invention, the antisense strand differs by no more than 4 nucleotides from any one of the nucleotide sequences as set forth in SEQ ID NO: 102 to SEQ ID NO: 192, SEQ ID NO: 201 to SEQ ID NO: 207, SEQ ID NO: 209 to SEQ ID NO: 220, SEQ ID NO: 222 to SEQ ID NO: 223, SEQ ID NO: 225 to SEQ ID NO: 233, SEQ ID NO: 235 to SEQ ID NO: 250, SEQ ID NO: 252 to SEQ ID NO: 291, SEQ ID NO: 293 to SEQ ID NO: 341, and SEQ ID NO: 343 to SEQ ID NO: 346; in some embodiments of the present invention, the antisense strand differs by no more than 3 nucleotides from any one of the nucleotide sequences as set forth in SEQ ID NO: 102 to SEQ ID NO: 192, SEQ ID NO: 201 to SEQ ID NO: 207, SEQ ID NO: 209 to SEQ ID NO: 220, SEQ ID NO: 222 to SEQ ID NO: 223, SEQ ID NO: 225 to SEQ ID NO: 233, SEQ ID NO: 235 to SEQ ID NO: 250, SEQ ID NO: 252 to SEQ ID NO: 291, SEQ ID NO: 293 to SEQ ID NO: 341, and SEQ ID NO: 343 to SEQ ID NO: 346; in some embodiments of the present invention, the antisense strand differs by no more than 2 nucleotides from any one of the nucleotide sequences as set forth in SEQ ID NO: 102 to SEQ ID NO: 192, SEQ ID NO: 201 to SEQ ID NO: 207, SEQ ID NO: 209 to SEQ ID NO: 220, SEQ ID NO: 222 to SEQ ID NO: 223, SEQ ID NO: 225 to SEQ ID NO: 233, SEQ ID NO: 235 to SEQ ID NO: 250, SEQ ID NO: 252 to SEQ ID NO: 291, SEQ ID NO: 293 to SEQ ID NO: 341, and SEQ ID NO: 343 to SEQ ID NO: 346; in some embodiments of the present invention, the antisense strand differs by no more than 1 nucleotides from any one of the nucleotide sequences as set forth in SEQ ID NO: 102 to SEQ ID NO: 192, SEQ ID NO: 201 to SEQ ID NO: 207, SEQ ID NO: 209 to SEQ ID NO: 220, SEQ ID NO: 222 to SEQ ID NO: 223, SEQ ID NO: 225 to SEQ ID NO: 233, SEQ ID NO: 235 to SEQ ID NO: 250, SEQ ID NO: 252 to SEQ ID NO: 291, SEQ ID NO: 293 to SEQ ID NO: 341, and SEQ ID NO: 343 to SEQ ID NO: 346; in some embodiments of the invention, the antisense strand is any one of the nucleotide sequences as set forth in SEQ ID NO: 102 to SEQ ID NO: 192, SEQ ID NO: 201 to SEQ ID NO: 207, SEQ ID NO: 209 to SEQ ID NO: 220, SEQ ID NO: 222 to SEQ ID NO: 223, SEQ ID NO: 225 to SEQ ID NO: 233, SEQ ID NO: 235 to SEQ ID NO: 250, SEQ ID NO: 252 to SEQ ID NO: 291, SEQ ID NO: 293 to SEQ ID NO: 341, and SEQ ID NO: 343 to SEQ ID NO: 346.

In some embodiments of the present invention, the sense strand and antisense strand have a mismatch of no more than 3 nucleotides; in some embodiments, the sense strand and antisense strand have a mismatch of no more than 2 nucleotides; in some embodiments, the sense strand and antisense strand have a mismatch of no more than 1 nucleotide; in some embodiments, the sense strand and antisense strand are completely complementary.
preferably, the sense strand and antisense strand are complementary by at least 15, 16, 17, 18, 19, 20, or 21 nucleotides.

In some embodiments of the present invention, the antisense strand is 19-27 nucleotides in length; and the sense strand is 19-25 nucleotides in length.

In some embodiments of the present invention, the antisense strand is 19-23 nucleotides in length; and the sense strand is 19-21 nucleotides in length.

In some embodiments of the present invention, the antisense strand is 23 nucleotides in length, and the sense strand is 21 nucleotides in length. In some embodiments of the present invention, the antisense strand is 22 nucleotides in length, and the sense strand is 20 nucleotides in length. In some embodiments of the invention, the antisense strand is 21 nucleotides in length, and the sense strand is 21 nucleotides in length. In some embodiments of the invention, the antisense strand is 21 nucleotides in length, and the sense strand is 19 nucleotides in length. In some embodiments of the invention, the antisense strand is 19 nucleotides in length, and the sense strand is 19 nucleotides in length.

In some embodiments of the invention, the siRNA comprises one or more single-stranded nucleotide overhangs, such as overhangs of 1, 2, 3, or 4 nucleotides. In some embodiments of the invention, the overhangs can be on the sense strand, the antisense strand, or any combination thereof. In some embodiments of the invention, the overhangs are present at the 5' end, 3' end, or both ends of the antisense strand or sense strand of the siRNA.

In some embodiments of the invention, the siRNA has a 2-nucleotide overhang at the 3' end of the antisense strand.

In some embodiments of the invention, the siRNA has a blunt end. In some embodiments of the invention, the siRNA has at least one blunt end located at the 5' end of the antisense strand (or the 3' end of the sense strand).

In some embodiments of the invention, the siRNA has two blunt ends.

In some embodiments of the present invention, the siRNA has a nucleotide sequence (5'→3') selected from Duplex 1 to Duplex 107:

| Duplex No. | Sense strand (5'→3') | SEQ ID NO: | Antisense strand (5'→3') | SEQ ID NO: |
|---|---|---|---|---|
| 1 | GAGGUGCUGAACAGCAU UUUU | 1 | AAAAAUGCUGUUCAGCAC CUCCC | 102 |
| 2 | GCUCCCACCUUUUCUUCU AA | 2 | UUAGAAGAAAAGGUGGG AGCCU | 103 |
| 3 | CACCUUUUCUUCUAAUGA A | 3 | UUCAUUAGAAGAAAAGG UGUU | 104 |
| 4 | CCAGCUUUUCUUCUAAUG AA | 4 | UUCAUUAGAAGAAAAGG UGGUU | 105 |
| 5 | CGAGCUUUUCUUCUAAUG AA | 5 | UUCAUUAGAAGAAAAGG UGGUU | 105 |
| 6 | AGAGUCUAUCCAACAGCU UA | 6 | UAAGCUGUUGGUAGAC UCUGU | 106 |
| 7 | AGAGUCUACUCAACAGCU UA | 7 | UAAGCUGUUGGUAGAC UCUGU | 106 |
| 8 | AGAGUCUACCUAACAGCU UA | 8 | UAAGCUGUUGGUAGAC UCUGU | 106 |
| 9 | GGGUCUCAUUUUCCAGCA AU | 9 | AUUGCUGGAAAGUGAGA CCCUC | 107 |
| 10 | GGGUCUCACUUUUCAGCA AU | 10 | AUUGCUGGAAAGUGAGA CCCUC | 107 |
| 11 | GGGUCUCACUUUCUAGCA AU | 11 | AUUGCUGGAAAGUGAGA CCCUC | 107 |
| 12 | AGGUGCUAAACAGCAUU UUU | 12 | AAAAAUGCUGUUCAGCAC CUCC | 108 |
| 13 | AGGUGCUGAAUAGCAUU UUU | 13 | AAAAAUGCUGUUCAGCAC CUCC | 108 |
| 14 | AGGUGCUGAACAGUAUU UUU | 14 | AAAAAUGCUGUUCAGCAC CUCC | 108 |
| 15 | AGGUGCUGAACAGCAUU | 15 | AAAAAUGCUGUUCAGCAC | 109 |
| | UUU | | CUUU | |
| 16 | AGGUGCUGAACAGCAUU UUA | 16 | UAAAAUGCUGUUCAGCAC CUUU | 110 |
| 17 | GGUGCUGAACAGCAUUU UU | 17 | AAAAAUGCUGUUCAGCAC CUU | 111 |
| 18 | GGUGCUGAACAGCAUUU UA | 18 | UAAAAUGCUGUUCAGCAC CUU | 112 |
| 19 | GGGUCUCACUUUCCAGCA AU | 19 | AUUGCUGGAAAGUGAGA CCCUU | 113 |
| 20 | AGAGUCUACCCAACAGCU UA | 20 | UAAGCUGUUGGUAGAC UCUUU | 114 |
| 21 | UCUCCCACCUUUUCUUCU AA | 21 | UUAGAAGAAAAGGUGGG AGAUU | 115 |
| 22 | CUCCCACCUUUUCUUCUA AA | 22 | UUUAGAAGAAAAGGUGG GAGUU | 116 |
| 23 | CCACCUUUUCUUCUAAUG AA | 23 | UUCAUUAGAAGAAAAGG UGGUU | 105 |
| 24 | AGGGUCUCACUUUCCAGC AAU | 24 | AUUGCUGGAAAGUGAGA CCCUCC | 117 |
| 25 | GGGUCUCACUUUCCAGCA AU | 19 | AUUGCUGGAAAGUGAGA CCCUC | 107 |
| 26 | GGUCUCACUUUCCAGCAA U | 25 | AUUGCUGGAAAGUGAGA CCCU | 118 |
| 27 | AGGUGCUGAACAGCAUU UUU | 15 | AAAAAUGCUGUUCAGCAC CUCC | 108 |
| 28 | GGUGCUGAACAGCAUUU UU | 17 | AAAAAUGCUGUUCAGCAC CUC | 119 |
| 29 | CAGAGUCUACCCAACAGC UUA | 26 | UAAGCUGUUGGGUAGAC UCUGUG | 120 |
| 30 | AGAGUCUACCCAACAGCU UA | 20 | UAAGCUGUUGGGUAGAC UCUGU | 106 |
| 31 | GAGUCUACCCAACAGCUU A | 27 | UAAGCUGUUGGUAGAC UCUG | 121 |
| 32 | AGGUGCUGAACAGCAUU UUA | 16 | UAAAAUGCUGUUCAGCAC CUCC | 122 |
| 33 | GGUGCUGAACAGCAUUU UA | 18 | UAAAAUGCUGUUCAGCAC CUC | 123 |
| 34 | UCUCCCACCUUUUCUUCU AA | 21 | UUAGAAGAAAAGGUGGG AGACU | 124 |
| 35 | CUCCCACCUUUUCUUCUA AA | 22 | UUUAGAAGAAAAGGUGG GAGAC | 125 |
| 36 | CUCCCACCUUUUCUUCUA A | 28 | UUAGAAGAAAAGGUGGG AGUU | 126 |
| 37 | CCACCUUUUCUUCUAAUG AA | 23 | UUCAUUAGAAGAAAAGG UGGGG | 127 |
| 38 | CCACCUUUUCUUCUAAUG AA | 23 | AUCAUUAGAAGAAAAGG UGGUU | 128 |
| 39 | CCACCUUUUCUUCUAAUG AU | 29 | AUCAUUAGAAGAAAAGG UGGUU | 128 |
| 40 | CUCCCACUUUUUCUUCUA AA | 30 | UUUAGAAGAAAAGGUGG GAGAC | 125 |
| 41 | CUACCUUUUCUUCUAAUG | 31 | UUCAUUAGAAGAAAAGG | 105 |
| | AA | | UGGUU | |
| 42 | GAGGUGCUGAACAGCAU UUUA | 32 | UAAAAUGCUGUUCAGCAC CUCCC | 129 |
| 43 | GCGGAUGAGAGAGAGCCC ACA | 33 | UGUGGGCUCUCUCUCAUC CGCUU | 130 |
| 44 | CGGAUGAGAGAGAGCCCA CAG | 34 | CUGUGGGCUCUCUCUCAU CCGCU | 131 |
| 45 | GAGAGAGAGCCCACAGAG UCU | 35 | AGACUCUGUGGCUCUCU CUCGG | 132 |
| 46 | AGCCCACAGAGUCUACCC AAU | 36 | AUUGGGUAGACUCUGUG GGCUCU | 133 |
| 47 | CGGAUGAGAGAGAGCCCA CAA | 37 | UUGUGGGCUCUCUCUCAU CCGGG | 134 |
| 48 | GCCCACAGAGUCUACCCA ACA | 38 | UGUUGGGUAGACUCUGU GGGCUC | 135 |
| 49 | GCCCACAGAGUCUACCCA ACA | 38 | UGUUGGGUAGACUCUGU GGGC | 136 |
| 50 | CCACAGAGUCUACCCAAC A | 39 | UGUUGGGUAGACUCUGU GG | 137 |
| 51 | CCACAGAGUCUACCCAAC A | 39 | UGUUGGGUAGACUCUGU GGGC | 136 |
| 52 | CCCACAGAGUCUACCCAA CAA | 40 | UUGUUGGGUAGACUCUG UGGGCU | 138 |
| 53 | CCCACAGAGUCUACCCAA CAA | 40 | UUGUUGGGUAGACUCUG UGGG | 139 |
| 54 | CACAGAGUCUACCCAACA A | 41 | UUGUUGGGUAGACUCUG UG | 140 |
| 55 | CACAGAGUCUACCCAACA A | 41 | UUGUUGGGUAGACUCUG UGGG | 139 |
| 56 | UCCCACCUUUUCUUCUAA A | 42 | UUUAGAAGAAAAGGUGG GAUU | 141 |
| 57 | CACCCCCAGUCUCCCACC UU | 43 | AAGGUGGGAGACUGGGG GUGAC | 142 |
| 58 | ACCCCCAGUCUCCCACCU UU | 44 | AAAGGUGGGAGACUGGG GGUGA | 143 |
| 59 | CCCCCAGUCUCCCACCUU UU | 45 | AAAAGGUGGGAGACUGG GGGUG | 144 |
| 60 | CCCCAGUCUCCCACCUUU UU | 46 | AAAAAGGUGGAGACUG GGGGU | 145 |
| 61 | CCCAGUCUCCCACCUUUU CU | 47 | AGAAAAGGUGGAGACU GGGGG | 146 |
| 62 | CCAGUCUCCCACCUUUUC UU | 48 | AAGAAAAGGUGGGAGAC UGGGG | 147 |
| 63 | CAGUCUCCCACCUUUUCU UU | 49 | AAAGAAAAGGUGGAGA CUGGG | 148 |
| 64 | AGUCUCCCACCUUUUCUU CU | 50 | AGAAGAAAAGGUGGGAG ACUGG | 149 |
| 65 | GUCUCCCACCUUUUCUUC UA | 51 | UAGAAGAAAAGGUGGGA GACUG | 150 |
| 66 | UCCCACCUUUUCUUCUAA UA | 52 | UAUUAGAAGAAAAGGUG GGAGA | 151 |
| 67 | CCCACCUUUUCUUCUAAU | 53 | UCAUUAGAAGAAAAGGU | 152 |
| | GA | | GGGAG | |
| 68 | CACCUUUUCUUCUAAUGA GU | 54 | ACUCAUUAGAAGAAAAG GUGGG | 153 |
| 69 | ACCUUUUCUUCUAAUGAG UU | 55 | AACUCAUUAGAAGAAAA GGUGG | 154 |
| 70 | CCUUUUCUUCUAAUGAGU CA | 56 | UGACUCAUUAGAAGAAA AGGUG | 155 |
| 71 | CUUUUCUUCUAAUGAGUC GA | 57 | UCGACUCAUUAGAAGAA AAGGU | 156 |
| 72 | UUUUCUUCUAAUGAGUC GAU | 58 | AUCGACUCAUUAGAAGA AAAGG | 157 |
| 73 | UUUCUUCUAAUGAGUCG ACU | 59 | AGUCGACUCAUUAGAAG AAAAG | 158 |
| 74 | ACCCCCAGUCUCCCACCU U | 60 | AAGGUGGGAGACUGGGG GUGA | 159 |
| 75 | CCCCCAGUCUCCCACCUU U | 61 | AAAGGUGGGAGACUGGG GGUG | 160 |
| 76 | CCCCAGUCUCCCACCUUU U | 62 | AAAAGGUGGGAGACUGG GGGU | 161 |
| 77 | CCCAGUCUCCCACCUUUU U | 63 | AAAAAGGUGGGAGACUG GGGG | 162 |
| 78 | CCAGUCUCCCACCUUUUC U | 64 | AGAAAAGGUGGGAGACU GGGG | 163 |
| 79 | CAGUCUCCCACCUUUUCU U | 65 | AAGAAAAGGUGGGAGAC UGGG | 164 |
| 80 | AGUCUCCCACCUUUUCUU U | 66 | AAAGAAAAGGUGGGAGA CUGG | 165 |
| 81 | GUCUCCCACCUUUUCUUC U | 67 | AGAAGAAAAGGUGGGAG ACUG | 166 |
| 82 | UCUCCCACCUUUUCUUCU A | 68 | UAGAAGAAAAGGUGGGA GACU | 167 |
| 83 | CCCACCUUUUCUUCUAAU A | 69 | UAUUAGAAGAAAAGGUG GGAG | 168 |
| 84 | CCACCUUUUCUUCUAAUG A | 70 | UCAUUAGAAGAAAAGGU GGGA | 169 |
| 85 | ACCUUUUCUUCUAAUGAG U | 71 | ACUCAUUAGAAGAAAAG GUGG | 170 |
| 86 | CCUUUUCUUCUAAUGAGU U | 72 | AACUCAUUAGAAGAAAA GGUG | 171 |
| 87 | CUUUUCUUCUAAUGAGUC A | 73 | UGACUCAUUAGAAGAAA AGGU | 172 |
| 88 | UUUUCUUCUAAUGAGUC GA | 74 | UCGACUCAUUAGAAGAA AAGG | 173 |
| 89 | UUUCUUCUAAUGAGUCG AU | 75 | AUCGACUCAUUAGAAGA AAAG | 174 |
| 90 | UUCUUCUAAUGAGUCGAC U | 76 | AGUCGACUCAUUAGAAG AAAA | 175 |
| 91 | UCACCCCCAGUCUCCCAC CUU | 77 | AAGGUGGGAGACUGGGG GUGACA | 176 |
| 92 | CACCCCCAGUCUCCCACC UUU | 78 | AAAGGUGGGAGACUGGG GGUGAC | 177 |
| 93 | ACCCCCAGUCUCCCACCU | 79 | AAAAGGUGGGAGACUGG | 178 |
| | UUU | | GGGUGA | |
| 94 | CCCCCAGUCUCCCACCUU UUU | 80 | AAAAAGGUGGGAGACUG GGGGUG | 179 |
| 95 | CCCCAGUCUCCCACCUUU UCU | 81 | AGAAAAGGUGGGAGACU GGGGGU | 180 |
| 96 | CCCAGUCUCCCACCUUUU CUU | 82 | AAGAAAAGGUGGAGAC UGGGGG | 181 |
| 97 | CCAGUCUCCCACCUUUUC UUU | 83 | AAAGAAAAGGUGGGAGA CUGGGG | 182 |
| 98 | CAGUCUCCCACCUUUUCU UCU | 84 | AGAAGAAAAGGUGGGAG ACUGGG | 183 |
| 99 | AGUCUCCCACCUUUUCUU CUA | 85 | UAGAAGAAAAGGUGGGA GACUGG | 184 |
| 100 | CUCCCACCUUUUCUUCUA AUA | 86 | UAUUAGAAGAAAAGGUG GGAGAC | 185 |
| 101 | UCCCACCUUUUCUUCUAA UGA | 87 | UCAUUAGAAGAAAAGGU GGGAGA | 186 |
| 102 | CCACCUUUUCUUCUAAUG AGU | 88 | ACUCAUUAGAAGAAAAG GUGGGA | 187 |
| 103 | CACCUUUUCUUCUAAUGA GUU | 89 | AACUCAUUAGAAGAAAA GGUGGG | 188 |
| 104 | ACCUUUUCUUCUAAUGAG UCA | 90 | UGACUCAUUAGAAGAAA AGGUGG | 189 |
| 105 | CCUUUUCUUCUAAUGAGU CGA | 91 | UCGACUCAUUAGAAGAA AAGGUG | 190 |
| 106 | CUUUUCUUCUAAUGAGUC GAU | 92 | AUCGACUCAUUAGAAGA AAAGGU | 191 |
| 107 | UUUUCUUCUAAUGAGUC GACU | 93 | AGUCGACUCAUUAGAAG AAAAGG | 192 |
| 108 | GUUUCUCCUUGGUCUAAG UG | 94 | CACUUAGACCAAGGAGAA ACUU | 193 |
| 109 | CCUGUGGUCCUCCCACGC UC | 95 | GAGCGUGGGAGGACCACA GGUU | 194 |
| 110 | ACAUACACCCCUUCCACC UC | 96 | GAGGUGGAAGGGUGUA UGUUU | 195 |
| 111 | GGUGUACAUACACCCCUU CC | 97 | GGAAGGGGUGUAUGUAC ACCUU | 196 |
| 112 | GUGGAGGGUCUCACUUUC CA | 98 | UGGAAAGUGAGACCCUCC ACUU | 197 |
| 113 | UACGUCCACUUCCAAGGG AA | 99 | UUCCCUUGGAAGUGGACG UAUU | 198 |
| 114 | CAGUCUCCCACCUUUUCU CC | 100 | UAAGAAAAGGUGGGAGA CUGUU | 199 |
| 115 | UGUGGUCCUCCCACGCUC UC | 101 | GAGAGCGUGGGAGGACC ACAUU | 200 |

In some embodiments of the present invention, the siRNA compirses at least one modified nucleotide.

In some embodiments of the present invention, all nucleotides in the sense and/or antisense strands of the siRNA are modified nucleotides or nucleotide analogs.

In some embodiments of the present invention, the modified nucleotide is selected from 2'-methoxynucleotides, 2'-fluoronucleotides, 2'-deoxynucleotides, 2',3'-seco-nucleotide analogs, 2'-fluoroarabinonucleotides, 2'-methoxyethylnucleotides, 2'-amino-modified nucleotides, 2'-alkyl-modified nucleotides, 3'-methoxynucleotides, 2'-allyl-modified nucleotides, phosphorothioate group-containing nucleotides, methylphosphonate group-containing nucleotides, 5'-phosphate group-containing nucleotides, 5'-phosphate mimic-containing nucleotides, diol-modified nucleotides, abasic nucleotides, morpholinonucleotides, locked nucleotides (LNA), unlocked nucleotides (UNA), threose nucleotides (TNA), or glycerol nucleotides (GNA), but the present invention is not limited thereto.

In some embodiments of the present invention, the antisense strand of the siRNA is 23 nucleotides in length, wherein positions 2, 6, 14, and 16 of the 5' end of the antisense strand are 2'-fluoronucleotides, and the remaining positions are 2'-methoxynucleotides; the sense strand of the siRNA is 21 nucleotides in length, wherein positions 7, 9, 10, and 11 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 6, 8, 9, 14, and 16 of the 5' end of the antisense strand are 2'-fluoronucleotides, and the remaining positions are 2'-methoxynucleotides; the sense strand of the siRNA is 21 nucleotides in length, wherein positions 7, 9, 10, and 11 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 6, 14, and 16 of the 5' end of the antisense strand are 2'-fluoronucleotides, and the remaining positions are 2'-methoxynucleotides; the sense strand of the siRNA is 21 nucleotides in length, wherein positions 9, 10, and 11 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 6, 8, 9, 10, 14, and 16 of the 5' end of the antisense strand are 2'-fluoronucleotides, and the remaining positions are 2'-methoxynucleotides; the sense strand of the siRNA is 21 nucleotides in length, wherein positions 7, 9, 10, and 11 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 6, 8, 9, 10, 14, and 16 of the 5' end of the antisense strand are 2'-fluoronucleotides, and the remaining positions are 2'-methoxynucleotides; the sense strand of the siRNA is 21 nucleotides in length, wherein positions 7, 9, 10, 11, and 15 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 6, 10, 14, and 16 of the 5' end of the antisense strand are 2'-fluoronucleotides, and the remaining positions are 2'-methoxynucleotides; the sense strand of the siRNA is 21 nucleotides in length, wherein positions 3, 7, 9, 10, and 11 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 6, 14, and 16 of the 5' end of the antisense strand are 2'-fluoronucleotides, and the remaining positions are 2'-methoxynucleotides; the sense strand of the siRNA is 21 nucleotides in length, wherein positions 7, 9, 10, and 11 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 3, 4, 6, 8, 10, 14, 16, 18, 20, and 22 of the 5' end of the antisense strand are 2'-fluoronucleotides, and the remaining positions are 2'-methoxynucleotides; the sense strand of the siRNA is 21 nucleotides in length, wherein position 2 of the 5' end of the sense strand is 2'-fluoronucleotide, and the remaining positions are 2'-methoxynucleotides.

In some embodiments of the present invention, the antisense strand of the siRNA is 23 nucleotides in length, wherein positions 2, 4, 5, 6, 8, 10, 14, 16, 18, 20, and 22 at the 5' end of the antisense strand are 2'-fluoronucleotides, and the remaining positions are 2'-methoxynucleotides; the sense strand of the siRNA is 21 nucleotides in length, wherein position 2 at the 5' end of the sense strand is 2'-fluoronucleotide, and the remaining positions are 2'-methoxynucleotides.

In some embodiments of the present invention, the antisense strand of the siRNA is 23 nucleotides in length, wherein positions 2, 4, 6, 7, 8, 10, 14, 16, 18, 20, and 22 at the 5' end of the antisense strand are 2'-fluoronucleotides, and the remaining positions are 2'-methoxynucleotides; the sense strand of the siRNA is 21 nucleotides in length, wherein position 2 at the 5' end of the sense strand is 2'-fluoronucleotide, and the remaining positions are 2'-methoxynucleotides.

In some embodiments of the present invention, the antisense strand of the siRNA is 23 nucleotides in length, wherein positions 2, 4, 6, 8, 10, 14, 16, 18, and 20 at the 5' end of the antisense strand are 2'-fluoronucleotides, and the remaining positions are 2'-methoxynucleotides; the sense strand of the siRNA is 21 nucleotides in length, wherein positions 1, 3, 5, 7, 9, 10, 11, 13, 15, 17, 19, and 21 at the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 4, 6, 8, 10, 14, 16, 18, 20, and 22 at the 5' end of the antisense strand are 2'-fluoronucleotides, and the remaining positions are 2'-methoxynucleotides; the sense strand of the siRNA is 21 nucleotides in length, wherein positions 1, 3, 5, 7, 9, 10, 11, 13, 15, 17, 19, and 21 at the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 6, 8, 9, 14, and 16 of the 5' end of the antisense strand are 2'-fluoronucleotides, position 7 is a glycerol nucleotide (GNA), and the remaining positions are 2'-methoxynucleotides; the sense strand of the siRNA is 21 nucleotides in length, wherein positions 7, 9, 10, and 11 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 8, 9, 10, 14, and 16 of the 5' end of the antisense strand are 2'-fluoronucleotides, position 6 is a glycerol nucleotide (GNA), and the remaining positions are 2'-methoxynucleotides; the sense strand of the siRNA is 21 nucleotides in length, wherein positions 7, 9, 10, 11, and 15 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 8, 9, 10, 14, and 16 of the 5' end of the antisense strand are 2'-fluoronucleotides, position 4 is a glycerol nucleotide (GNA), and the remaining positions are 2'-methoxynucleotides; the sense strand of the siRNA is 21 nucleotides in length, wherein positions 7, 9, 10, 11, and 15 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 8, 9, 10, 14, and 16 of the 5' end of the antisense strand are 2'-fluoronucleotides, position 5 is a glycerol nucleotide (GNA), and the remaining positions are 2'-methoxynucleotides; the sense strand of the siRNA is 21 nucleotides in length, wherein positions 7, 9, 10, 11, and 15 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 8, 9, 10, 14, and 16 of the 5' end of the antisense strand are 2'-fluoronucleotides, position 7 is a glycerol nucleotide (GNA), and the remaining positions are 2'-methoxynucleotides; the sense strand of the siRNA is 21 nucleotides in length, wherein positions 7, 9, 10, 11, and 15 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 8, 9, 10, 14, and 16 of the 5' end of the antisense strand are 2'-fluoronucleotides, position 6 is a glycerol nucleotide (GNA), and the remaining positions are 2'-methoxynucleotides; the sense strand of the siRNA is 21 nucleotides in length, wherein positions 9, 10, and 11 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 5, 7, and 12 of the 5' end of the antisense strand are 2'-deoxynucleotides, position 14 is a 2'-fluoronucleotide, and the remaining positions are 2'-methoxynucleotides. In some embodiments of the present invention, the sense strand of the siRNA is 21 nucleotides in length, wherein positions 9, 10, and 11 of the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 5, 7, and 12 of the 5' end of the antisense strand are 2'-deoxynucleotides, positions 6, 8, 9, 10, 14, and 16 are 2'-fluoronucleotides, and the remaining positions are 2'-methoxynucleotides. In some embodiments of the present invention, the sense strand of the siRNA is 21 nucleotides in length, wherein positions 7, 9, 10, and 11 of the 5' end of the sense strand are 2'-fluoronucleotides, and the remaining positions are 2'-methoxynucleotides.

In some embodiments of the present invention, the antisense strand of the siRNA is 21 nucleotides in length, wherein positions 2, 6, 14, and 16 of the 5' end of the antisense strand are 2'-fluoronucleotides, and the remaining positions are 2'-methoxynucleotides; the sense strand of the siRNA is 21 nucleotides in length, wherein positions 5, 7, and 9 of the 5' end of the sense strand are 2'-fluoronucleotides, and the remaining positions are 2'-methoxynucleotides.

In some embodiments of the present invention, the antisense strand of the siRNA is 21 nucleotides in length, wherein positions 2, 6, 14, and 16 of the 5' end of the antisense strand are 2'-fluoronucleotides, and the remaining positions are 2'-methoxynucleotides; the sense strand of the siRNA is 21 nucleotides in length, wherein positions 7, 9, 10, and 11 of the 5' end of the sense strand are 2'-fluoronucleotides, and the remaining positions are 2'-methoxynucleotides.

In some embodiments of the present invention, the antisense strand of the siRNA is 21 nucleotides in length, wherein positions 2, 4, 6, 8, 10, 12, 14, 16, 18, and 20 at the 5' end of the antisense strand are 2'-fluoronucleotides, and the remaining positions are 2'-methoxynucleotides; the sense strand of the siRNA is 21 nucleotides in length, wherein positions 9, 10, and 11 at the 5' end of the sense strand are 2'-fluoronucleotides, and the remaining positions are 2'-methoxynucleotides.

In some embodiments of the present invention, the antisense strand of the siRNA is 21 nucleotides in length, wherein positions 2, 4, 6, 8, 10, 12, 14, 16, 18, and 20 at the 5' end of the antisense strand are 2'-fluoronucleotides, and the remaining positions are 2'-methoxynucleotides; the sense strand of the siRNA is 21 nucleotides in length, wherein positions 1, 3, 5, 7, 9, 11, 13, 15, 17, 19, and 21 at the 5' end of the sense strand are 2'-fluoronucleotides, and the remaining positions are 2'-methoxynucleotides.

In some embodiments of the present invention, the antisense strand of the siRNA is 21 nucleotides in length, wherein positions 2, 6, 14, and 16 at the 5' end of the antisense strand are 2'-fluoronucleotides, and the remaining positions are 2'-methoxynucleotides; the sense strand of the siRNA is 19 nucleotides in length, wherein positions 7, 8, and 9 at the 5' end of the sense strand are 2'-fluoronucleotides, and the remaining positions are 2'-methoxynucleotides.

In some embodiments of the present invention, the antisense strand of the siRNA is 19 nucleotides in length, wherein positions 2, 4, 6, 8, 10, 12, 14, 16, and 18 at the 5' end of the antisense strand are 2'-fluoronucleotides, and the remaining positions are 2'-methoxynucleotides; the sense strand of the siRNA is 19 nucleotides in length, wherein positions 7, 8, and 9 at the 5' end of the sense strand are 2'-fluoronucleotides, and the remaining positions are 2'-methoxynucleotides.

In some embodiments of the present invention, the antisense strand of the siRNA is 23 nucleotides in length, wherein position 14 at the 5' end of the antisense strand is a 2'-fluoronucleotide, positions 2, 5, and 7 are 2'-deoxynucleotides, position 12 is a threose nucleotide, and the remaining positions are 2'-methoxynucleotides; the sense strand of the siRNA is 21 nucleotides in length, wherein positions 9, 10, and 11 at the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 6, 14, and 16 at the 5' end of the antisense strand is 2'-fluoronucleotides, and the remaining positions are 2'-methoxynucleotides; the sense strand of the siRNA is 21 nucleotides in legnth, wherein positions 9, 10, and 11 at the 5' end of the sense strand are 2'-fluoronucleotides, position 1 is a threose nucleotide, and the remaining positions are 2'-methoxynucleotides.

In some embodiments of the present invention, the antisense strand of the siRNA is 23 nucleotides in length, wherein positions 2, 6, 14, and 16 at the 5' end of the antisense strand are 2'-fluoronucleotides, position 22 is a threose nucleotide, and the remaining positions are 2'-methoxynucleotides; the sense strand of the siRNA is 21 nucleotides in length, wherein positions 9, 10, and 11 at the 5' end of the sense strand are 2'-fluoronucleotides, and the remaining positions are 2'-methoxynucleotides.

In some embodiments of the present invention, the antisense strand of the siRNA is 23 nucleotides in length, wherein positions 2, 6, 14, and 16 at the 5' end of the antisense strand are 2'-fluoronucleotides, position 23 is a threose nucleotide, and the remaining positions are 2'-methoxynucleotides; the sense strand of the siRNA is 21 nucleotides in length, wherein positions 9, 10, and 11 at the 5' end of the sense strand are 2'-fluoronucleotides, and the remaining positions are 2'-methoxynucleotides.

In some embodiments of the present invention, the antisense strand of the siRNA is 22 nucleotides in length, wherein positions 2, 6, 14, and 16 at the 5' end of the antisense strand are 2'-fluoronucleotides, and the remaining positions are 2'-methoxynucleotides; the sense strand of the siRNA is 20 nucleotides in length, wherein positions 8, 9, and 10 at the 5' end of the sense strand are 2'-fluoronucleotides, position 1 is a threose nucleotide, and the remaining positions are 2'-methoxynucleotides.

In some embodiments of the present invention, the antisense strand of the siRNA is 22 nucleotides in length, wherein positions 2, 6, 14, and 16 at the 5' end of the antisense strand are 2'-fluoronucleotides, position 21 is a threose nucleotide, and the remaining positions are 2'-methoxynucleotides; the sense strand of the siRNA is 20 nucleotides in length, wherein positions 8, 9, and 10 at the 5' end of the sense strand are 2'-fluoronucleotides, and the remaining positions are 2'-methoxynucleotides.

In some embodiments of the present invention, the antisense strand of the siRNA is 22 nucleotides in length, wherein positions 2, 6, 14, and 16 at the 5' end of the antisense strand are 2'-fluoronucleotides, position 22 is a threose nucleotide, and the remaining positions are 2'-methoxynucleotides; the sense strand of the siRNA is 20 nucleotides in length, wherein positions 8, 9, and 10 at the 5' end of the sense strand are 2'-fluoronucleotides, and the remaining positions are 2'-methoxynucleotides.

In some embodiments of the present invention, the antisense strand of the siRNA is 22 nucleotides in length, wherein positions 2, 6, 14, and 16 at the 5' end of the antisense strand are 2'-fluoronucleotides, and the remaining positions are 2'-methoxynucleotides; the sense strand of the siRNA is 20 nucleotides in length, wherein positions 8, 9, and 10 at the 5' end of the sense strand are 2'-fluoronucleotides, and the remaining positions are 2'-methoxynucleotides.

In some embodiments of the present invention, the antisense strand of the siRNA is 22 nucleotides in length, wherein positions 2, 6, 14, and 16 at the 5' end of the antisense strand are 2'-fluoronucleotides, and the remaining positions are 2'-methoxynucleotides; the sense strand of the siRNA is 20 nucleotides in length, wherein positions 6, 8, 9, and 10 at the 5' end of the sense strand are 2'-fluoronucleotides, and the remaining positions are 2'-methoxynucleotides.

In some embodiments of the present invention, the antisense strand of the siRNA is 22 nucleotides in length, wherein positions 2, 6, 14, and 16 at the 5' end of the antisense strand are 2'-fluoronucleotides, and the remaining positions are 2'-methoxynucleotides; the sense strand of the siRNA is 20 nucleotides in length, wherein positions 8, 9, and 10 at the 5' end of the sense strand are 2'-fluoronucleotides, position 6 is a 2'-deoxynucleotide, and the remaining positions are 2'-methoxynucleotides.

In some embodiments of the present invention, the antisense strand of the siRNA is 22 nucleotides in length, wherein positions 2, 6, 8, 9, 14, and 16 at the 5' end of the antisense strand are 2'-fluoronucleotides, and the remaining positions are 2'-methoxynucleotides; the sense strand of the siRNA is 20 nucleotides in length, wherein positions 6, 8, 9, and 10 at the 5' end of the sense strand are 2'-fluoronucleotides, and the remaining positions are 2'-methoxynucleotides.

In some embodiments of the present invention, the antisense strand of the siRNA is 22 nucleotides in length, wherein position 14 at the 5' end of the antisense strand is a 2'-fluoronucleotide, positions 2, 5, and 7 are 2'-deoxynucleotides, position 12 is a threose nucleotide, and the remaining positions are 2'-methoxynucleotides; the sense strand of the siRNA is 20 nucleotides in length, wherein positions 8, 9, and 10 at the 5' end of the sense strand are 2'-fluoronucleotides, and the remaining positions are 2'-methoxynucleotides.

In some embodiments of the present invention, the antisense strand of the siRNA is 22 nucleotides in length, wherein 3-10 positions (e.g., positions 2, 14, 16; positions 2, 5, 14, 16; positions 2, 4, 6, 14, 16; positions 2, 6, 10, 14, 16; positions 2, 6, 12, 14, 16; positions 2, 5, 10, 14, 16; positions 2, 3, 12, 14, 16; positions 2, 9, 12, 14, 16; positions 2, 6, 8, 9, 14, 16; positions 2, 3, 5, 12, 14, 16; positions 2, 8, 9, 12, 14, 16; positions 2, 7, 9, 12, 14, 16; positions 2, 4, 6, 8, 10, 14, 16, 18, 20; positions 2, 4, 5, 6, 8, 10, 12, 14, 16, 18 at the 5' end) of the antisense strand are 2'-fluoronucleotides, and the remaining positions are 2'-methoxynucleotides;
the sense strand of the siRNA is 20 nucleotides in length, wherein positions 8, 9, and 10 at the 5' end of the sense strand are 2'-fluoronucleotides, optionally one position (preferably position 1 at the 5' end) is a threose nucleotide, optionally one position (preferably position 6 at the 5' end) is a 2'-deoxynucleotide, and the remaining positions are 2'-methoxynucleotide conjugates.

In some embodiments of the present invention, the antisense strand of the siRNA is 22 nucleotides in length, wherein the following positions: positions 2, 14, 16; or positions 2, 5, 14, 16; or positions 2, 6, 14, 16; or positions 2, 6, 10, 14, 16; or positions 2, 6, 12, 14, 16; or positions 2, 5, 10, 14, 16; or positions 2, 3, 12, 14, 16; or positions 2, 9, 12, 14, 16; or positions 2, 6, 8, 9, 14, 16; or positions 2, 3, 5, 12, 14, 16; or positions 2, 8, 9, 12, 14, 16; or positions 2, 7, 9, 12, 14, 16; or positions 2, 4, 6, 8, 10, 14, 16, 18, 20; or positions 2, 4, 5, 6, 8, 10, 12, 14, 16, 18, at the 5' end of the antisense strand are 2'-fluoronucleotides, and the remaining positions are 2'-methoxynucleotides;
the sense strand of the siRNA is 20 nucleotides in length, wherein positions 8, 9, and 10 at the 5' end of the sense strand are 2'-fluoronucleotides, optionally, position 1 at the 5' end is a threose nucleotide, or optionally, position 6 at the 5' end is a 2'-deoxynucleotide, and the remaining positions are 2'-methoxynucleotides.

In some embodiments of the present invention, the antisense strand of the siRNA is 22 nucleotides in length, wherein the following positions: positions 2, 14, 16; or positions 2, 5, 14, 16; or positions 2, 4, 6, 14, 16; or positions 2, 6, 10, 14, 16; or positions 2, 6, 12, 14, 16; or positions 2, 5, 10, 14, 16; or positions 2, 3, 12, 14, 16; or positions 2, 9, 12, 14, 16; or positions 2, 6, 8, 9, 14, 16; or positions 2, 3, 5, 12, 14, 16; or positions 2, 8, 9, 12, 14, 16; or positions 2, 7, 9, 12, 14, 16; or positions 2, 4, 6, 8, 10, 14, 16, 18, 20; or positions 2, 4, 5, 6, 8, 10, 12, 14, 16, 18, at the 5' end of the antisense strand are 2'-fluoronucleotides, and the remaining positions are 2'-methoxynucleotides; the sense strand of the siRNA is 20 nucleotides in length, wherein positions 8, 9, and 10 at the 5' end of the sense strand are 2'-fluoronucleotides, and the remaining positions are 2'-methoxynucleotides.

In some embodiments of the present invention, the antisense strand of the siRNA is 21 nucleotides in length, wherein positions 2, 6, 14 and 16 at the 5' end of the antisense strand are 2'-fluoronucleotides, and the remaining positions are 2'-methoxynucleotides; the sense strand of the siRNA is 19 nucleotides in length, wherein positions 7, 8, and 9 at the 5' end of the sense strand are 2'-fluoronucleotides, position 1 is a threose nucleotide, and the remaining positions are 2'-methoxynucleotides.

In some embodiments of the present invention, the antisense strand of the siRNA is 21 nucleotides in length, wherein positions 2, 6, 14, and 16 at the 5' end of the antisense strand are 2'-fluoronucleotides, position 20 is a threose nucleotide, and the remaining positions are 2'-methoxynucleotides; the sense strand of the siRNA is 19 nucleotides in length, wherein positions 7, 8, and 9 at the 5' end of the sense strand are 2'-fluoronucleotides, and the remaining positions are 2'-methoxynucleotides.

In some embodiments of the present invention, the antisense strand of the siRNA is 21 nucleotides in length, wherein positions 2, 6, 14, and 16 at the 5' end of the antisense strand are 2'-fluoronucleotides, position 21 is a threose nucleotide, and the remaining positions are 2'-methoxynucleotides; the sense strand of the siRNA is 19 nucleotides in length, wherein positions 7, 8, and 9 at the 5' end of the sense strand are 2'-fluoronucleotides, and the remaining positions are 2'-methoxynucleotides.

In some embodiments of the present invention, the antisense strand of the siRNA is 21 nucleotides in length, wherein position 14 at the 5' end of the antisense strand is a 2'-fluoronucleotide, positions 2, 5, and 7 are 2'-deoxynucleotides, position 12 is a threose nucleotide, and the remaining positions are 2'-methoxynucleotides; the sense strand of the siRNA is 19 nucleotides in length, wherein positions 7, 8, and 9 at the 5' end of the sense strand are 2'-fluoronucleotides, and the remaining positions are 2'-methoxynucleotides.

In some embodiments of the present invention, the antisense strand of the siRNA is 22 nucleotides in length, wherein 3-10 positions (e.g., positions 2, 14, 16; positions 2, 5, 14, 16; positions 2, 6, 14, 16; positions 2, 4, 6, 14, 16; positions 2, 6, 10, 14, 16; positions 2, 6, 12, 14, 16; positions 2, 5, 10, 14, 16; positions 2, 3, 12, 14, 16; positions 2, 9, 12, 14, 16; positions 2, 6, 8, 9, 14, 16; positions 2, 3, 5, 12, 14, 16; positions 2, 8, 9, 12, 14, 16; positions 2, 7, 9, 12, 14, 16; positions 2, 4, 6, 8, 10, 14, 16, 18, 20; positions 2, 4, 5, 6, 8, 10, 12, 14, 16, 18) of the antisense strand are 2'-fluoronucleotides, and the remaining positions are 2'-methoxynucleotides,
the sense strand of the siRNA is 20 nucleotides in length, wherein positions 8, 9, and 10 at the 5' end of the sense strand are 2'-fluoronucleotides, optionally one position (preferably position 1 at the 5' end) is a threose nucleotide, optionally one position (preferably position 6 at the 5' end) is a 2'-deoxynucleotide, and the remaining positions are 2'-methoxynucleotide conjugates.

In some embodiments of the present invention, the antisense strand of the siRNA is 22 nucleotides in length, wherein the following positions: positions 2, 14, 16; or positions 2, 5, 14, 16; or positions 2, 6, 14, 16; or positions 2, 4, 6, 14, 16; or positions 2, 6, 10, 14, 16; or positions 2, 6, 12, 14, 16; or positions 2, 5, 10, 14, 16; or positions 2, 3, 12, 14, 16; or positions 2, 9, 12, 14, 16; or positions 2, 6, 8, 9, 14, 16; or positions 2, 3, 5, 12, 14, 16; or positions 2, 8, 9, 12, 14, 16; or positions 2, 7, 9, 12, 14, 16; or positions 2, 4, 6, 8, 10, 14, 16, 18, 20; or positions 2, 4, 5, 6, 8, 10, 12, 14, 16, 18, at the 5' end of the antisense strand are 2'-fluoronucleotides, and the remaining positions are 2'-methoxynucleotides;
the sense strand of the siRNA is 20 nucleotides in length, wherein positions 8, 9, and 10 at the 5' end of the sense strand are 2'-fluoronucleotides, optionally position 1 at the 5' end is a threose nucleotide, or optionally position 6 at the 5' end is a 2'-deoxynucleotide, and the remaining positions are 2'-methoxynucleotides;
preferably, the 2'-deoxynucleotide at position 6 is independently selected from: deoxyadenosine monophosphate (dA), deoxyguanosine monophosphate (dG), deoxycytidine monophosphate (dC), deoxythymidine monophosphate (dT), deoxyuridine monophosphate (dU); more preferably, the 2'-deoxynucleotide at position 6 is deoxythymidine monophosphate (dT).

In some embodiments of the present invention, the antisense strand of the siRNA is 22 nucleotides in length, wherein the following positions: positions 2, 14, 16; or positions 2, 5, 14, 16; or positions 2, 6, 14, 16; or positions 2, 4, 6, 14, 16; or positions 2, 6, 10, 14, 16; or positions 2, 6, 12, 14, 16; or positions 2, 5, 10, 14, 16; or positions 2, 3, 12, 14, 16; or positions 2, 9, 12, 14, 16; or positions 2, 6, 8, 9, 14, 16; or positions 2, 3, 5, 12, 14, 16; or positions 2, 8, 9, 12, 14, 16; or positions 2, 7, 9, 12, 14, 16; or positions 2, 4, 6, 8, 10, 14, 16, 18, 20; or positions 2, 4, 5, 6, 8, 10, 12, 14, 16, 18, at the 5' end of the antisense strand are 2'-fluoronucleotides, and the remaining positions are 2'-methoxynucleotides; the sense strand of the siRNA is 20 nucleotides in length, wherein positions 8, 9, 10 at the 5' end of the sense strand are 2'-fluoronucleotides, and the remaining positions are 2'-methoxynucleotides; or
the antisense strand of the siRNA is 22 nucleotides in length, wherein positions 2, 6, 14, 16 at the 5' end of the antisense strand are 2'-fluoronucleotides, and the remaining positions are 2'-methoxynucleotides; the sense strand of the siRNA is 20 nucleotides in length, wherein positions 8, 9, and 10 at the 5' end of the sense strand are 2'-fluoronucleotides, position 1 is a threose nucleotide, and the remaining positions are 2'-methoxynucleotides; or
the antisense strand of the siRNA is 22 nucleotides in length, wherein positions 2, 6, 14, 16 at the 5' end of the antisense strand are 2'-fluoronucleotides, and the remaining positions are 2'-methoxynucleotides; the sense strand of the siRNA is 20 nucleotides in length, wherein positions 8, 9, 10 at the 5' end of the sense strand are 2'-fluoronucleotides, position 6 is a 2'-deoxynucleotide, and the remaining positions are 2'-methoxynucleotides; preferably, the 2'-deoxynucleotide at position 6 is independently selected from: deoxyadenosine monophosphate (dA), deoxyguanosine monophosphate (dG), deoxycytidine monophosphate (dC), deoxythymidine monophosphate (dT), deoxyuridine monophosphate (dU); more preferably, the 2'-deoxynucleotide at position 6 is deoxythymidine monophosphate (dT);
more preferably, the antisense strand of the siRNA is the nucleotide sequence as set forth in SEQ ID NO: 105, and the sense strand of the siRNA is the nucleotide sequence as set forth in SEQ ID NO: 23.

In some embodiments of the present invention, the antisense strand of the siRNA is 22 nucleotides in length, wherein positions 2, 6, 14, 16 of the 5' end of the antisense strand are 2'-fluoronucleotides, and the remaining positions are 2'-methoxynucleotides; the sense strand of the siRNA is 20 nucleotides in length, wherein positions 8, 9, 10 of the 5' end of the sense strand are 2'-fluoronucleotides, and the remaining positions are 2'-methoxynucleotides;
preferably, the antisense strand of the siRNA is the nucleotide sequence as set forth in SEQ ID NO: 116, and the sense strand of the siRNA is the nucleotide sequence as set forth in SEQ ID NO: 22.

In some embodiments of the present invention, the antisense strand of the siRNA is 22 nucleotides in length, wherein position 14 at the 5' end of the antisense strand is a 2'-fluoronucleotide, positions 2, 5, 7 are 2'-deoxynucleotides, position 12 is a threose nucleotide, and the remaining positions are 2'-methoxynucleotides; the sense strand of the siRNA is 20 nucleotides in length, wherein positions 8, 9, 10 at the 5' end of the sense strand are 2'-fluoronucleotides, and the remaining positions are 2'-methoxynucleotides;
preferably, the 2'-deoxynucleotides at positions 2, 5, 7 are independently selected from: deoxyadenosine monophosphate (dA), deoxyguanosine monophosphate (dG), deoxycytidine monophosphate (dC), deoxythymidine monophosphate (dT), deoxyuridine monophosphate (dU);
more preferably, the 2'-deoxyribonucleotides at positions 2, 5, and 7 are respectively: deoxythymidine monophosphate (dT), deoxythymidine monophosphate (dT), and deoxyadenosine monophosphate (dA);
more preferably, the antisense strand of the siRNA is the nucleotide sequence as set forth in SEQ ID NO: 105, and the sense strand of the siRNA is the nucleotide sequence as set forth in SEQ ID NO: 23.

In some embodiments of the present invention, the antisense strand of the siRNA is 21 nucleotides in length, wherein positions 2, 6, 14, 16 at the 5' end of the antisense strand are 2'-fluoronucleotides, and the remaining positions are 2'-methoxynucleotides; the sense strand of the siRNA is 19 nucleotides in length, wherein positions 7, 8, 9 at the 5' end of the sense strand are 2'-fluoronucleotides, position 1 is a threose nucleotide, and the remaining positions are 2'-methoxynucleotides; or
the antisense strand of the siRNA is 21 nucleotides in length, wherein position 14 at the 5' end of the antisense strand is a 2'-fluoronucleotide, positions 2, 5, 7 are 2'-deoxynucleotides, position 12 is a threose nucleotide, and the remaining positions are 2'-methoxynucleotides; the sense strand of the siRNA is 19 nucleotides in length, wherein positions 7, 8, 9 at the 5' end of the sense strand are 2'-fluoronucleotides, and the remaining positions are 2'-methoxynucleotides;
preferably, the 2'-deoxynucleotides at positions 2, 5, and 7 are independently selected from: deoxyadenosine monophosphate (dA), deoxyguanosine monophosphate (dG), deoxycytidine monophosphate (dC), deoxythymidine monophosphate (dT), and deoxyuridine monophosphate (dU);
more preferably, the 2'-deoxynucleotides at positions 2, 5, and 7 are respectively: deoxythymidine monophosphate (dT), deoxyuridine monophosphate (dU), and deoxyadenosine monophosphate (dA);
more preferably, the antisense strand of the siRNA is the nucleotide sequence as set forth in SEQ ID NO: 104, and the sense strand of the siRNA is the nucleotide sequence as set forth in SEQ ID NO: 3.

In some embodiments of the present invention, the modified nucleotide is a nucleotide whose phosphate group is modified with a phosphorothioate group. That is, a sulfur atom replaces a non-bridging oxygen atom in a phosphodiester bond, thereby replacing the phosphodiester bond with a phosphorothioate bond.

In some embodiments of the present invention, the 5' and 3' ends of the sense chain each independently comprise 0, 1, or 2 phosphorothioate groups; and/or, the 5' and 3' ends of the antisense chain each independently comprise 1 or 2 phosphorothioate groups.

In some embodiments of the present invention, between the nucleotides at positions 1 and 2 of the 5' end of the sense strand, between the nucleotides at positions 2 and 3 of the 5' end of the sense strand, between the nucleotides at positions 1 and 2 of the 3' end of the sense strand, between the nucleotides at positions 2 and 3 of the 3' end of the sense strand, between the nucleotides at positions 1 and 2 of the 3' end of the antisense strand, between the nucleotides at positions 2 and 3 of the 3' end of the antisense strand, between the nucleotides at positions 1 and 2 of the 5' end of the antisense strand, and between the nucleotides at positions 2 and 3 of the 5' end of the antisense strand, at least one linkage is phosphorothioate linkage; preferably, at least four linkages are phosphorothioate linkages; in some embodiments of the present invention, at least six linkages are phosphorothioate linkages; in some embodiments of the present invention, all eight linkages are phosphorothioate linkages.

In some embodiments of the present invention, the linkages between the nucleotides at positions 1 and 2, and between the nucleotides at positions 2 and 3 of the 5' end of the sense strand are phosphorothioate linkages.

In some embodiments of the present invention, the linkages between the nucleotides at positions 1 and 2, and between the nucleotides at positions 2 and 3 of the 5' end of the sense strand are phosphorothioate linkages, and the linkages between the nucleotides at positions 1 and 2, and between the nucleotides at positions 2 and 3 of the 3' end of the sense strand are also phosphorothioate linkages.

In some embodiments of the present invention, the linkages between the nucleotides at positions 1 and 2, and between the nucleotides at positions 2 and 3 of the 3' end of the antisense strand are phosphorothioate linkages, and the linkages between the nucleotides at positions 1 and 2, and between the nucleotides at positions 2 and 3 of the 5' end are also phosphorothioate linkages.

In some embodiments of the present invention, the linkages between the nucleotides at positions 1 and 2 of the 5' end of the sense strand, between the nucleotides at positions 2 and 3 of the 5' end of the sense strand, between the nucleotides at positions 1 and 2 of the 3' end of the sense strand, between the nucleotides at positions 2 and 3 of the 3' end of the sense strand, between the nucleotides at positions 1 and 2 of the 3' end of the antisense strand, between the nucleotides at positions 2 and 3 of the 3' end of the antisense strand, between the nucleotides at positions 1 and 2 of the 5' end of the antisense strand, and between the nucleotides at positions 2 and 3 of the 5' end of the antisense strand are all phosphorothioate linkages.

In some embodiments of the present invention, the sense strand may comprise one or more capping residues or moieties, referred to as "capping residues". A "capping residue" is a non-nucleotide compound or other moiety that can be incorporated into one or more ends of the nucleotide sequence of the siRNA. In some embodiments of the present invention, capping residues are present at the 5' end, the 3' end, or both of the 5' end and the 3' end of the sense strand.

In some embodiments of the present invention, an inverted abasic residue (iab or invAb) is added as a capping residue. See F. Czauderna, Nucleic Acids Res., 2003, 31(11), 2705-16. In some embodiments of the present invention, the 5' end and/or 3' end of the sense strand may comprise more than one reverse deoxyabasic deoxyribose moiety as a capping residue.

In some embodiments of the present invention, one or more inverted abasic residues (iab or invAb) are added to the 3' end of the sense strand. In some embodiments of the present invention, one or more inverted abasic residues (iab or invAb) are added to the 5' end of the sense strand. In some embodiments of the present invention, one or more inverted abasic residues may be inserted between the delivery carrier moiety and the nucleotide sequence of the siRNA's sense strand. In some embodiments of the present invention, one or more inverted abasic residues are included at or near one or more ends of the siRNA's sense strand. The inverted abasic residue (iab or invAb) is selected from the following structures: 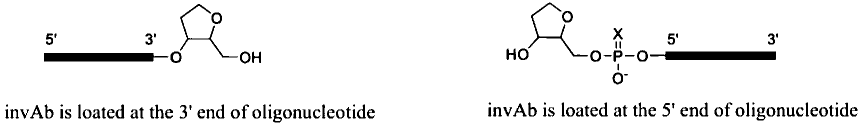 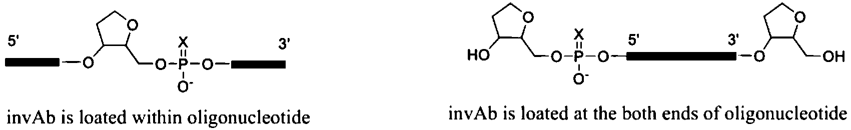 where X = O or S.

In some embodiments of the present invention, one or more inverted abasic residues (iab or invAb) are added to the 5' end of the sense strand. In some embodiments of the present invention, one or more inverted abasic residues may be inserted between the delivery carrier moiety and the nucleotide sequence of the siRNA's sense strand.

The inverted abasic residues may be linked via phosphate, phosphorothioate, or other nucleoside linkages.

In some embodiments of the present invention, the first nucleotide at the 5' end of the antisense strand is selected from the following structures: 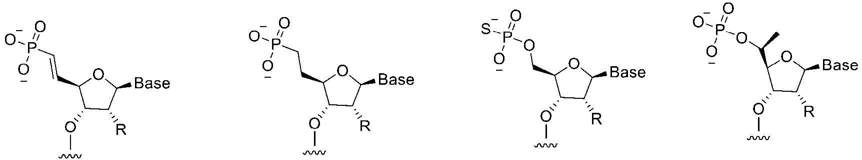
where Basse represents base A, U, G, C, T, or other nucleotide base.
where Basse represents base A, U, G, C, T, or other nucleotide base.

In some embodiments of the present invention, the first nucleotide at the 5' end of the antisense strand is a (E)-vinyl phosphate-modified nucleotide.

In some embodiments of the present invention, the siRNA comprises at least one base-modified nucleotide.

In some embodiments of the present invention, the base of the base-modified nucleotide is selected from the following structures:

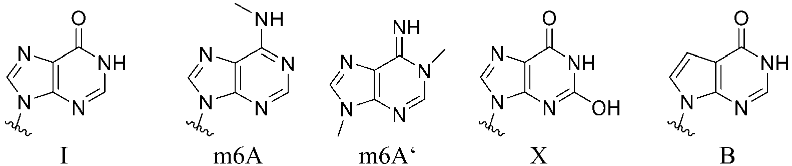

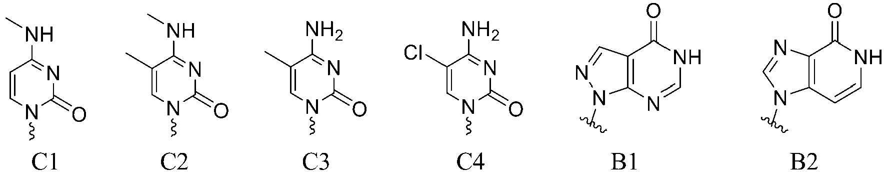

In some embodiments of the present invention, the base-modified nucleotide is located at positions 5, 6, 7, 8 of the siRNA's antisense strand.

In some embodiments of the present invention, the base-modified nucleotide is located at a single-stranded nucleotide overhang in the siRNA.

Preferably, the siRNA's antisense strand comprises a two-nucleotide overhang, and the base-modified nucleotide is the first nucleotide of the siRNA's antisense strand overhang.

Preferably, the siRNA's antisense strand comprises a two-nucleotide overhang, and the base-modified nucleotide is the second nucleotide of the siRNA's antisense strand overhang.

The present invention also provides a siRNA conjugate obtained by conjugating the above-mentioned siRNA with a conjugation molecule.

In the present invention, unless otherwise specified, "conjugation" refers to the covalent connection between two or more chemical moieties; "conjugate" refers to a compound formed by the covalent connection of various chemical moieties; and "siRNA conjugate" refers to a compound formed by the covalent connection of one or more chemical moieties to siRNA. It should be noted that the chemical moieties can be directly attached to the siRNA or attached to the siRNA via a linker.

In some embodiments of the present invention, the delivery carrier forms a conjugating molecule and is attached to the siRNA via a covalent bond.

In some embodiments of the present invention, the delivery carrier forms a conjugating molecule and is attached to the 3' or 5' end of the sense strand of the siRNA, either independently or simultaneously.

In some embodiments of the present invention, the siRNA of the present invention can be conjugated with a pharmaceutically acceptable conjugating molecule to obtain an siRNA conjugate. In some embodiments of the present invention, the siRNA is covalently conjugated to the conjugating molecule. To reduce the potential impact of conjugation on siRNA activity, the conjugation site between the siRNA and the conjugating molecule can be at the 3' or 5' end of the sense strand of the siRNA, or at the 5' end of the antisense strand. In some embodiments, the conjugation site of the siRNA to the conjugating molecule may also be located within the internal sequence of the siRNA.

In the present invention, a "delivery carrier" refers to a chemical moiety covalently linked to siRNA and affecting the targeting, activity, cellular distribution, cellular uptake, or stability of the oligonucleotide. Conjugation of siRNA to one or more delivery carriers can improve the pharmacological properties of siRNA. In some embodiments, the delivery carrier moiety modifies or enhances the pharmacokinetic properties of the oligonucleotide by improving its cellular distribution, bioavailability, metabolism, excretion, permeability, or cellular uptake. Specifically, the delivery carrier can target the oligonucleotide to a specific organ, tissue, or cell type, thereby enhancing the oligonucleotide's effectiveness in that organ, tissue, or cell type. Simultaneously, the delivery carrier moiety can be used to reduce the activity of the oligonucleotide in non-target cell types, tissues, or organs, for example, off-target activity or activity in non-target cell types, tissues, or organs.

The pharmaceutically acceptable delivery carrier may be a delivery carrier conventionally used in the field of siRNA drug delivery, for example, but not limited to, one or more of the following delivery carriers or their derivatives: lipophilic molecules, such as cholesterol, bile acids, vitamins (e.g., vitamin E), lipid molecules of different chain lengths; polymers, such as polyethylene glycol; polypeptides, such as transmembrane peptides; aptamers; antibodies; quantum dots; carbohydrates, such as lactose, polylactose, mannose, galactose, N-acetylgalactosamine (GalNAc); folic acid or folate; or receptor ligands expressed by hepatocytes, such as asialoglycoprotein, desialylated sugar residue, lipoprotein (e.g., high-density lipoprotein, low-density lipoprotein, etc.), glucagon, neurotransmitter (e.g., adrenaline), growth factors, transferrin, etc.

In some embodiments of the present invention, the delivery carrier comprises an N-acetylgalactosamine group.

In some embodiments of the present invention, the delivery carrier is directly attached to the 3' end of the siRNA's sense strand. In some embodiments of the present invention, the delivery carrier is directly attached to the 5' end of the siRNA's sense strand. In some embodiments of the present invention, the delivery carrier is attached to the 3' end of the siRNA's sense strand via an adapter. In some embodiments of the invention, the delivery carrier is attached to the 5' end of the siRNA's sense strand via an adapter.

In some embodiments of the invention, the delivery carrier comprises N-acetylgalactosamine, covalently linked to the 3' end of the siRNA's sense strand.

In some embodiments of the invention, the delivery carrier moiety is GalNAc(L96), having the following structure:

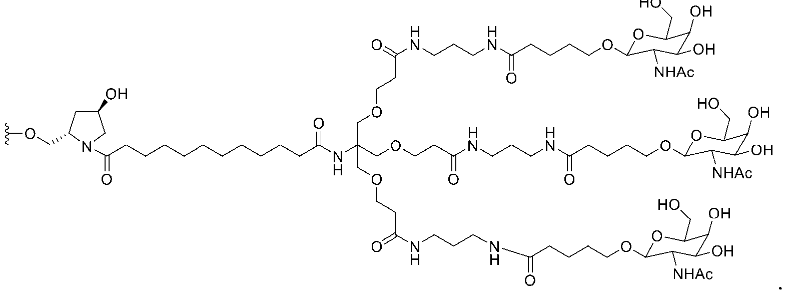

In some embodiments of the invention, GalNAc(L96) is attached to the 3' end of the siRNA's sense strand.

In some embodiments of the invention, GalNAc(L96) is attached to the 5' end of the siRNA's sense strand.

In some embodiments of the invention, GalNAc(L96) is attached to an inverted abasic residue (iab or invAb) at the 3' end of the siRNA's sense strand.

In some embodiments of the invention, GalNAc(L96) is attached to an inverted abasic residue (iab or invAb) at the 5' end of the siRNA's sense strand.

In some embodiments of the present invention, the delivery carrier moiety is Ser(GN), having the following structure:

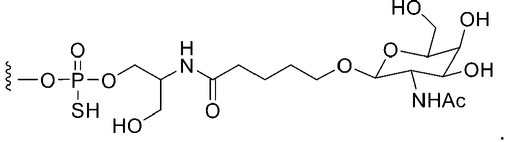

In some embodiments of the present invention, Ser(GN) is attached to the 3' end of the siRNA's sense strand. In some embodiments of the present invention, Ser(GN) is attached to the 5' end of the siRNA's sense strand.

In some embodiments of the present invention, Ser(GN) moieties are attached to both the 3' end and the 5' end of the siRNA's sense strand.

In some embodiments of the present invention, the delivery carrier moiety is LP-GalNAc (attached to the 5' end of the sense strand), having the following structure:

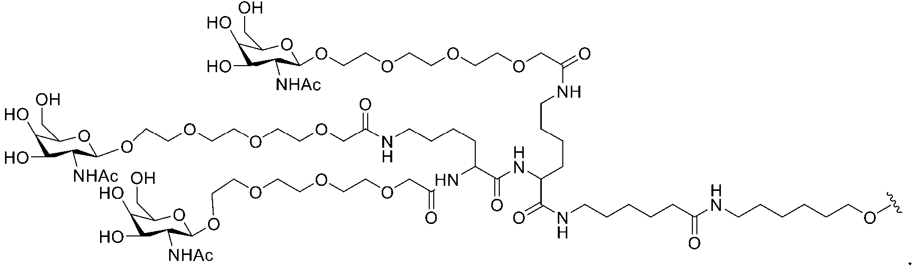

In some embodiments of the present invention, the delivery carrier moiety is XY-GalNAc (attached to the 3' end of the sense strand), having the following structure: 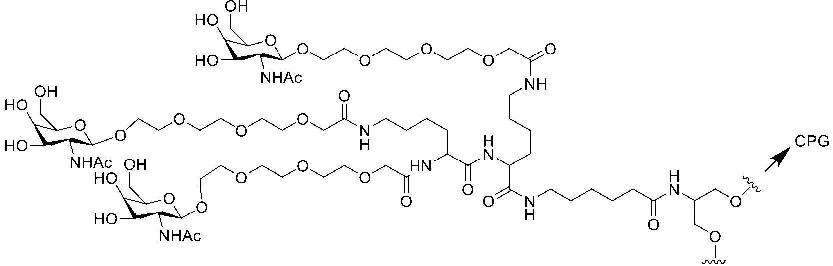 or

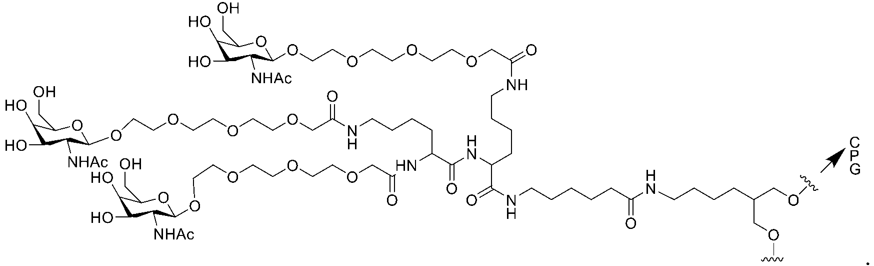

In some embodiments of the present invention, the structures of other delivery carrier moieties used (attached to the 5' end of the sense strand) are as follows:

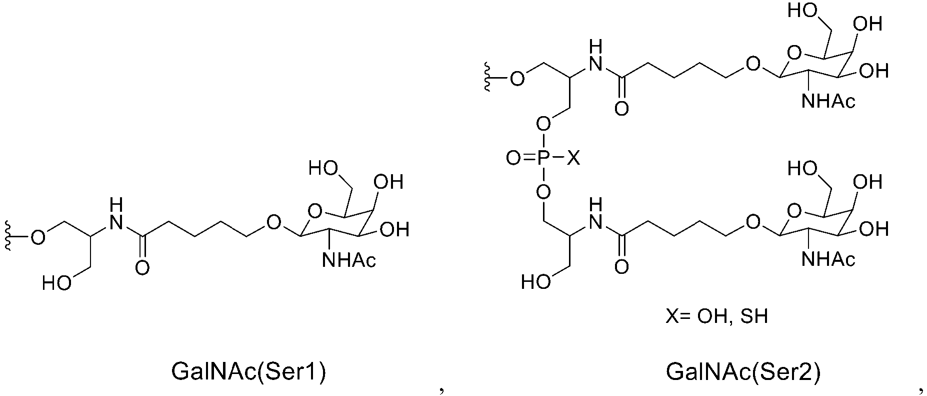

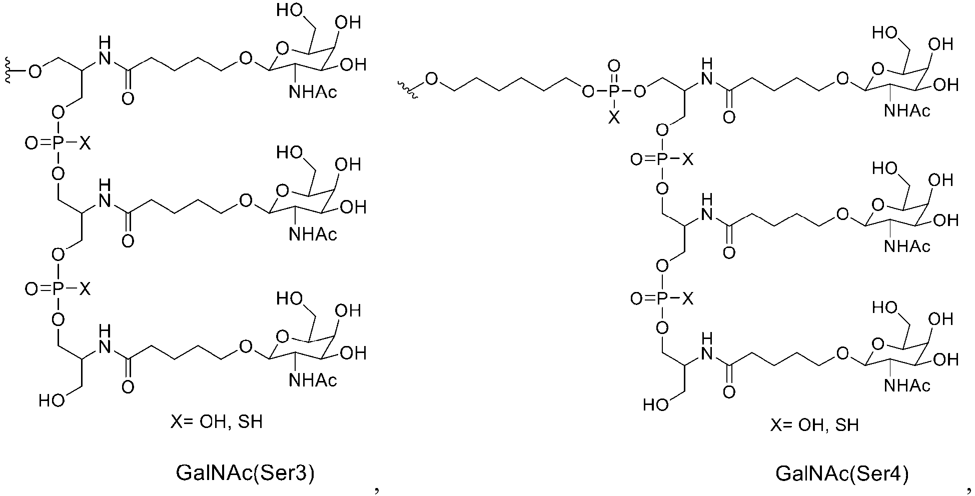

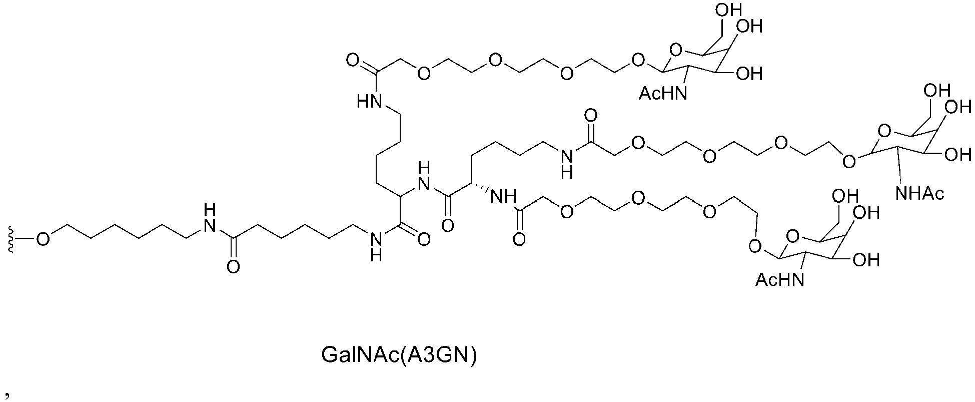

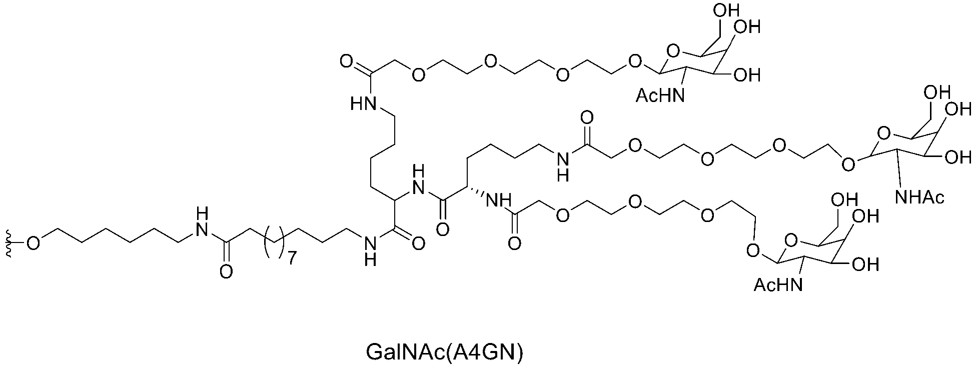

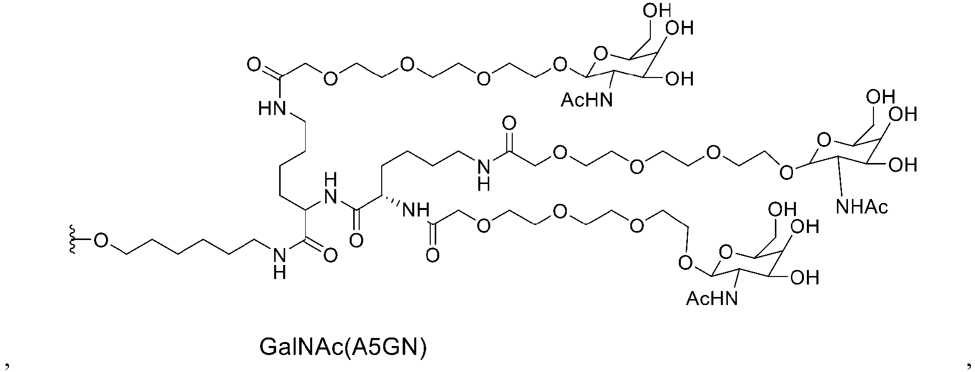

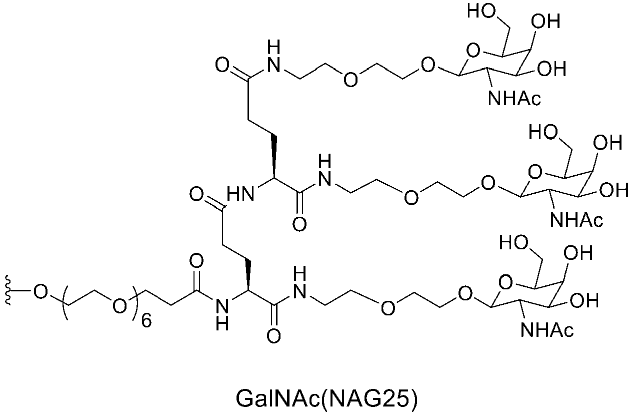

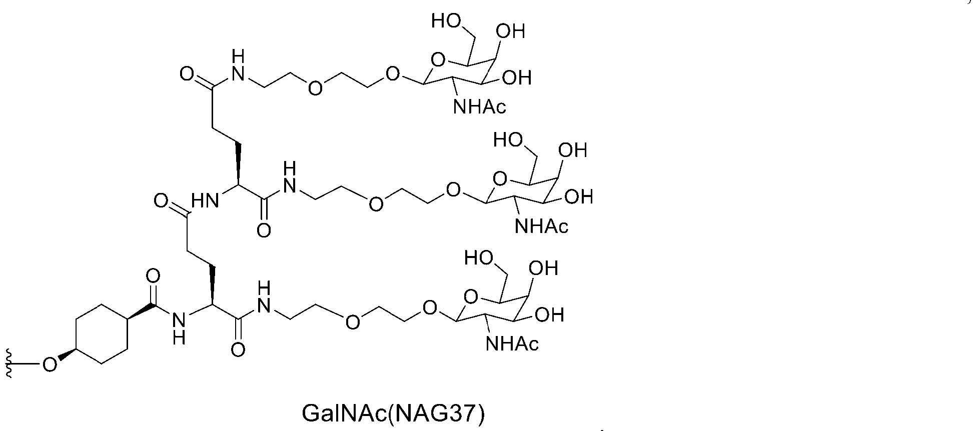

In some embodiments of the present invention, the structures of other delivery carrier moieties used (attached to the 3' end of the sense strand) are as follows:

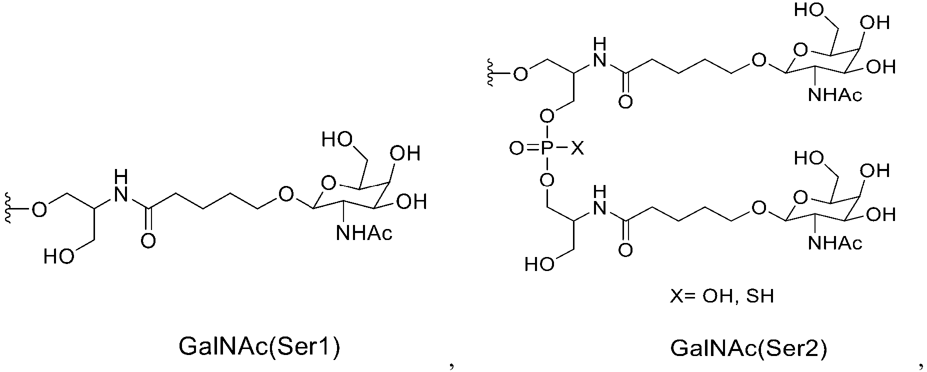

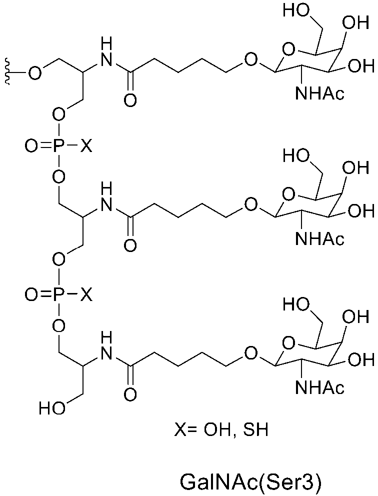

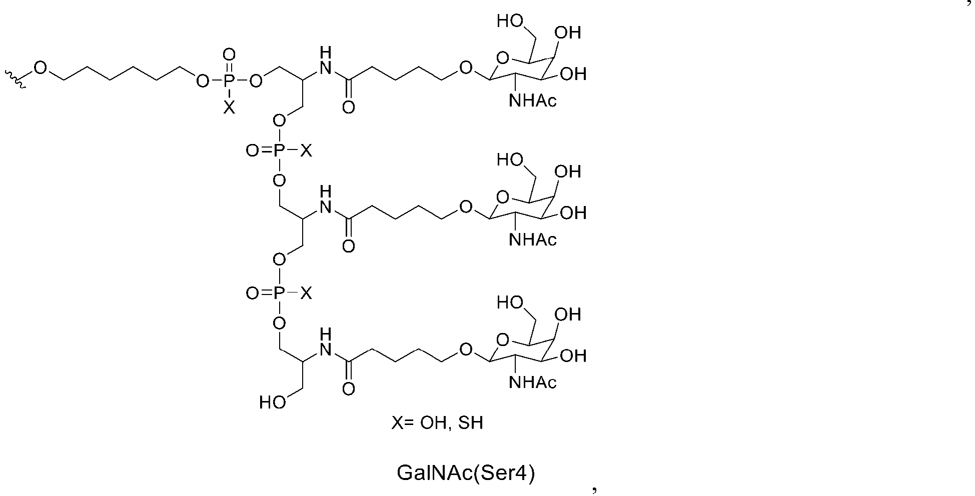

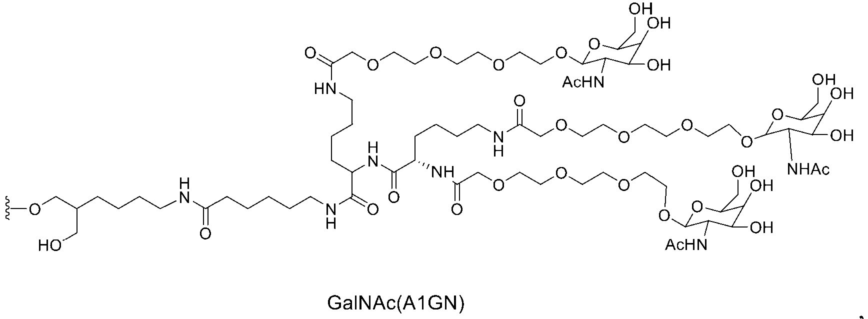

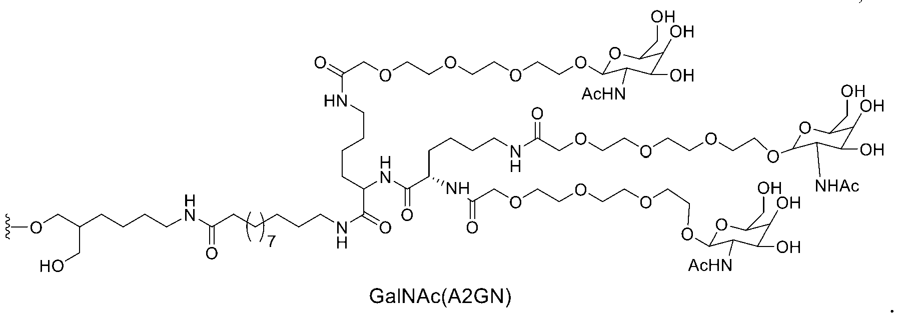

Optionally, the delivery carrier moiety is attached to the 3' end of the siRNA's sense strand, or to the 5' end of the siRNA's sense strand, or to an inverted abasic residue (iab) at the 3' end of the siRNA's sense strand, or to an inverted abasic residue (iab) at the 5' end of the siRNA's sense strand, or one or more of the delivery carrier moieties are simultaneously attached to the 3' end, the 5' end, an inverted abasic residue (iab) at the 3' end of the siRNA's sense strand, and an inverted abasic residue (iab) at the 5' end of the siRNA's sense strand. Preferably, the delivery carrier moiety GalNAc(L96) is attached to the 3' end of the siRNA's sense strand or to an inverted abasic residue (iab) at the 3' end of the siRNA's sense strand. The delivery carrier moiety Ser(GN) is attached to both the 3' and 5' ends of the siRNA's sense strand. The delivery carrier moiety LP-GalNAc is attached to the 5' end of the siRNA's sense strand. The delivery carrier moiety XY-GalNAc is attached to the 3' end of the siRNA's sense strand. The delivery carrier moiety GalNAc(Ser2) is attached to either the 3' or 5' end of the siRNA's sense strand, or both. The delivery carrier moiety GalNAc(Ser3) is attached to either the 3' or 5' end of the siRNA's sense strand. The delivery carrier moiety GalNAc(Ser4) is attached to the 3' end of the siRNA's sense strand. The delivery carrier moiety GalNAc (A1GN) is attached to the 3' end of the siRNA's sense strand; the delivery carrier moiety GalNAc (A2GN) is attached to the 3' end of the siRNA's sense strand; the delivery carrier moiety GalNAc (A3GN) is attached to the 5' end of the siRNA's sense strand; the delivery carrier moiety GalNAc (A4GN) is attached to the 5' end of the siRNA's sense strand; the delivery carrier moiety GalNAc (A5GN) is attached to the 5' end of the siRNA's sense strand; the delivery carrier moiety GalNAc (NAG25) is attached to the 5' end of the siRNA's sense strand; the delivery carrier moiety GalNAc (NAG37) is attached to the 5' end of the siRNA's sense strand; and the delivery carrier moieties GalNAc (Ser1) and GalNAc (Ser2) are simultaneously attached to the 3' end and the 5' end of the siRNA's sense strand, respectively, or simultaneously attached to the 5' end and the 3' end of the siRNA's sense strand, respectively;
preferably, the delivery carrier moiety is selected from the following: GalNAc (L96) attached to the 3' end of the siRNA's sense strand, GalNAc (A1GN) attached to the 3' end of the siRNA's sense strand, GalNAc (A2GN) attached to the 3' end of the siRNA's sense strand, GalNAc (A3GN) attached to the 5' end of the siRNA's sense strand, GalNAc (A4GN) attached to the 5' end of the siRNA's sense strand, and GalNAc (Ser2) simultaneously attached to both the 3' and 5' ends of the siRNA's sense strand.

The siRNA conjugates listed below are selected from Conjugates 1 to 1668 and the positive control PC c. The unmodified base sequences of the sense and antisense strands of each conjugate correspond to one of SEQ ID NO: 1 to SEQ ID NO: 200 listed above and SEQ ID NO: 201 to SEQ ID NO: 351 listed here, respectively.

| **Unmodified base sequence** | **SEQ ID NO:** |
|---|---|
| AAAAAUGCUGUTCAGCACCUCCC | 201 |
| IUUGCUGGAAAGUGAGACCCUCC | 202 |
| IUUGCTGGAAAGUGAGACCCUCC | 203 |
| ITUGCUGGAAAGUGAGACCCUCC | 204 |
| IAAAAUGCUGUUCAGCACCUCCC | 205 |
| IAAAATGCUGUUCAGCACCUCCC | 206 |
| UUCAUUAGAAGAAAAGGUGGUI | 207 |
| GCACCUUUUCUUCUAAUGAA | 208 |
| UUCAUUAGAAGAAAAGGUGCUI | 209 |
| UUCAUUAGAAGAAAAGIUGGUU | 210 |
| UUCAUUAGAAGAAAAGGUGIUU | 211 |
| AAAAAUGCUGUUCAGCACCUTU | 212 |
| AAAAAUGCUGUTCAGCACCUUU | 213 |
| UAAAAUGCUGUUCAGCACCUTU | 214 |
| UAAAAUGCUGUTCAGCACCUUU | 215 |
| AAAAAUGCUGUUCAGCACCTU | 216 |
| AAAAAUGCUGUTCAGCACCUU | 217 |
| UAAAAUGCUGUUCAGCACCTU | 218 |
| UAAAAUGCUGUTCAGCACCUU | 219 |
| AUUGCUGGAAAGUGAGACCCTU | 220 |
| GGGUCTCACUUUCCAGCAAU | 221 |
| ATUGCUGGAAAGUGAGACCCUU | 222 |
| UAAGCUGUUGGGUAGACUCUTU | 223 |
| TCUCCCACCUUUUCUUCUAA | 224 |
| UUAGAAGAAAAGGUGGGAGATU | 225 |
| UTAGAAGAAAAGGUGGGAGAUU | 226 |
| UUUAGAAGAAAAGGUGGGAGTU | 227 |
| UTUAGAAGAAAAGGUGGGAGUU | 228 |
| UUCAUUAGAAGAAAAGGUGGTU | 229 |
| UTCATUAGAAGAAAAGGUGGUU | 230 |
| UUCAUUAGAAGAAAAGGUGTU | 231 |
| UTCAUUAGAAGAAAAGGUGUU | 232 |
| UAAGCUGUUGGGUAGACUCUGTG | 233 |
| CCACCTUUUCUUCUAAUGAA | 234 |
| AUUGCTGGAAAGUGAGACCCUCC | 235 |
| UUCAUTAGAAGAAAAGGUGGUU | 236 |
| UUCAUTAGAAGAAAAGGUGUU | 237 |
| UUCATUAGAAGAAAAGGUGGUU | 238 |
| UUAGIAGAAAAGGUGGGAGAUU | 239 |
| UUUAIAAGAAAAGGUGGGAGUU | 240 |
| UUCAIUAGAAGAAAAGGUGGUU | 241 |
| UUAGAIGAAAAGGUGGGAGAUU | 242 |
| UUUAGIAGAAAAGGUGGGAGUU | 243 |
| UUCAUIAGAAGAAAAGGUGGUU | 244 |
| UUAGAAIAAAAGGUGGGAGAUU | 245 |
| UUUAGAIGAAAAGGUGGGAGUU | 246 |
| UUCAUUIGAAGAAAAGGUGGUU | 247 |
| UUAGAAGIAAAGGUGGGAGAUU | 248 |
| UUUAGAAIAAAAGGUGGGAGUU | 249 |
| UUCAUUAIAAGAAAAGGUGGUU | 250 |
| GUCAUCCACAAUGAGAGUACA | 251 |
| UGUAIUCUCAUUGUGGAUGACGA | 252 |
| UGUACICUCAUUGUGGAUGACGA | 253 |
| UGUACUIUCAUUGUGGAUGACGA | 254 |
| UGUACUCICAUUGUGGAUGACGA | 255 |
| UUAGBAGAAAAGGUGGGAGAUU | 256 |
| UUUABAAGAAAAGGUGGGAGUU | 257 |
| UUCABUAGAAGAAAAGGUGGUU | 258 |
| UUAGABGAAAAGGUGGGAGAUU | 259 |
| UUUAGBAGAAAAGGUGGGAGUU | 260 |
| UUCAUBAGAAGAAAAGGUGGUU | 261 |
| UUAGAABAAAAGGUGGGAGAUU | 262 |
| UUUAGABGAAAAGGUGGGAGUU | 263 |
| UUCAUUBGAAGAAAAGGUGGUU | 264 |
| UUAGAAGBAAAGGUGGGAGAUU | 265 |
| UUUAGAABAAAAGGUGGGAGUU | 266 |
| UUCAUUABAAGAAAAGGUGGUU | 267 |
| UUAGXAGAAAAGGUGGGAGAUU | 268 |
| UUUAXAAGAAAAGGUGGGAGUU | 269 |
| UUCAXUAGAAGAAAAGGUGGUU | 270 |
| UUAGAXGAAAAGGUGGGAGAUU | 271 |
| UUUAGXAGAAAAGGUGGGAGUU | 272 |
| UUCAUXAGAAGAAAAGGUGGUU | 273 |
| UUAGAAXAAAAGGUGGGAGAUU | 274 |
| UUUAGAXGAAAAGGUGGGAGUU | 275 |
| UUCAUUXGAAGAAAAGGUGGUU | 276 |
| UUAGAAGXAAAGGUGGGAGAUU | 277 |
| UUUAGAAXAAAAGGUGGGAGUU | 278 |
| UUCAUUAXAAGAAAAGGUGGUU | 279 |
| UUUAAAAGAAAAGGUGGGAGUU | 280 |
| UUCAAUAGAAGAAAAGGUGGUU | 281 |
| UUCAUAAGAAGAAAAGGUGGUU | 282 |
| UUAGAAAAAAAGGUGGGAGAUU | 283 |
| UUUAGAAAAAAAGGUGGGAGUU | 284 |
| UUCAUUAAAAGAAAAGGUGGUU | 285 |
| UTGUGGGCUCUCUCUCAUCCGGG | 286 |
| UUAGAAGAAAAGGUGGGAGAUI | 287 |
| UUUAGAAGAAAAGGUGGGAGUI | 288 |
| UUAGAAGAAAAGGUGGGAGAIU | 289 |
| UUUAGAAGAAAAGGUGGGAGIU | 290 |
| UUCAUUAGAAGAAAAGGUGGIU | 291 |
| TCCCACCUUUUCUUCUAAA | 292 |
| UUAGAAGAAAAGGUGGGAGUI | 293 |
| UUUAGAAGAAAAGGUGGGAUI | 294 |
| UUCAUUAGAAGAAAAGGUGUI | 295 |
| UUAGAAGAAAAGGUGGGAGIU | 296 |
| UUUAGAAGAAAAGGUGGGAIU | 297 |
| UUCAUUAGAAGAAAAGGUGIU | 298 |
| UUCAUUAGAAGAAAAGGUGGGA | 299 |
| UUCAUUAGAAGAAAAGGUGGG | 300 |
| BUCAUUAGAAGAAAAGGUGGGA | 301 |
| BUCAUUAGAAGAAAAGGUGGG | 302 |
| BUCAUUAGAAGAAAAGGUGGUU | 303 |
| BUCAUUAGAAGAAAAGGUGUU | 304 |
| BUCAUUAGAAGAAAAGGUGGIU | 305 |
| BUCAUUAGAAGAAAAGGUGIU | 306 |
| BUCAUUAGAAGAAAAGGUGGUI | 307 |
| BUCAUUAGAAGAAAAGGUGUI | 308 |
| XUCAUUAGAAGAAAAGGUGGGA | 309 |
| XUCAUUAGAAGAAAAGGUGGG | 310 |
| XUCAUUAGAAGAAAAGGUGGUU | 311 |
| XUCAUUAGAAGAAAAGGUGUU | 312 |
| XUCAUUAGAAGAAAAGGUGGIU | 313 |
| XUCAUUAGAAGAAAAGGUGIU | 314 |
| XUCAUUAGAAGAAAAGGUGGUI | 315 |
| XUCAUUAGAAGAAAAGGUGUI | 316 |
| UTCATUAGAAGAAAAGGUGGGA | 317 |
| UTCATUAGAAGAAAAGGUGGG | 318 |
| UTCATUAGAAGAAAAGGUGUU | 319 |
| BTCATUAGAAGAAAAGGUGGGA | 320 |
| BTCATUAGAAGAAAAGGUGGG | 321 |
| BTCATUAGAAGAAAAGGUGGUU | 322 |
| BTCATUAGAAGAAAAGGUGUU | 323 |
| XTCATUAGAAGAAAAGGUGGGA | 324 |
| XTCATUAGAAGAAAAGGUGGG | 325 |
| XTCATUAGAAGAAAAGGUGGUU | 326 |
| XTCATUAGAAGAAAAGGUGUU | 327 |
| UTCATUAGAAGAAAAGGUGGIU | 328 |
| UTCATUAGAAGAAAAGGUGIU | 329 |
| BTCATUAGAAGAAAAGGUGGIU | 330 |
| BTCATUAGAAGAAAAGGUGIU | 331 |
| XTCATUAGAAGAAAAGGUGGIU | 332 |
| XTCATUAGAAGAAAAGGUGIU | 333 |
| UTCATUAGAAGAAAAGGUGGUI | 334 |
| UTCATUAGAAGAAAAGGUGUI | 335 |
| BTCATUAGAAGAAAAGGUGGUI | 336 |
| BTCATUAGAAGAAAAGGUGUI | 337 |
| XTCATUAGAAGAAAAGGUGGUI | 338 |
| XTCATUAGAAGAAAAGGUGUI | 339 |
| UUCAUUAGAAGAAAAGGUGG | 340 |
| UUCAUUAGAAGAAAAGGUG | 341 |
| CCCACCUUUUCUUCUAAUGAA | 342 |
| UUCAUUAGAAGAAAAGGUGGGUU | 343 |
| UUCAUUAGAAGAAAAGGUGGGTT | 344 |
| UUCAUUAGAAGAAAAGGUGGTT | 345 |
| UUCAUUAGAAGAAAAGGUGTT | 346 |
| UUCAUUAGAAGAAAAGGUGGGAG | 347 |
| UGUACTCUCAUUGUGGAUGACGA | 348 |
| AAUUUAGACCAAGGAGAAACGUC | 349 |
| CGUUUCUCCUUGGUCUAAAUU | 350 |
| CACUUAGACCAAGGAGAAACGUC | 351 |

wherein C, G, U, A, and T represent base moieties of a nucleotide, including modified and unmodified nucleotides; I represents a base moiety of a modified nucleotide, wherein the base is 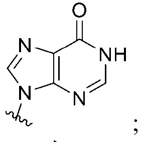 m6A represents a base moiety of a modified nucleotide, wherein the base is 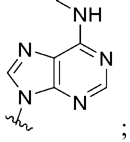 X represents a base moiety of a modified nucleotide, wherein the base is 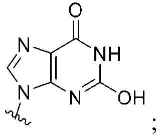 and B represents a base moiety of a modified nucleotide, wherein the base is

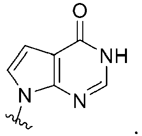

In some embodiments of the present invention, the siRNA conjugate is selected from Conjugates 1 to 1668.

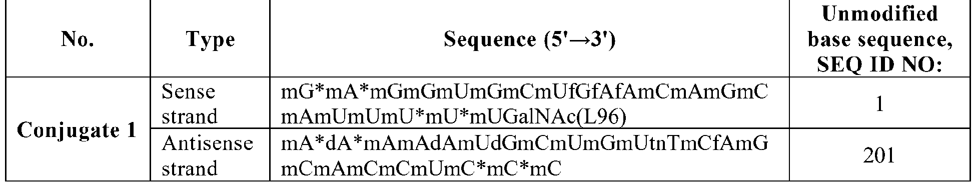

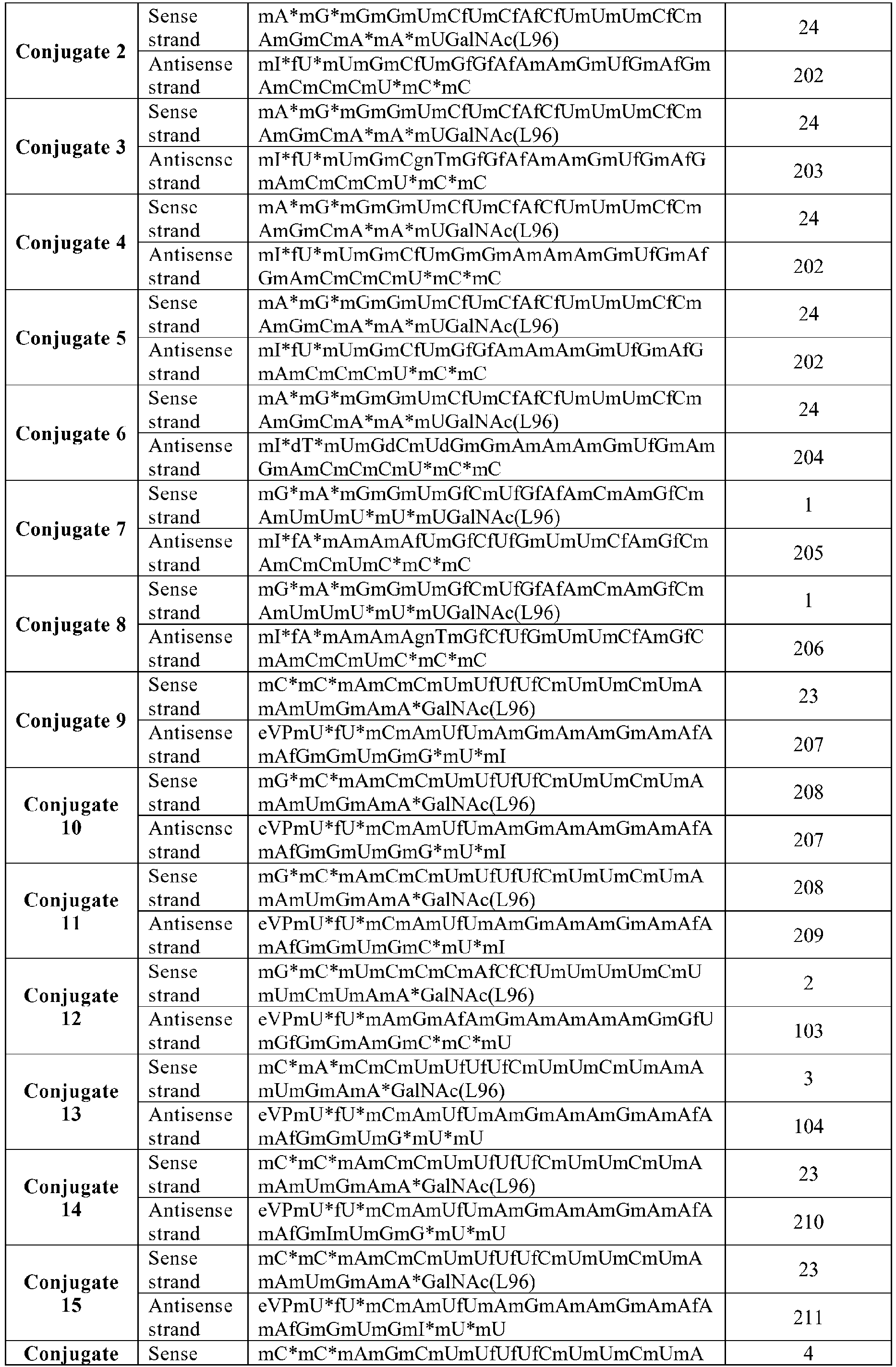

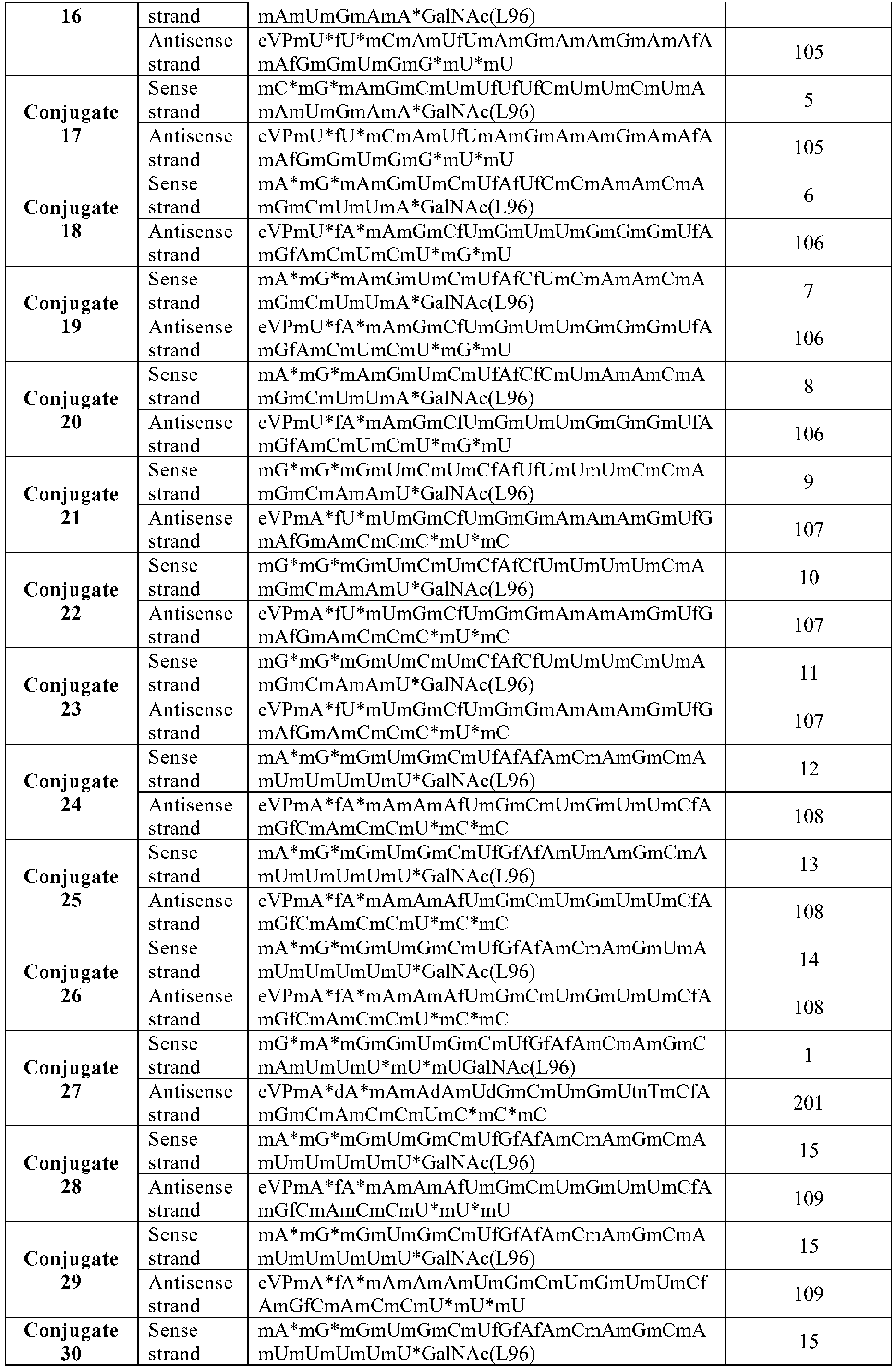

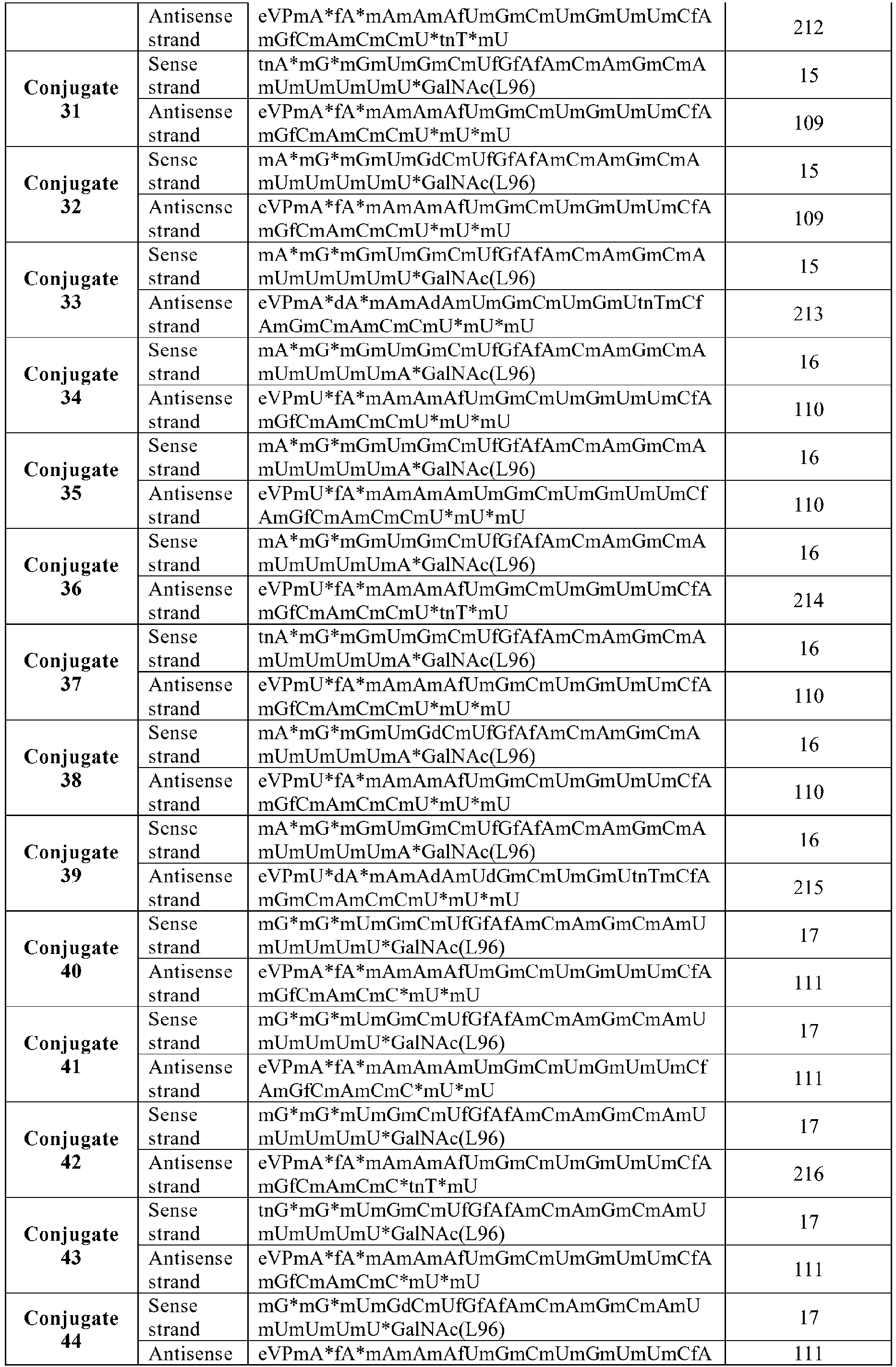

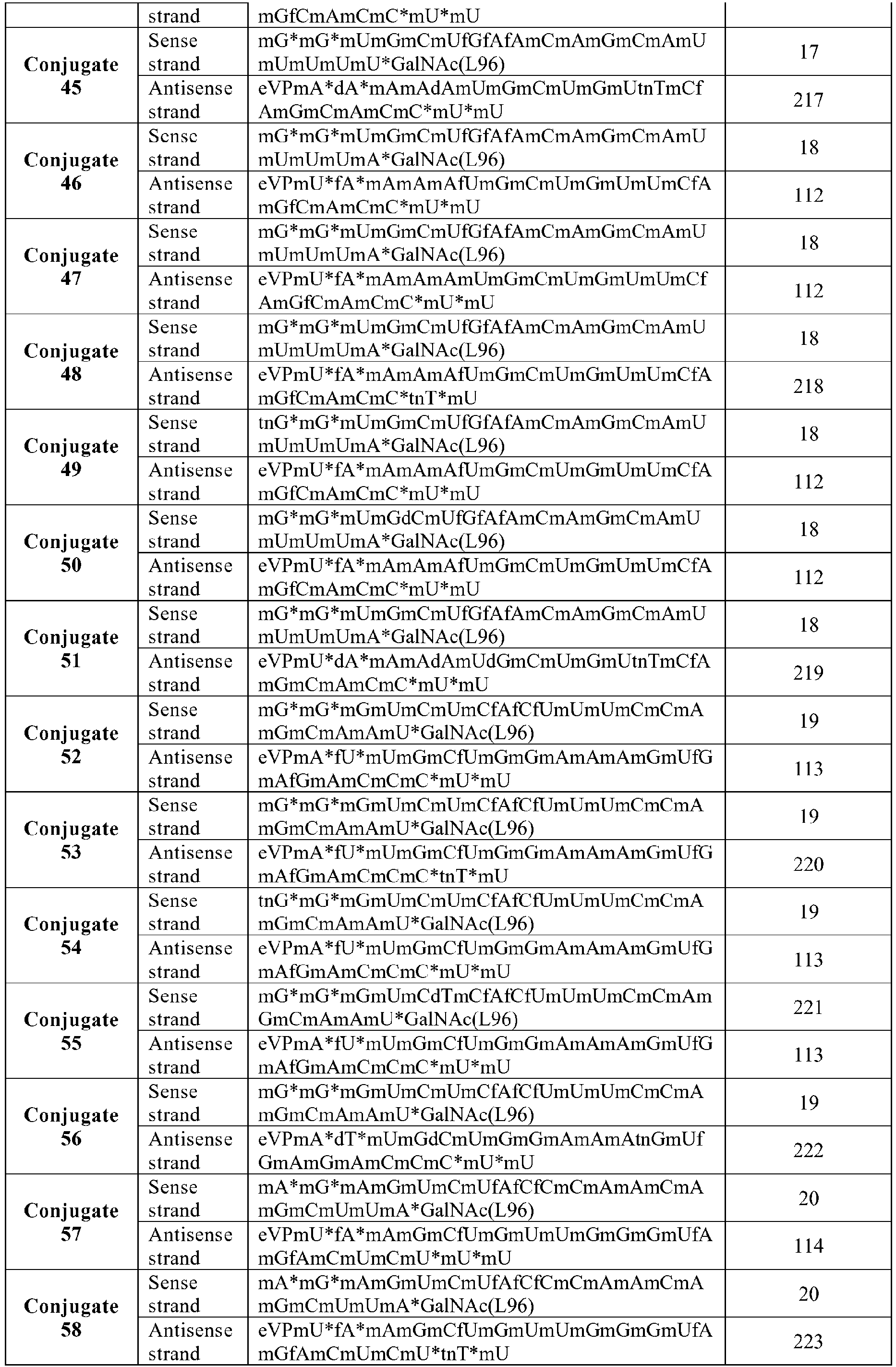

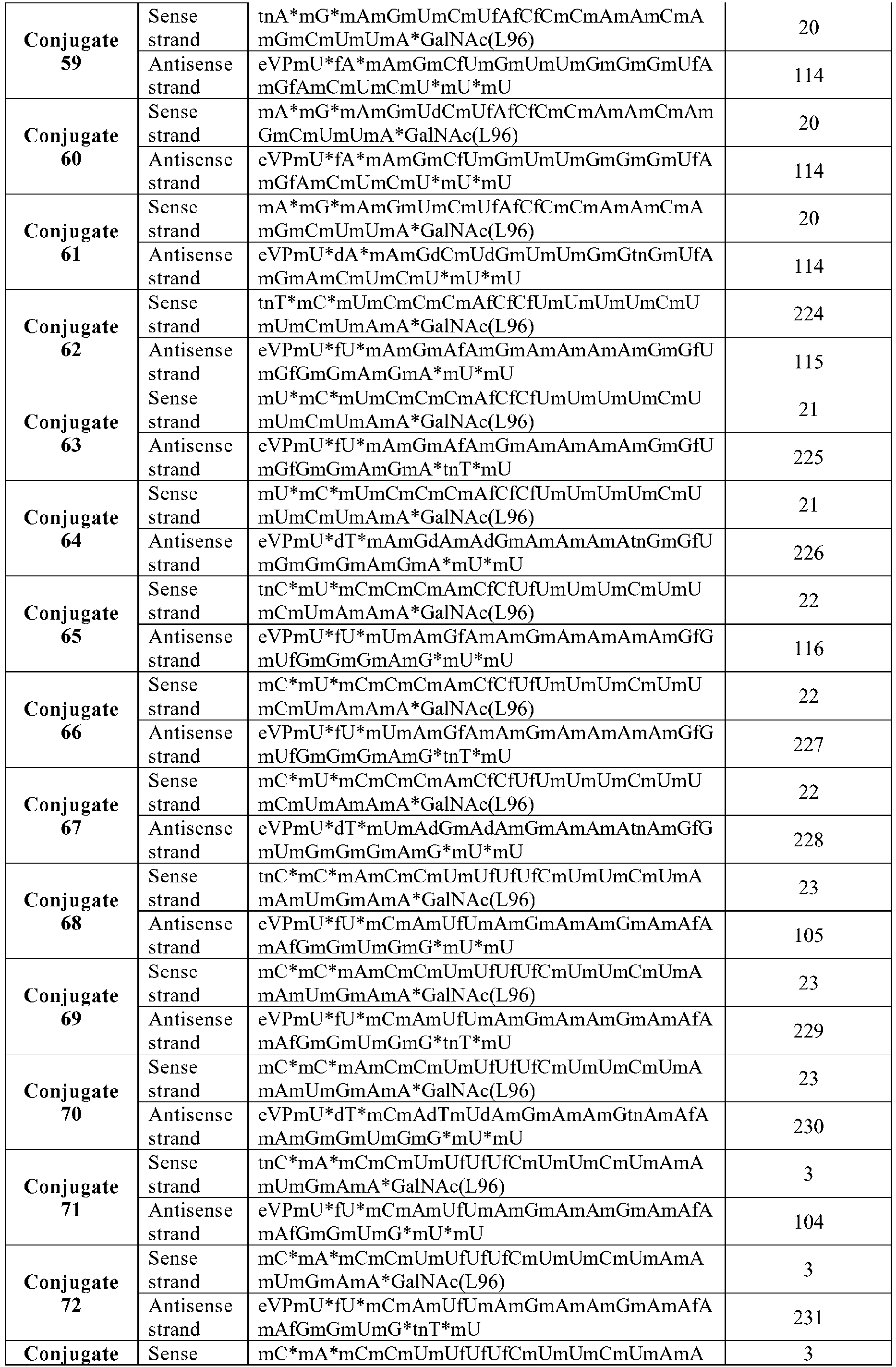

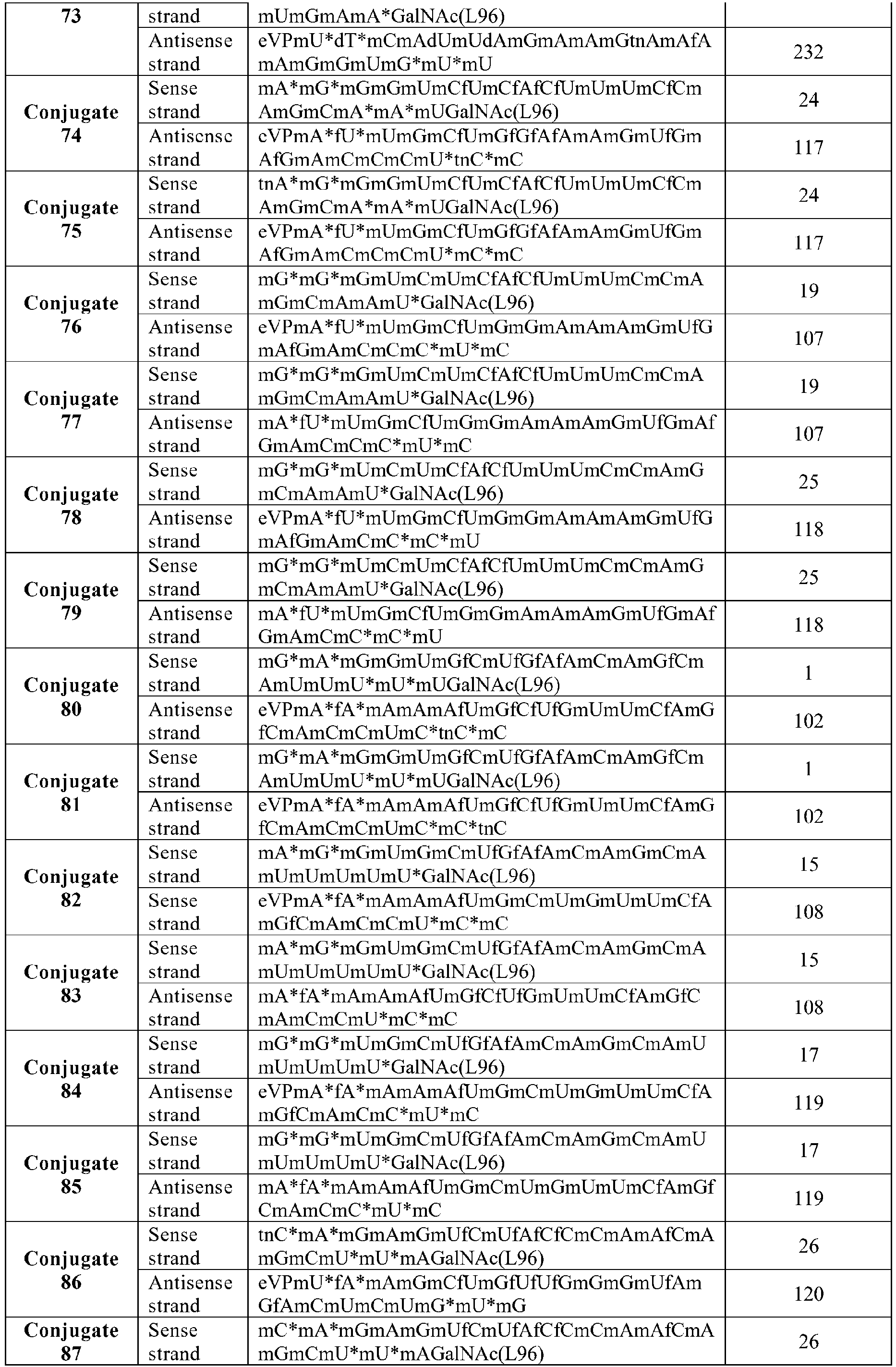

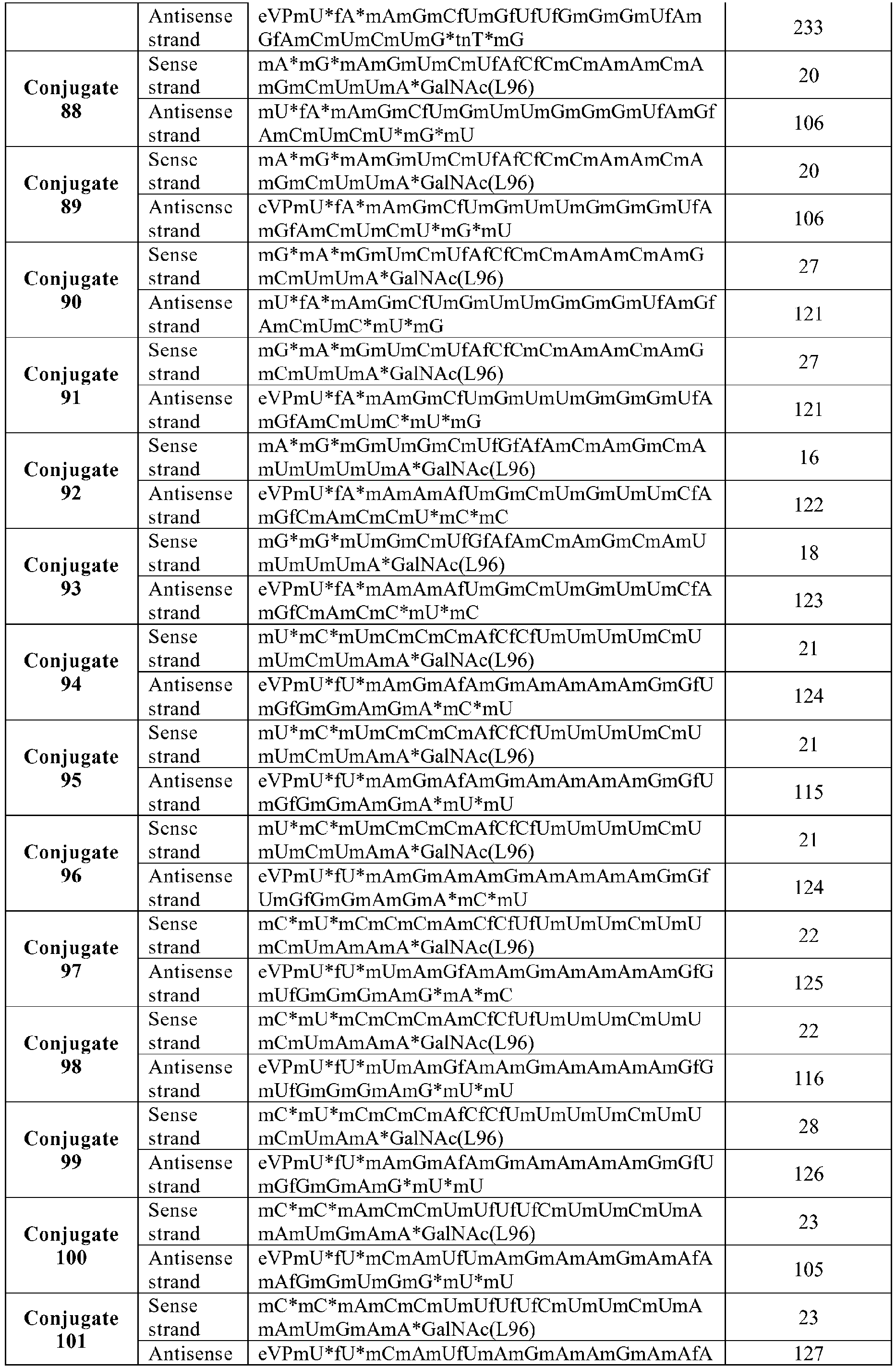

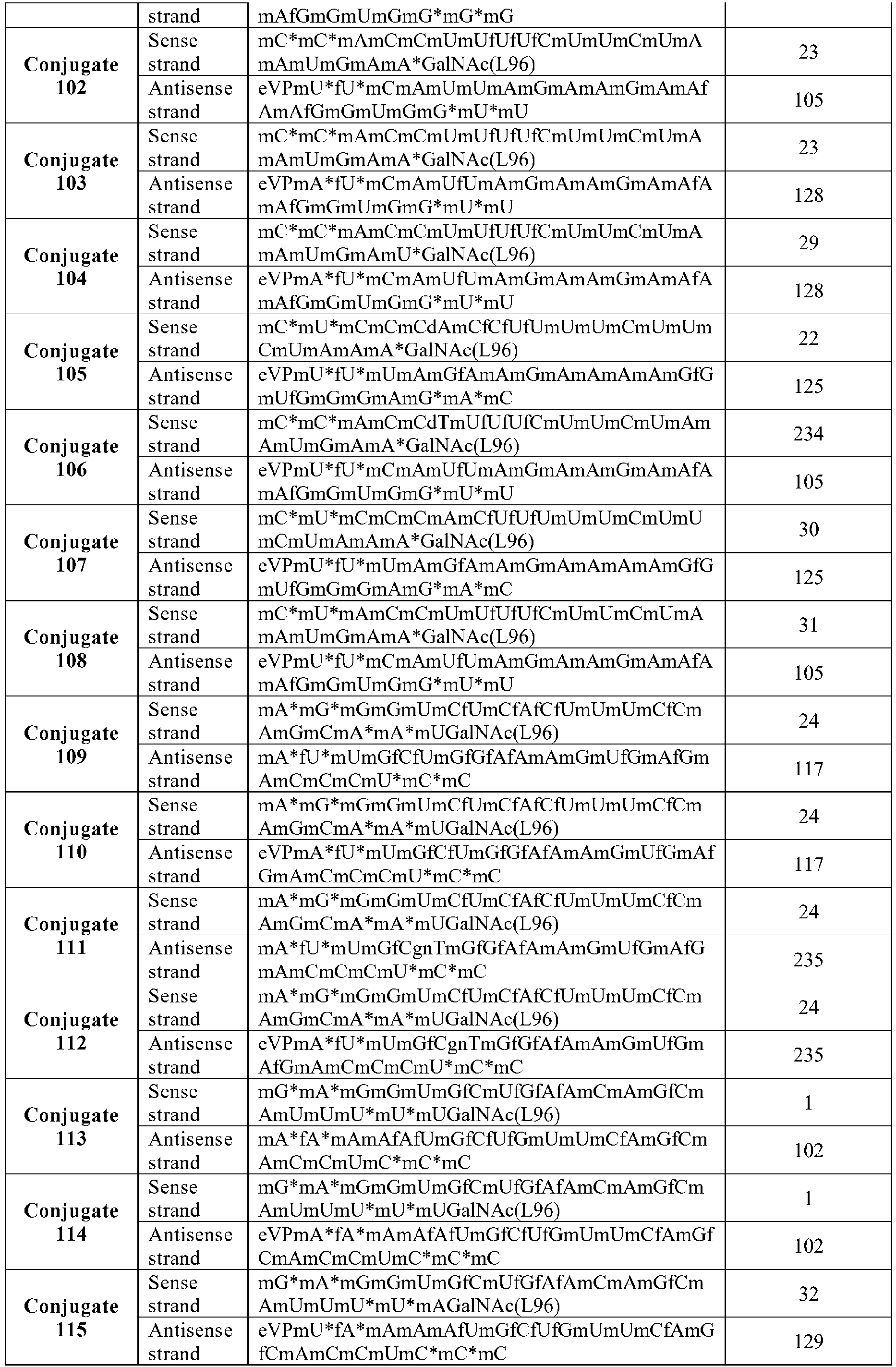

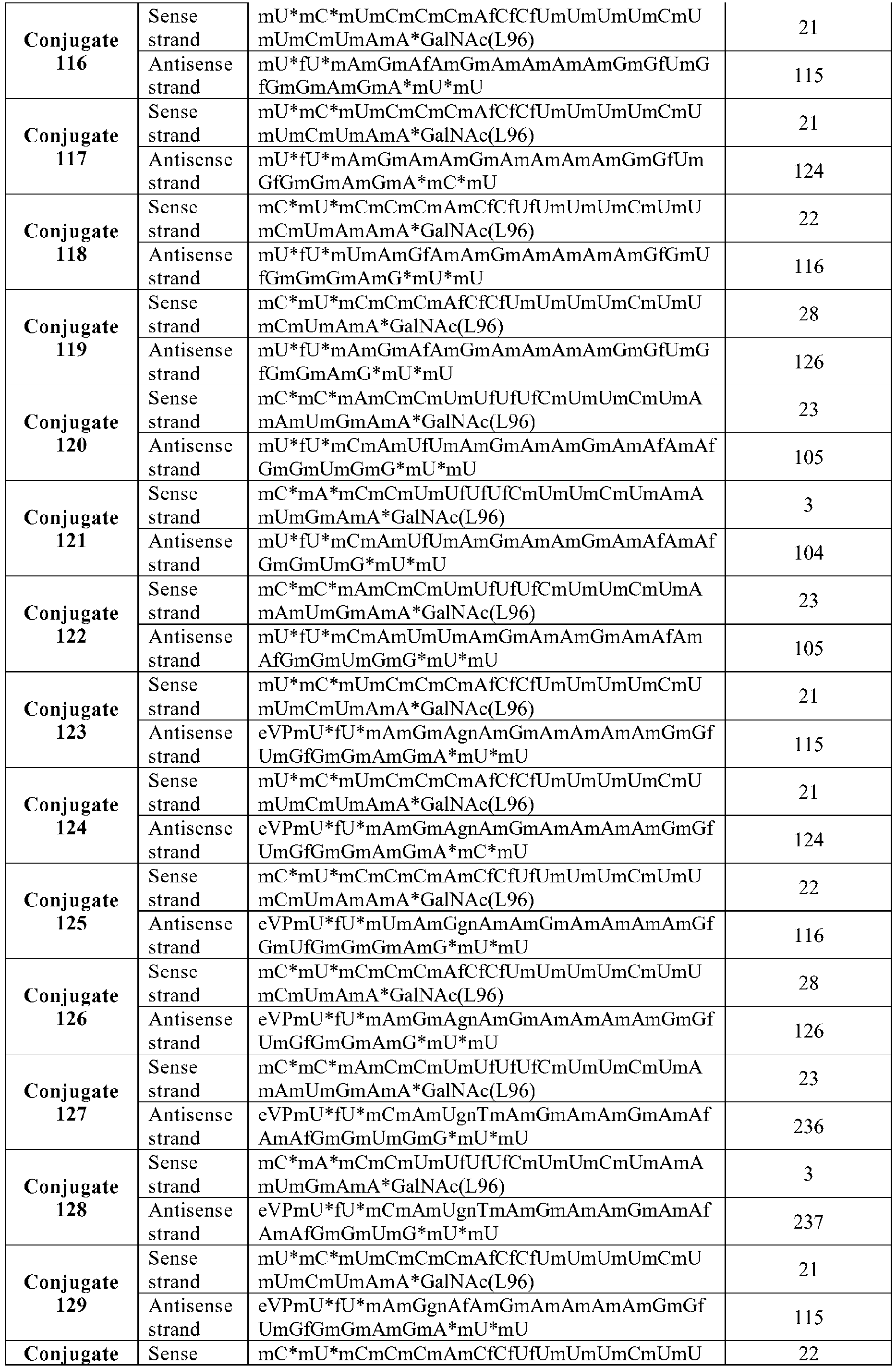

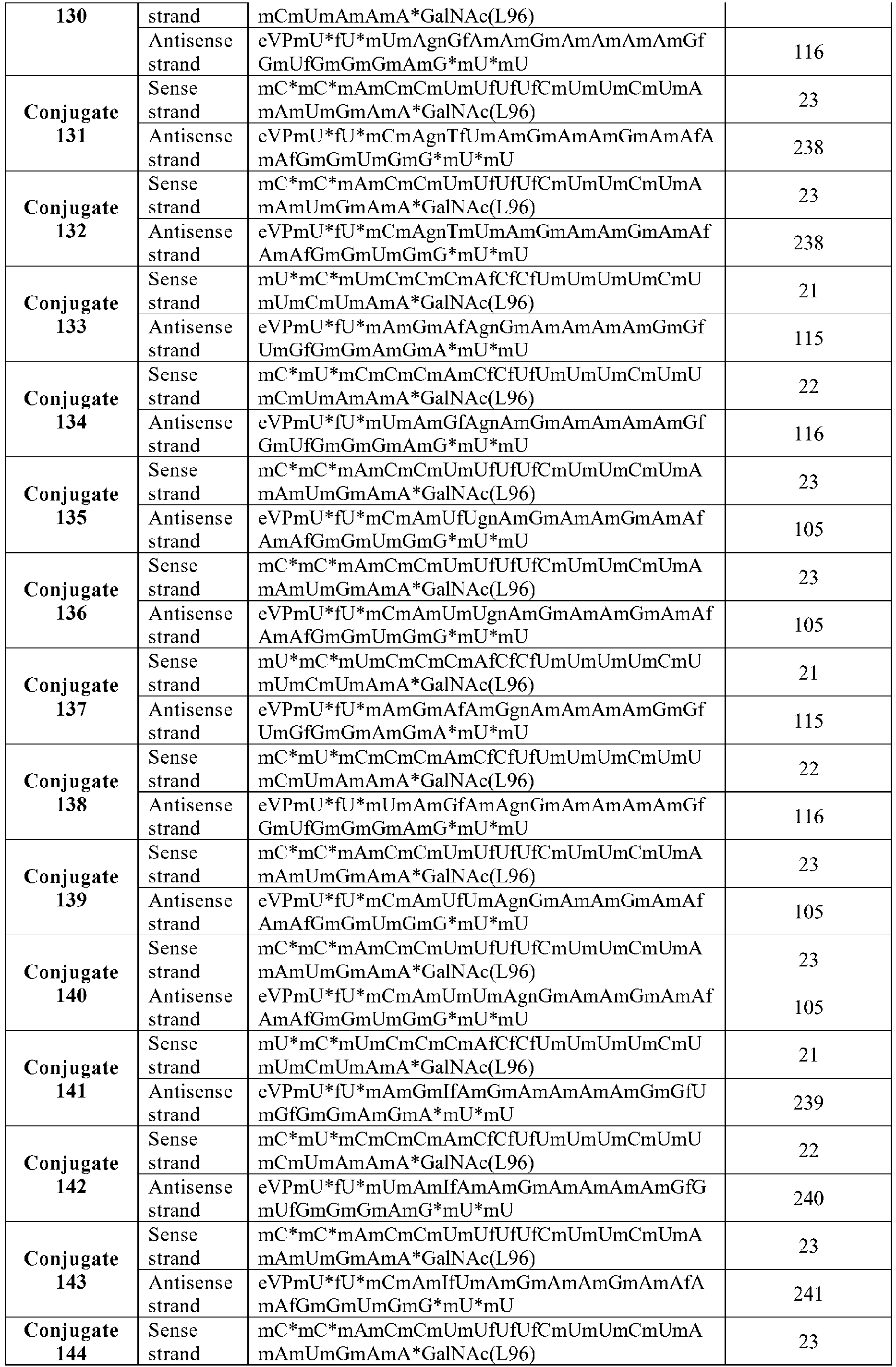

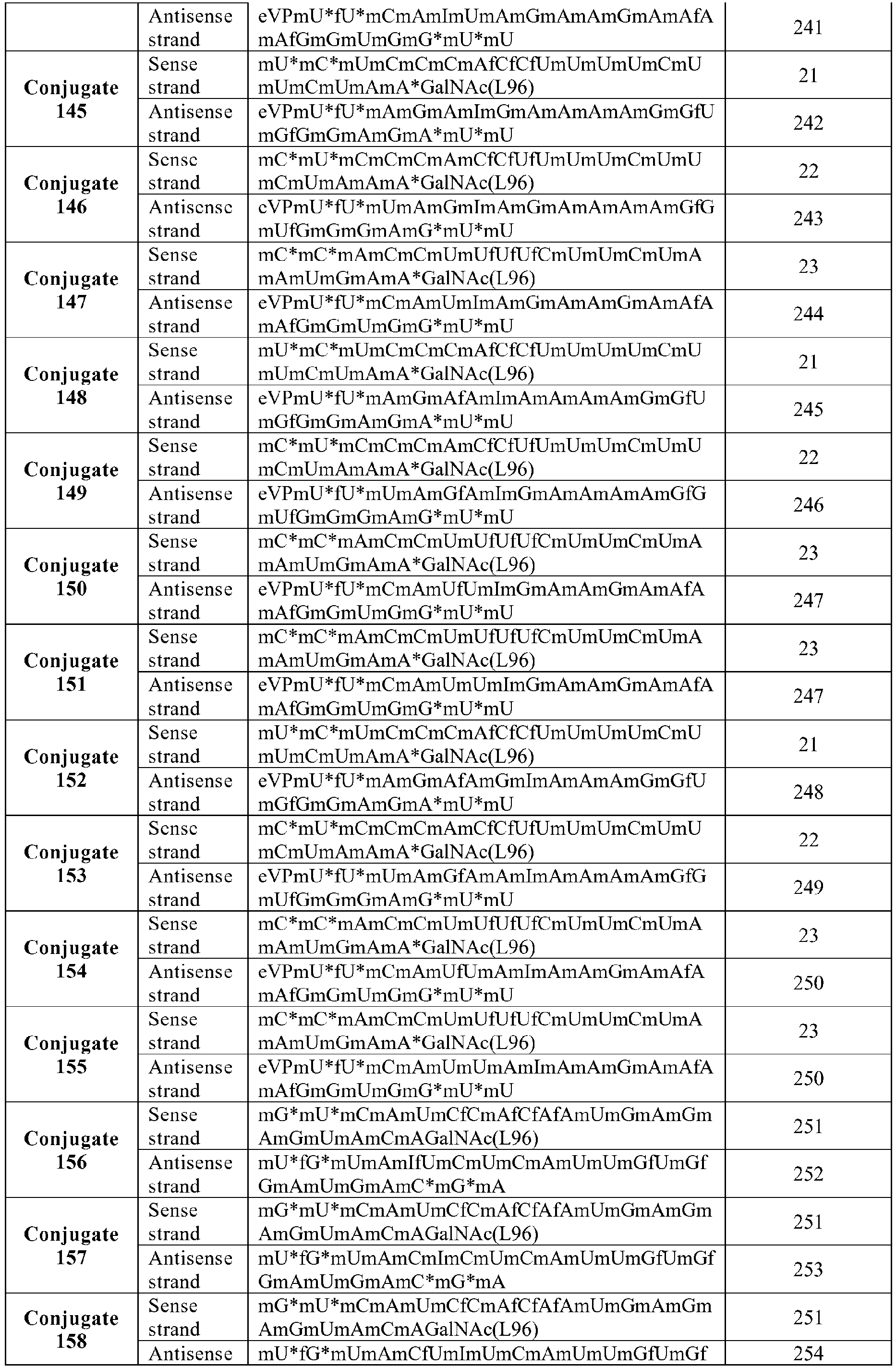

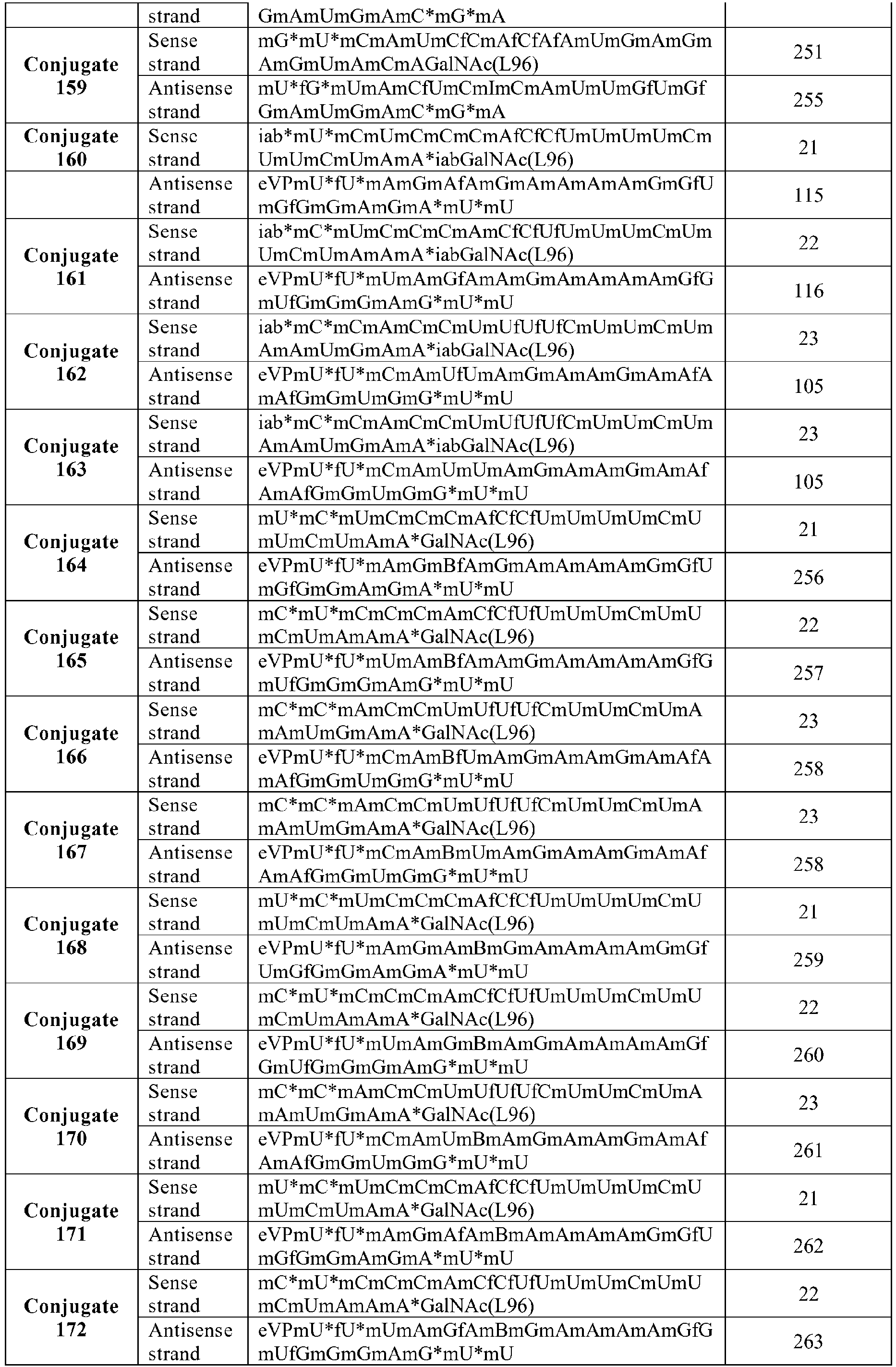

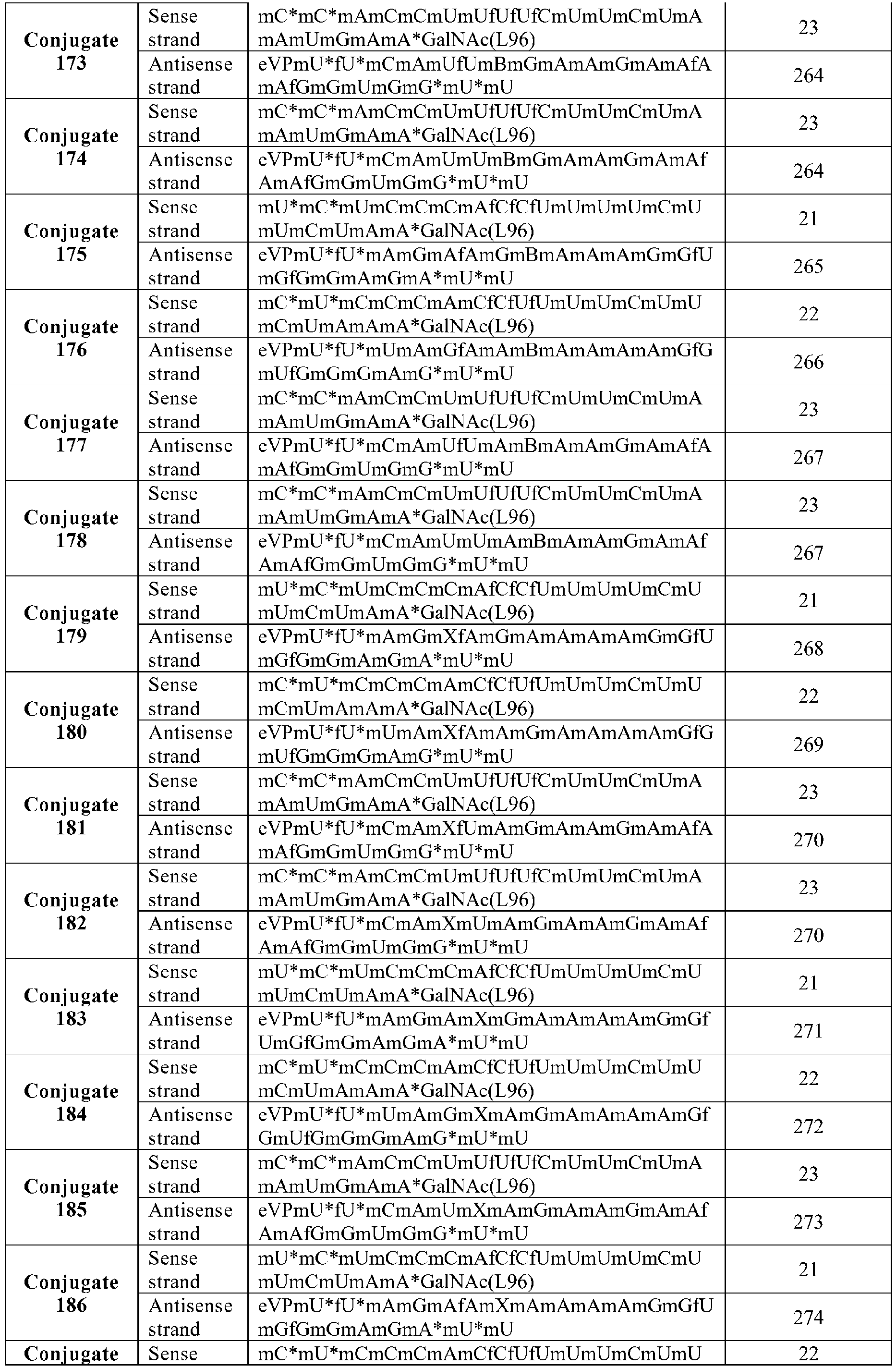

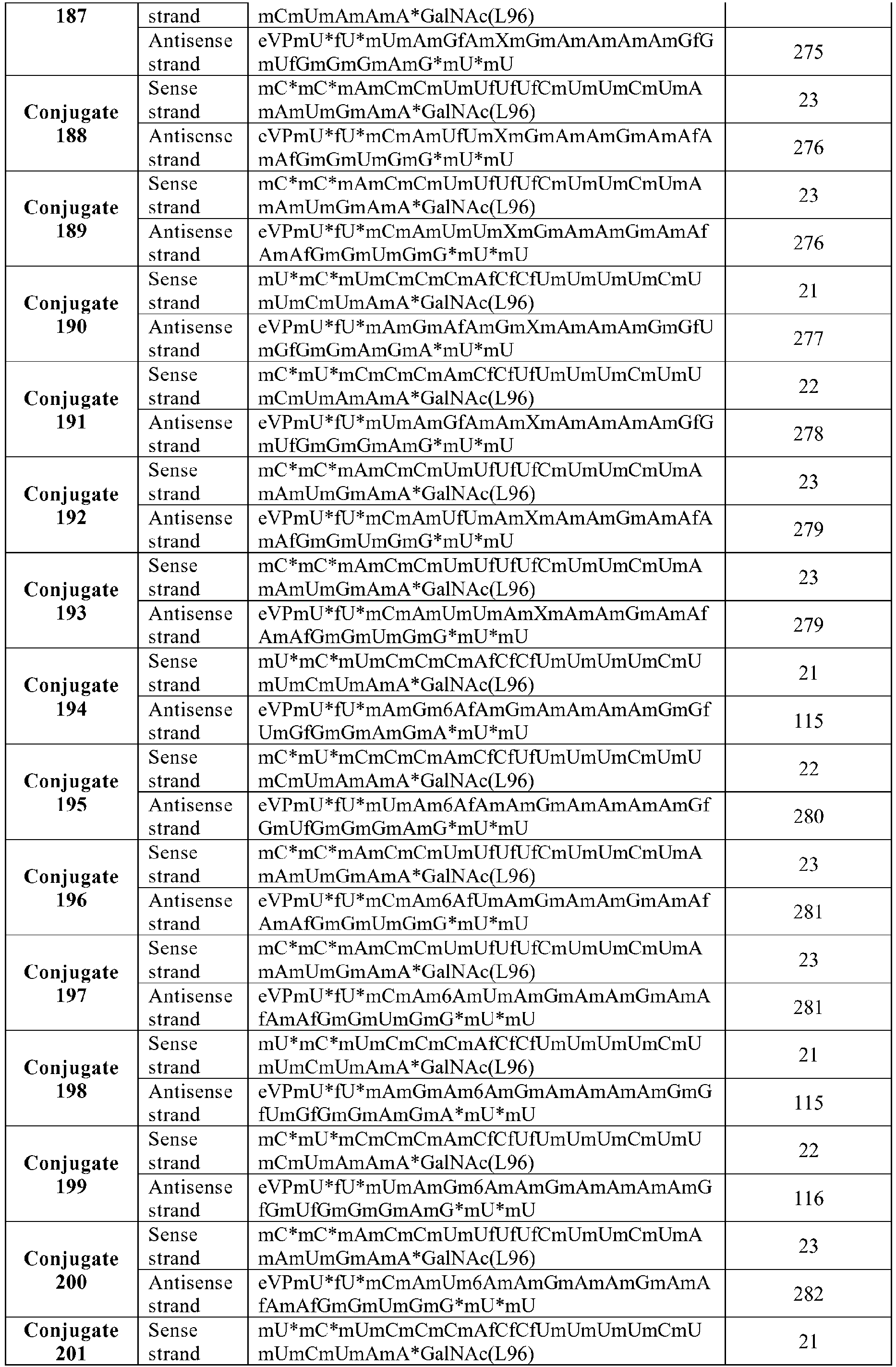

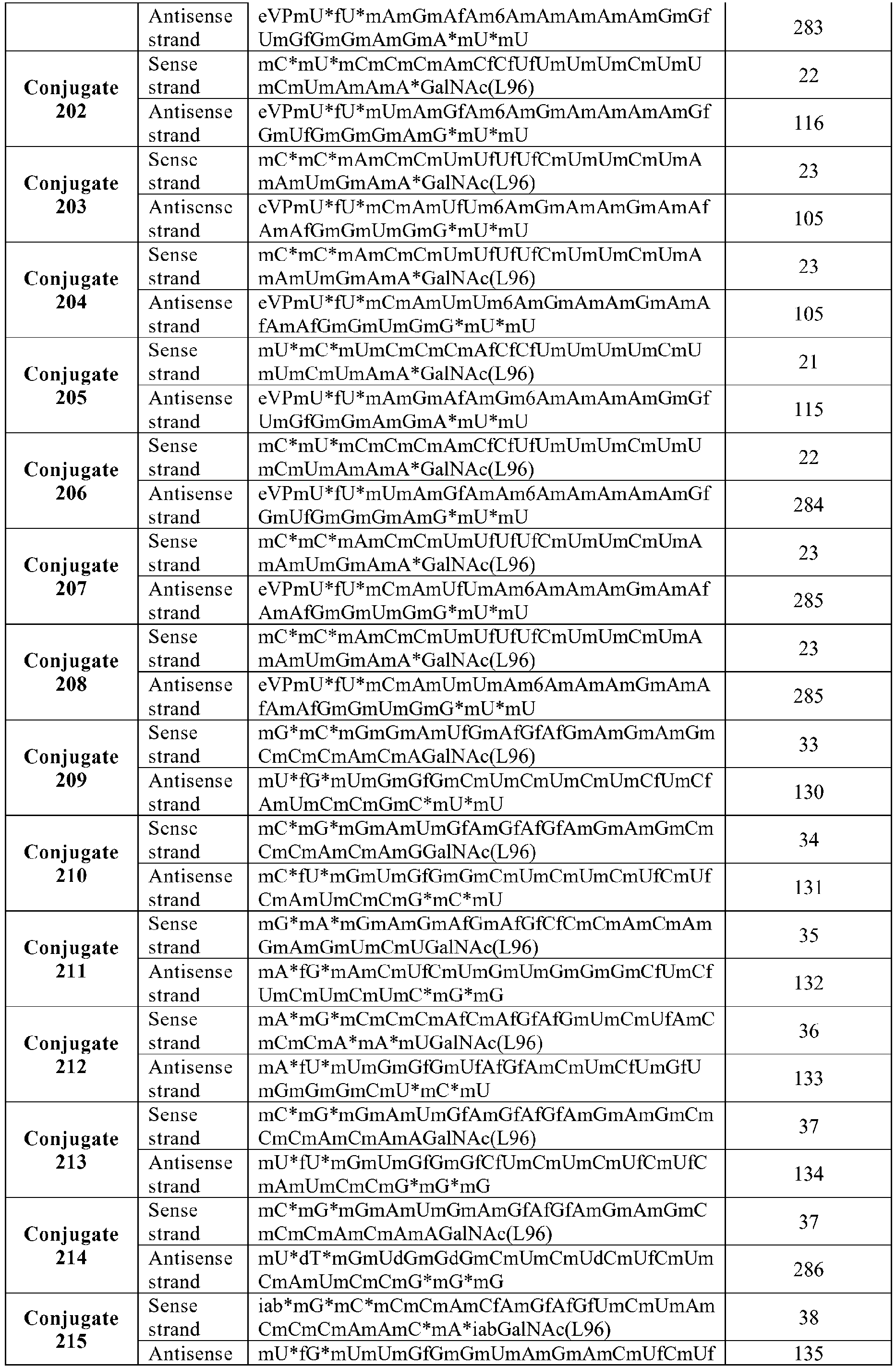

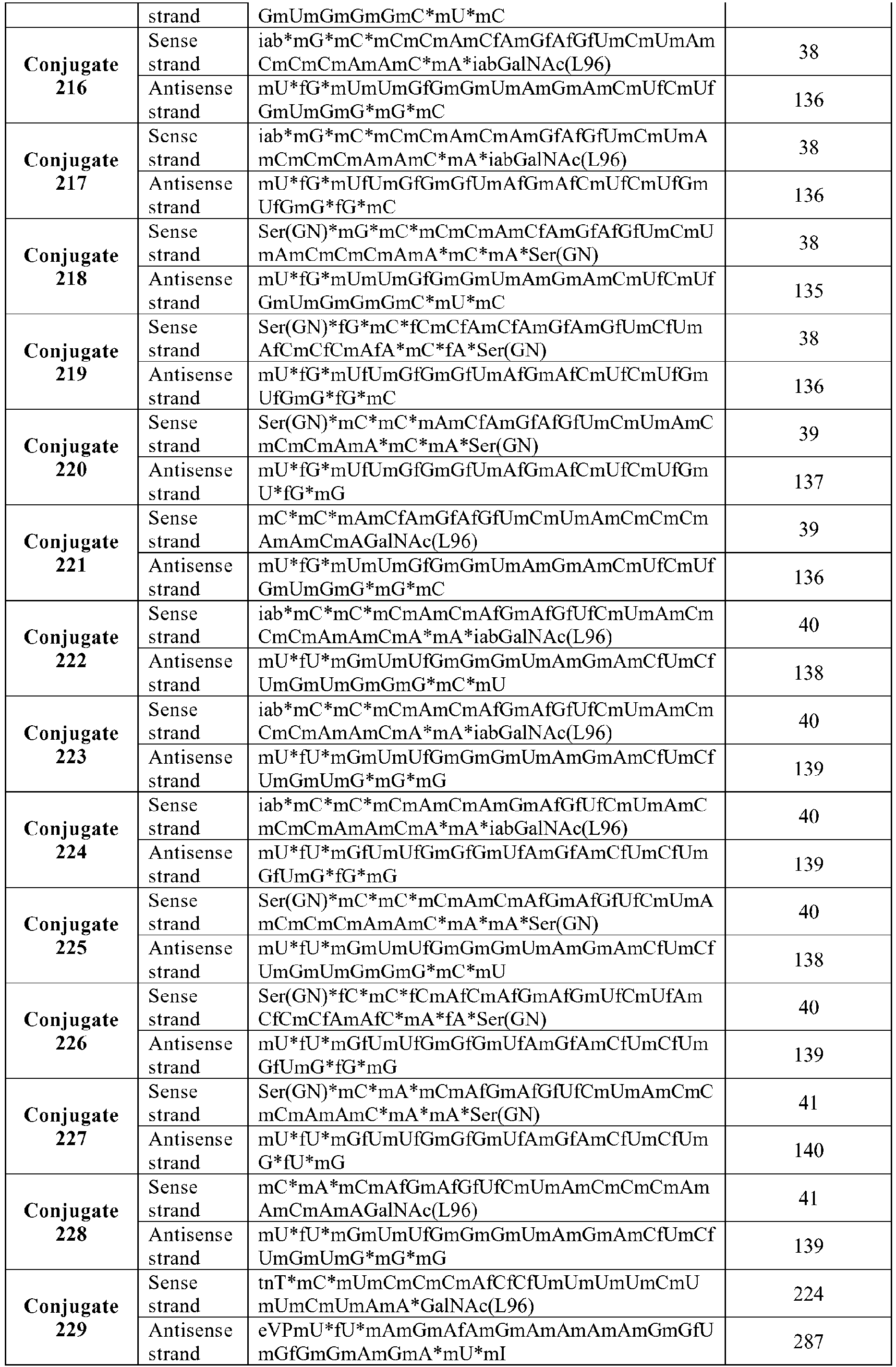

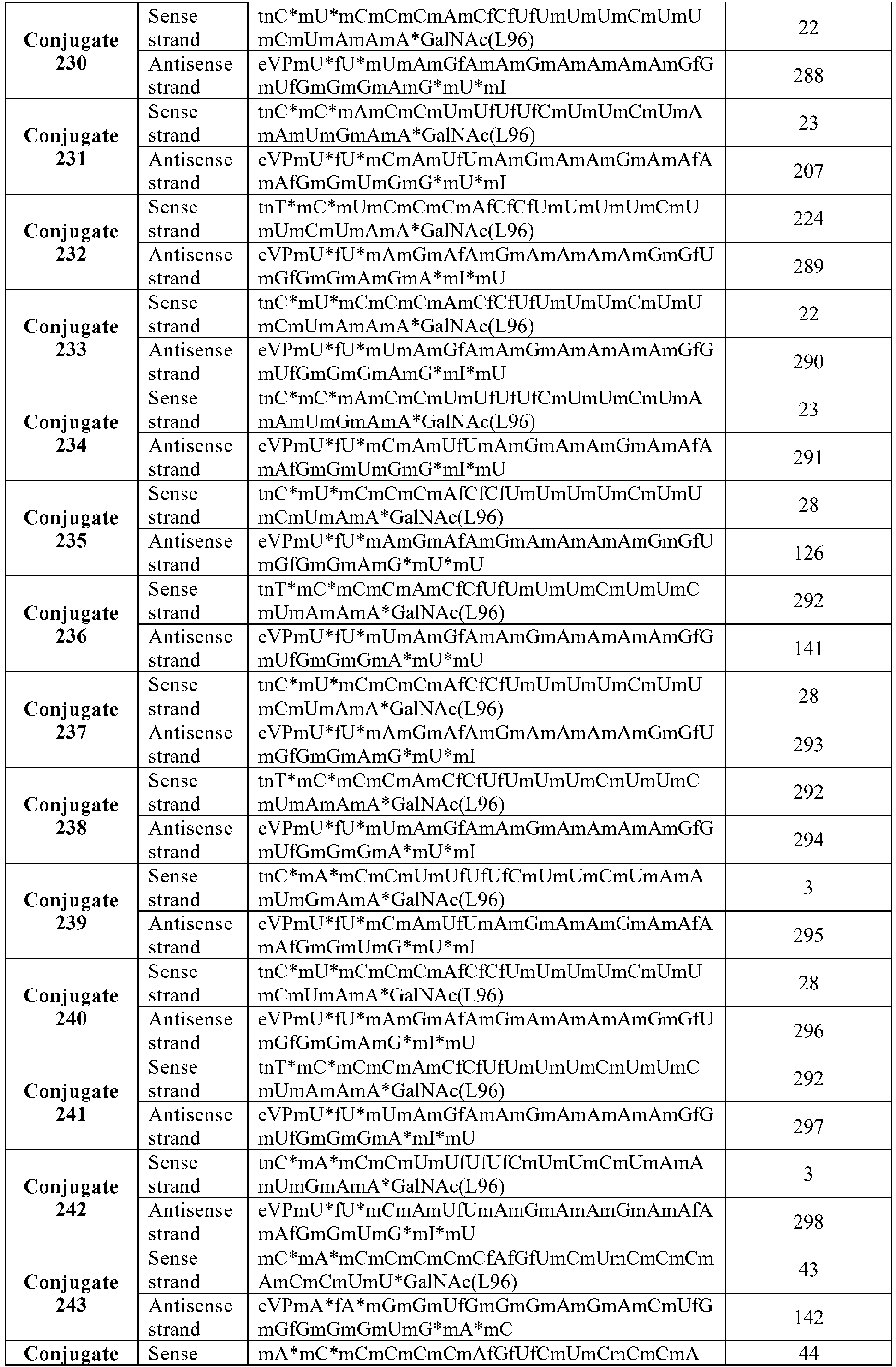

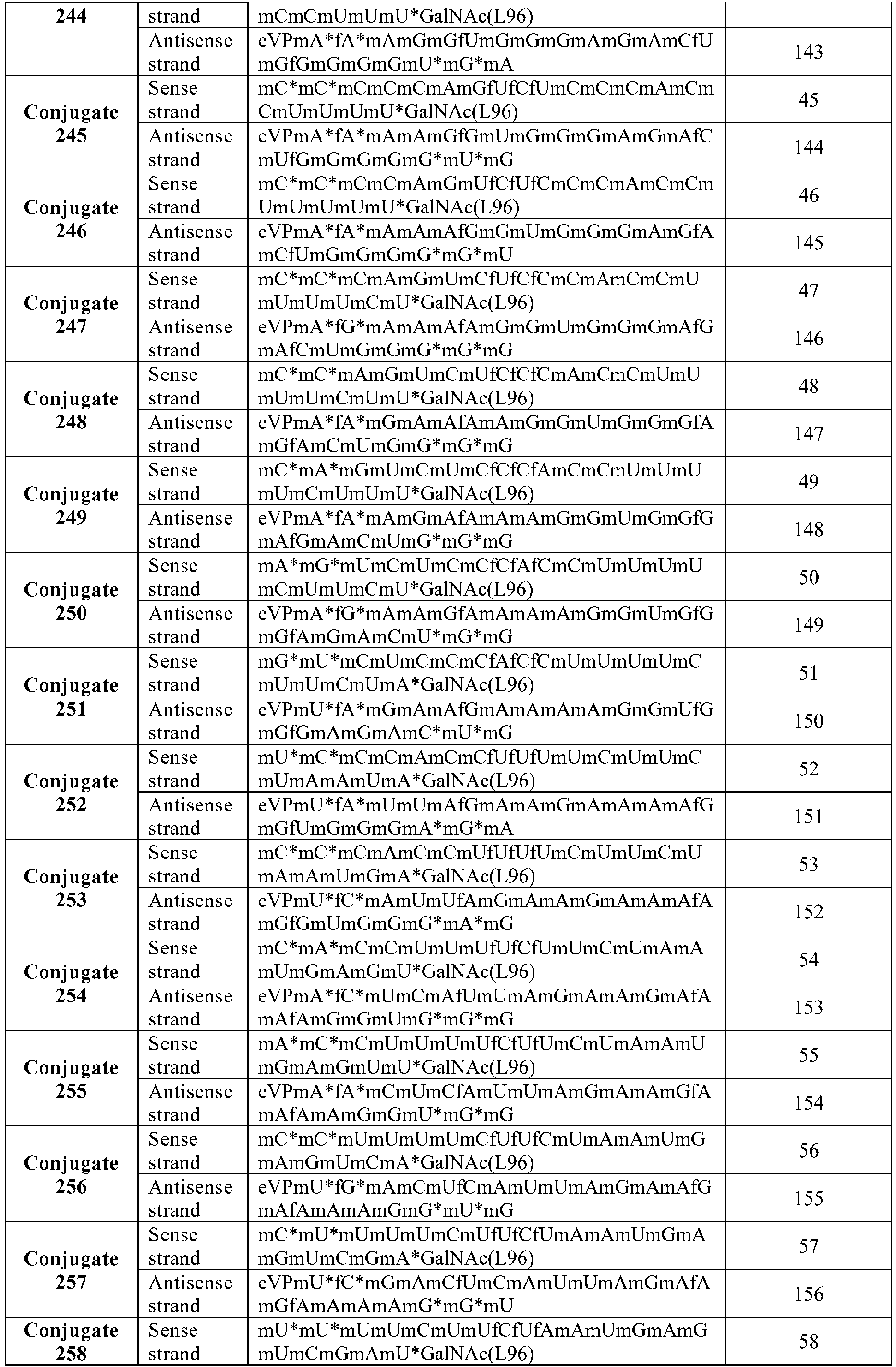

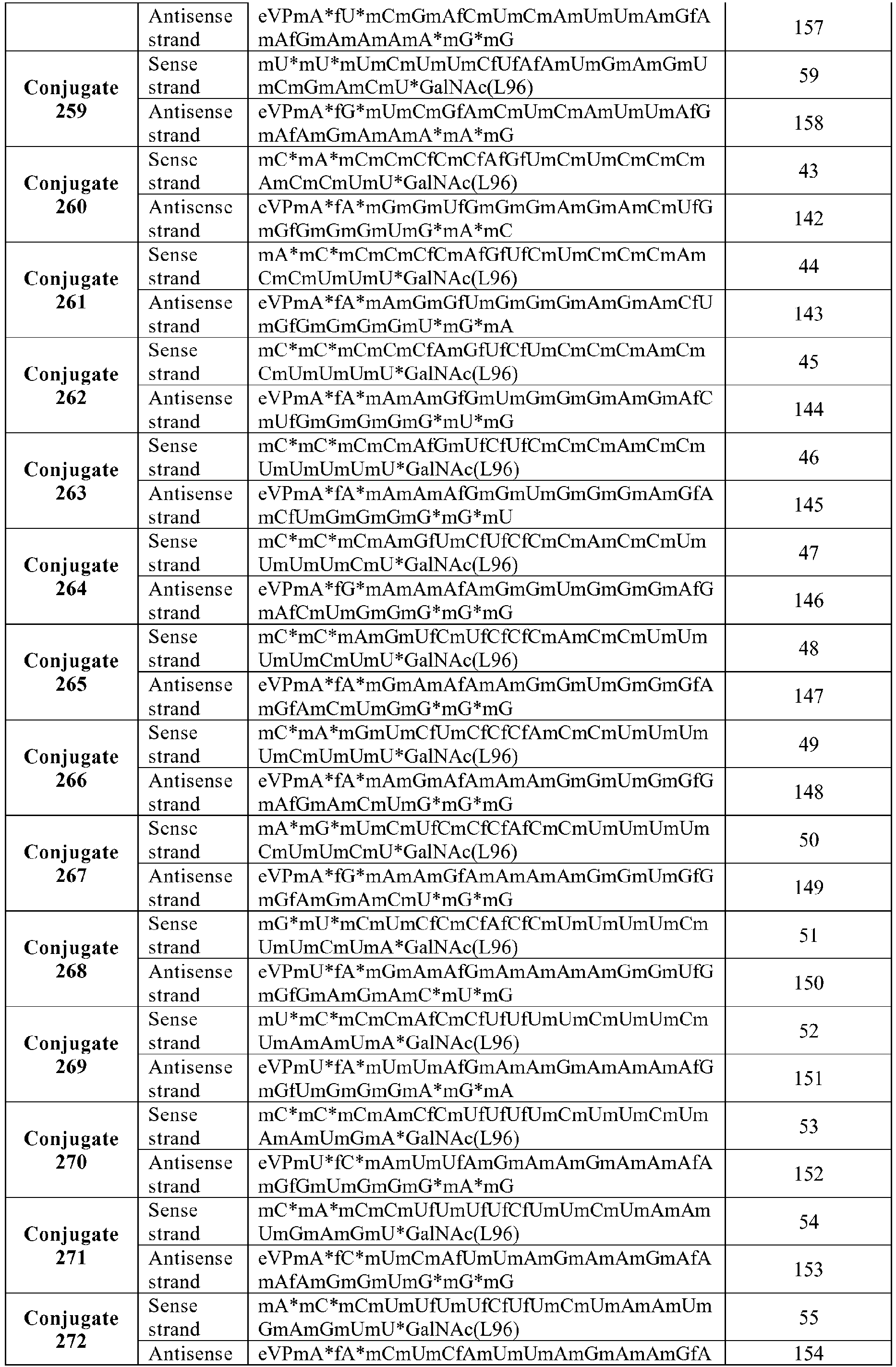

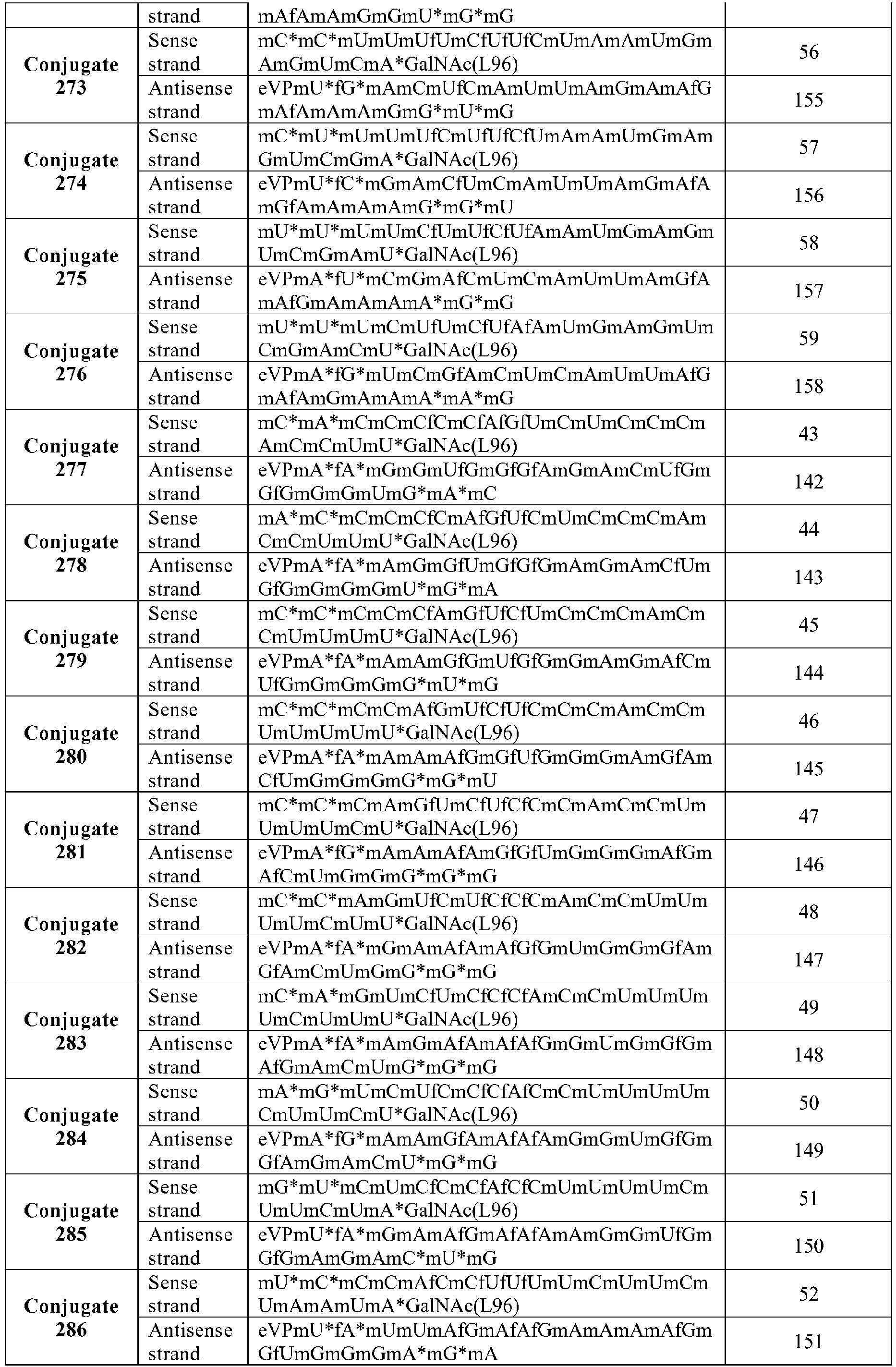

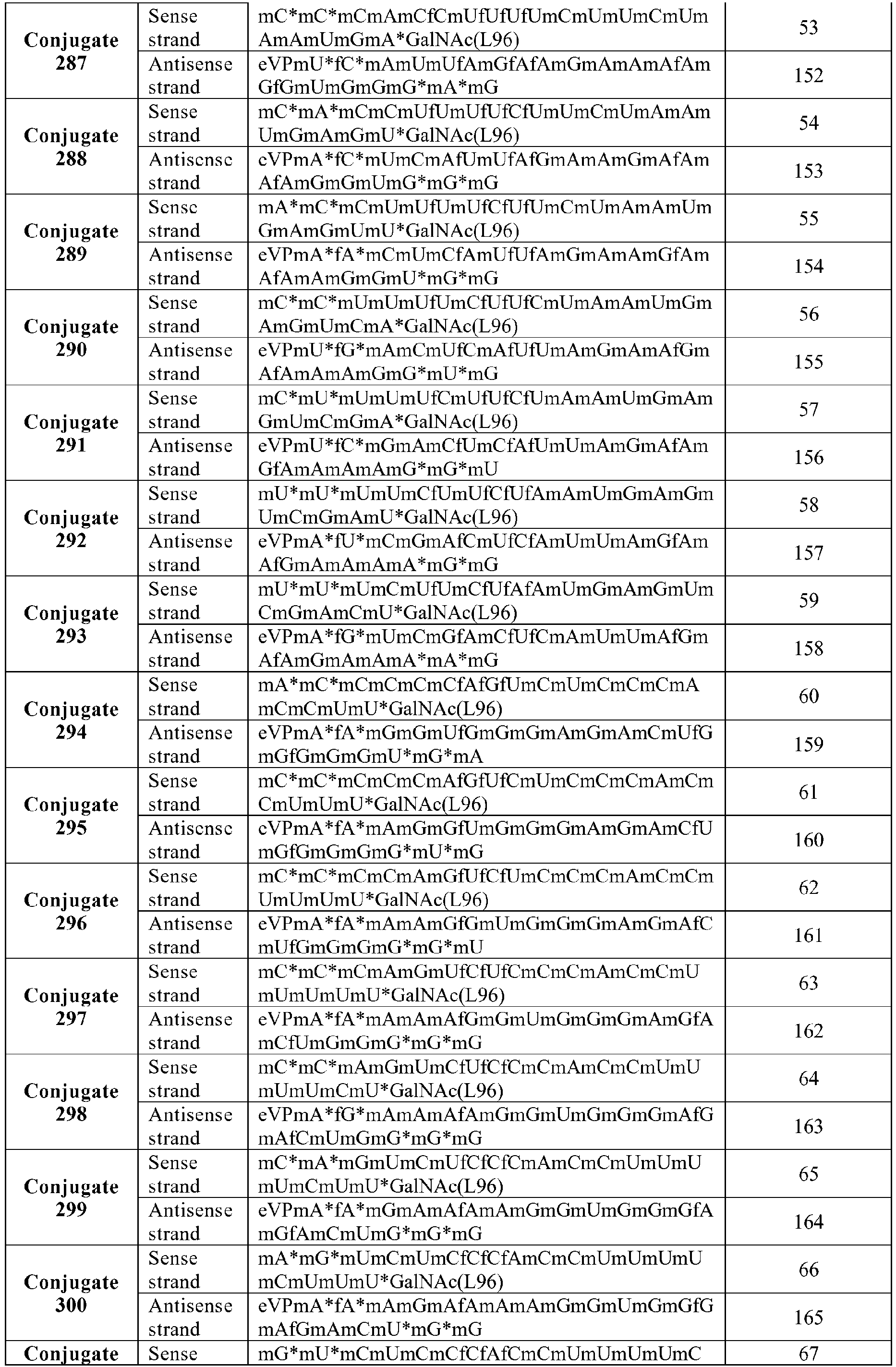

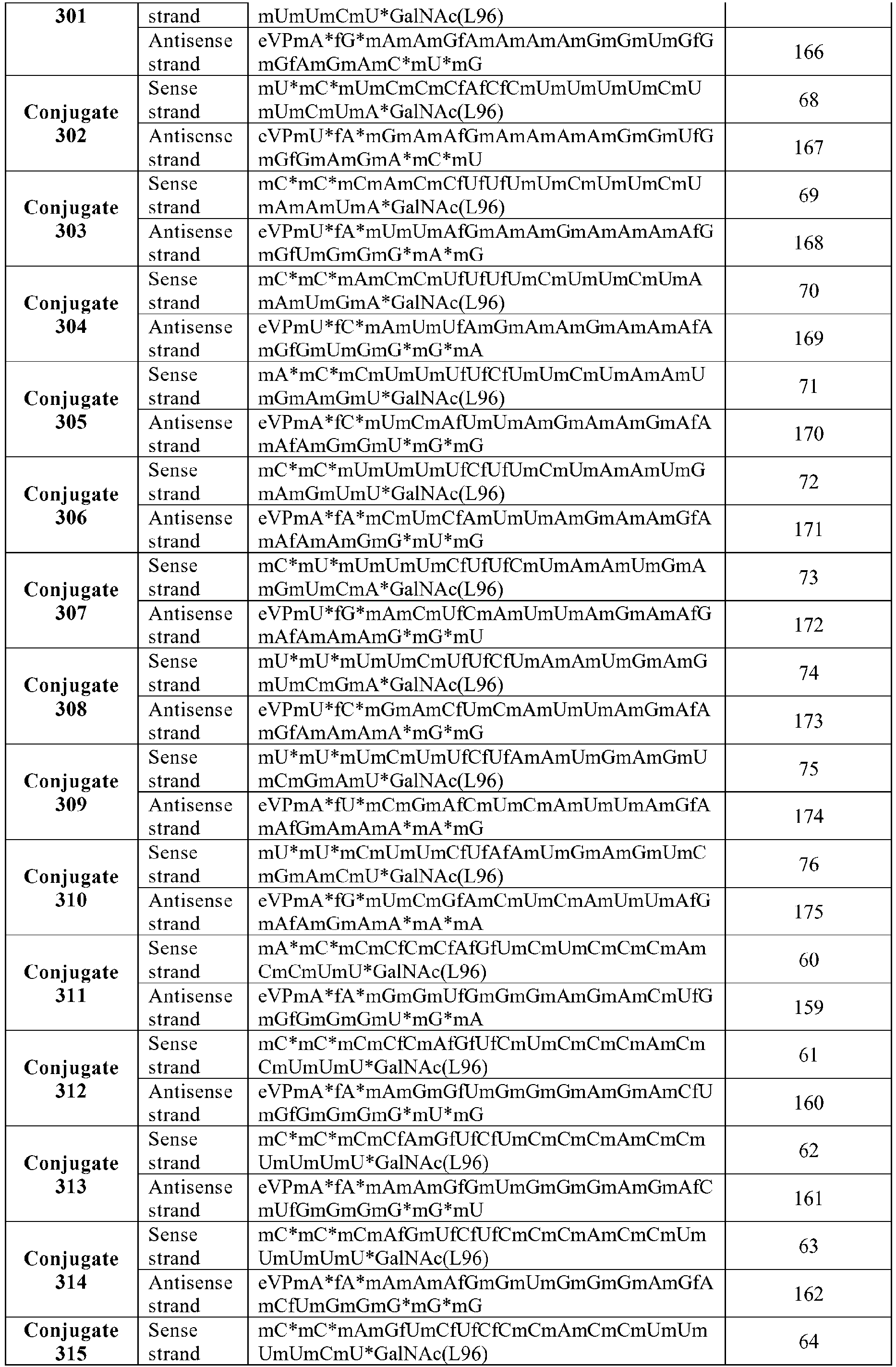

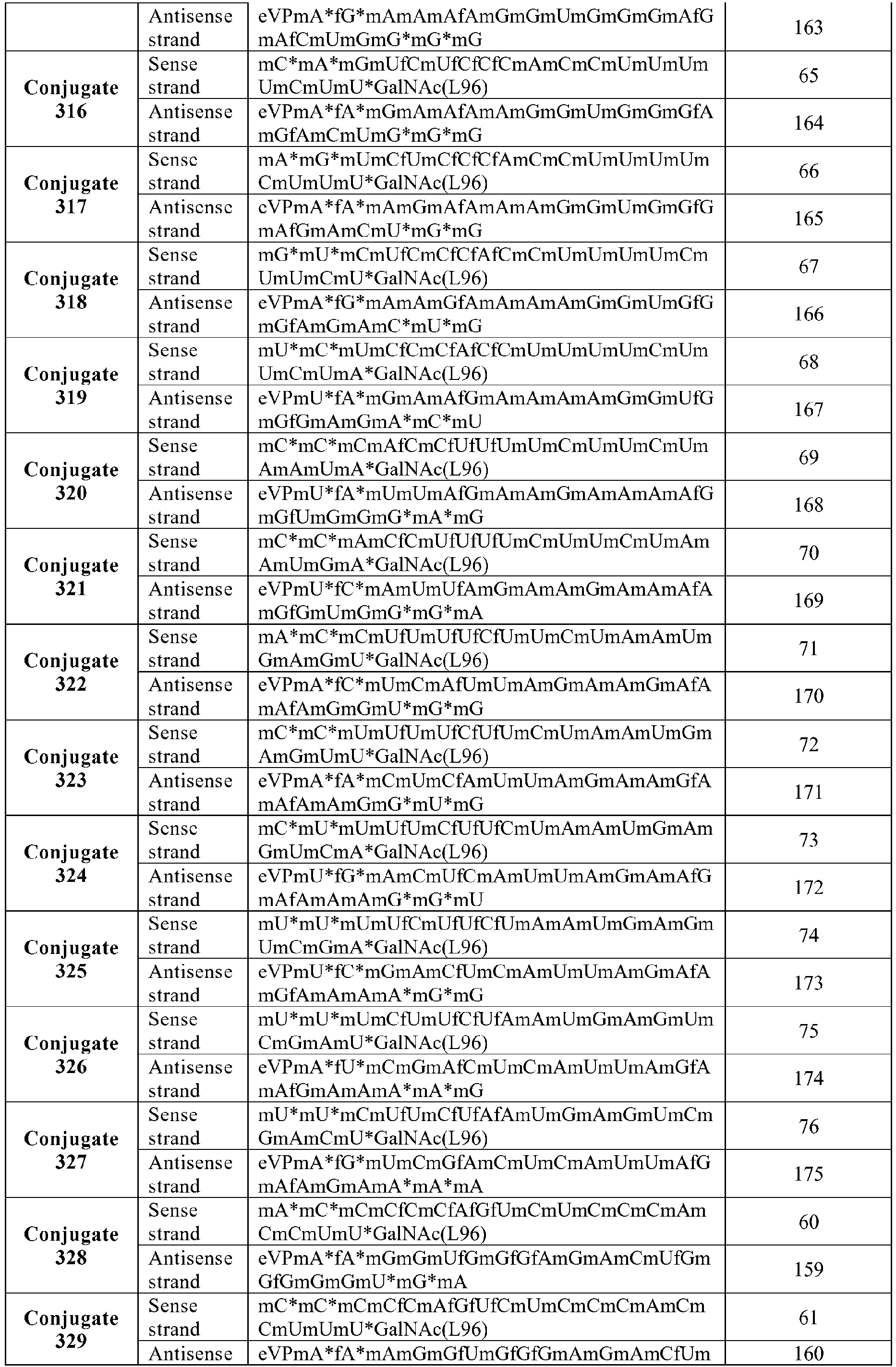

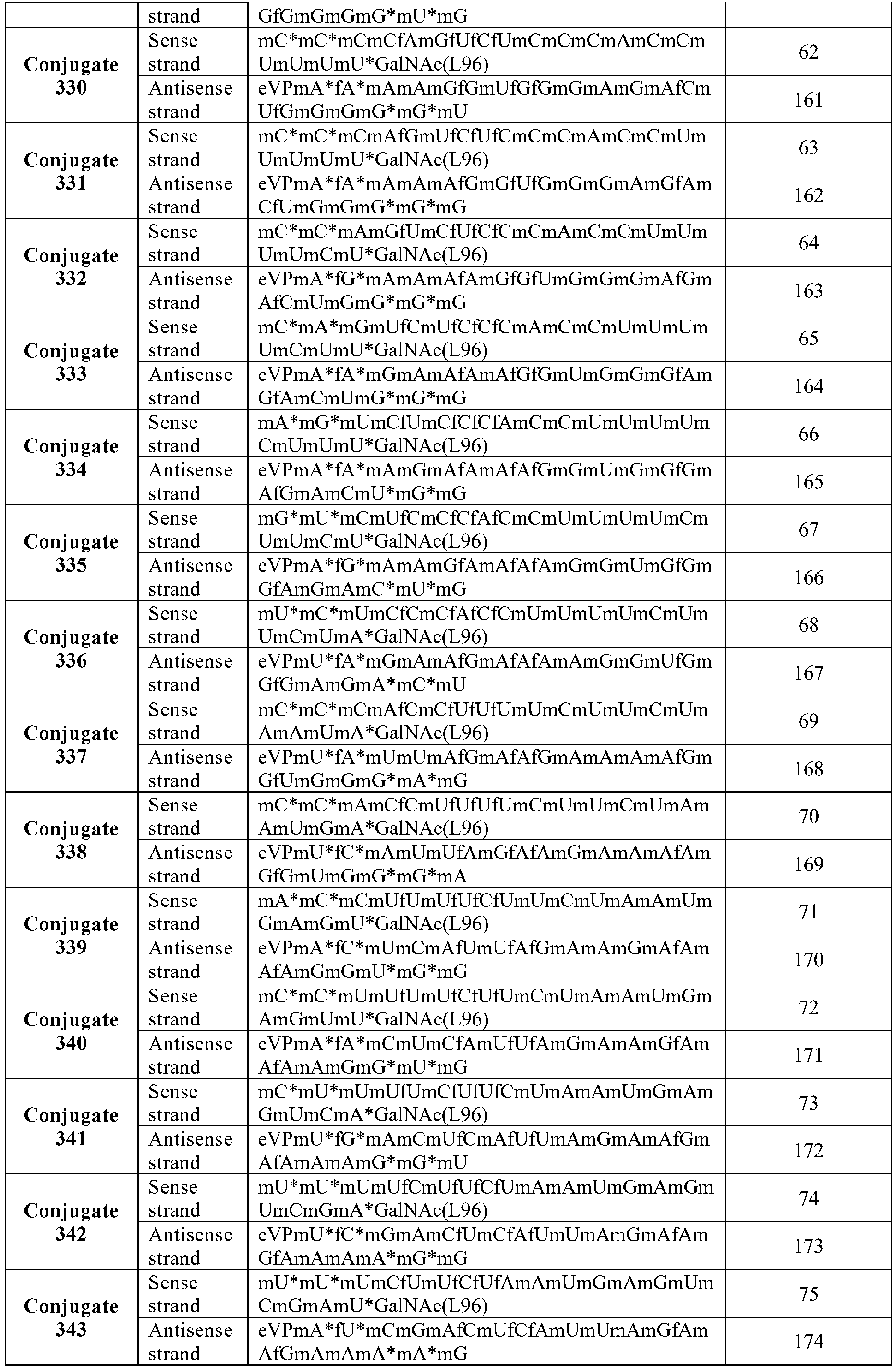

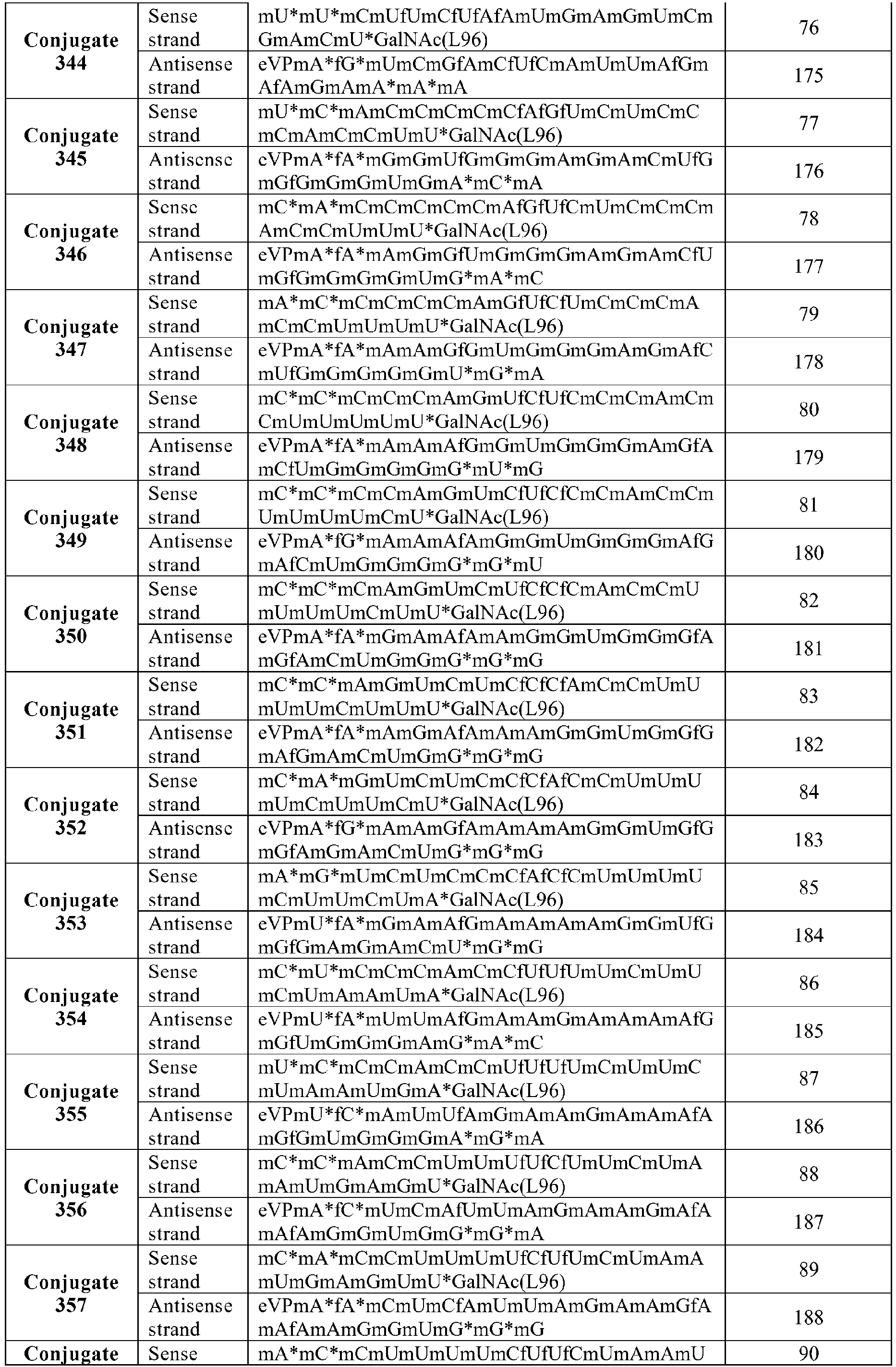

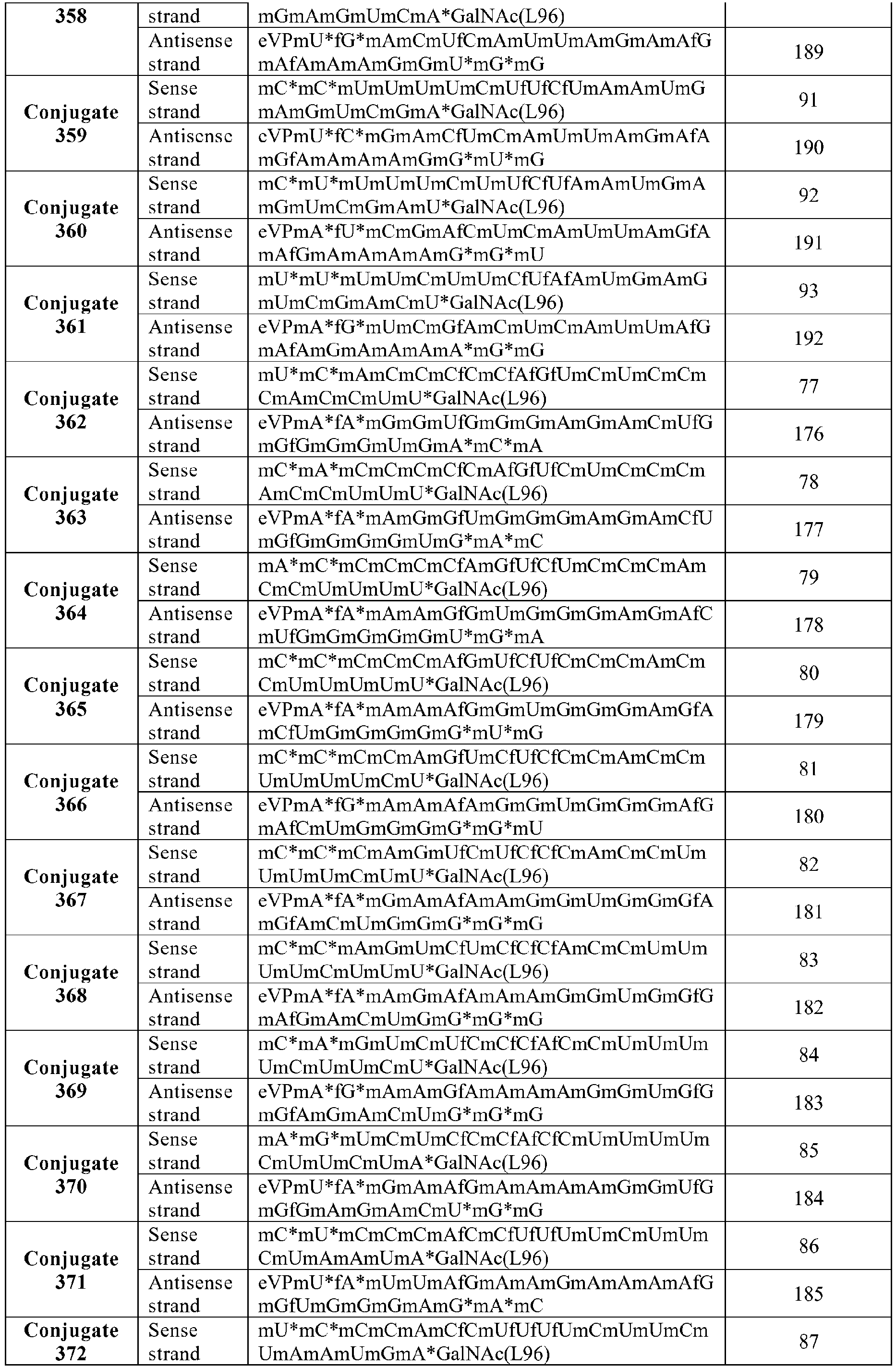

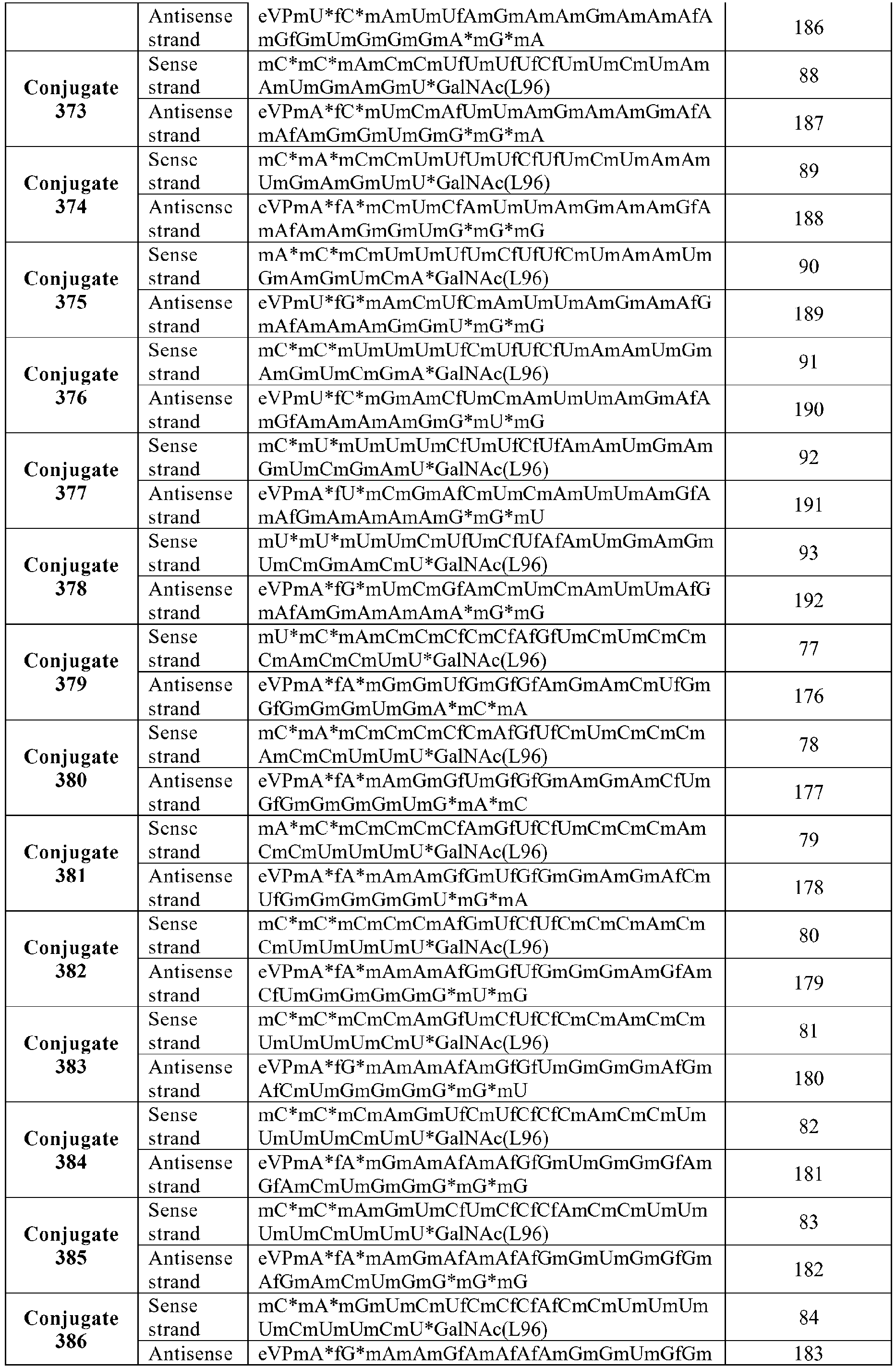

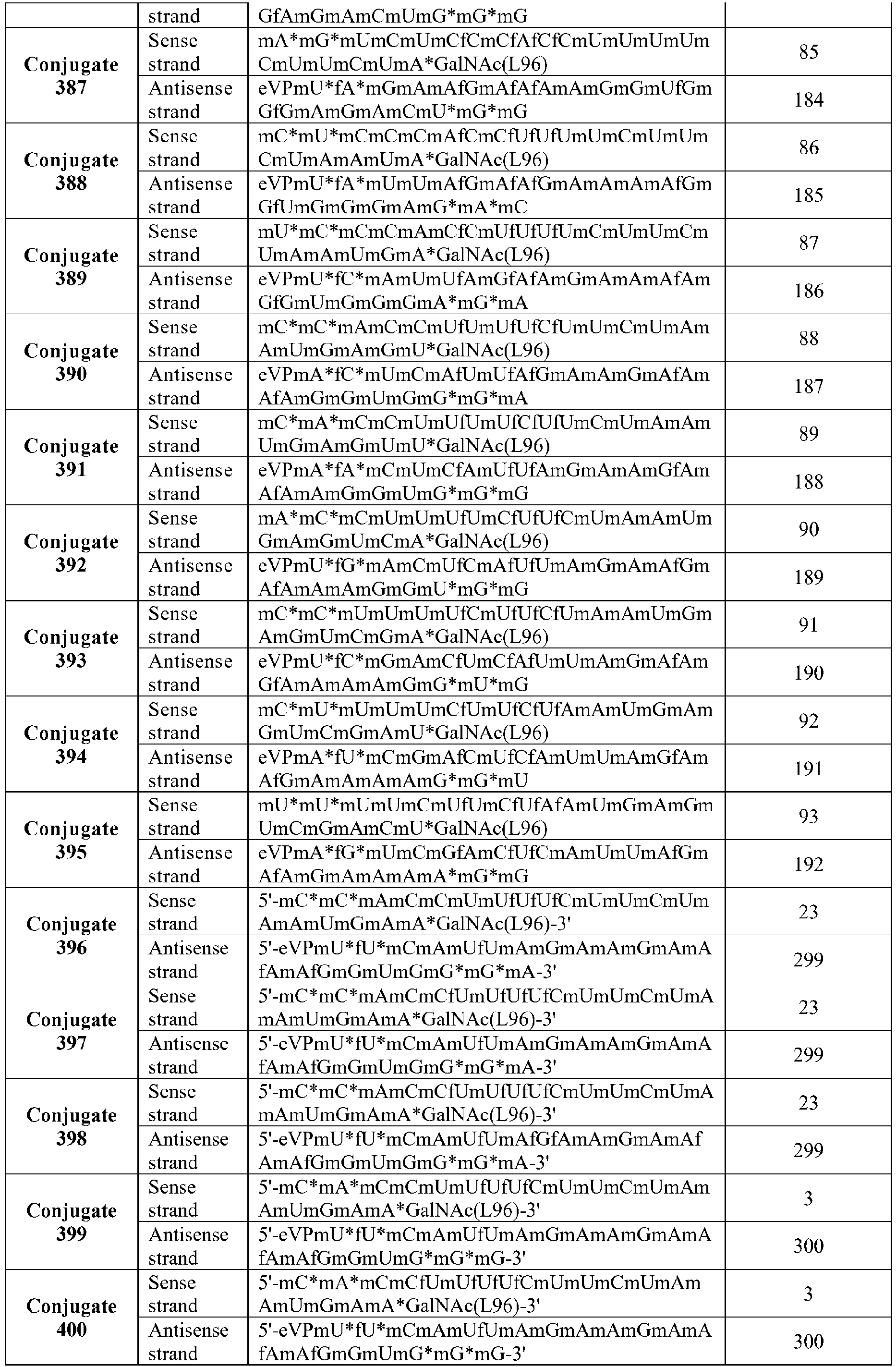

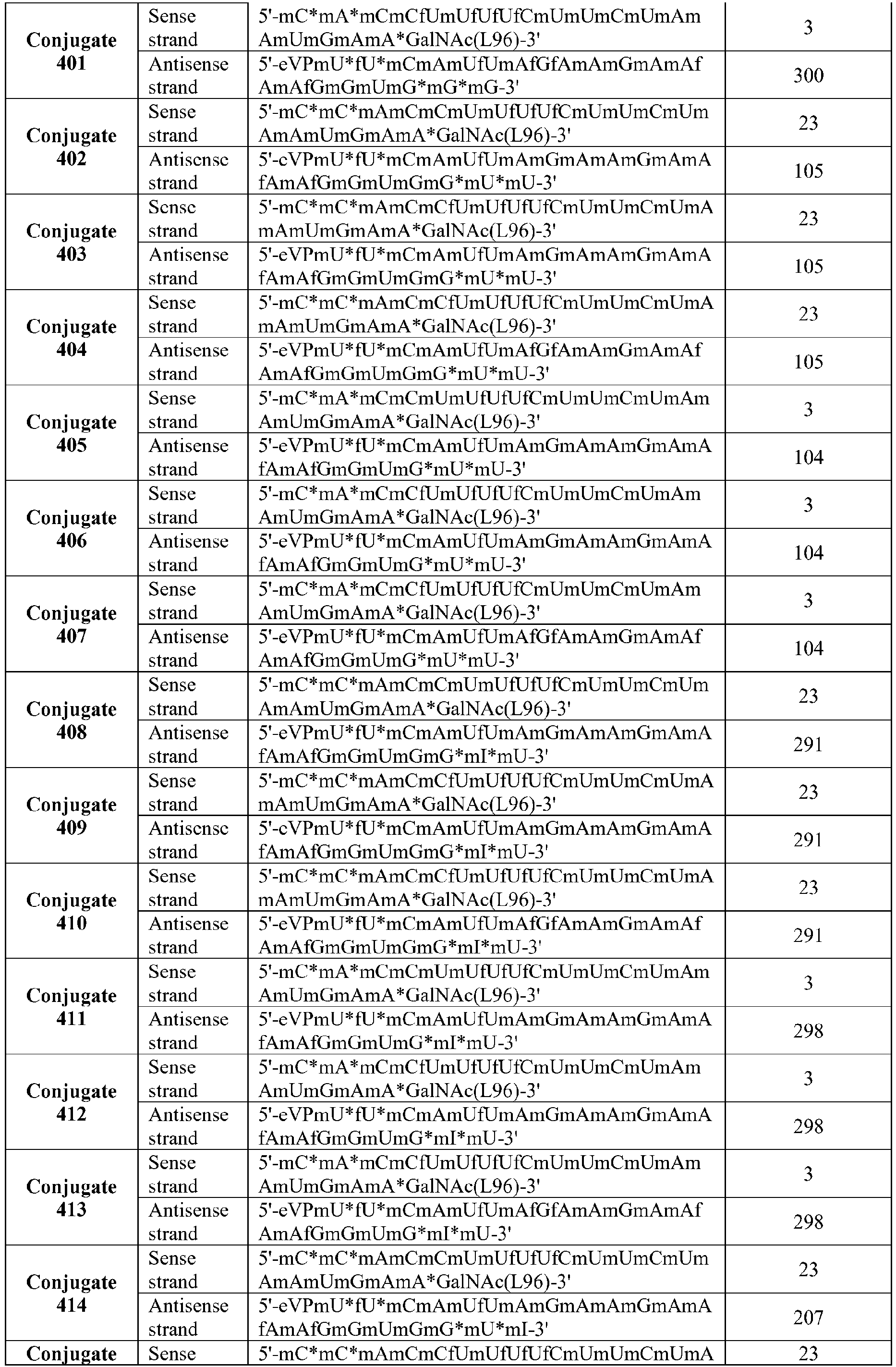

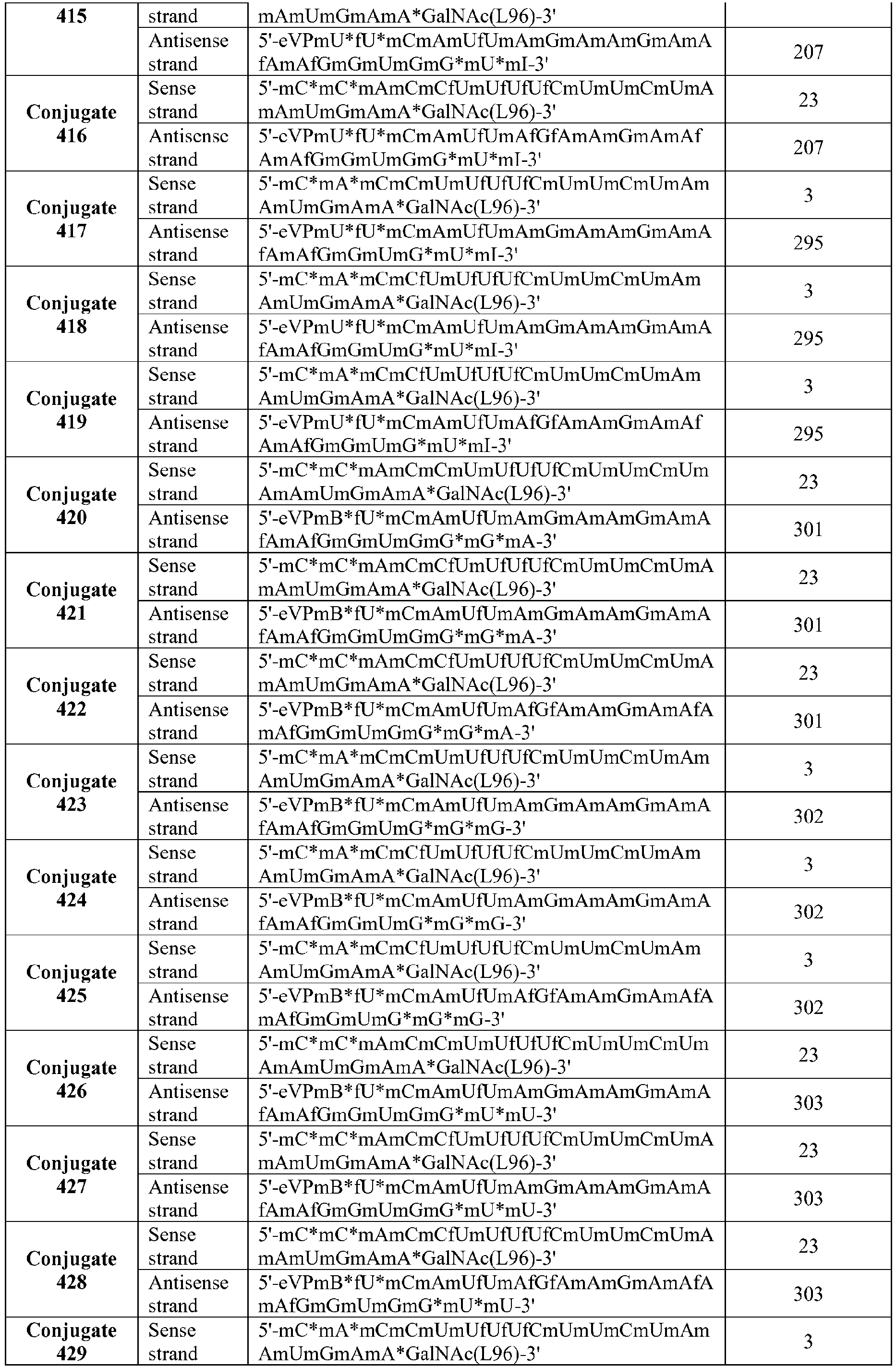

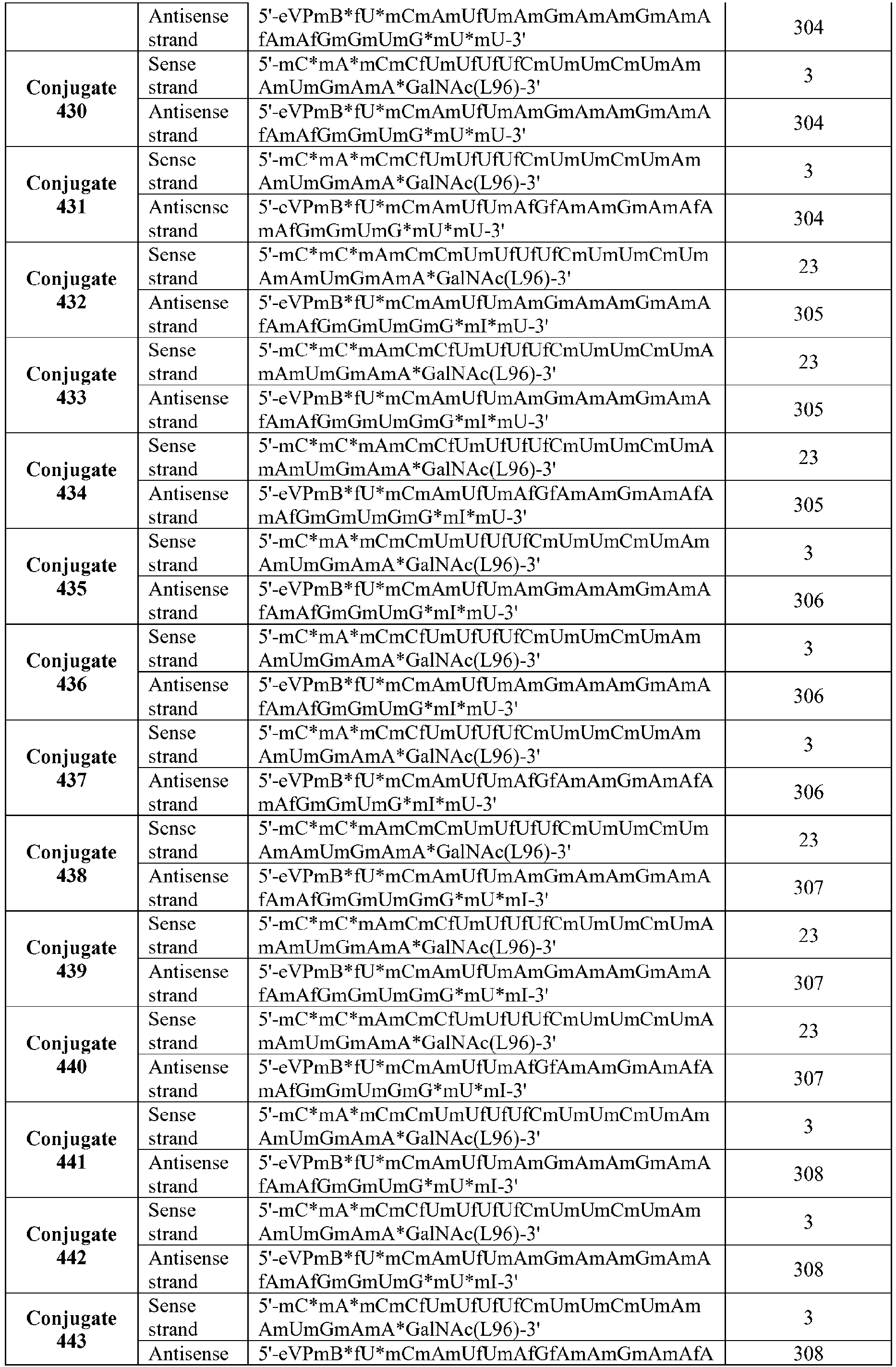

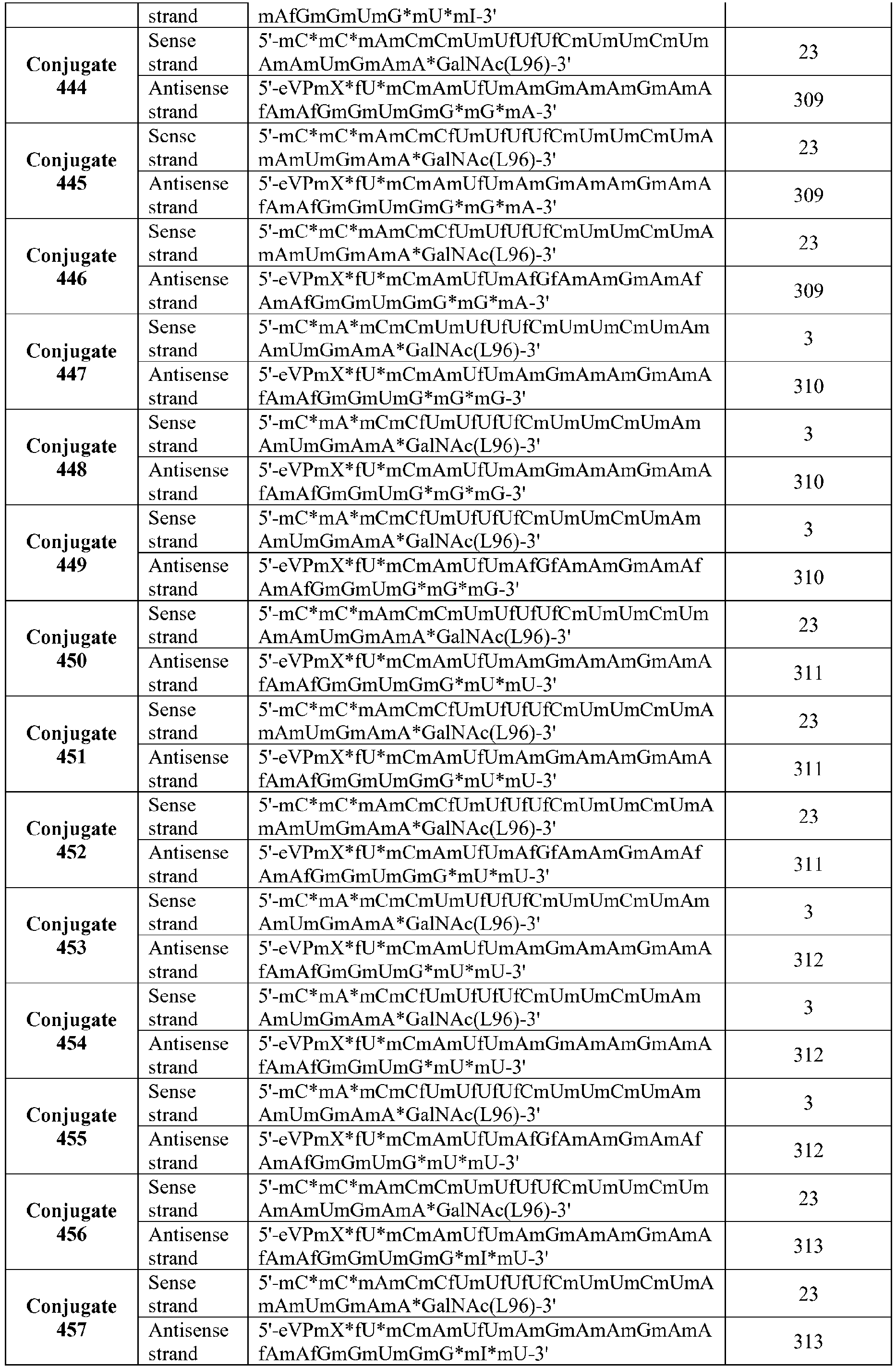

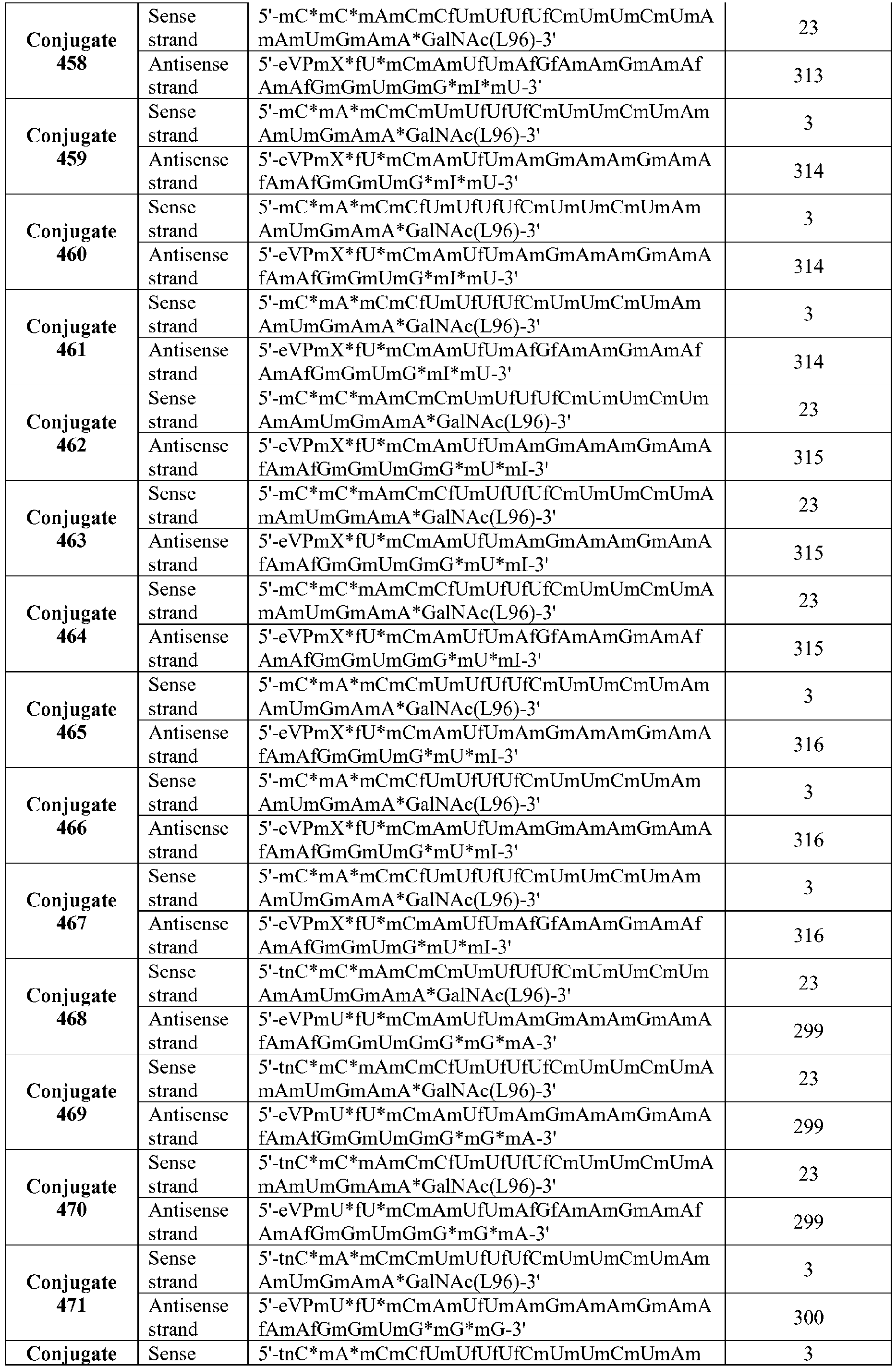

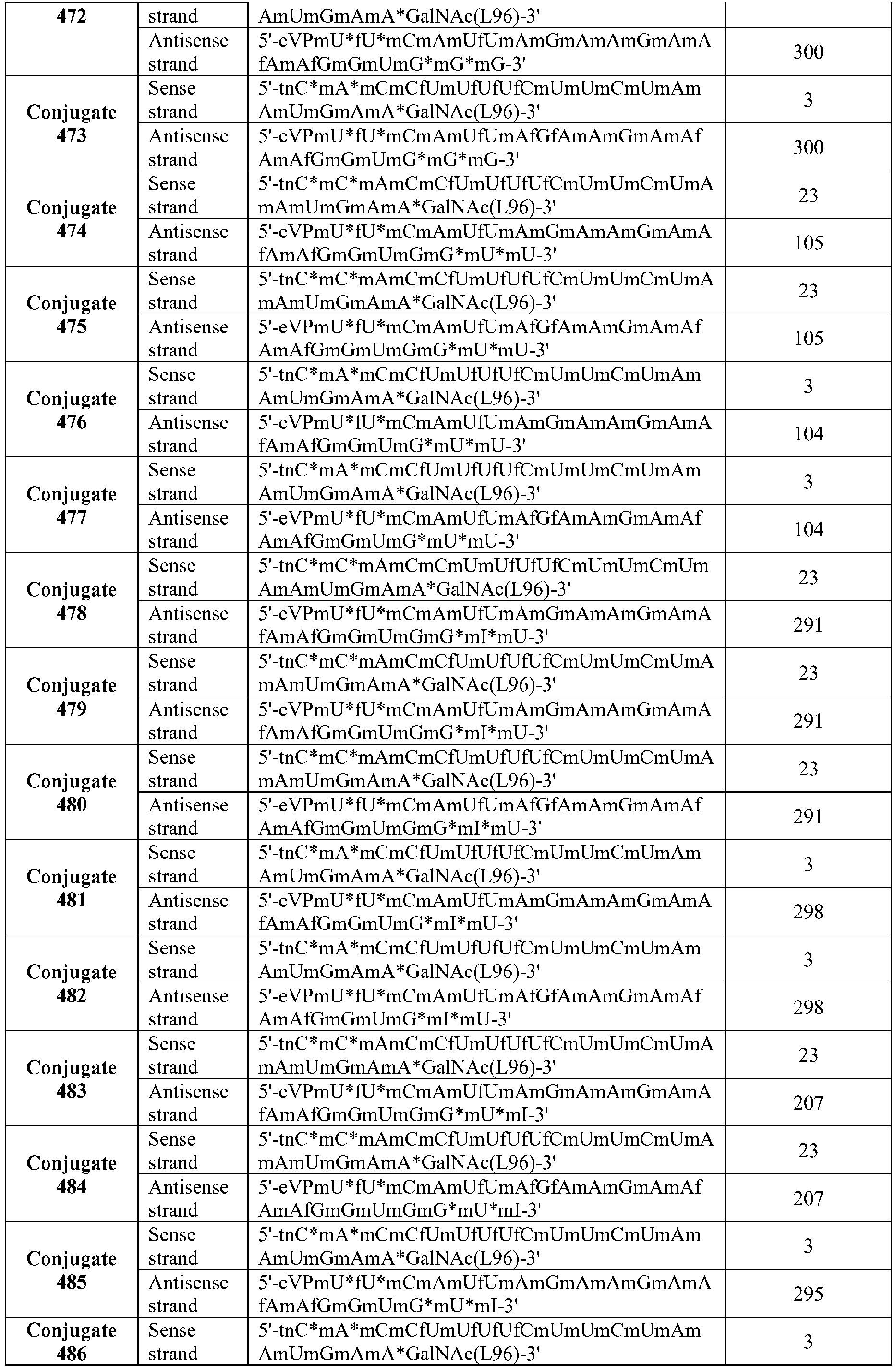

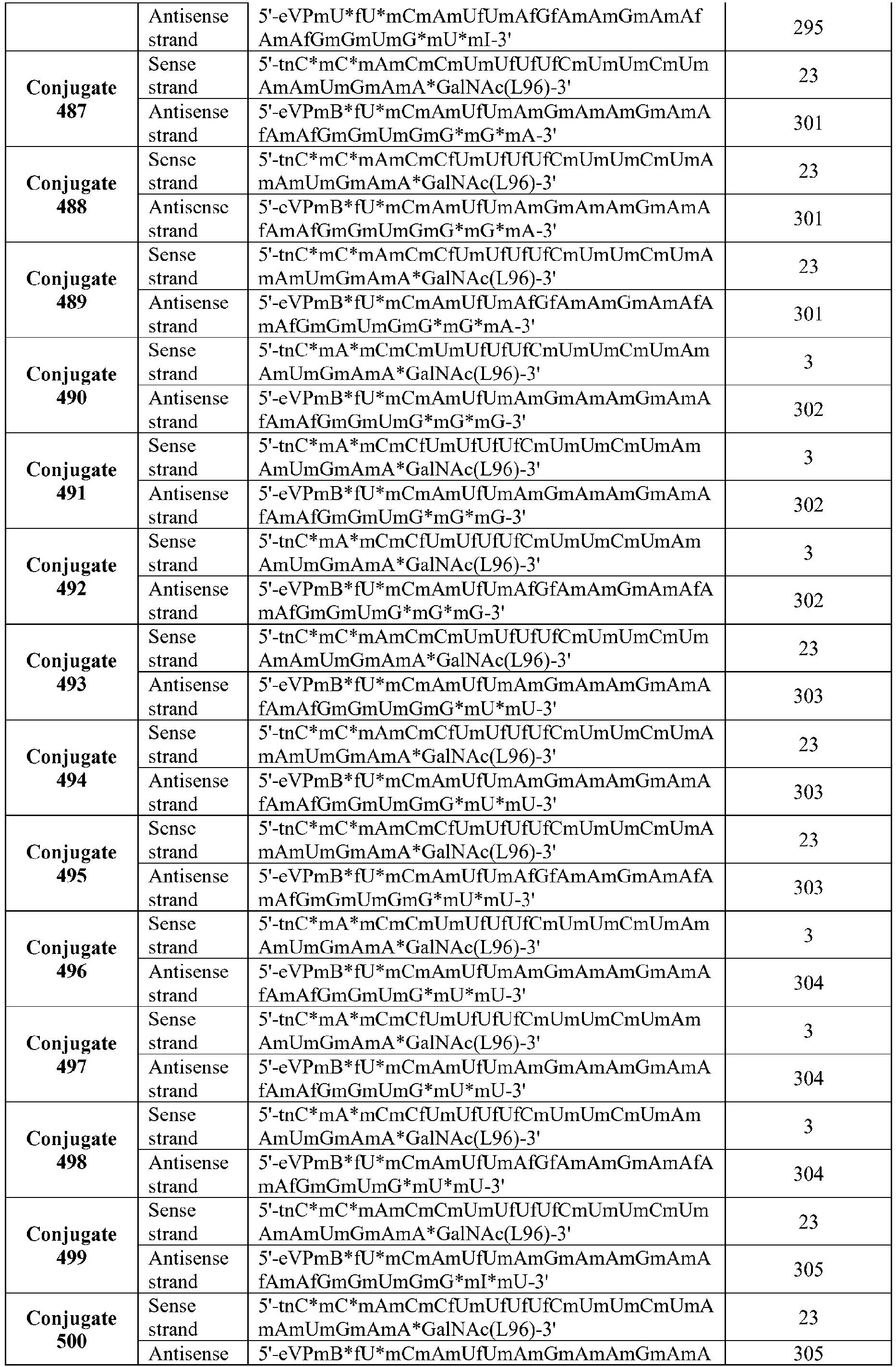

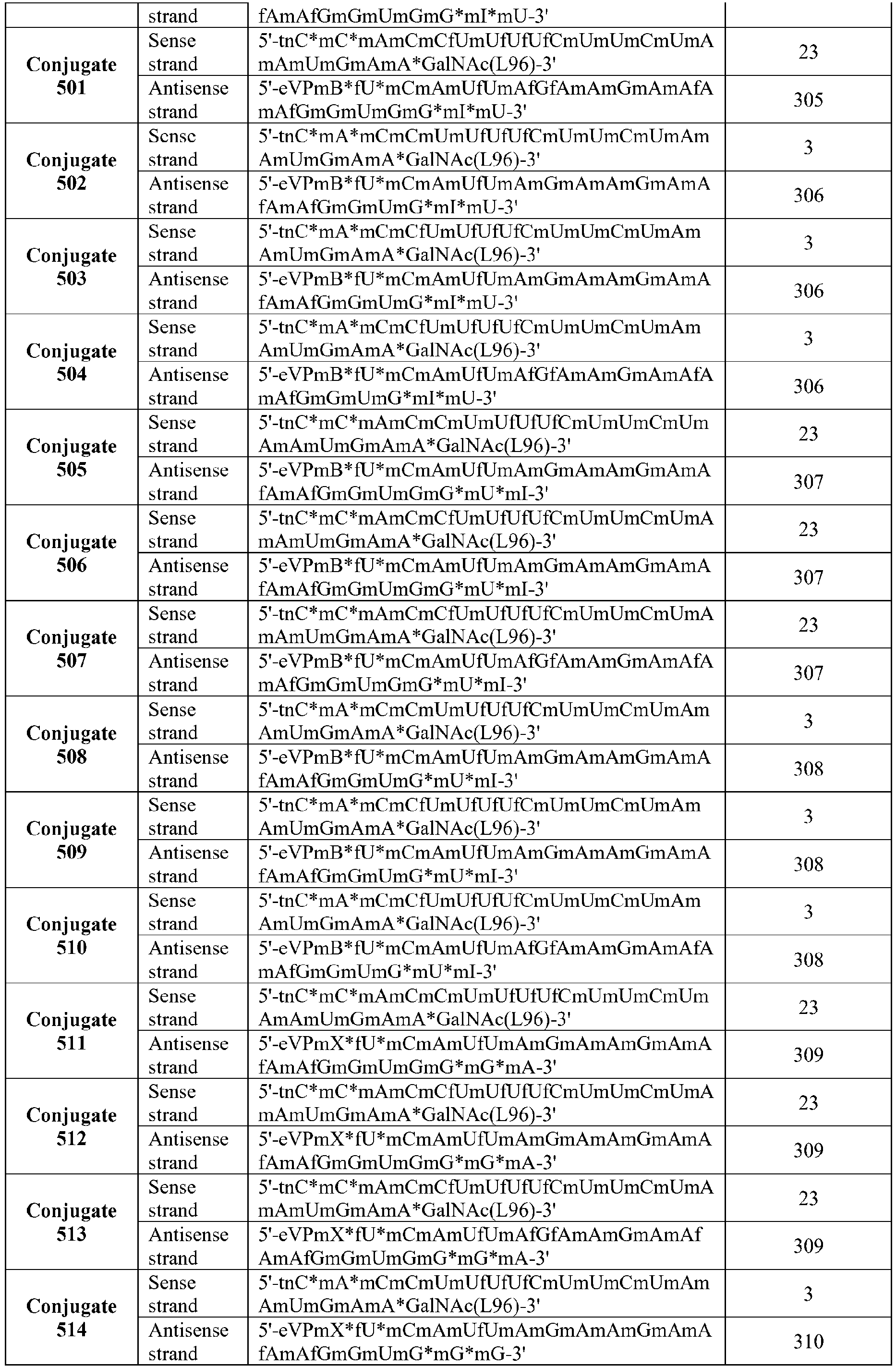

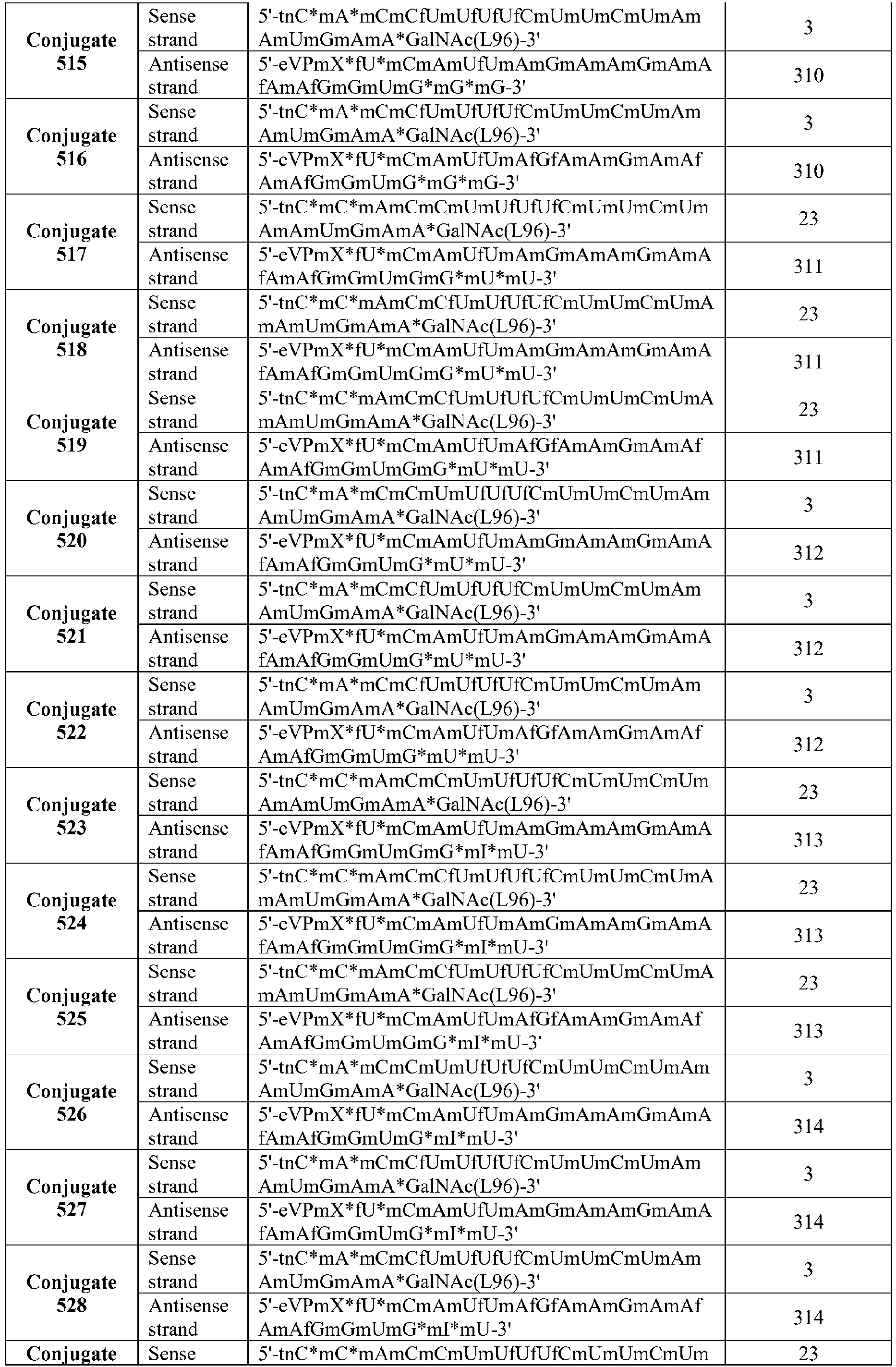

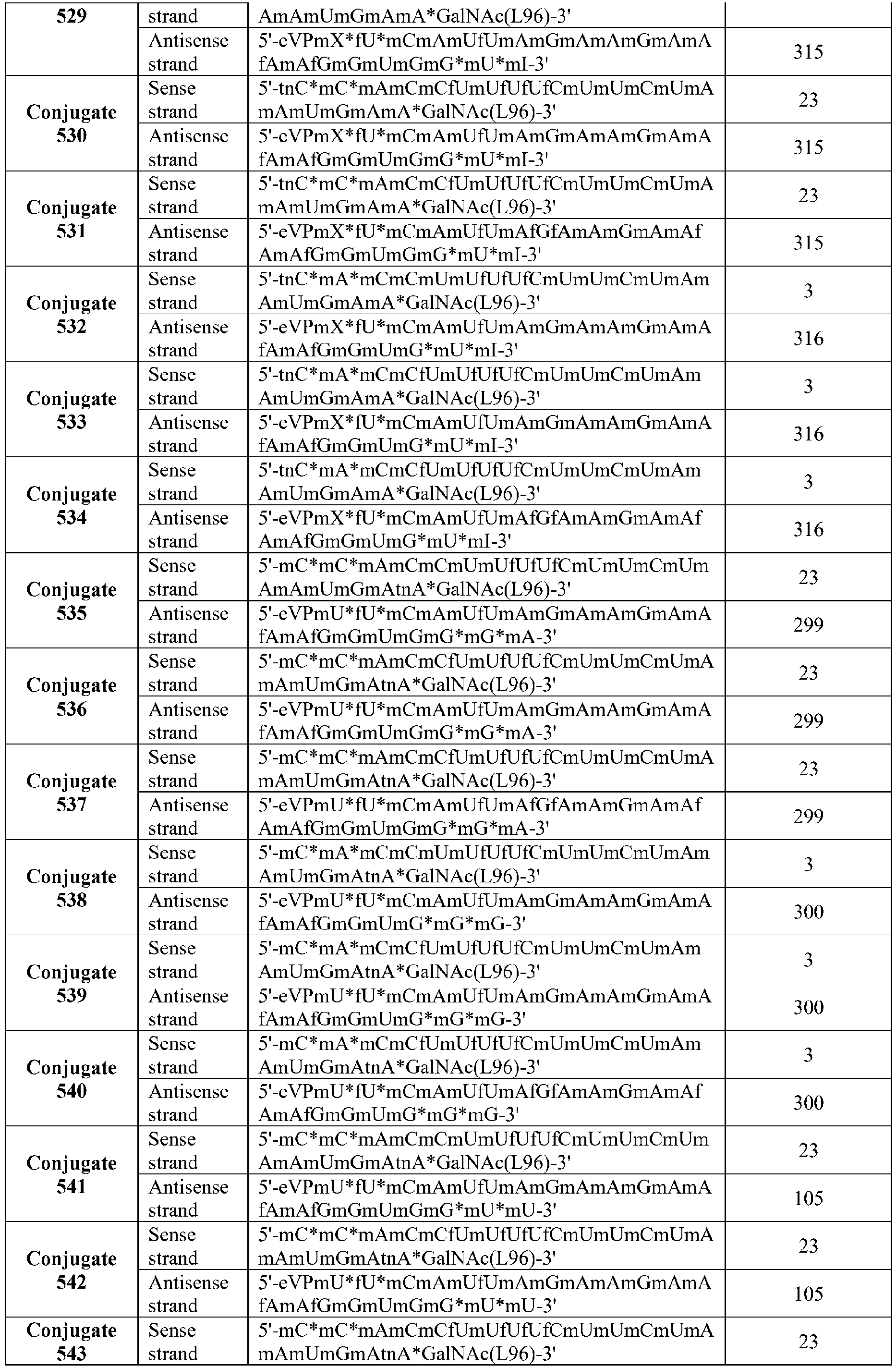

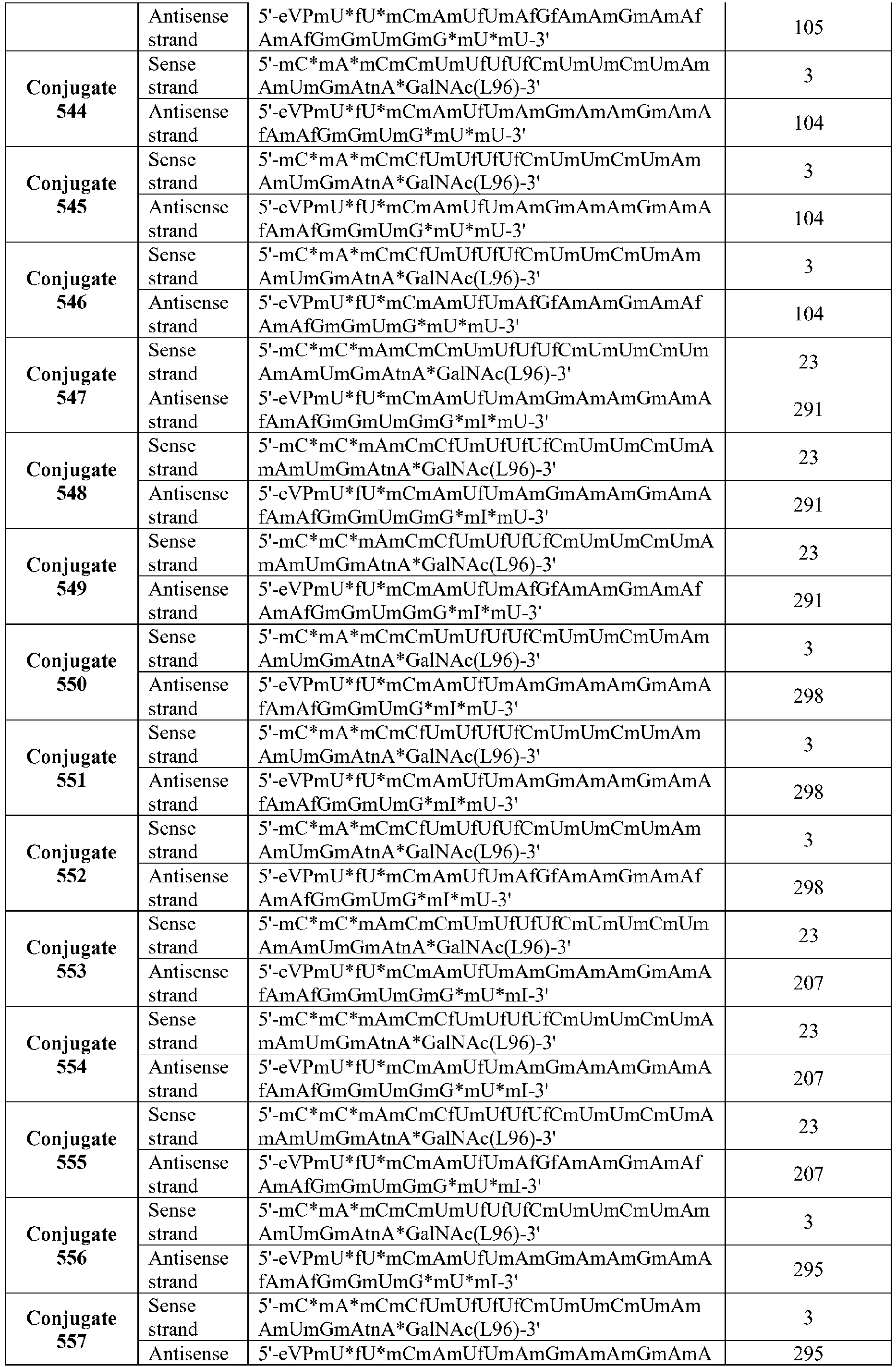

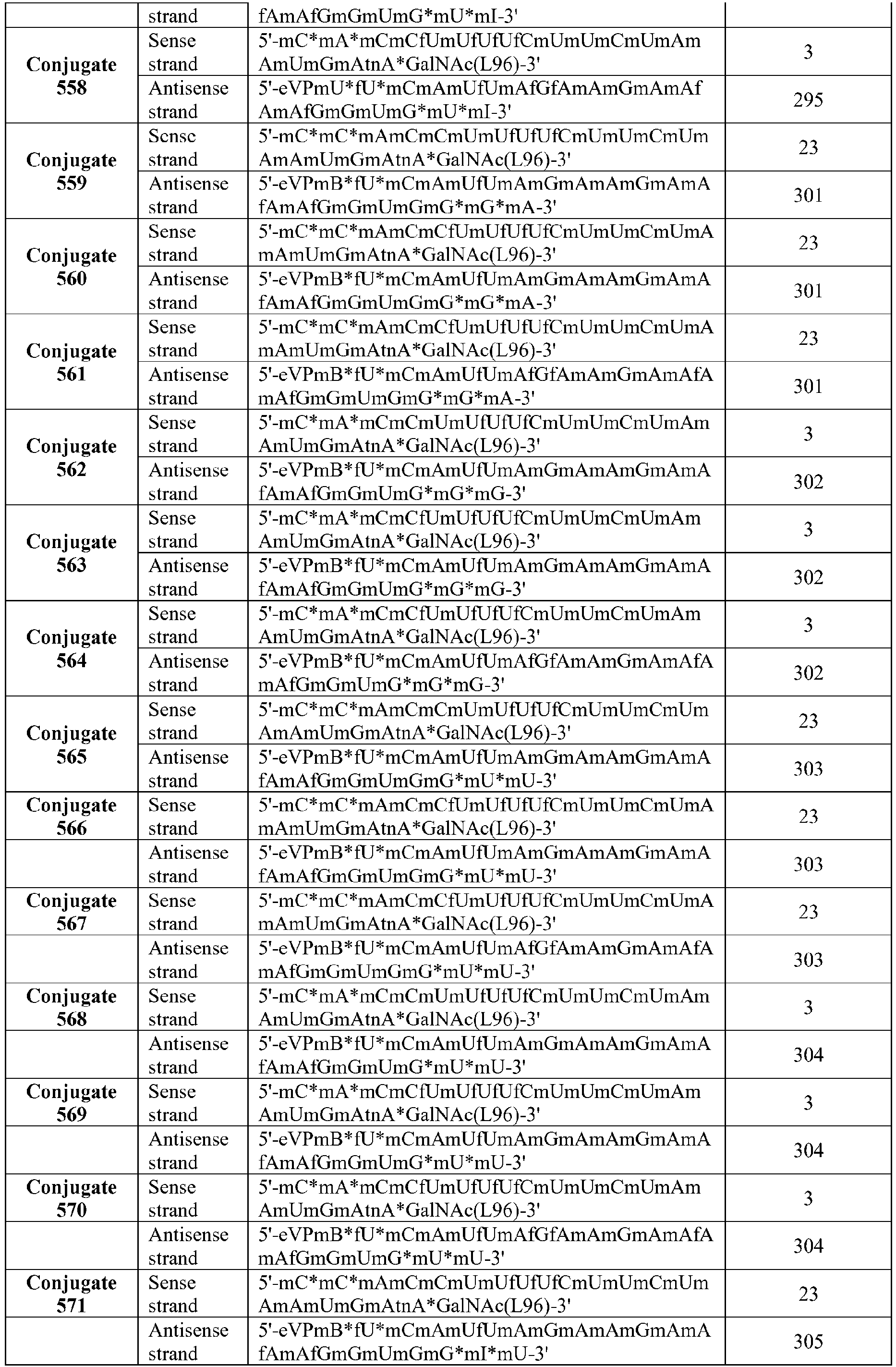

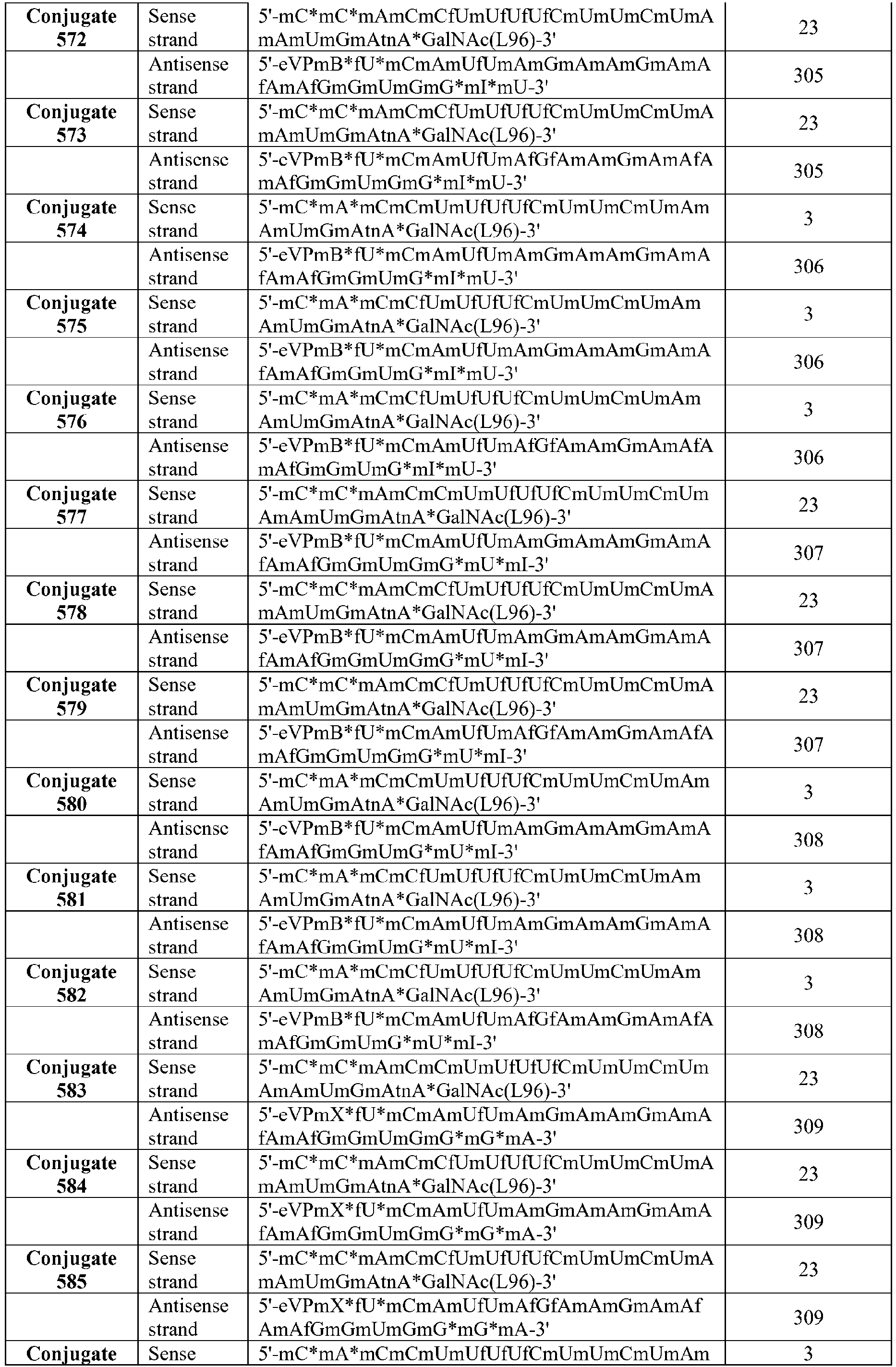

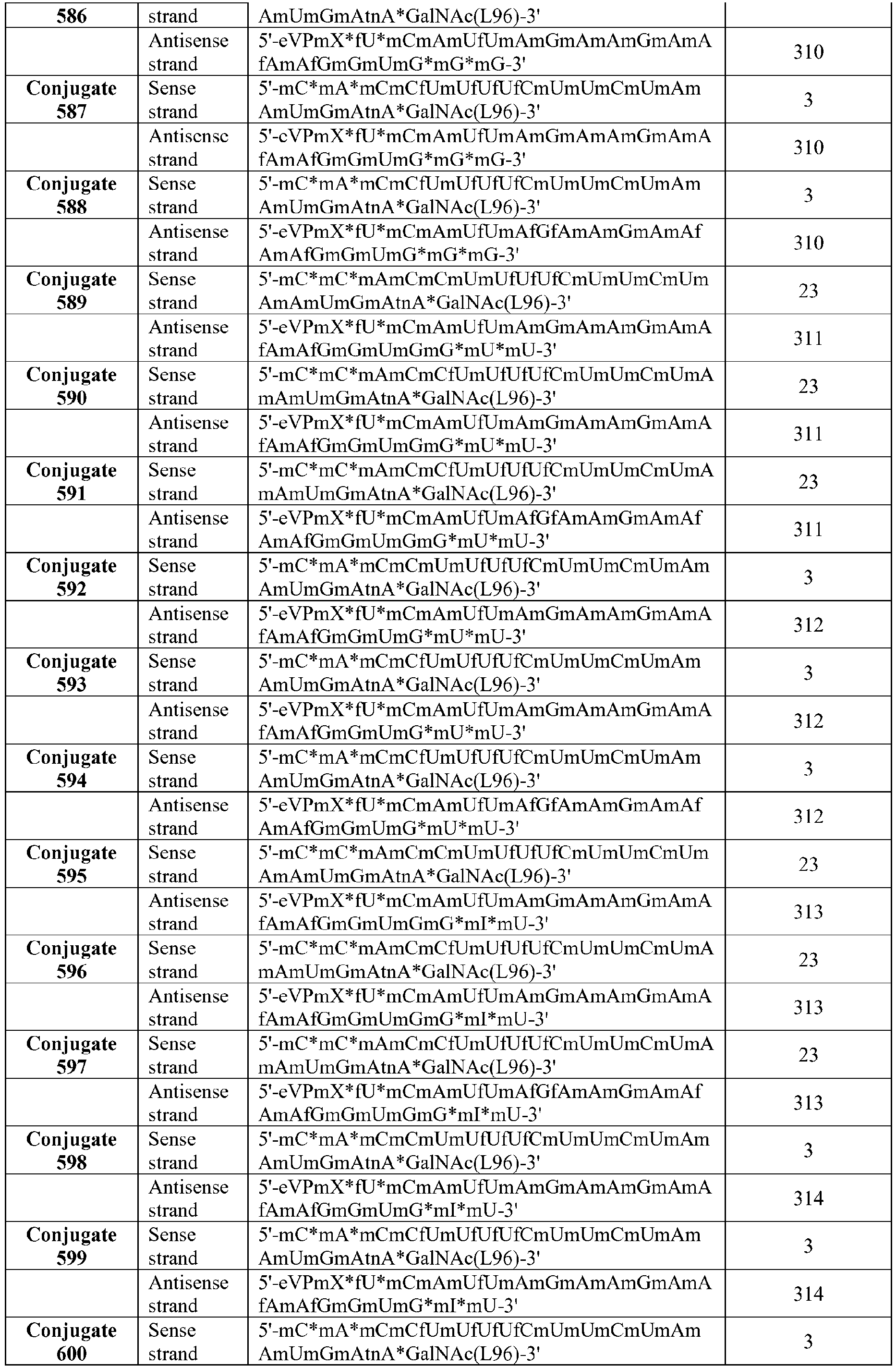

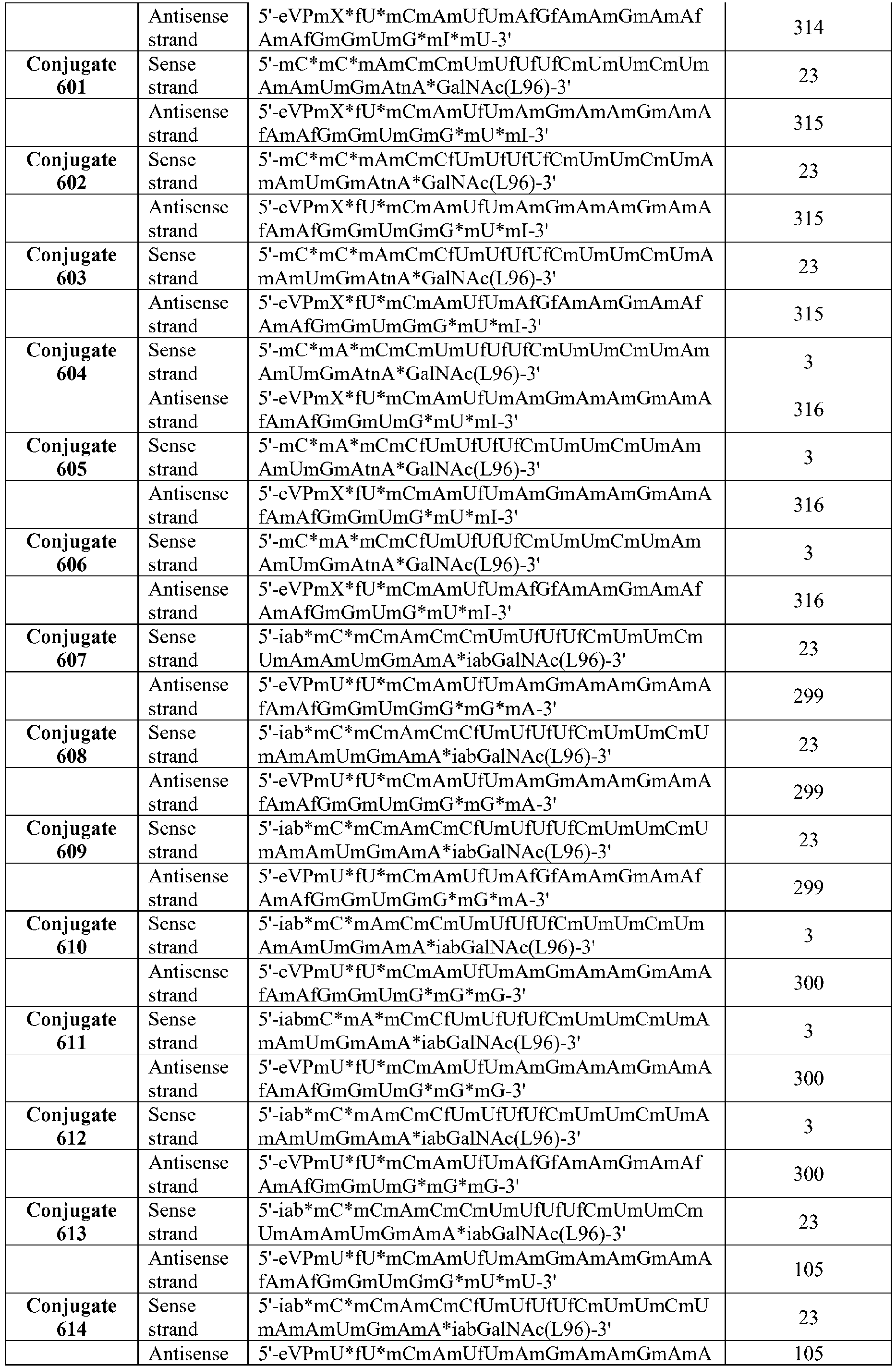

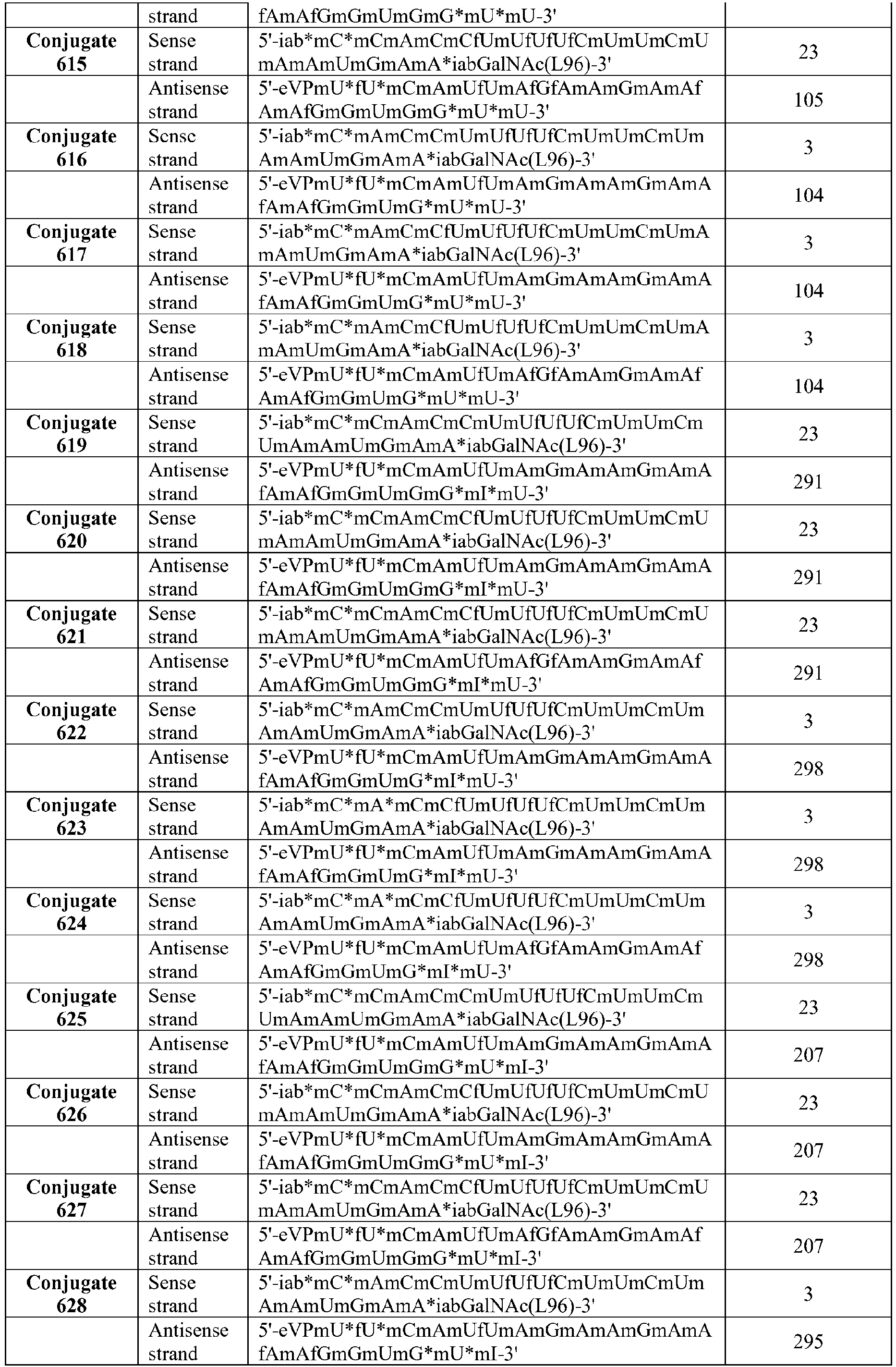

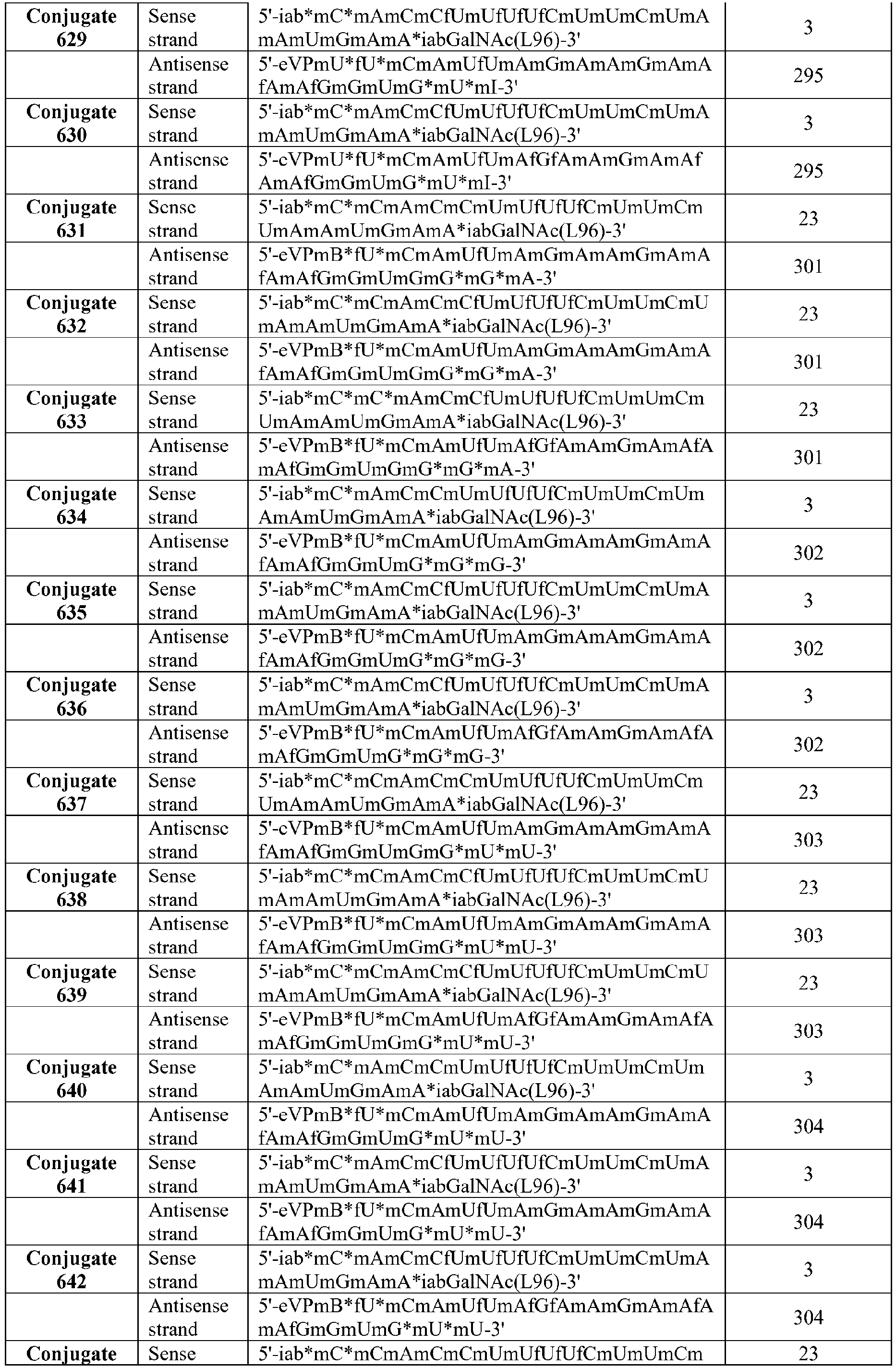

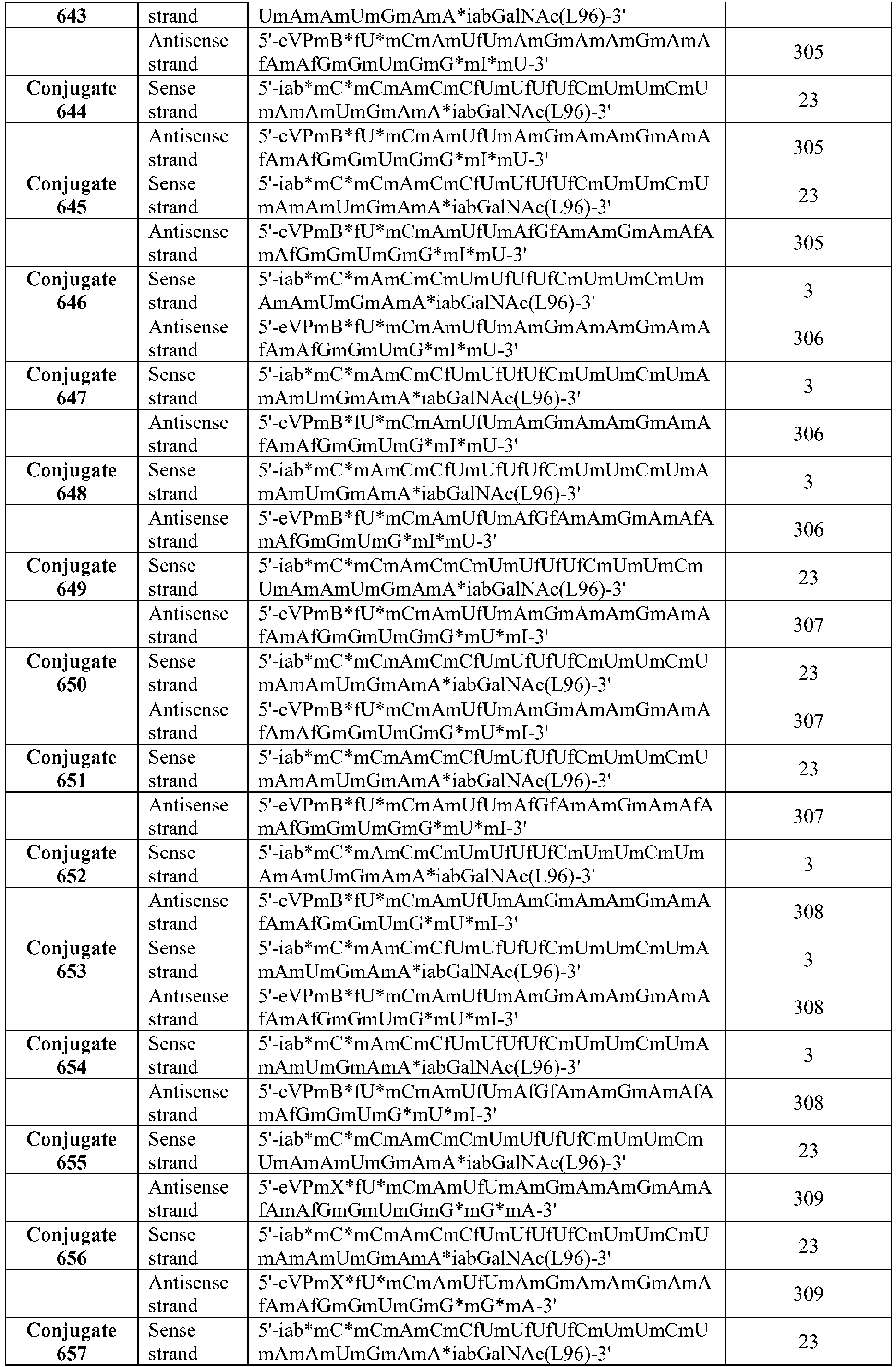

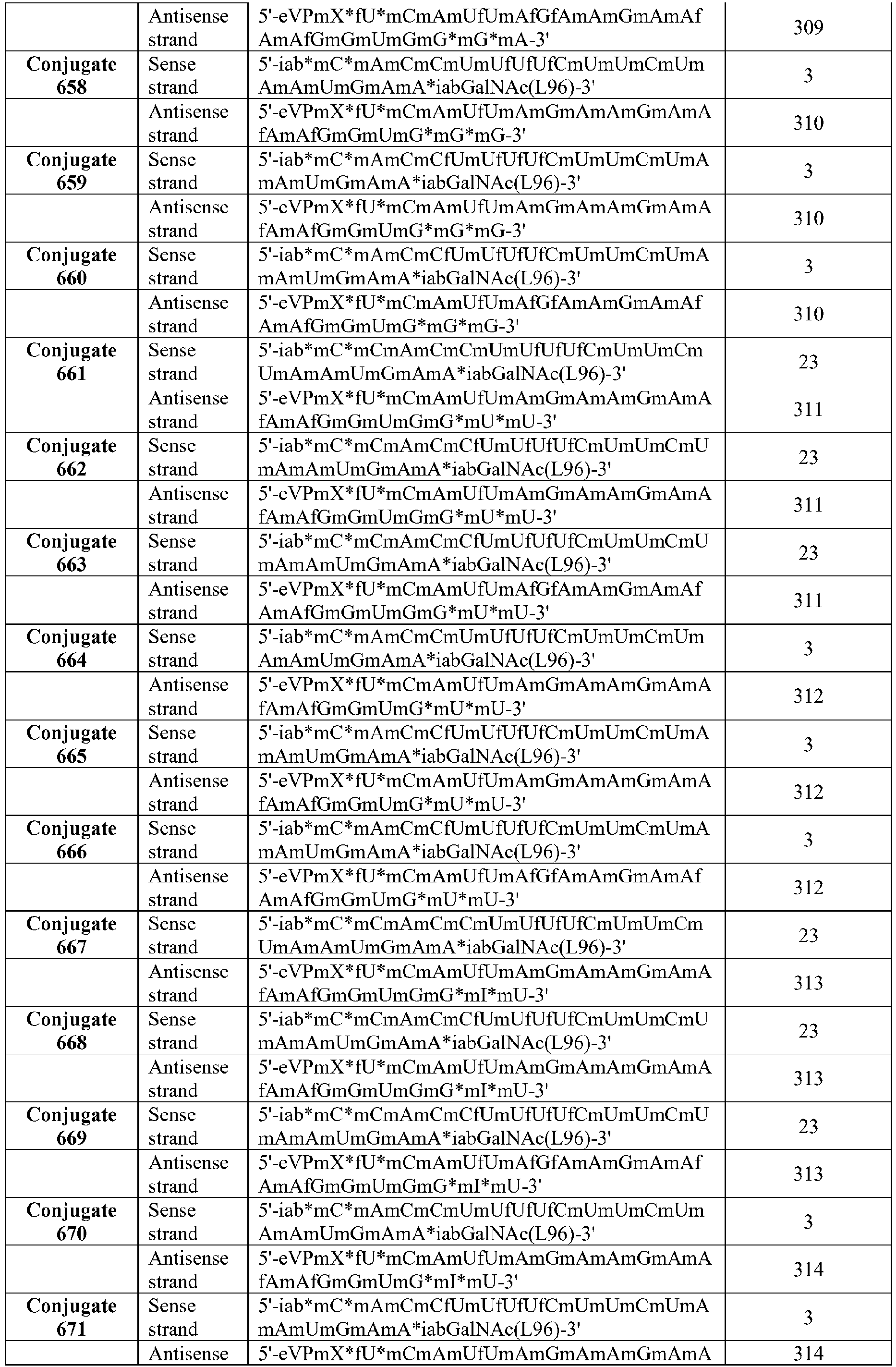

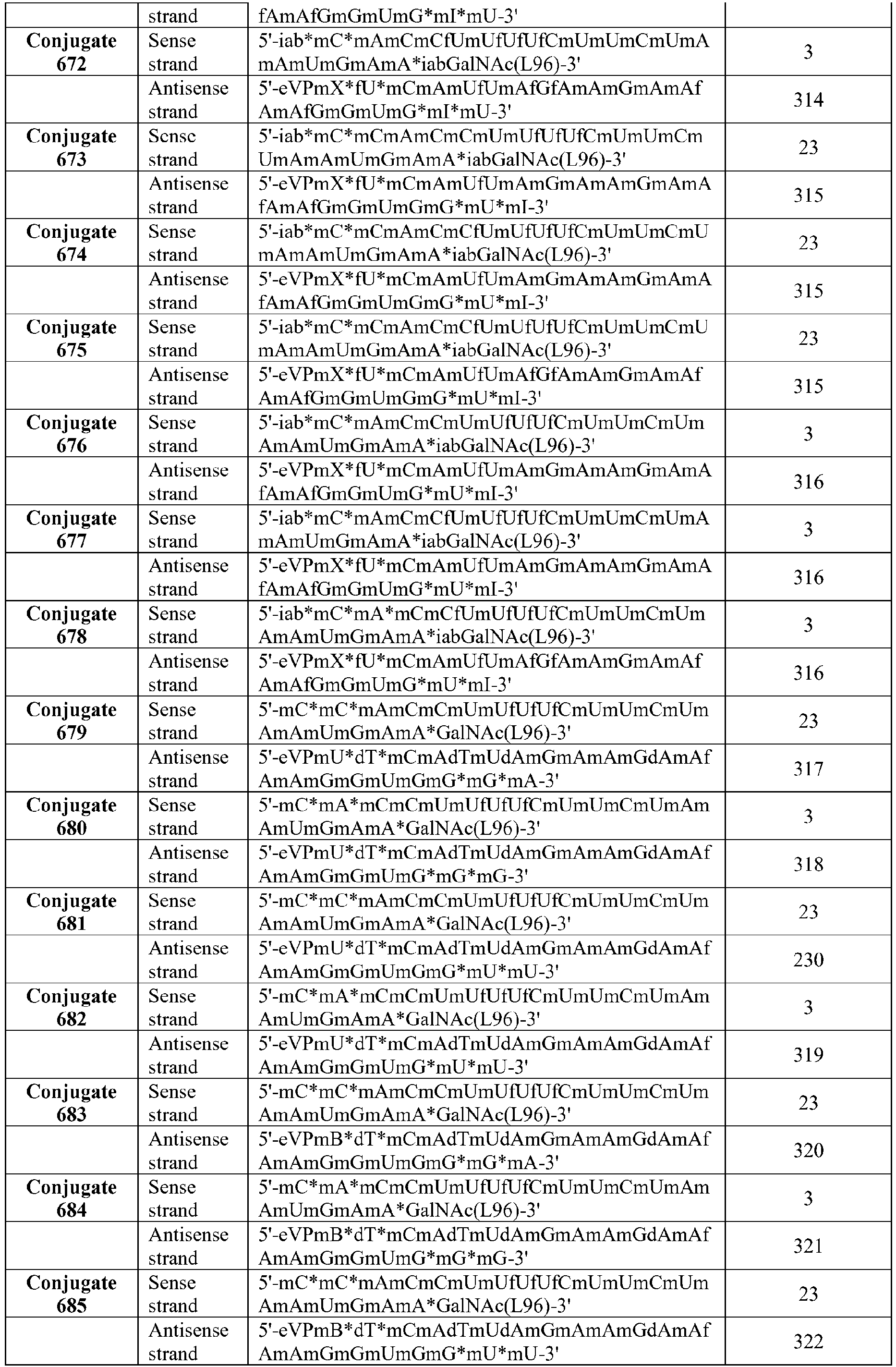

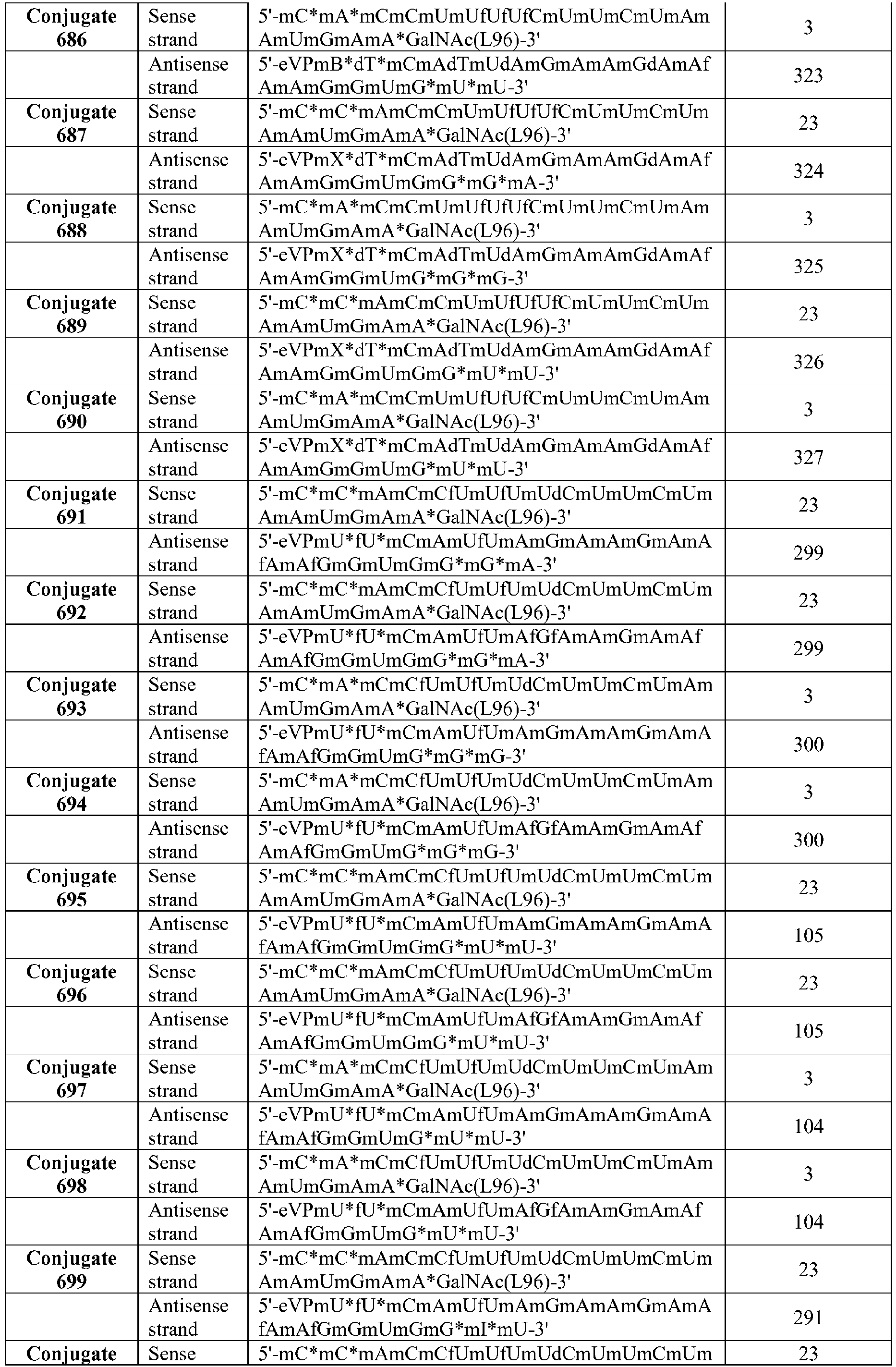

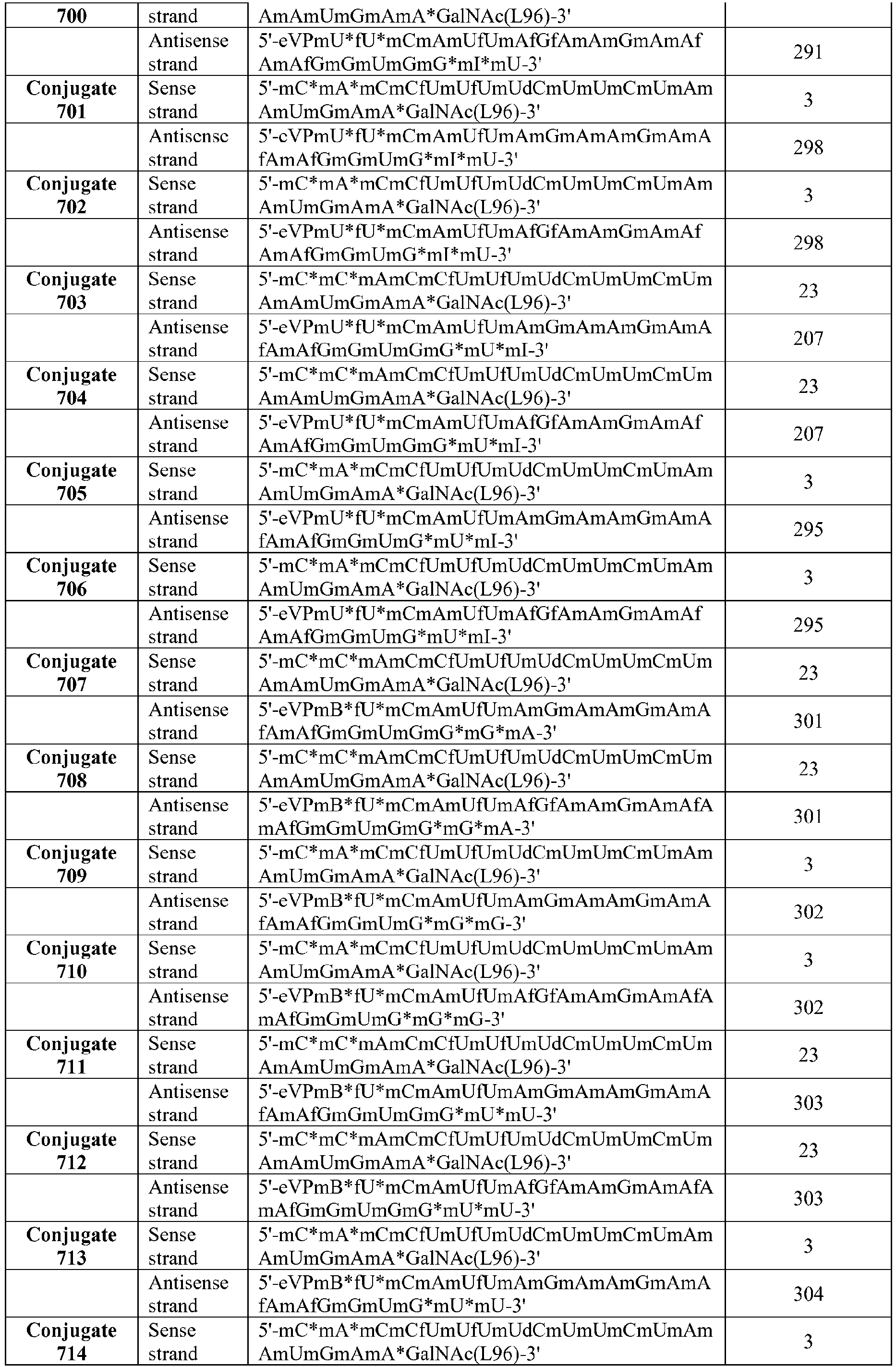

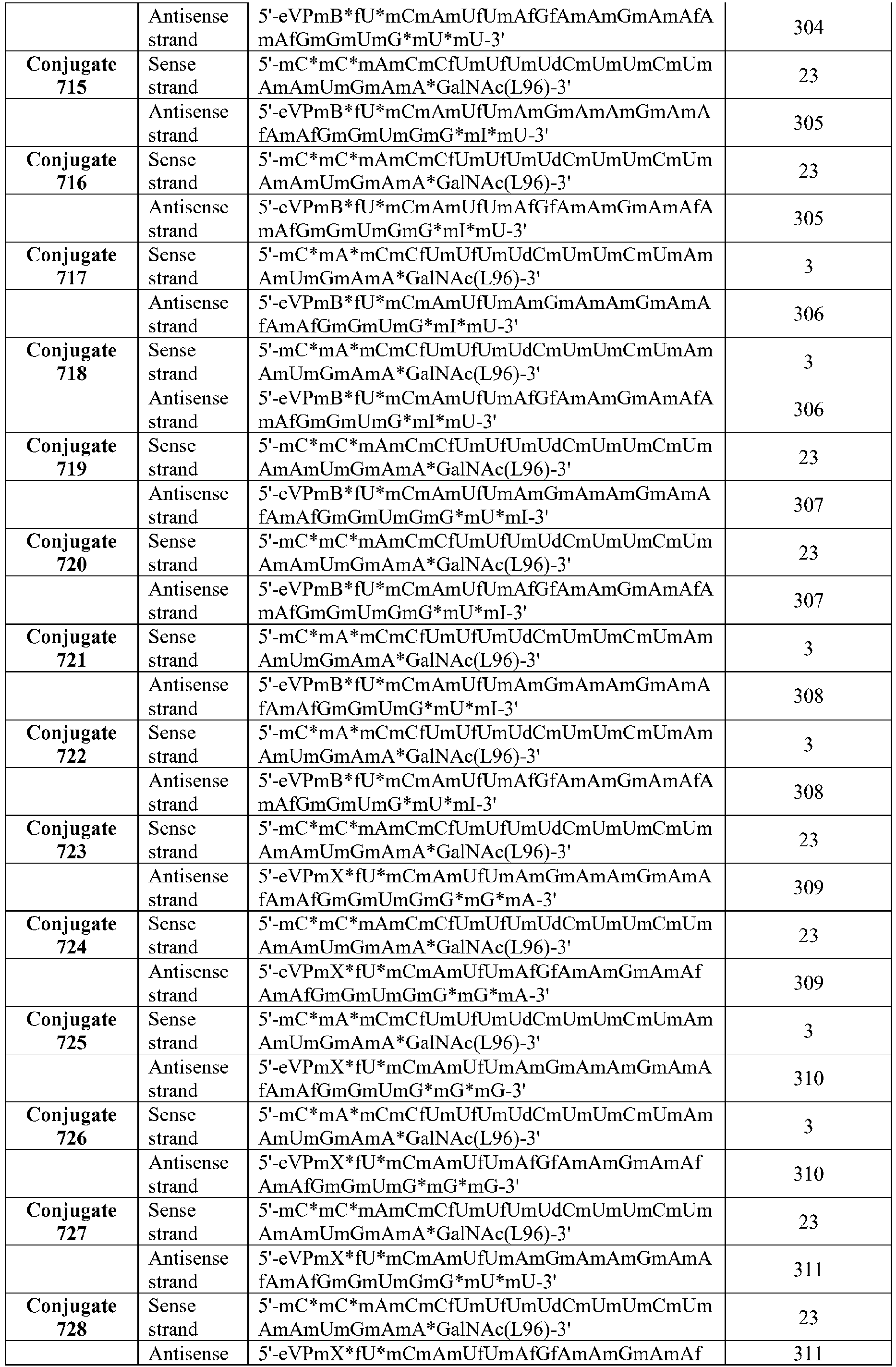

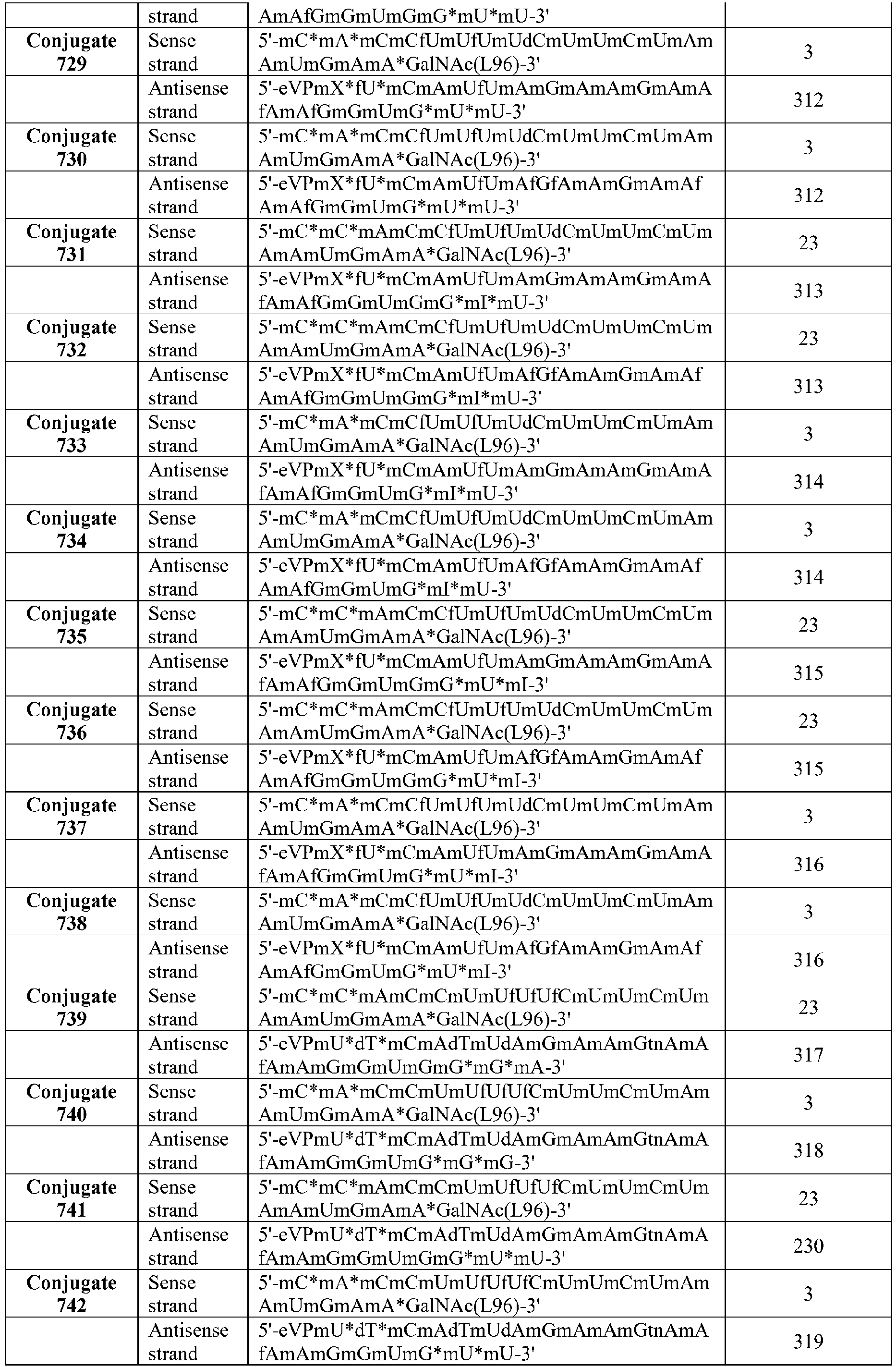

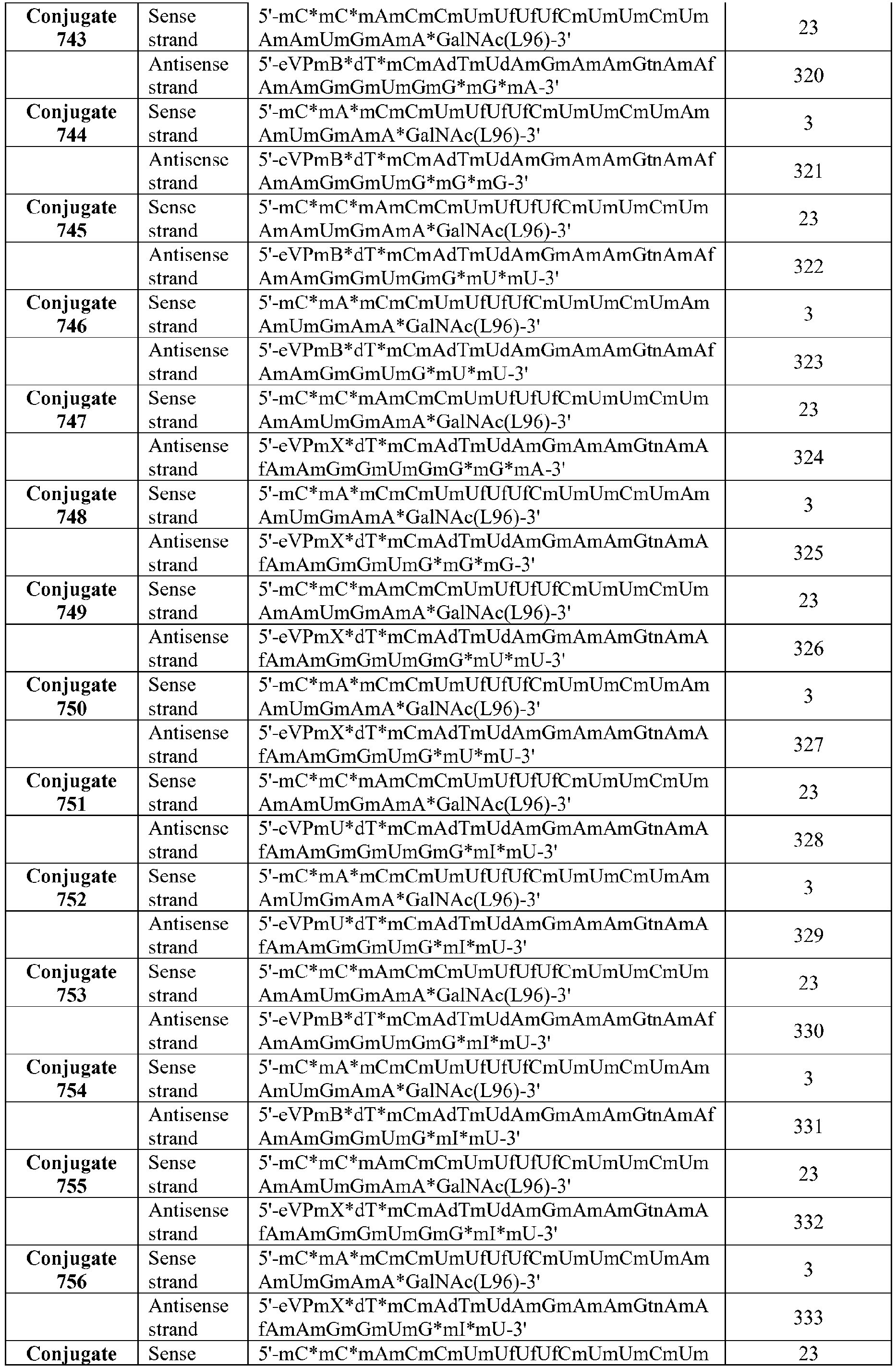

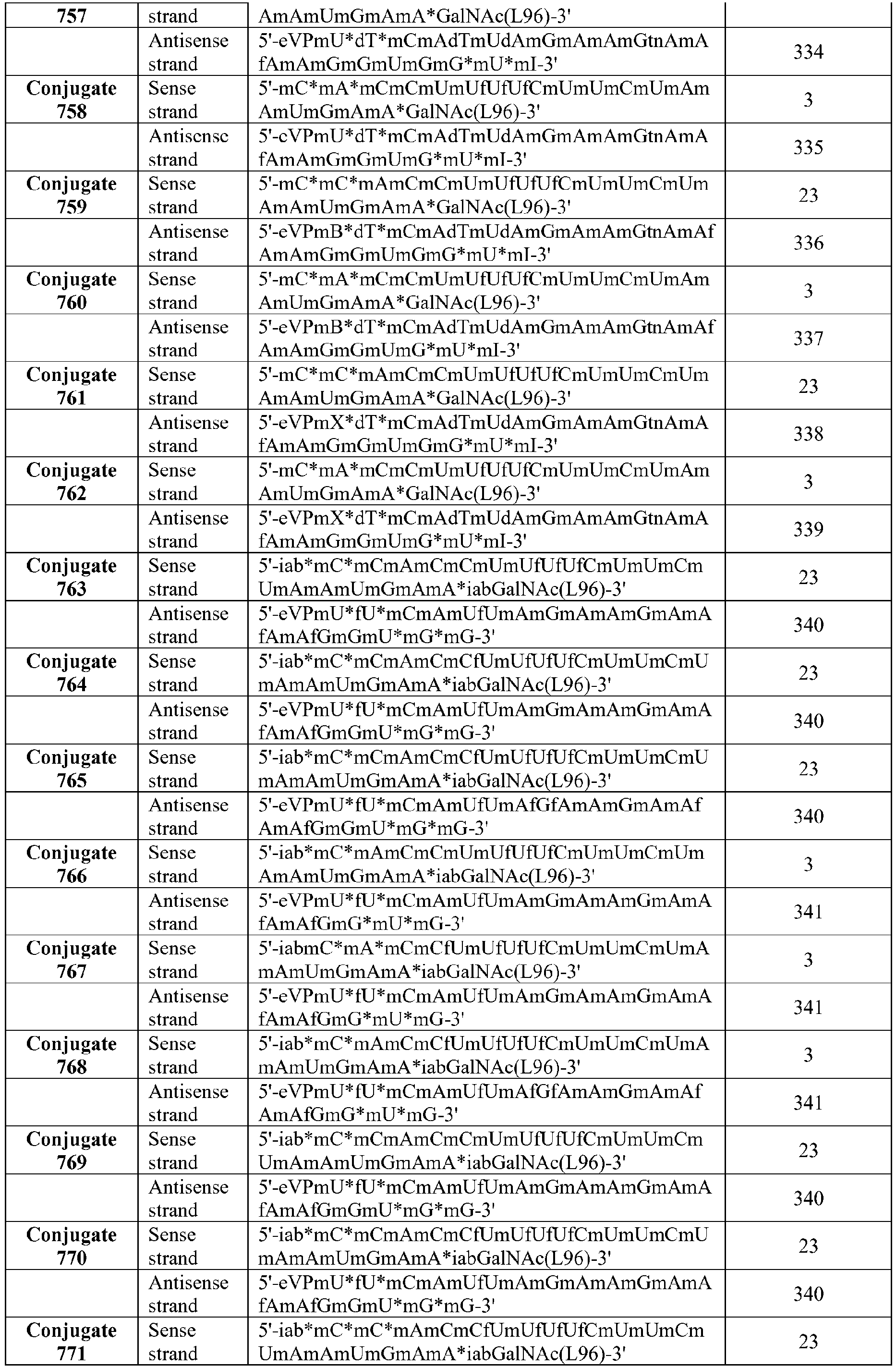

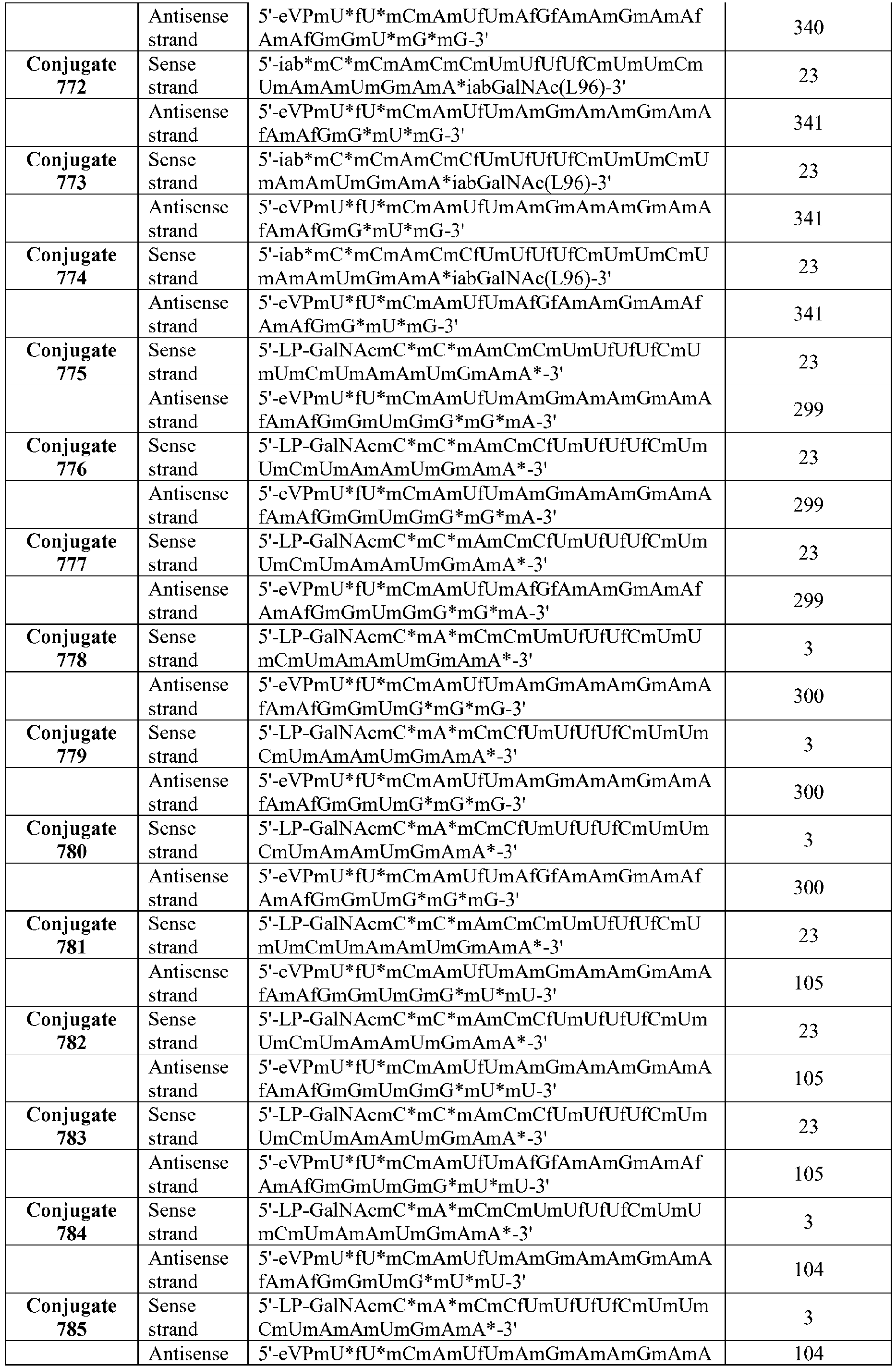

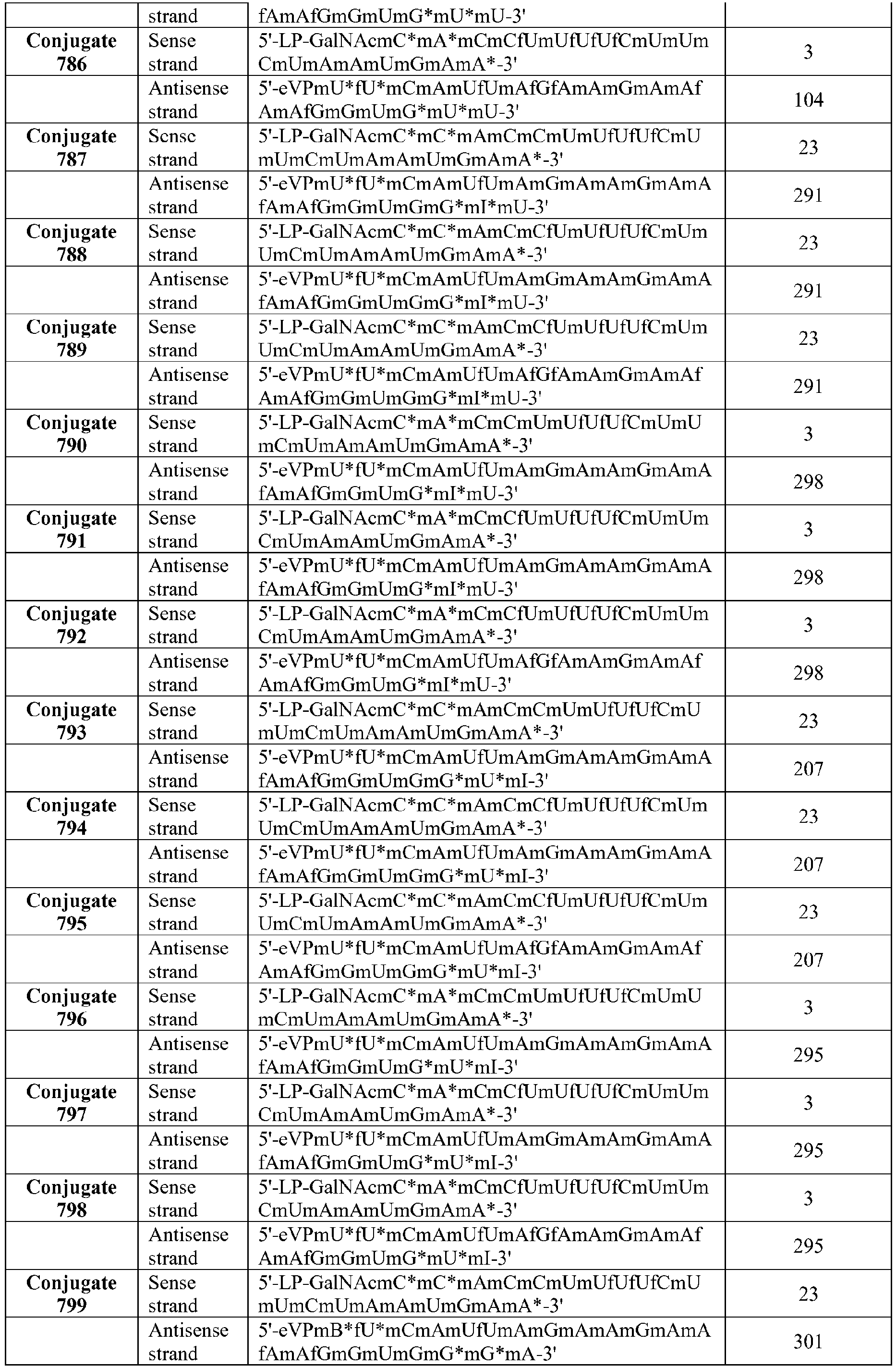

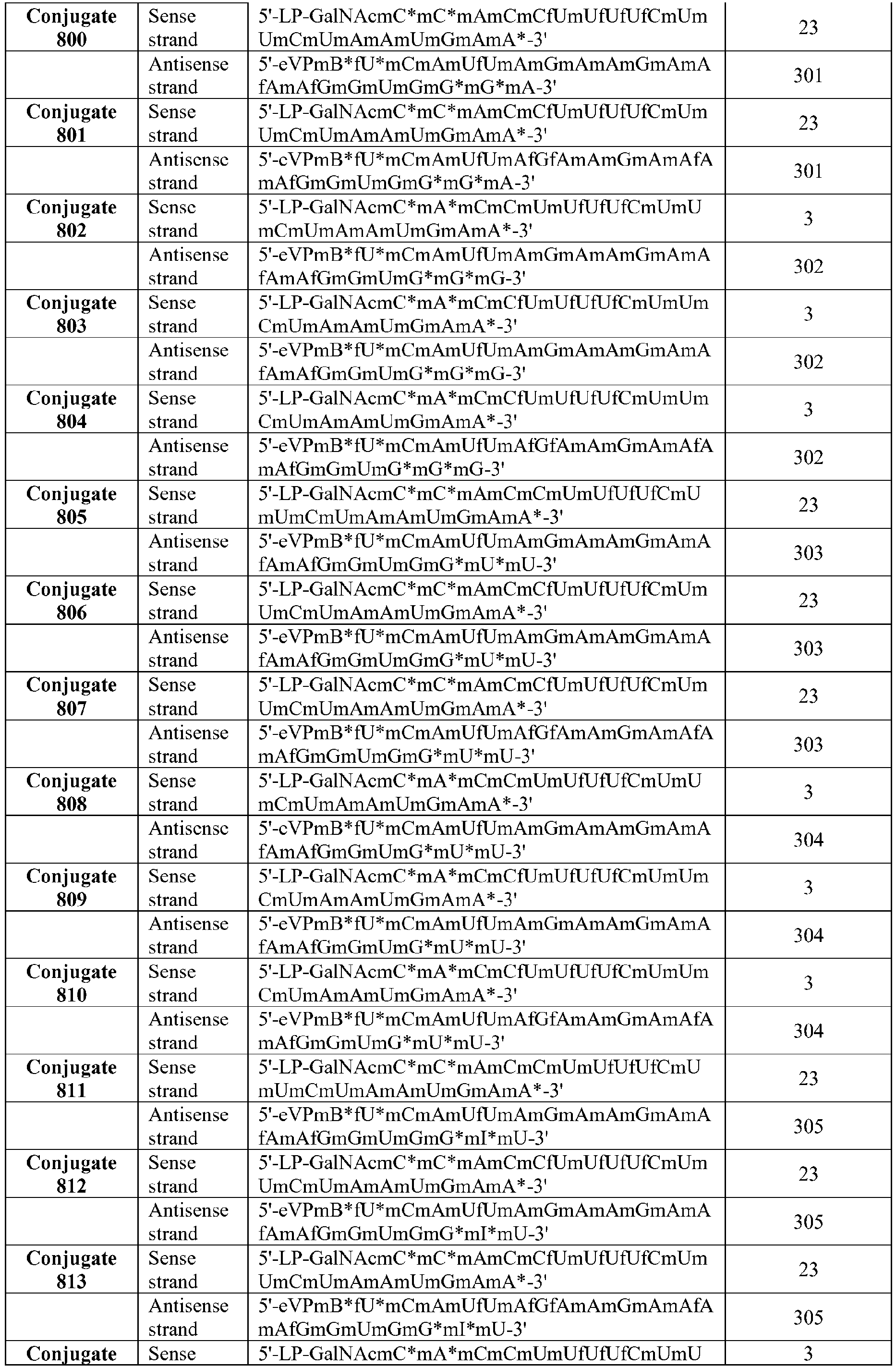

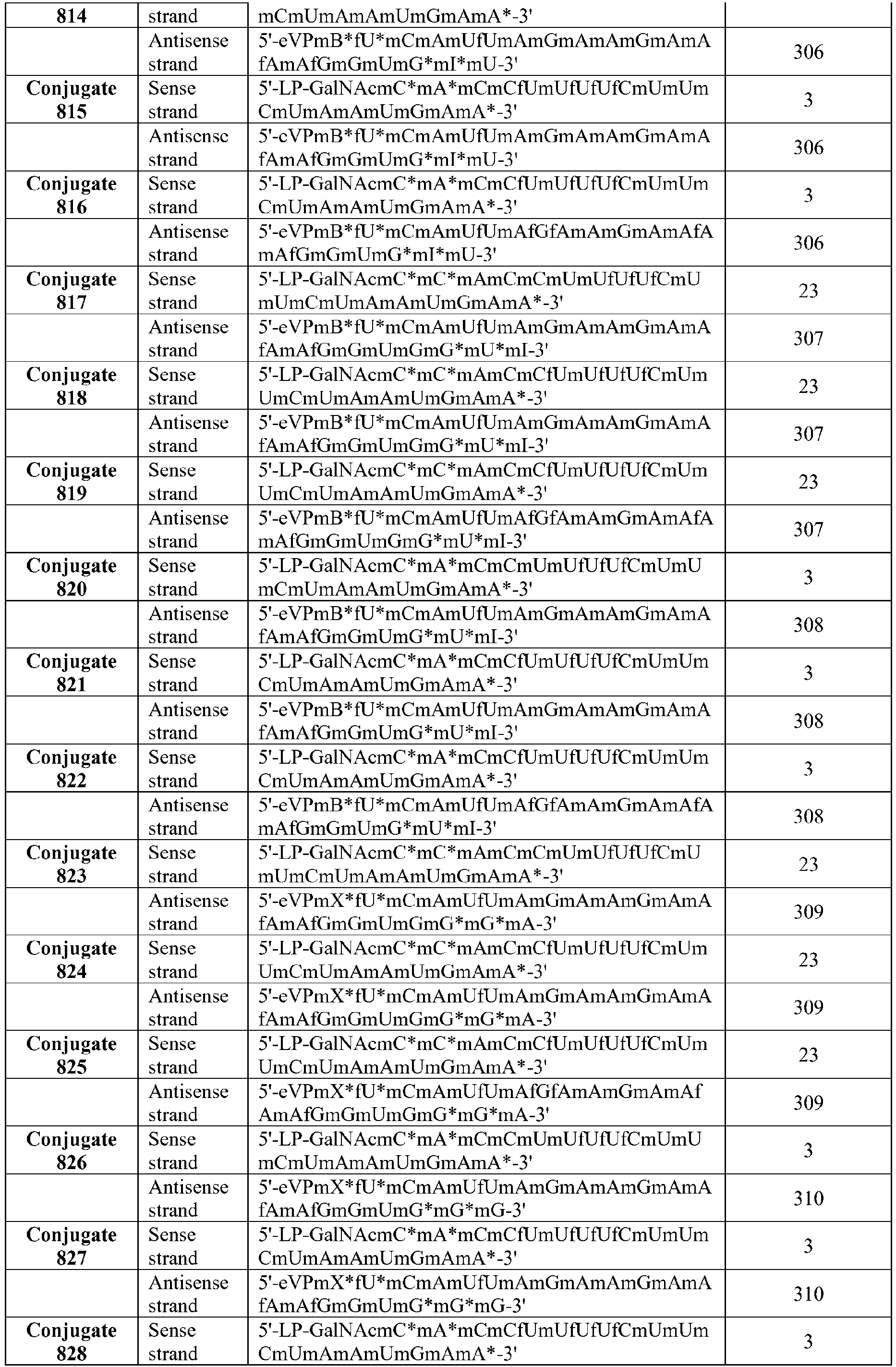

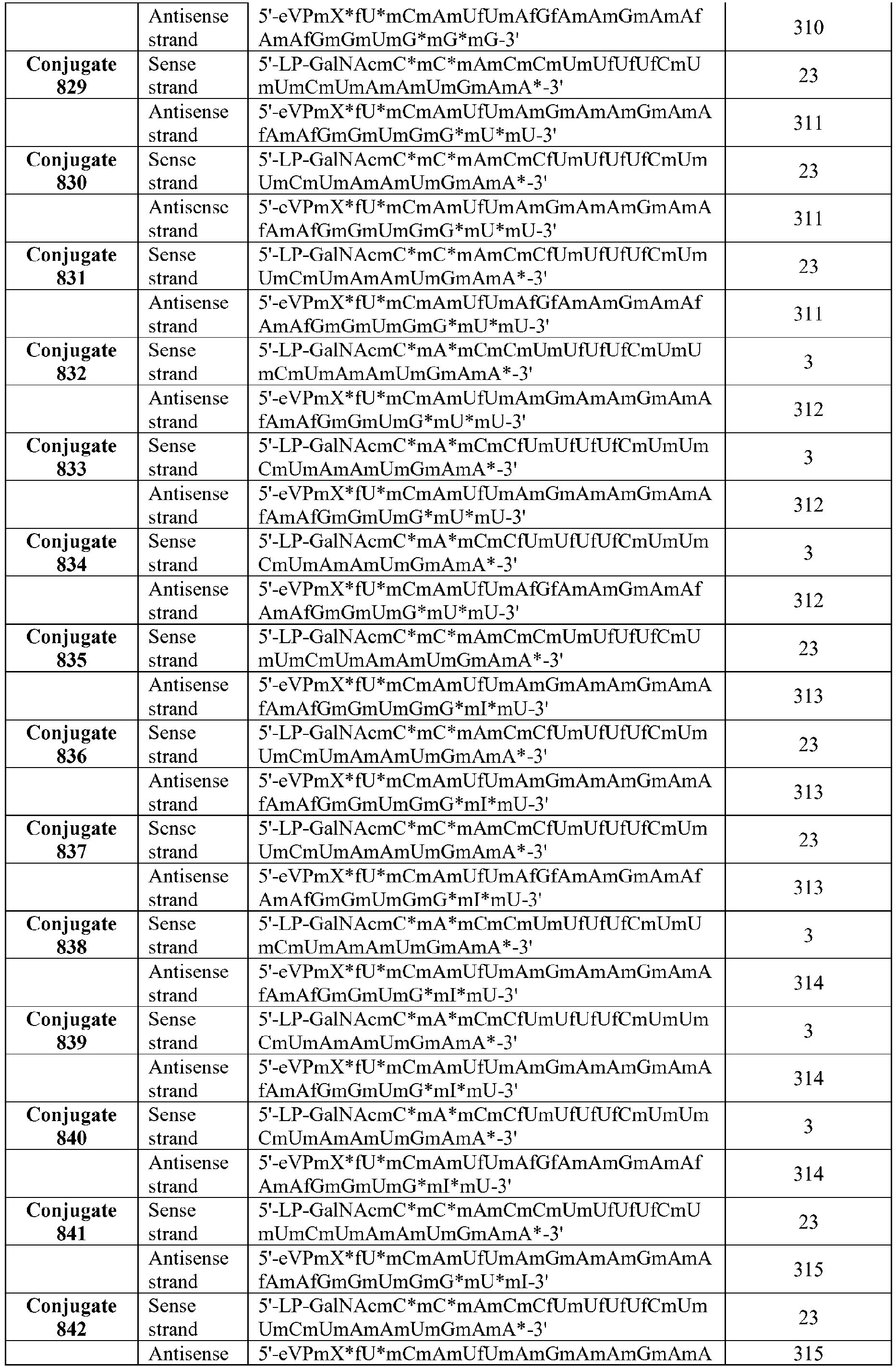

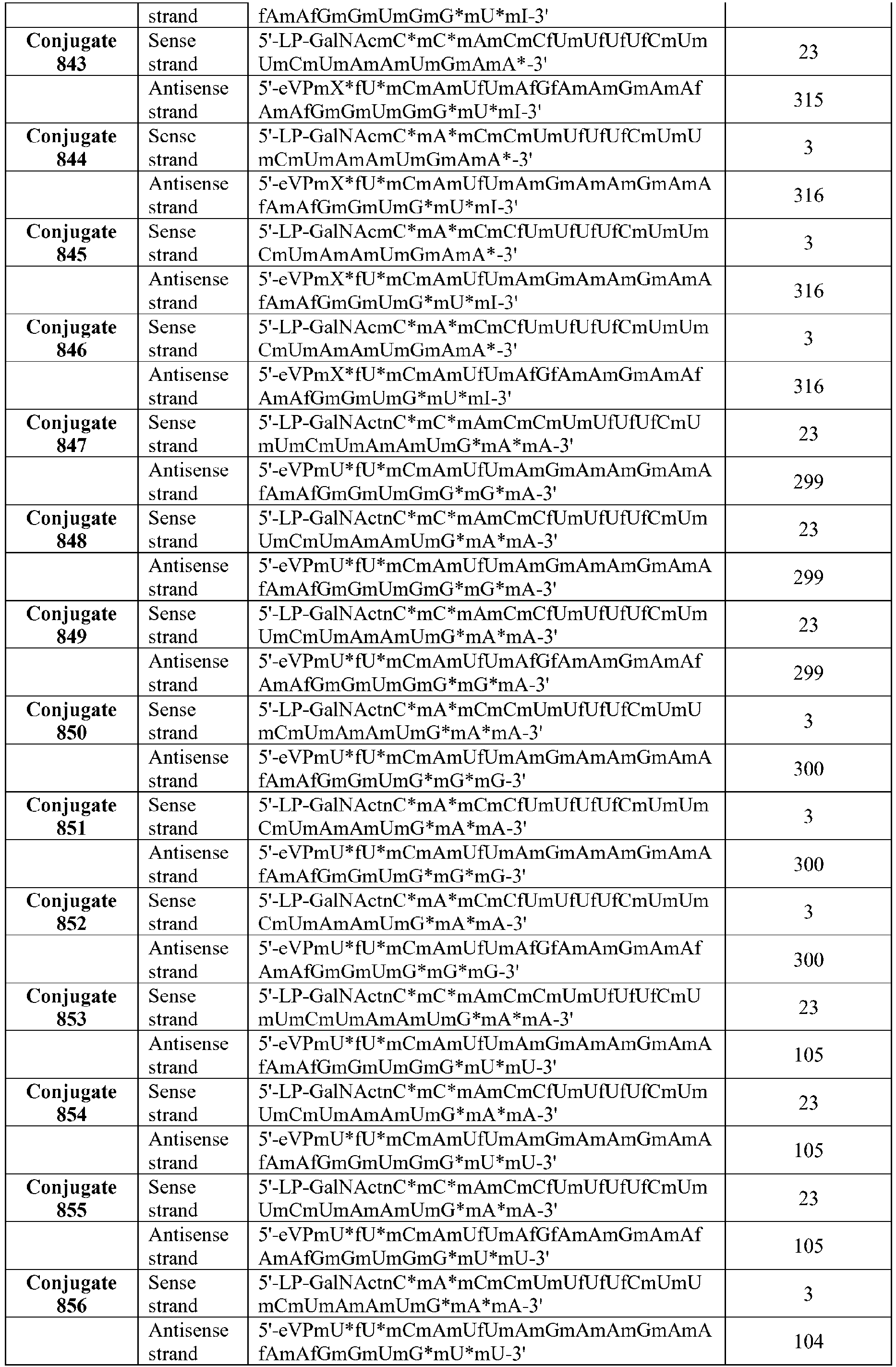

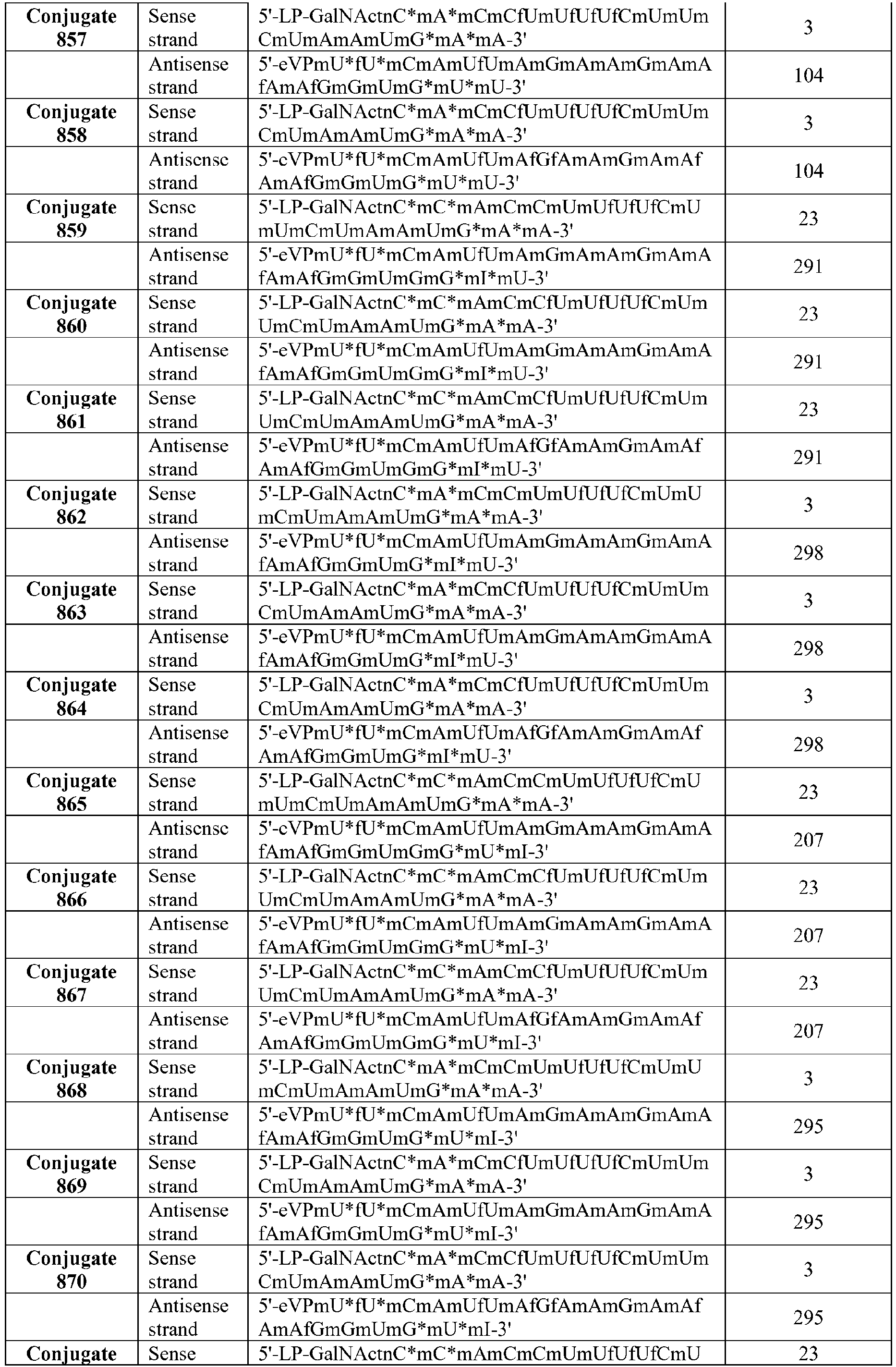

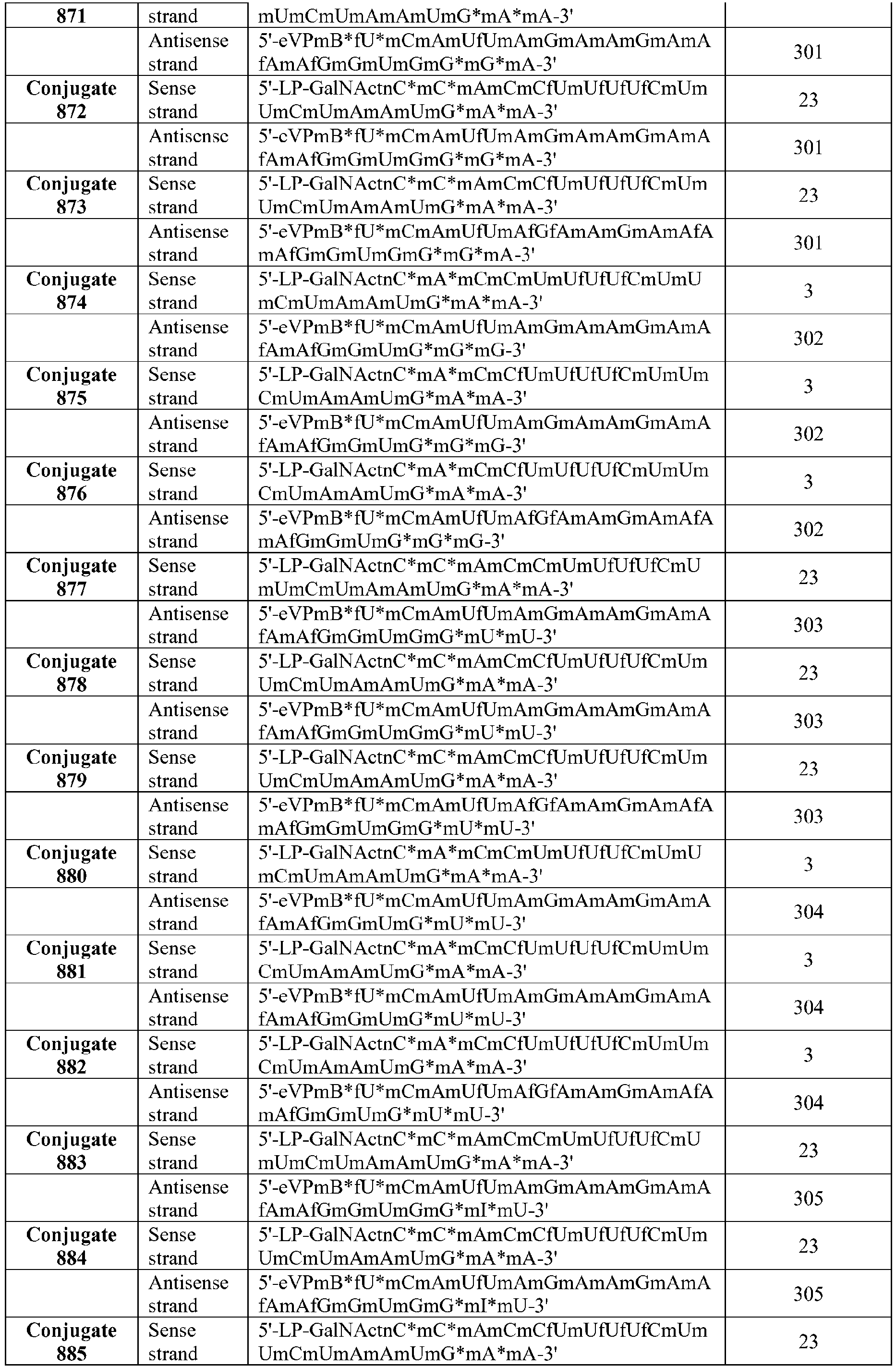

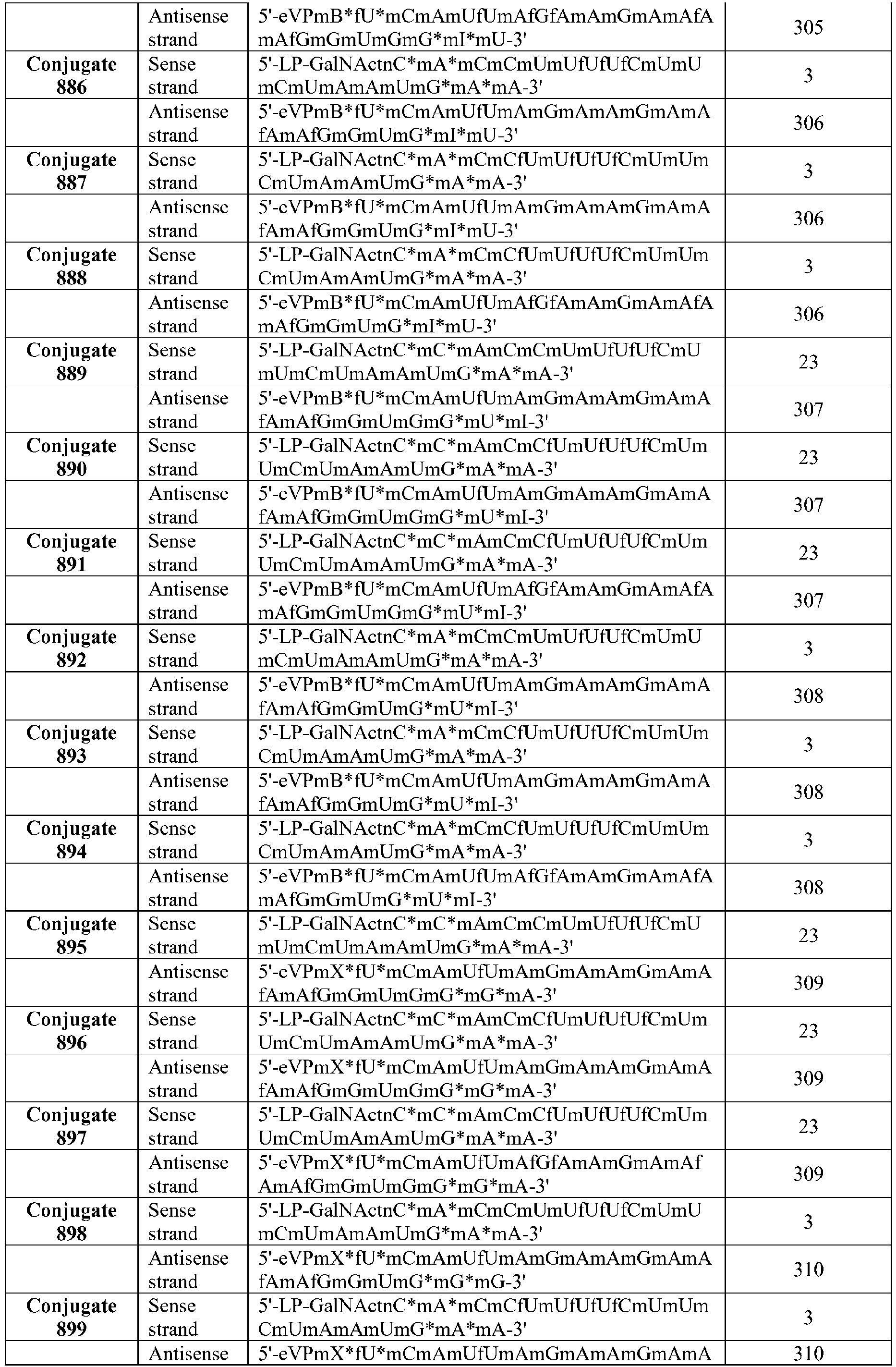

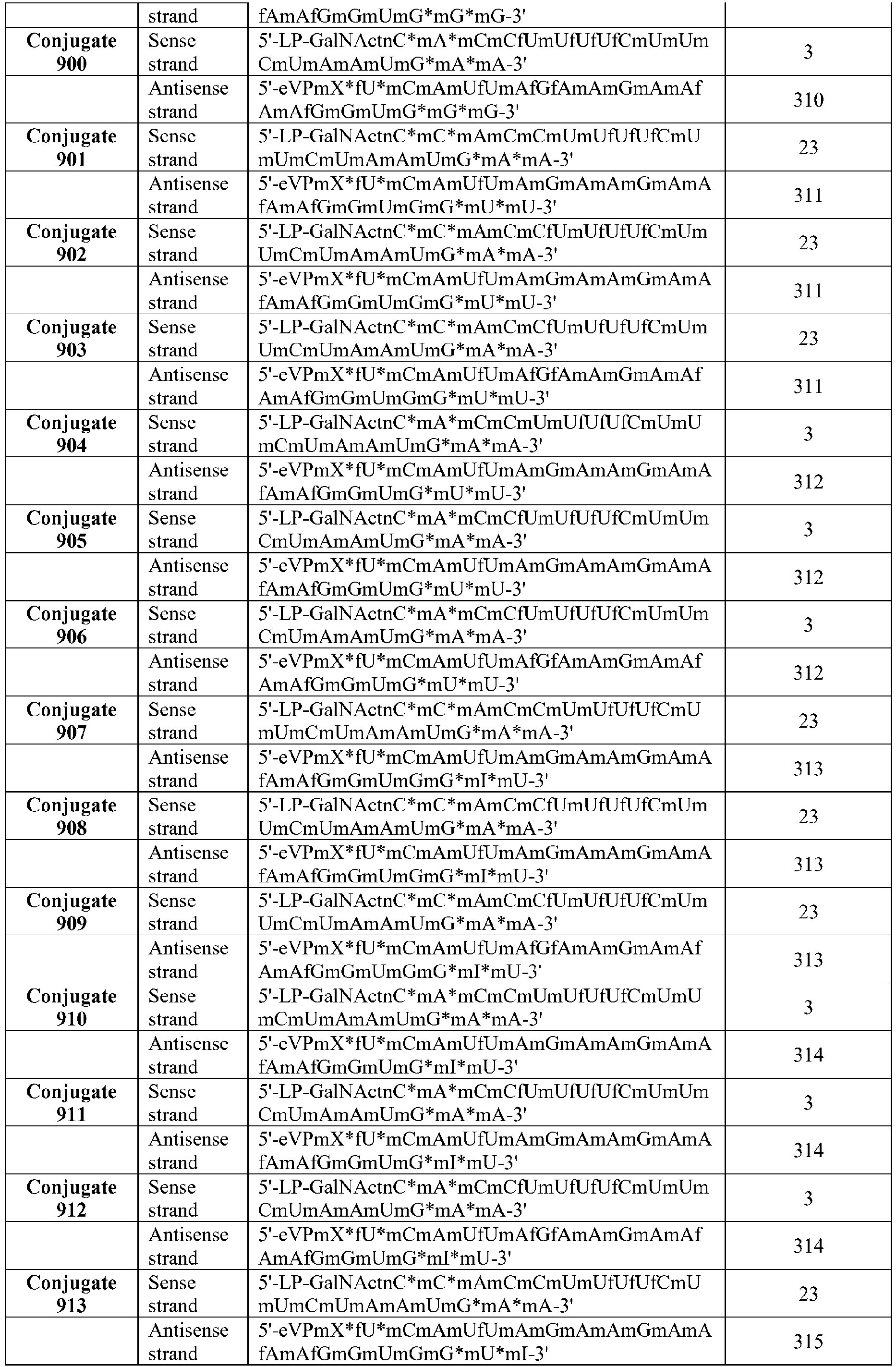

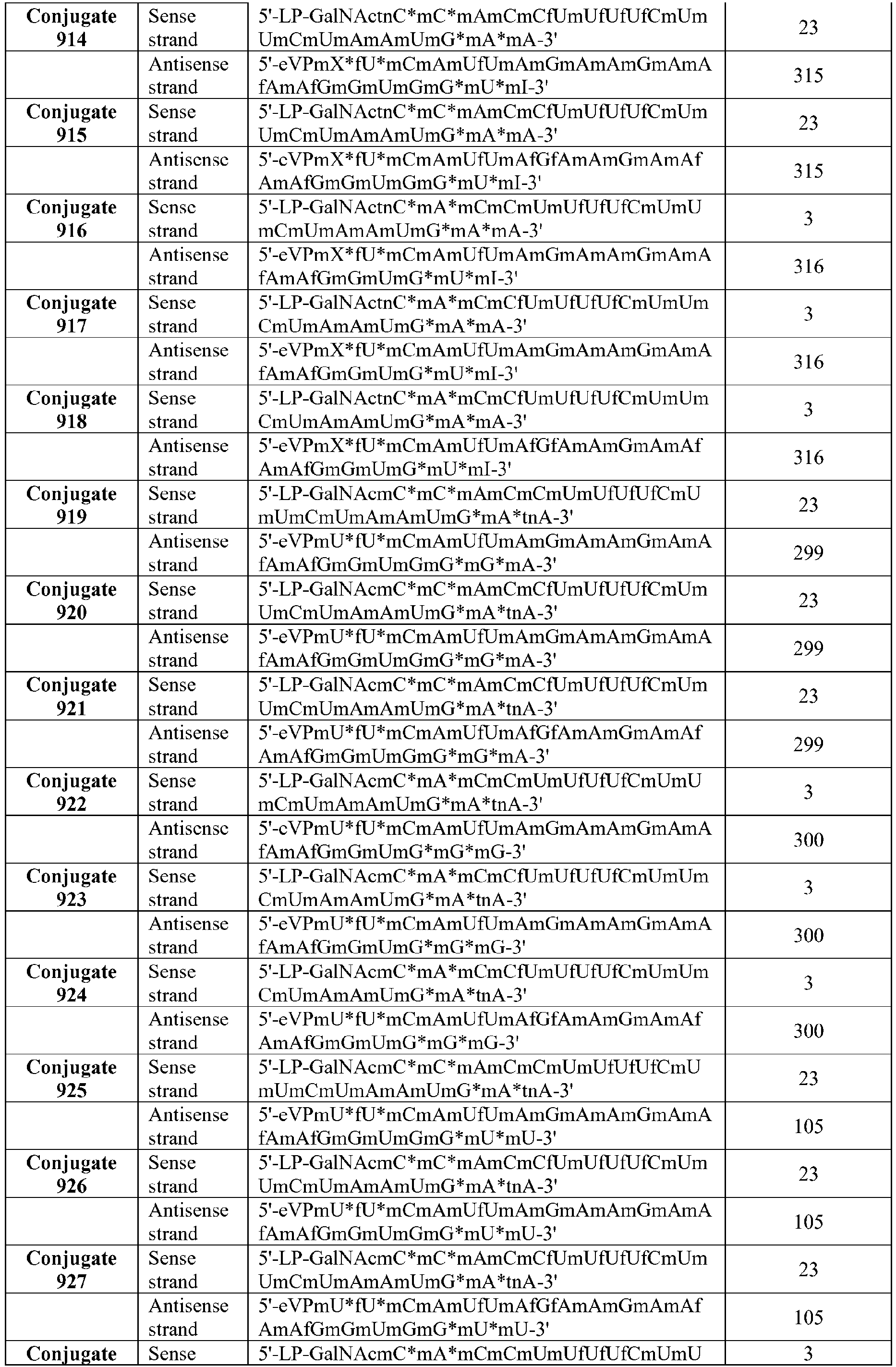

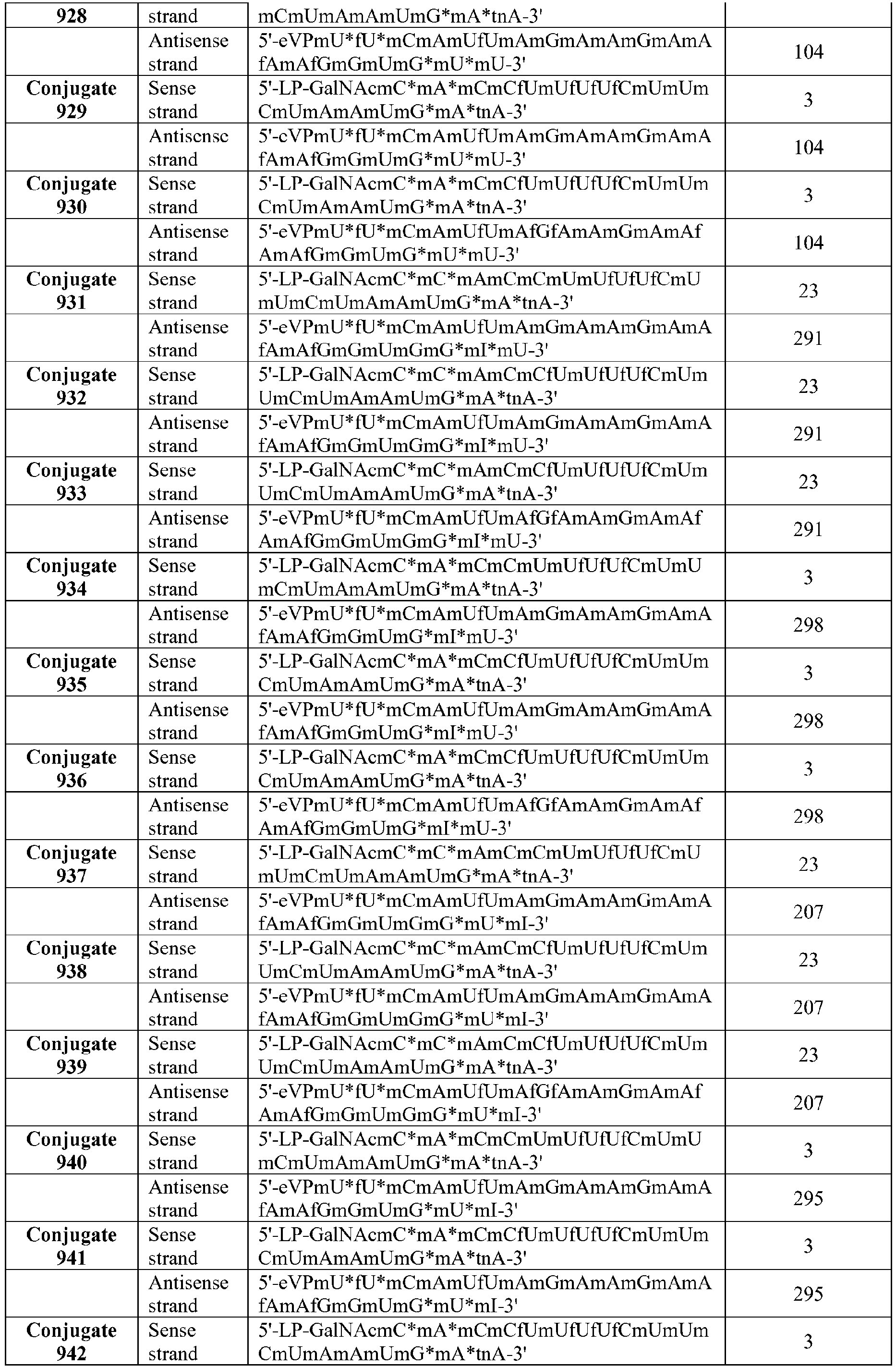

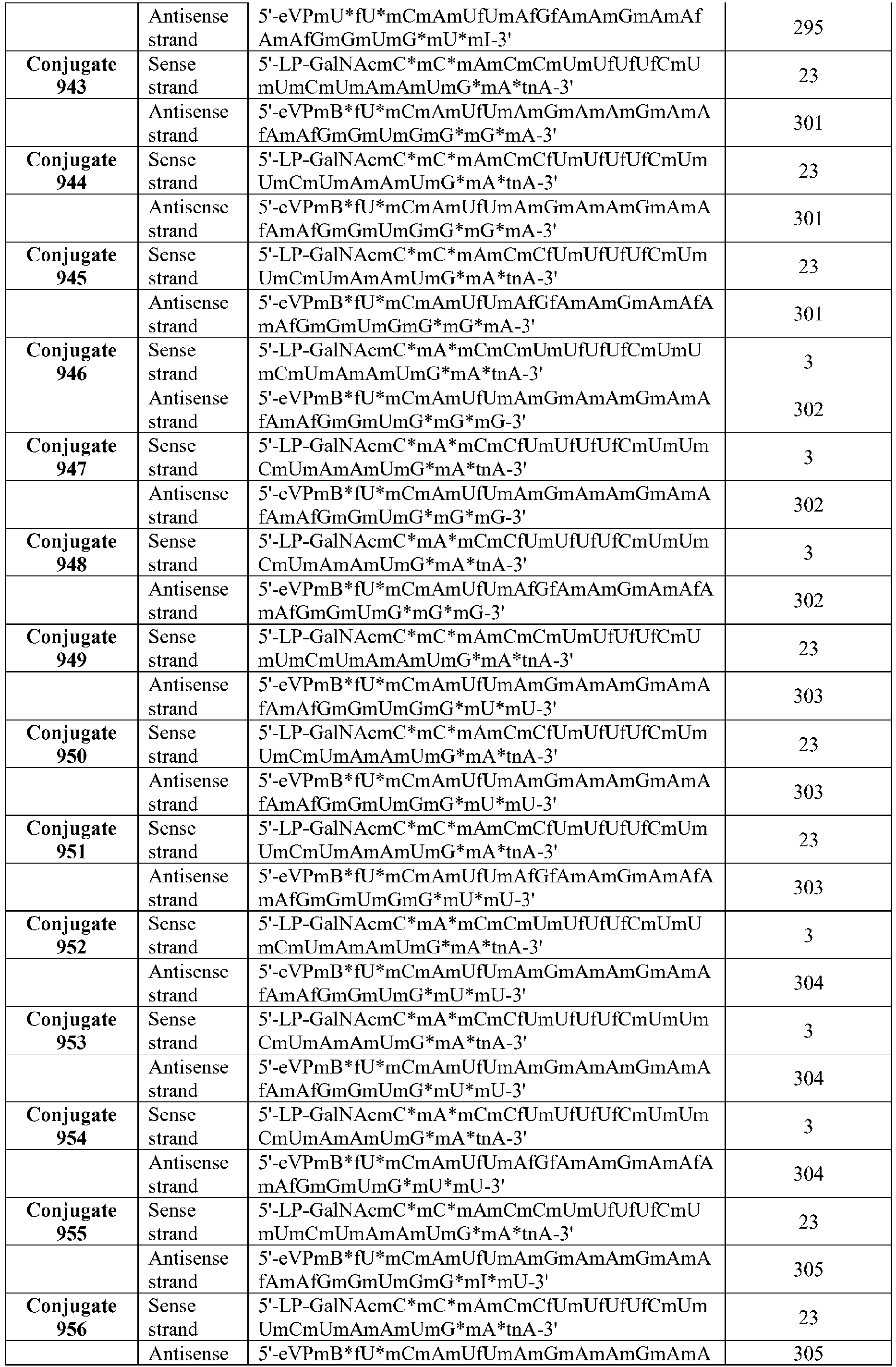

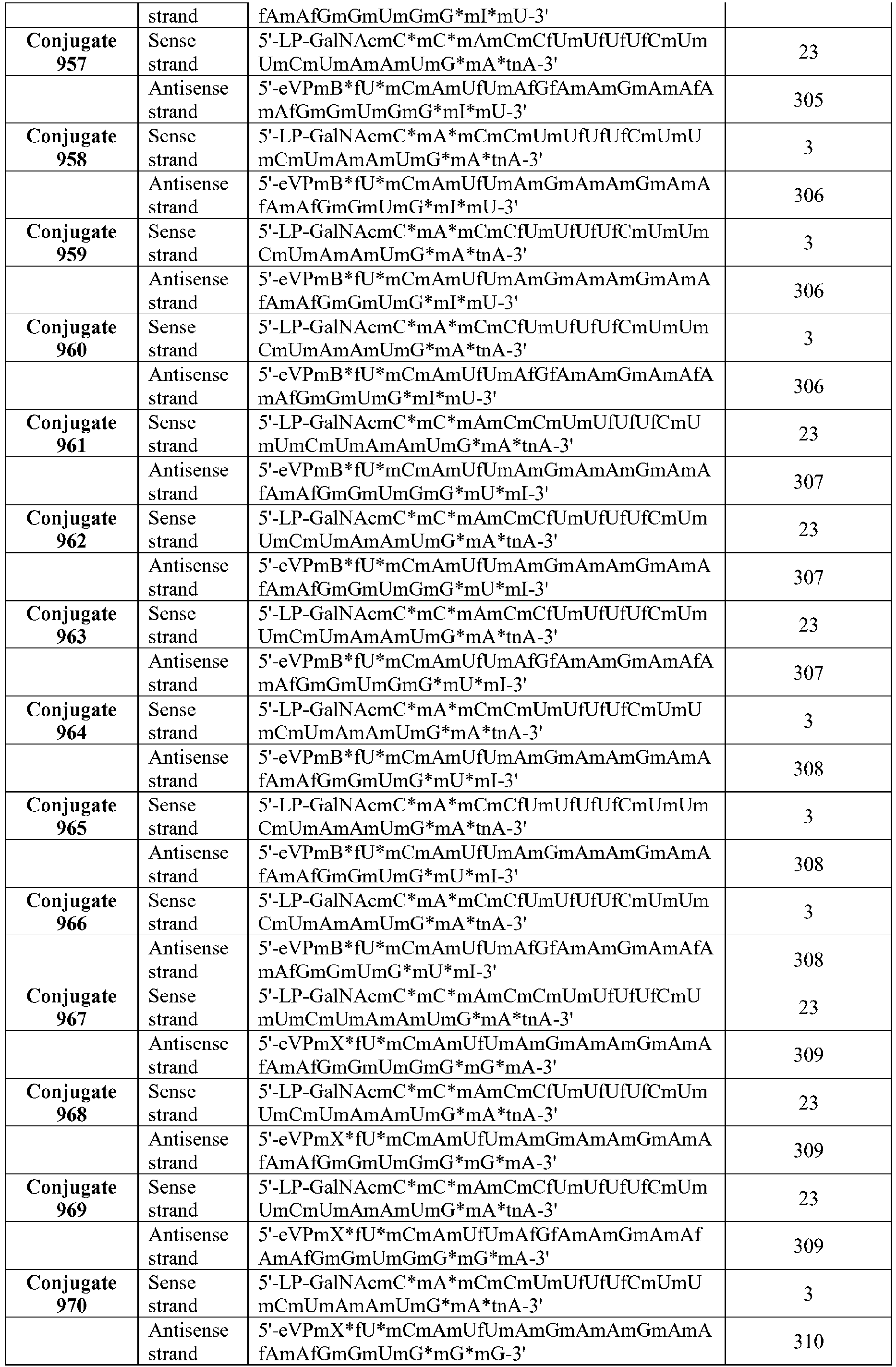

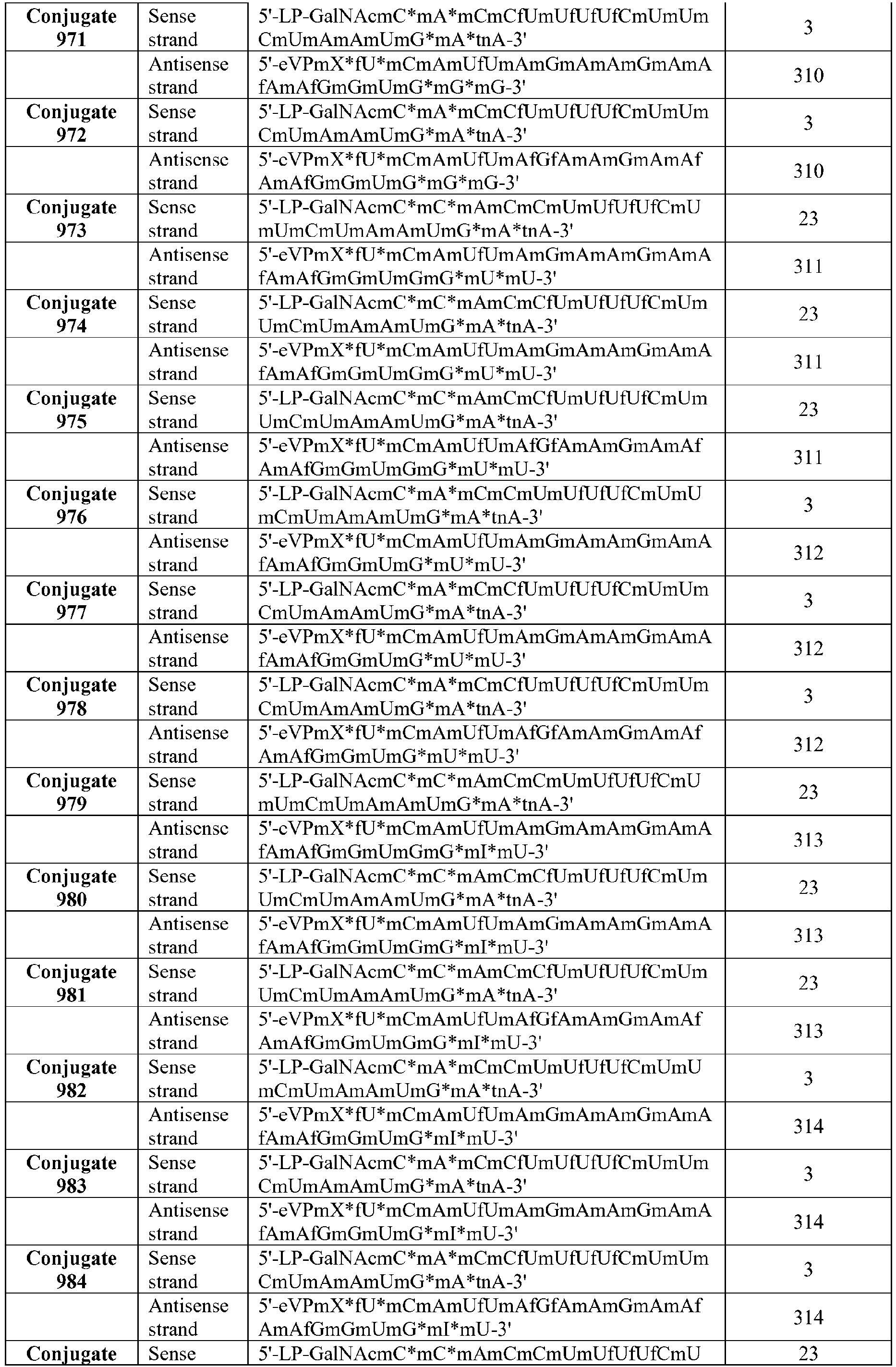

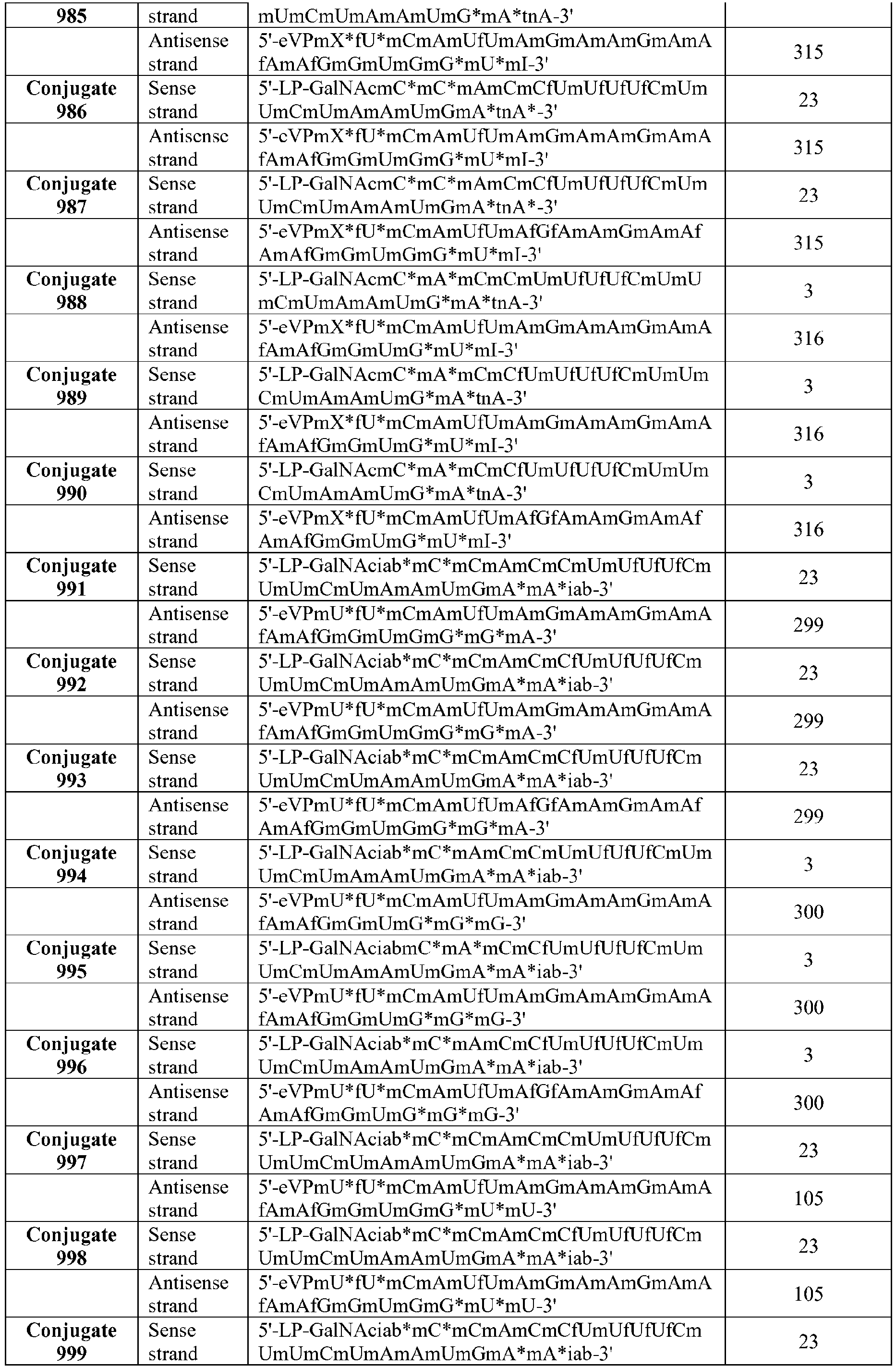

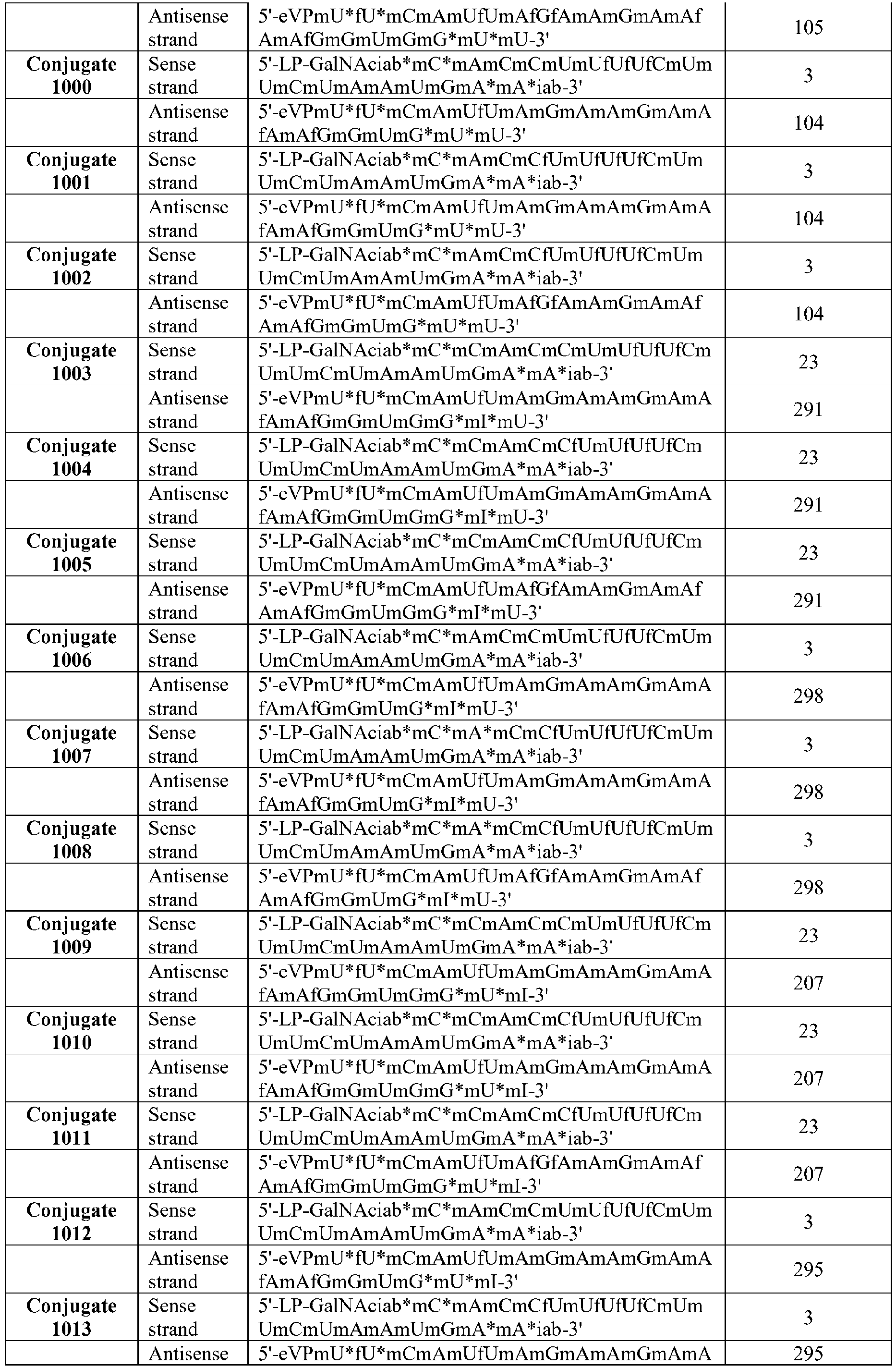

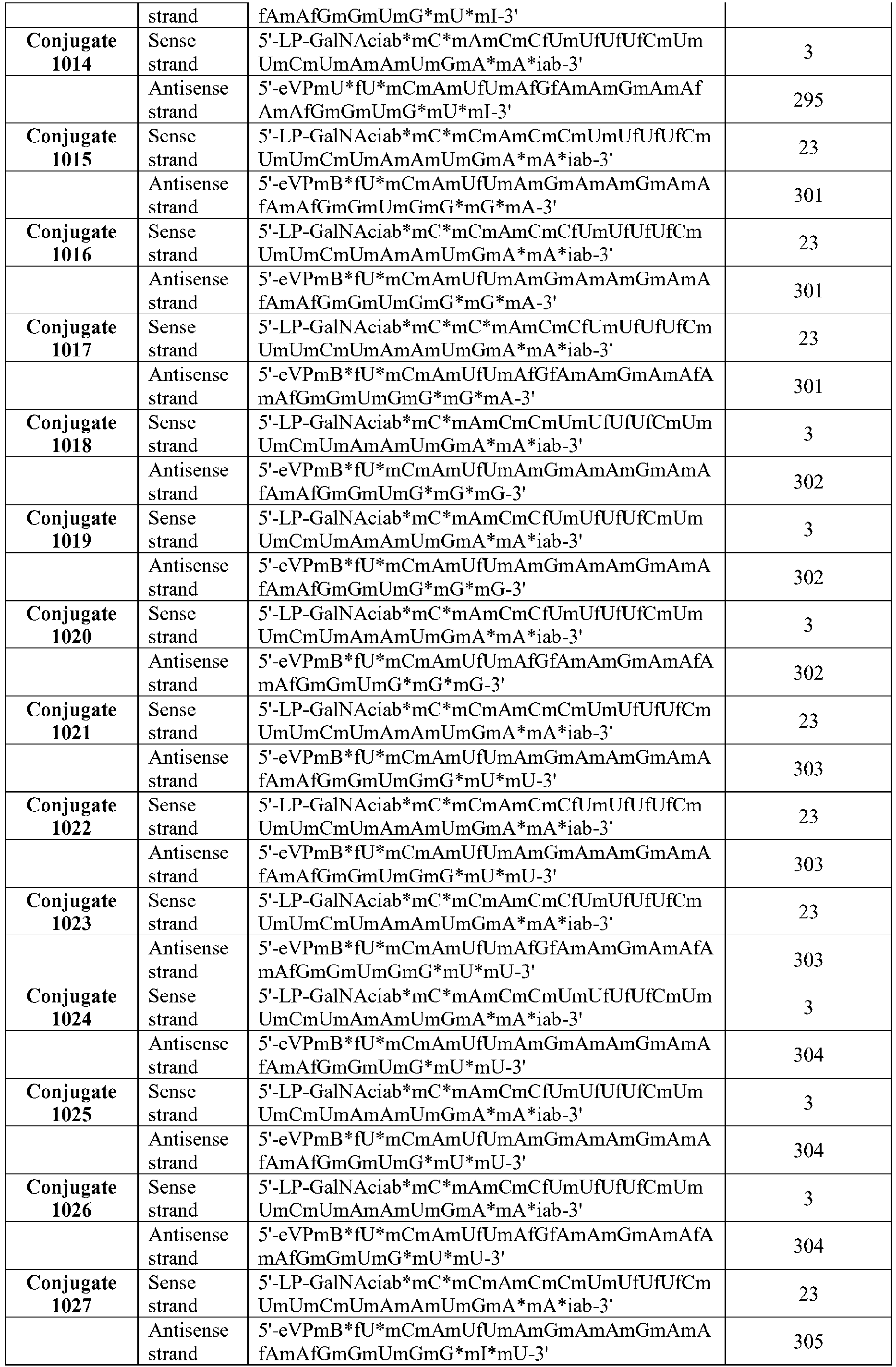

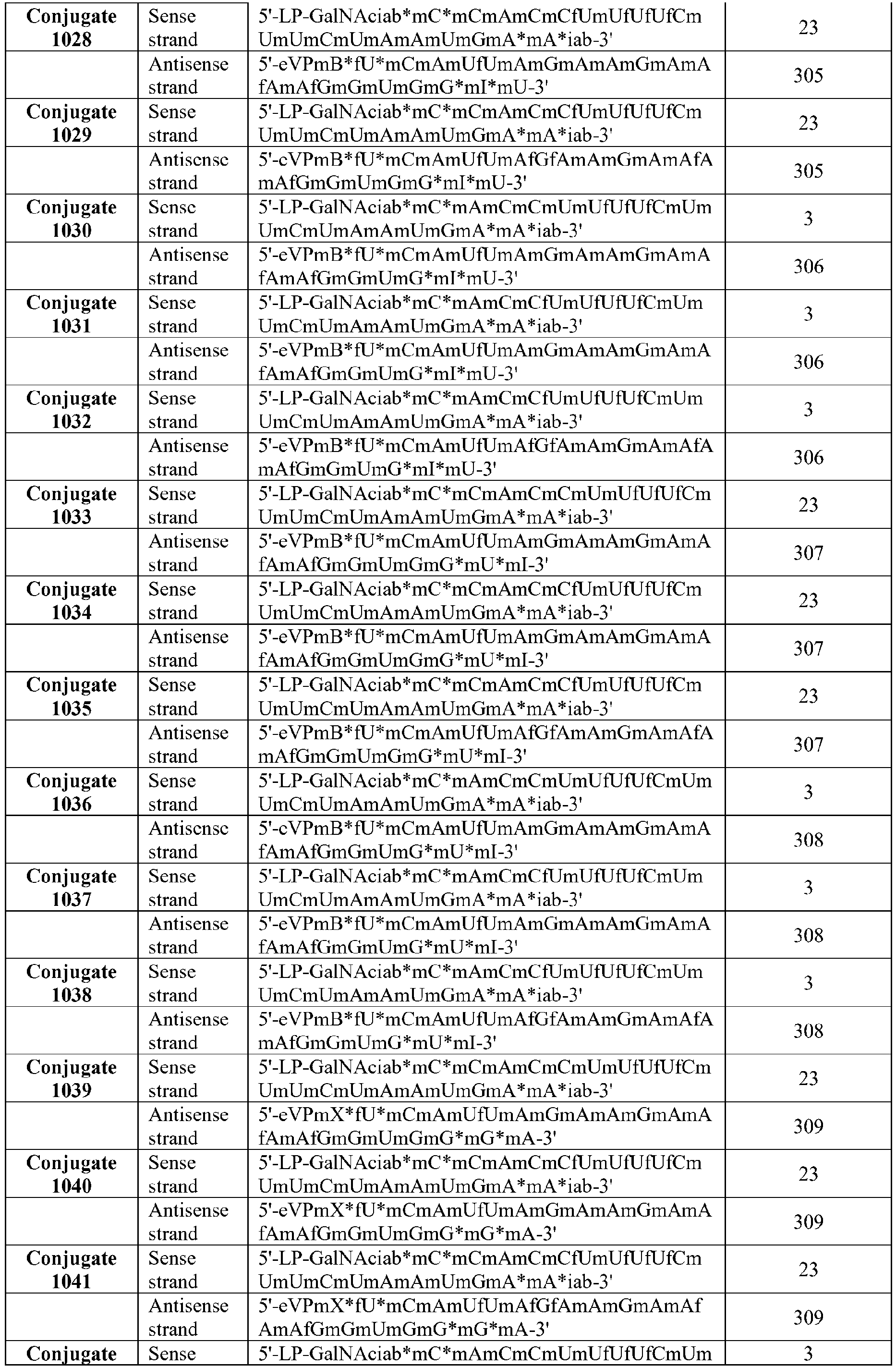

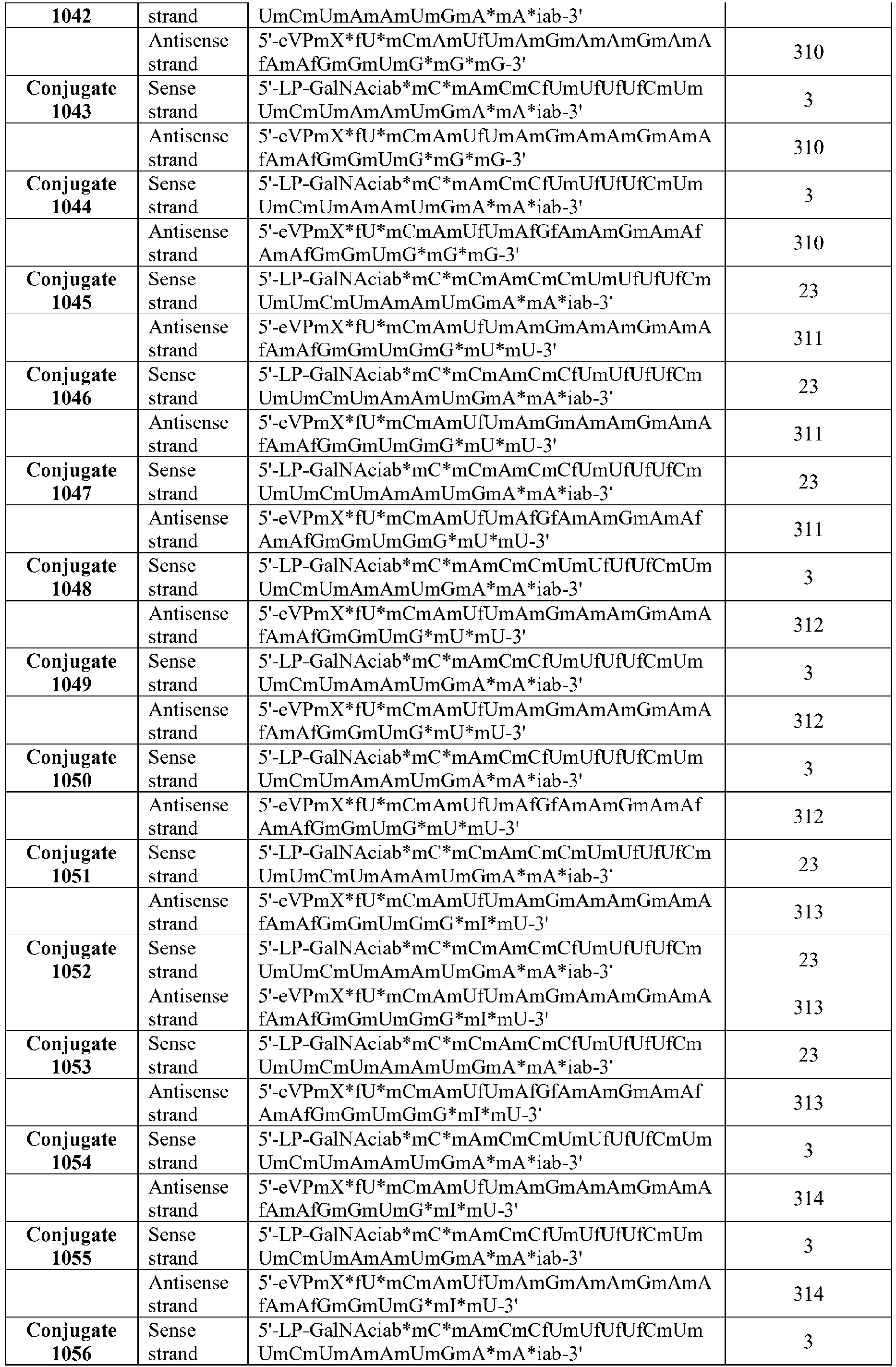

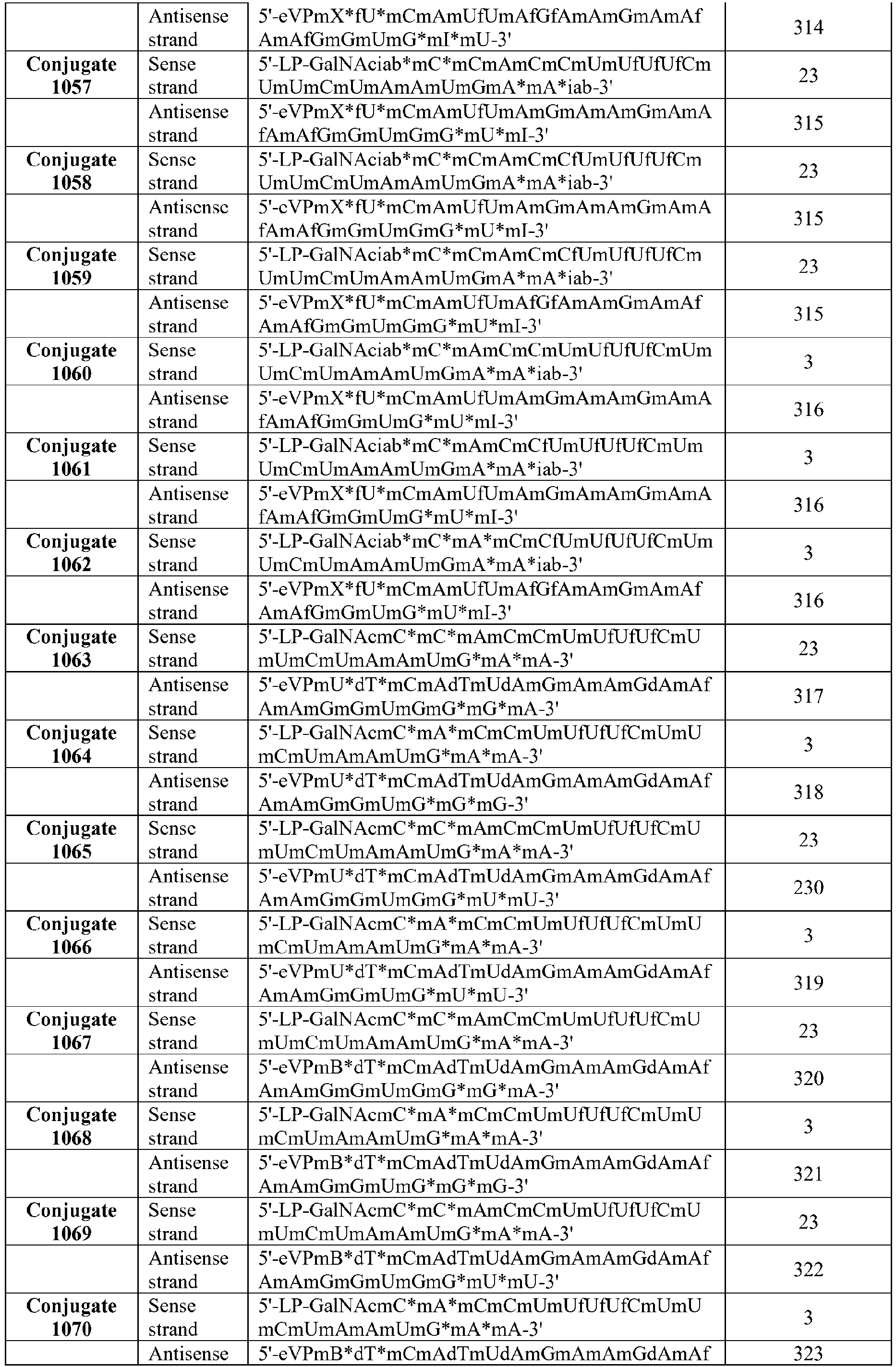

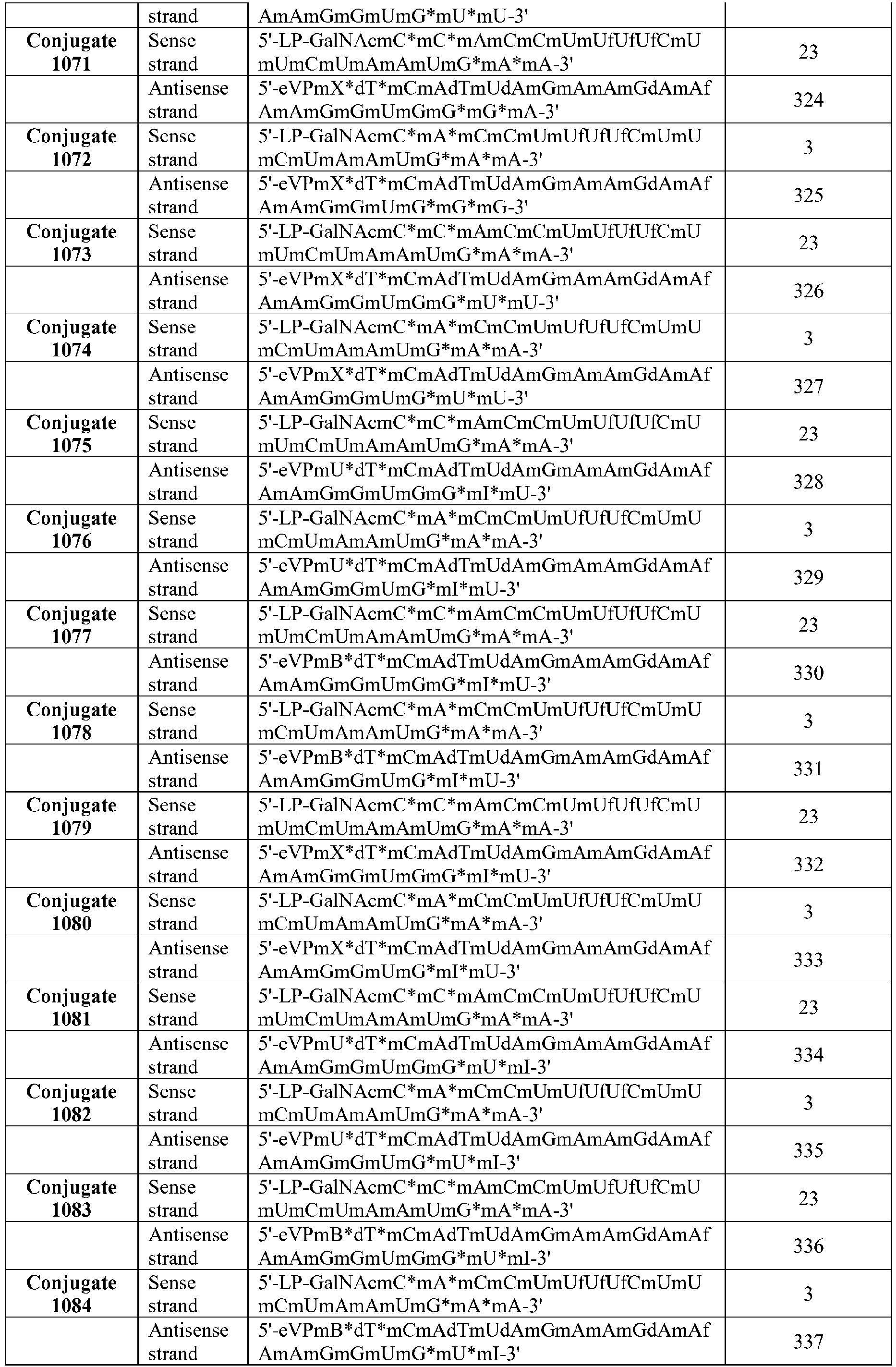

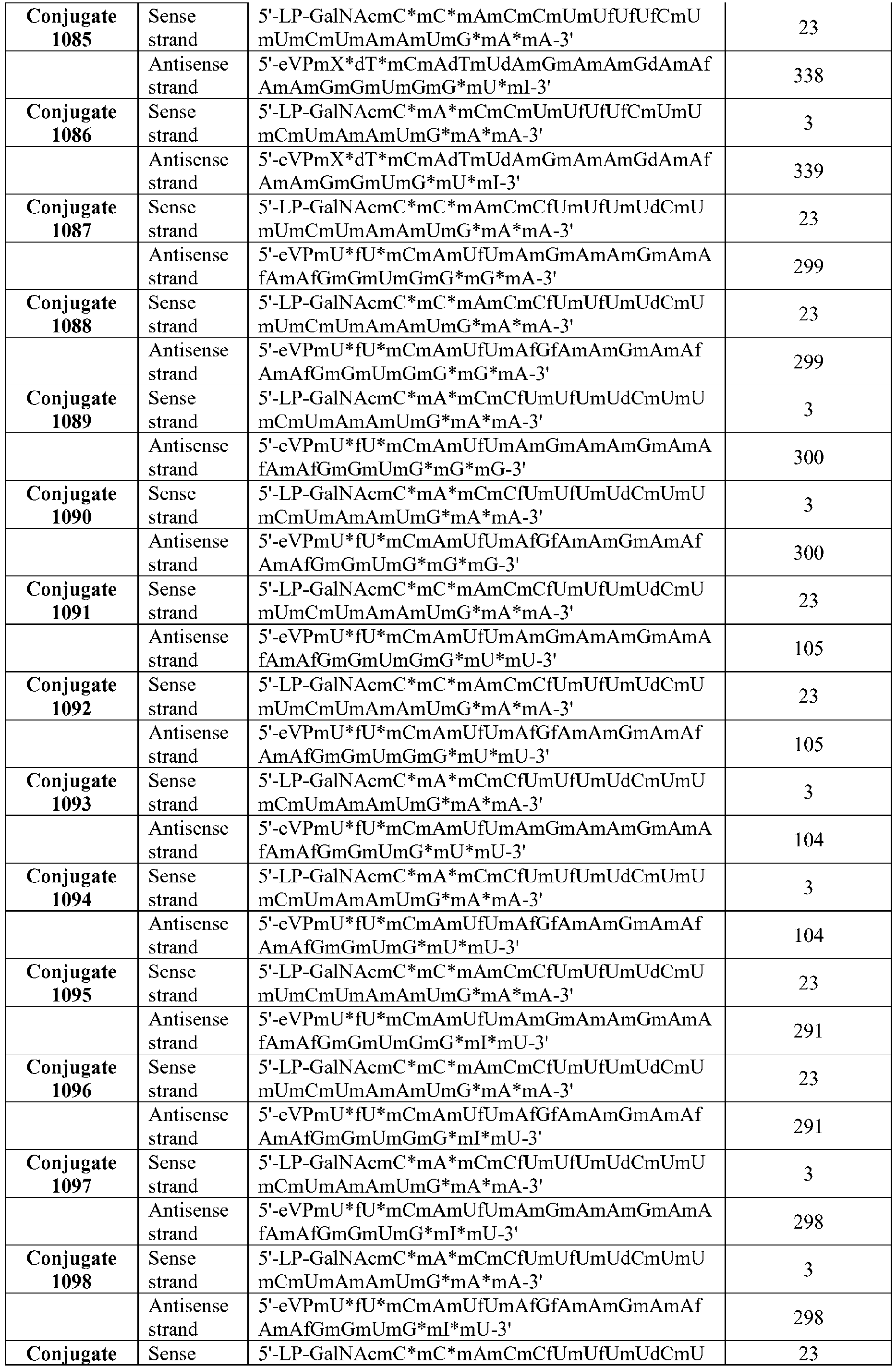

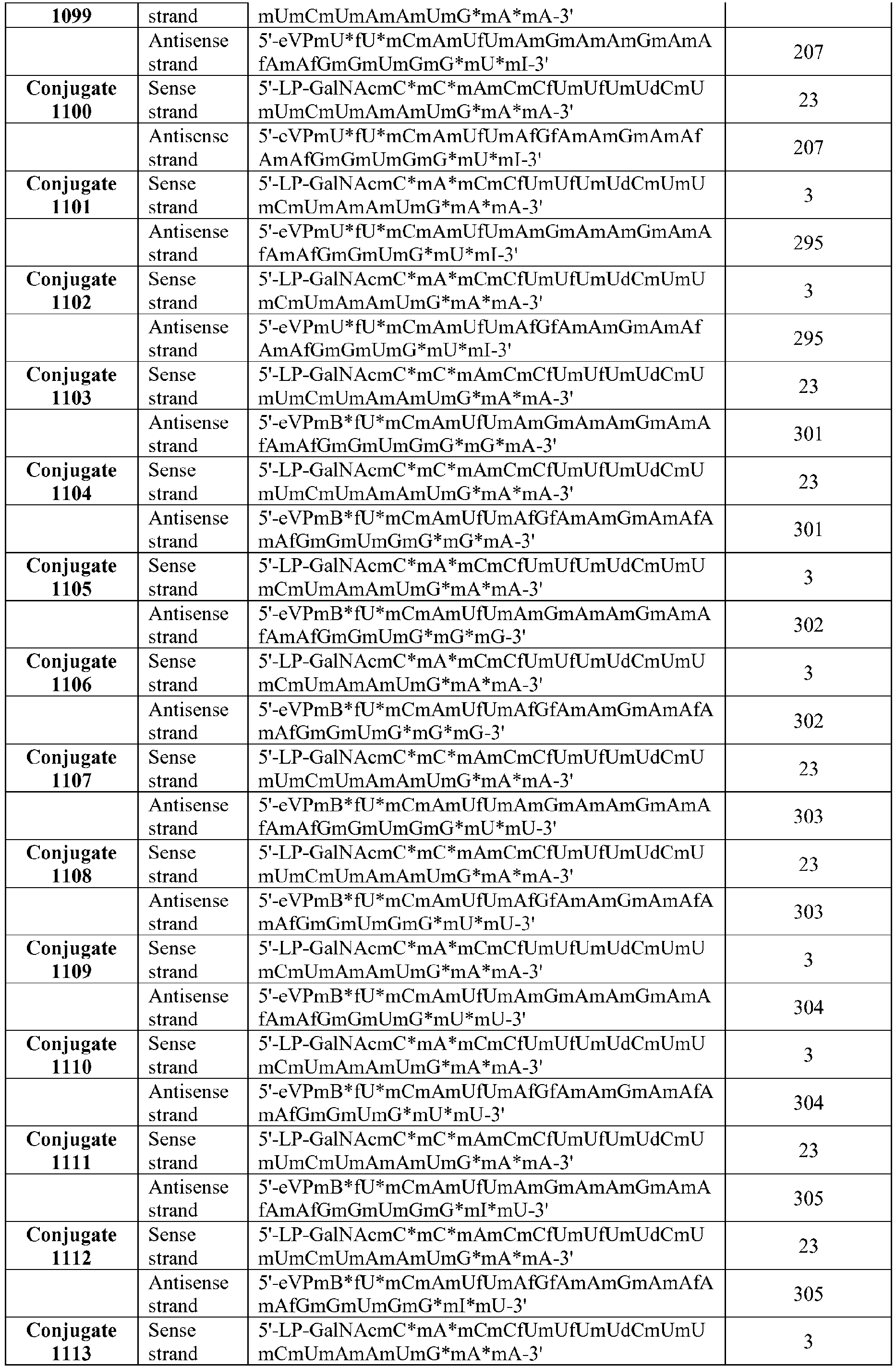

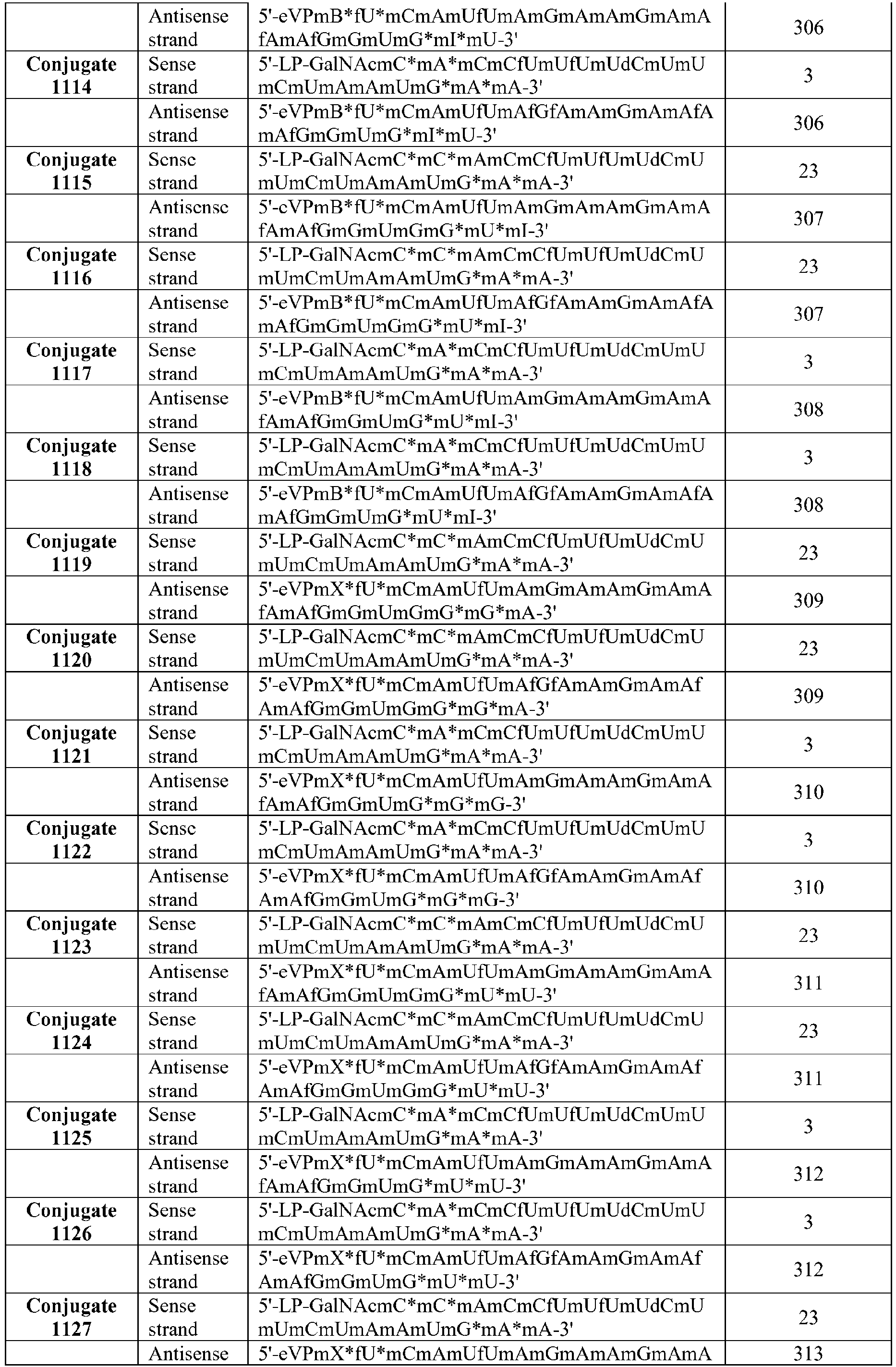

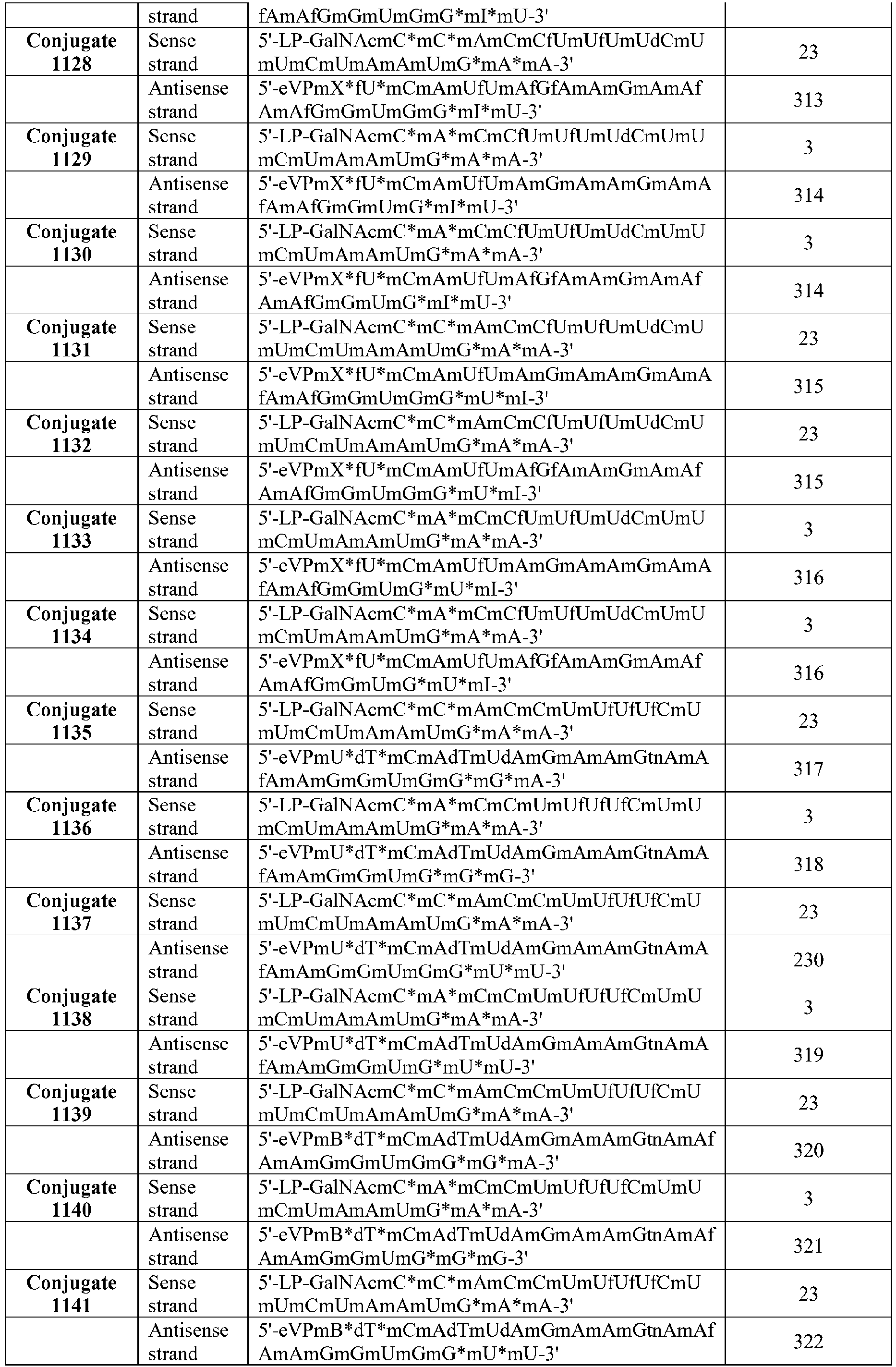

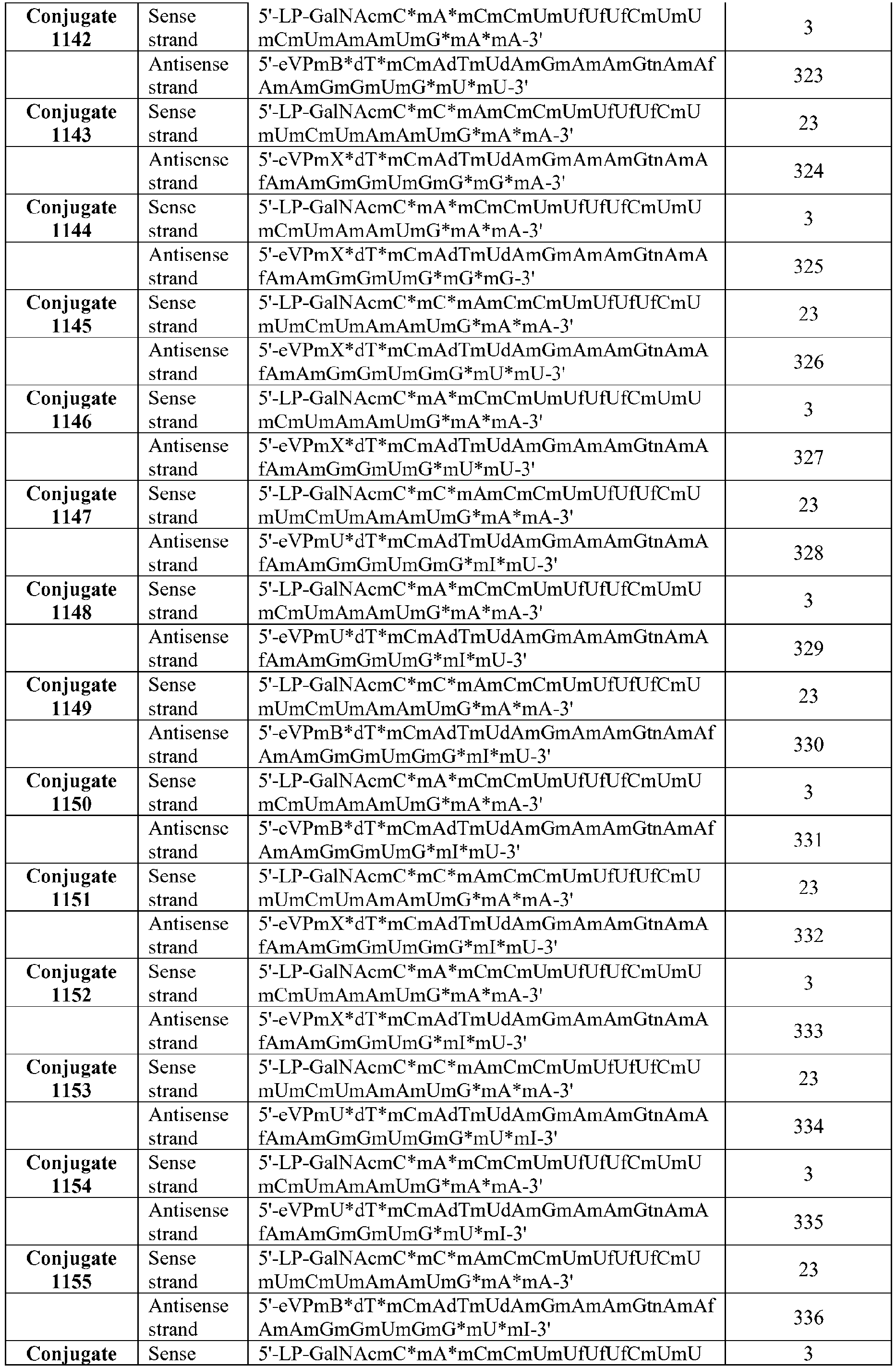

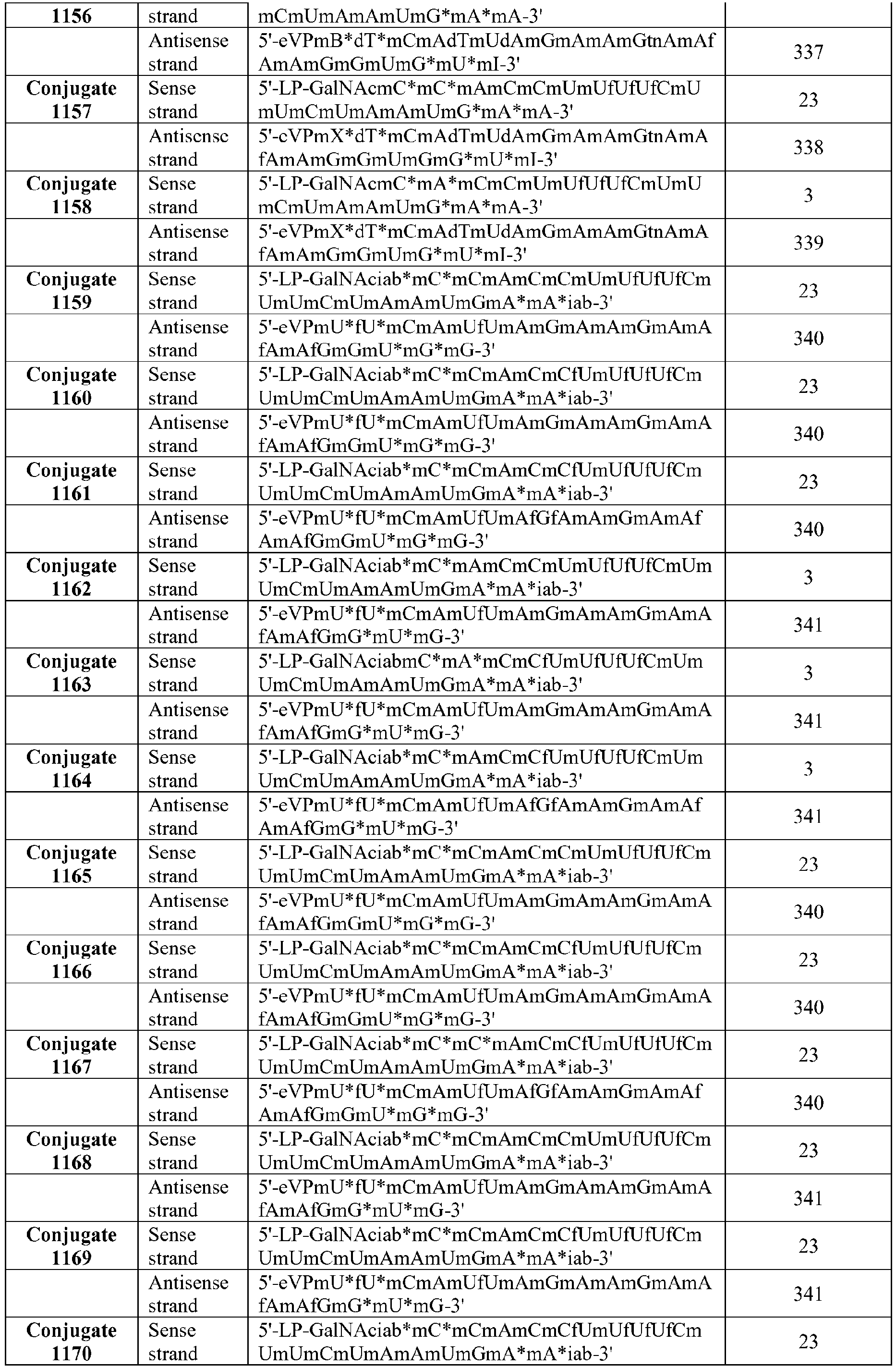

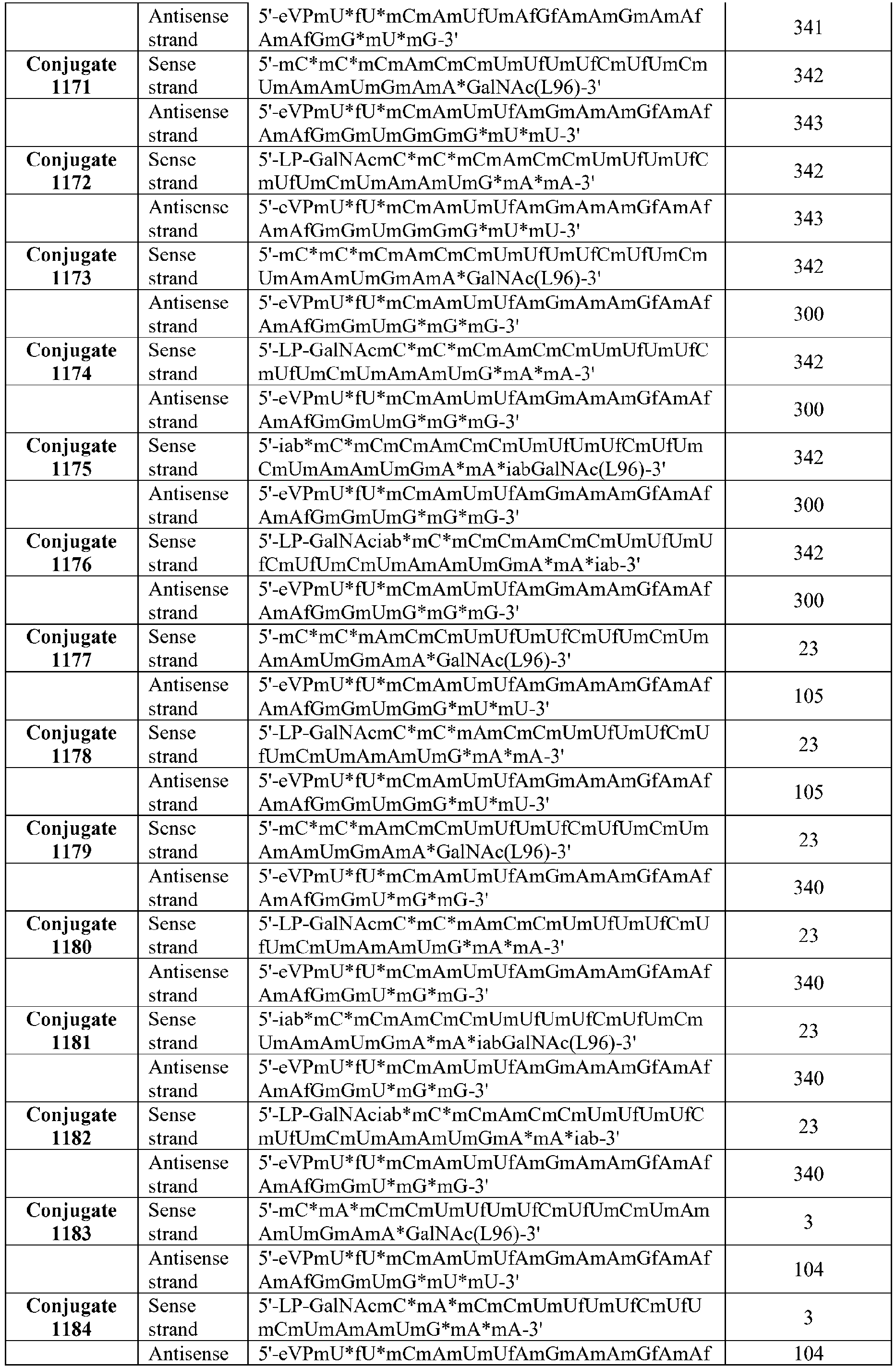

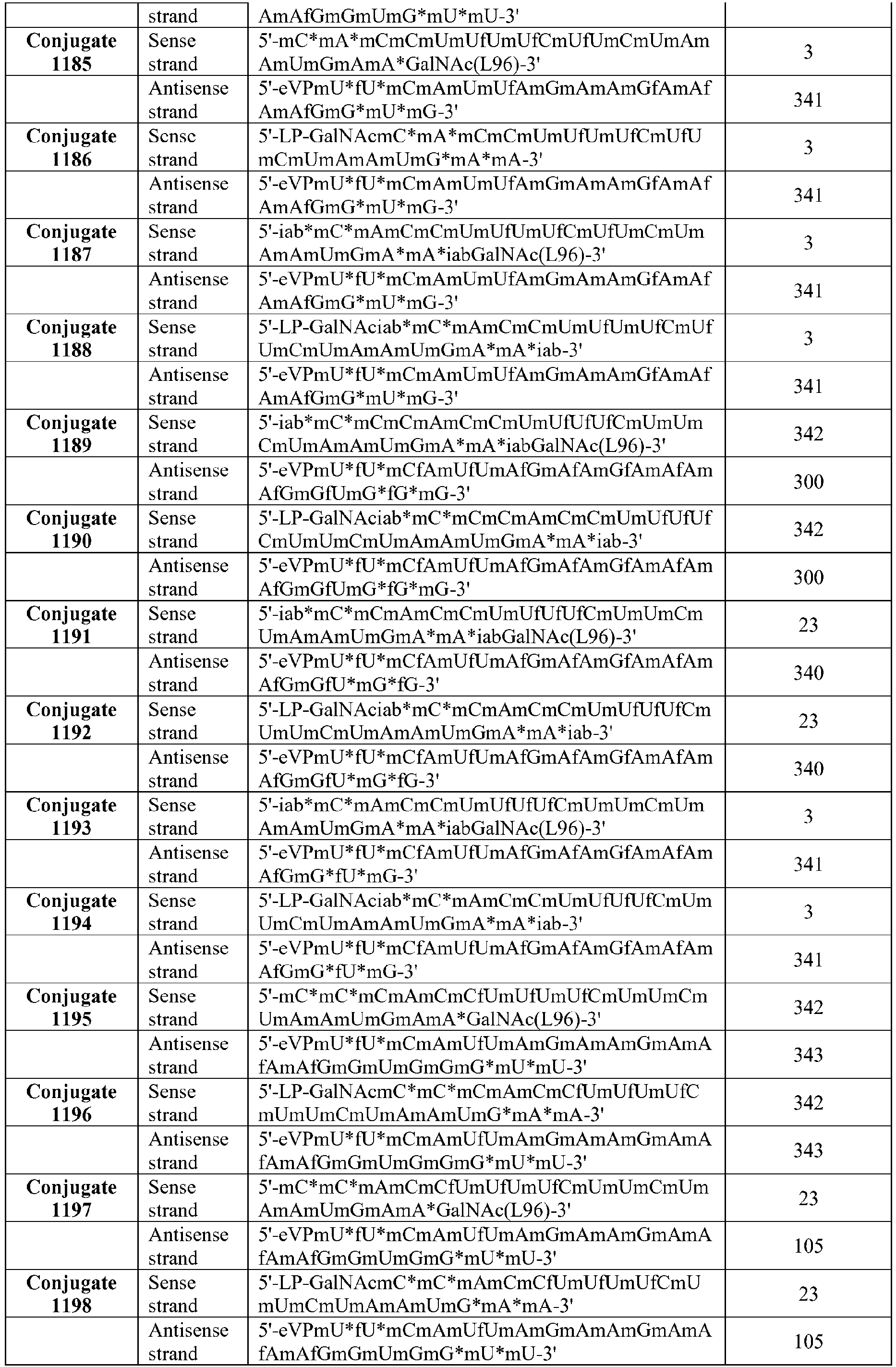

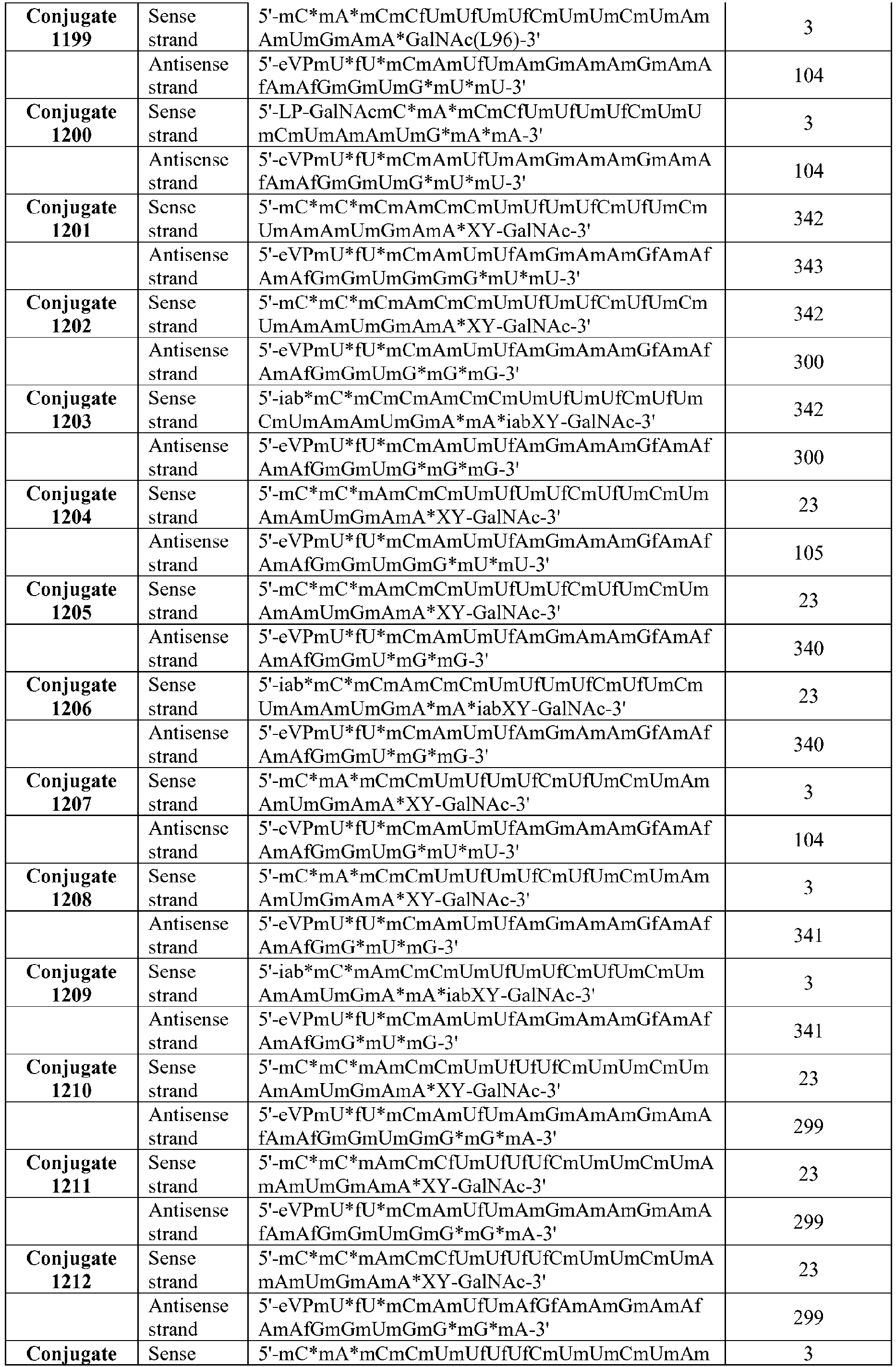

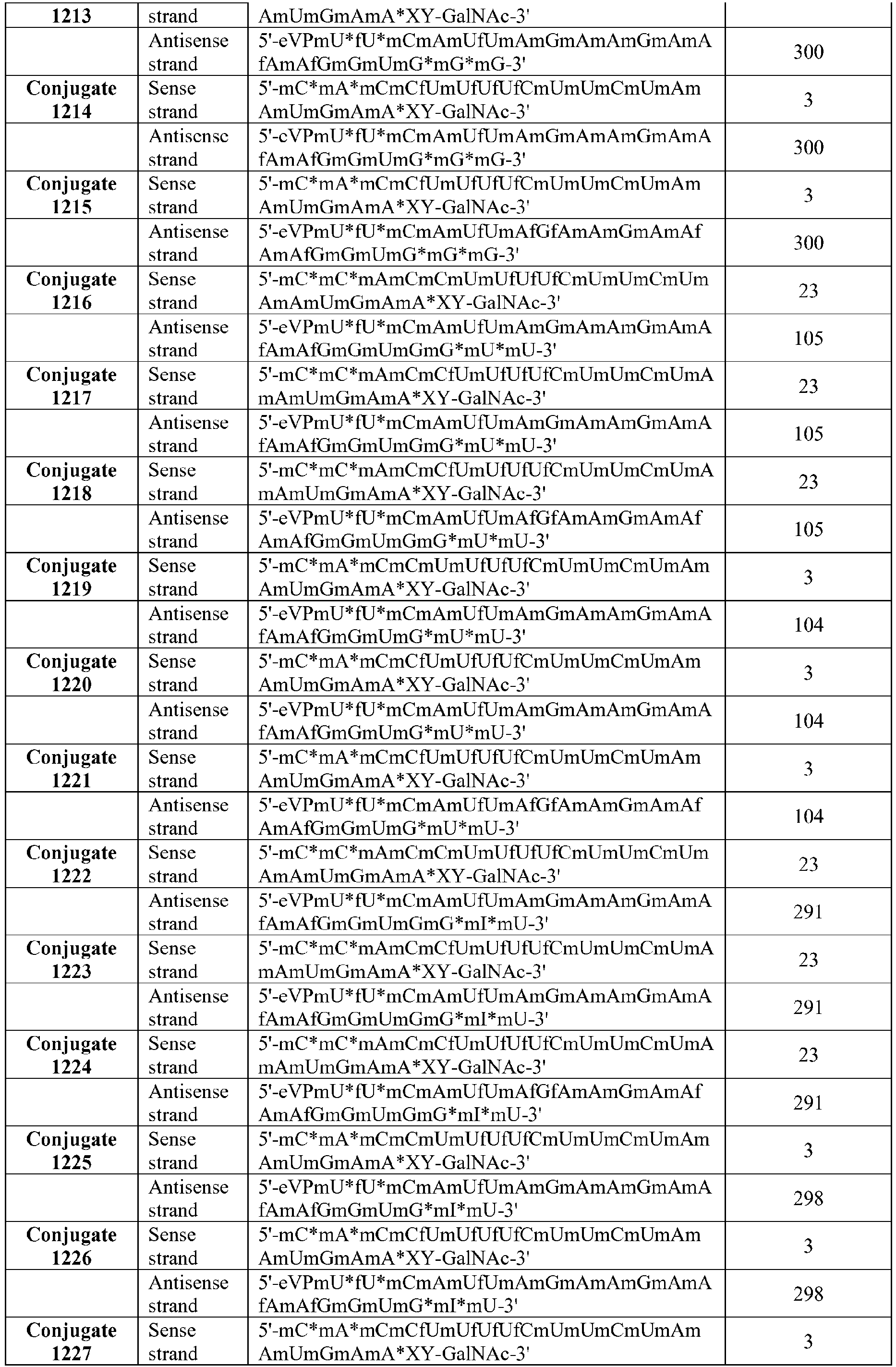

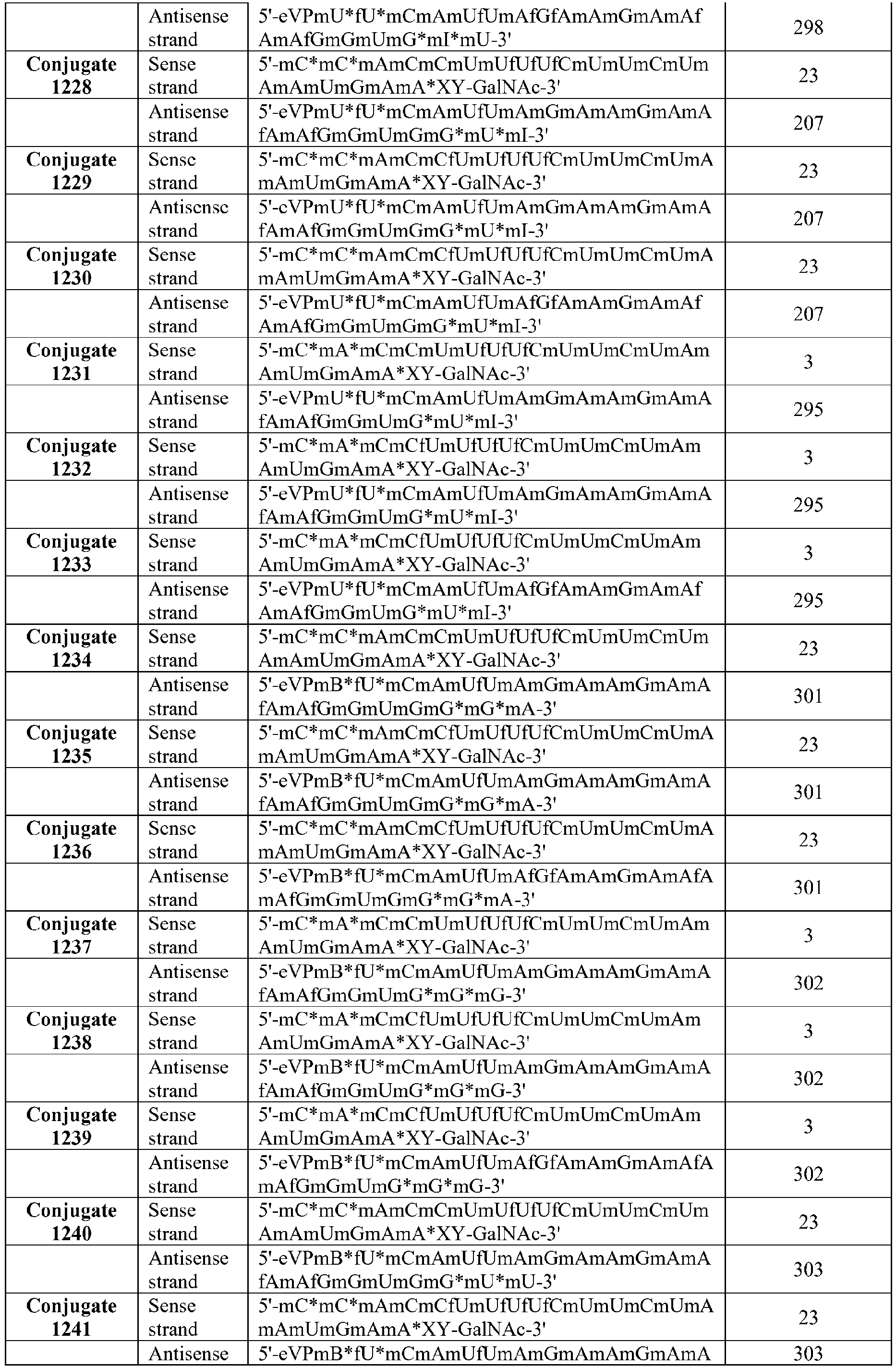

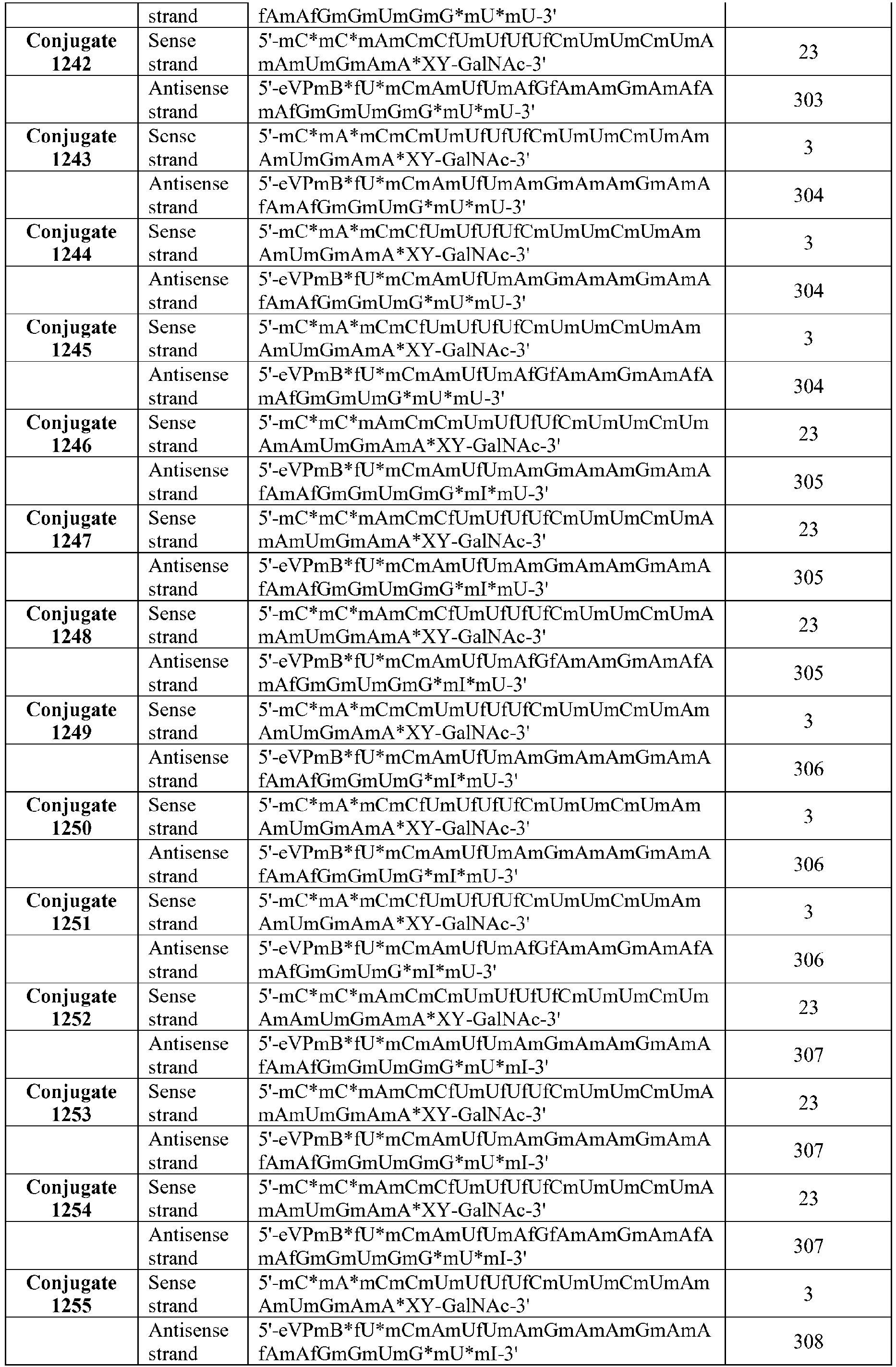

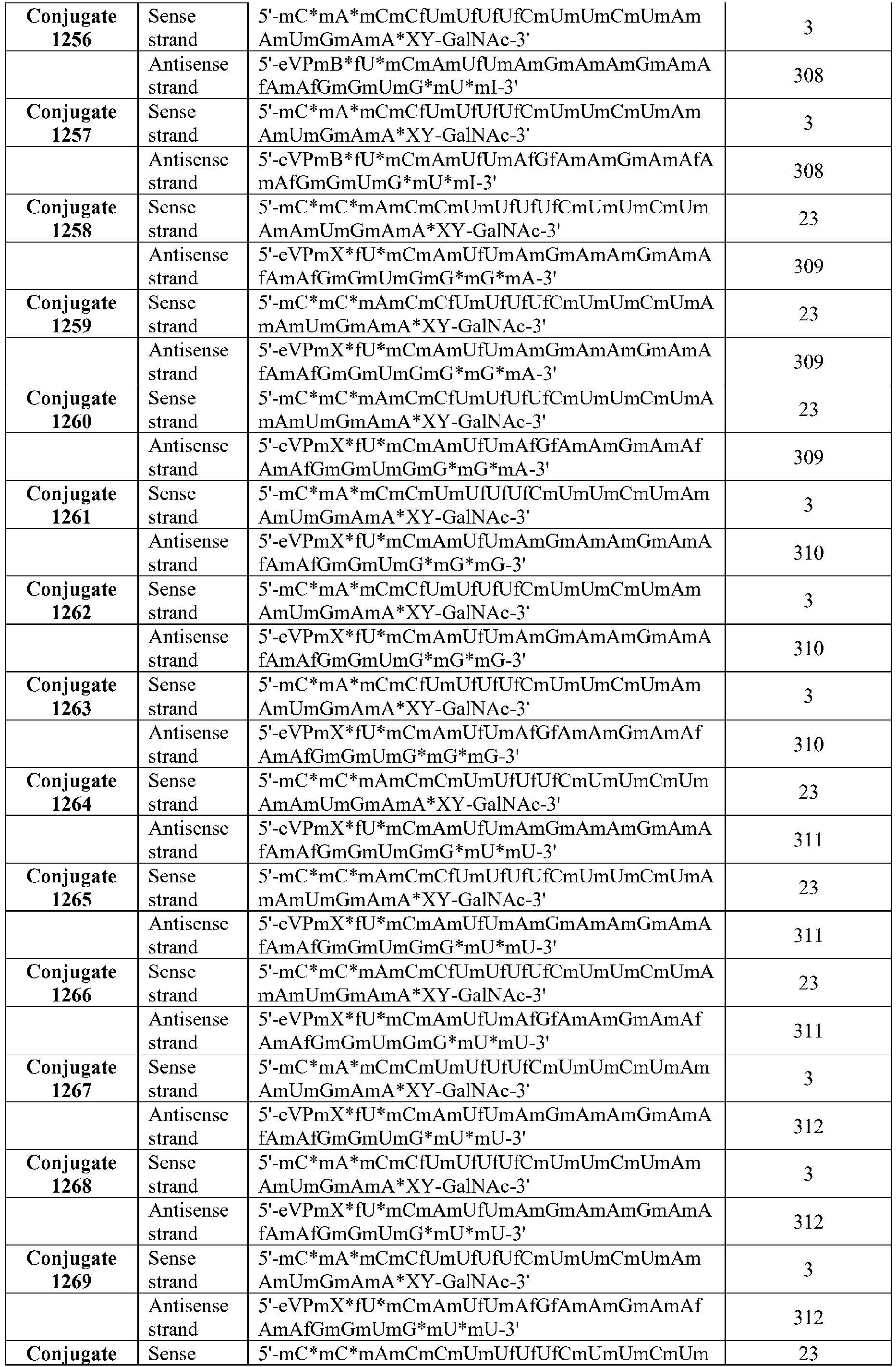

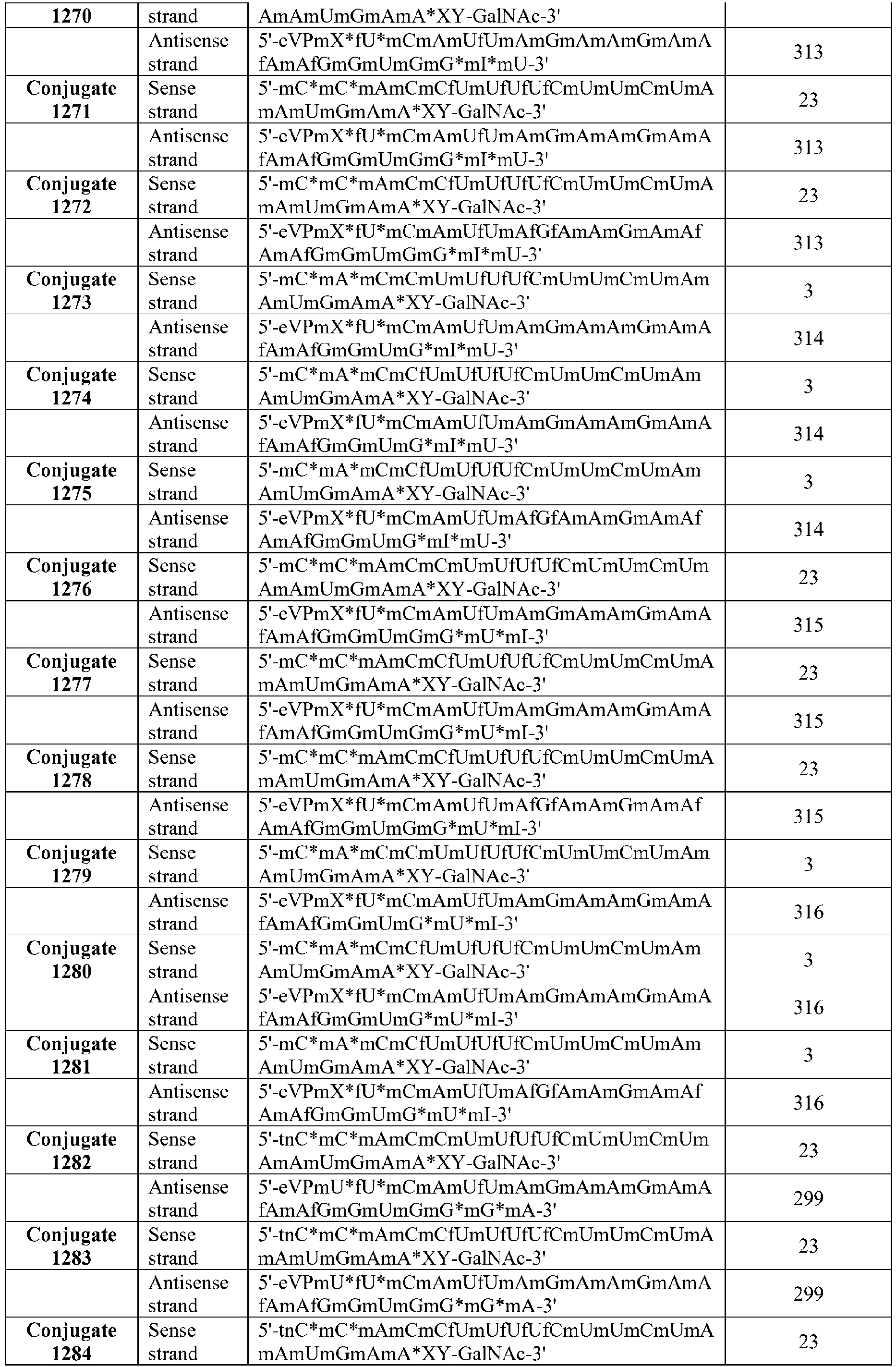

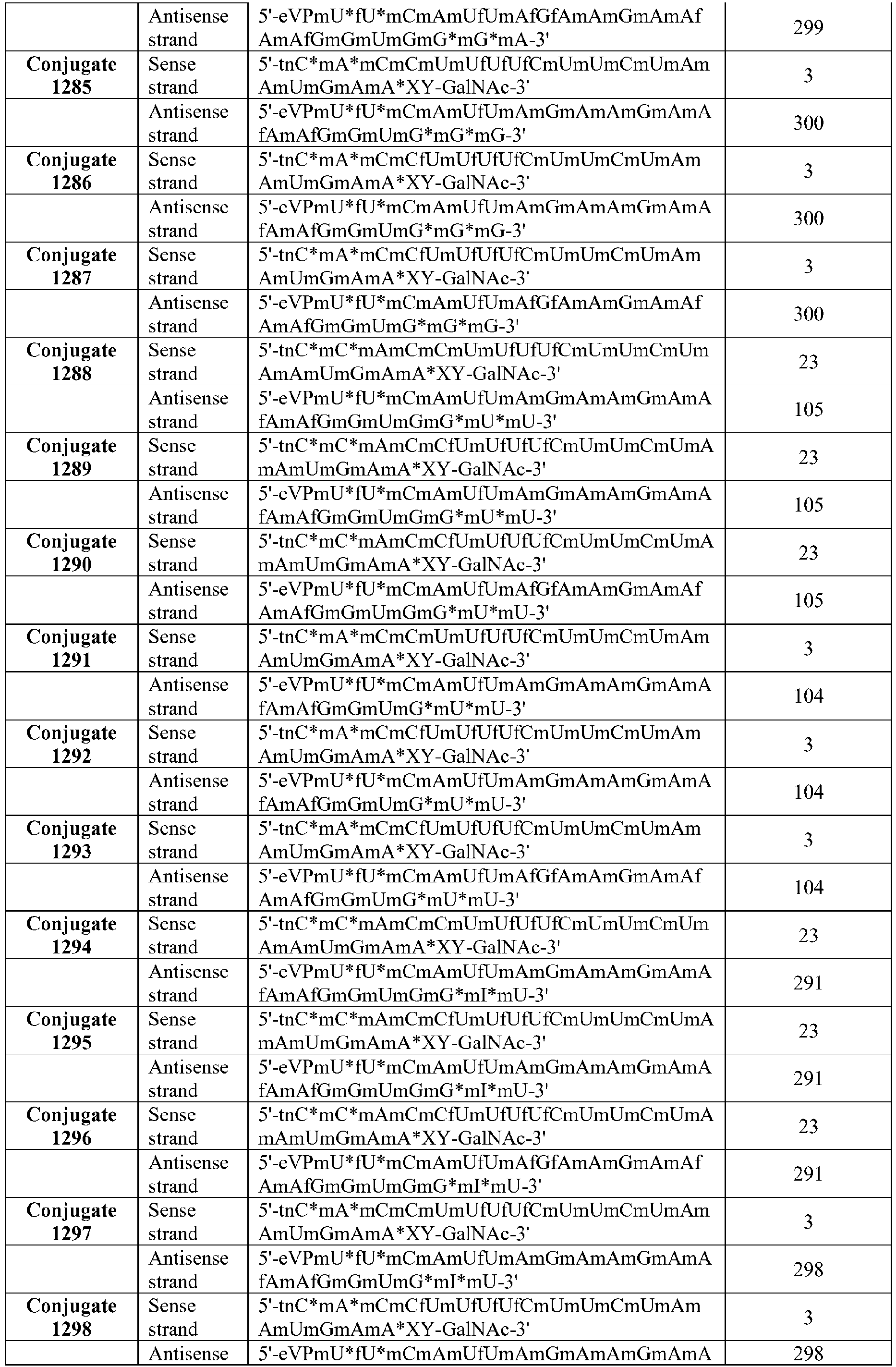

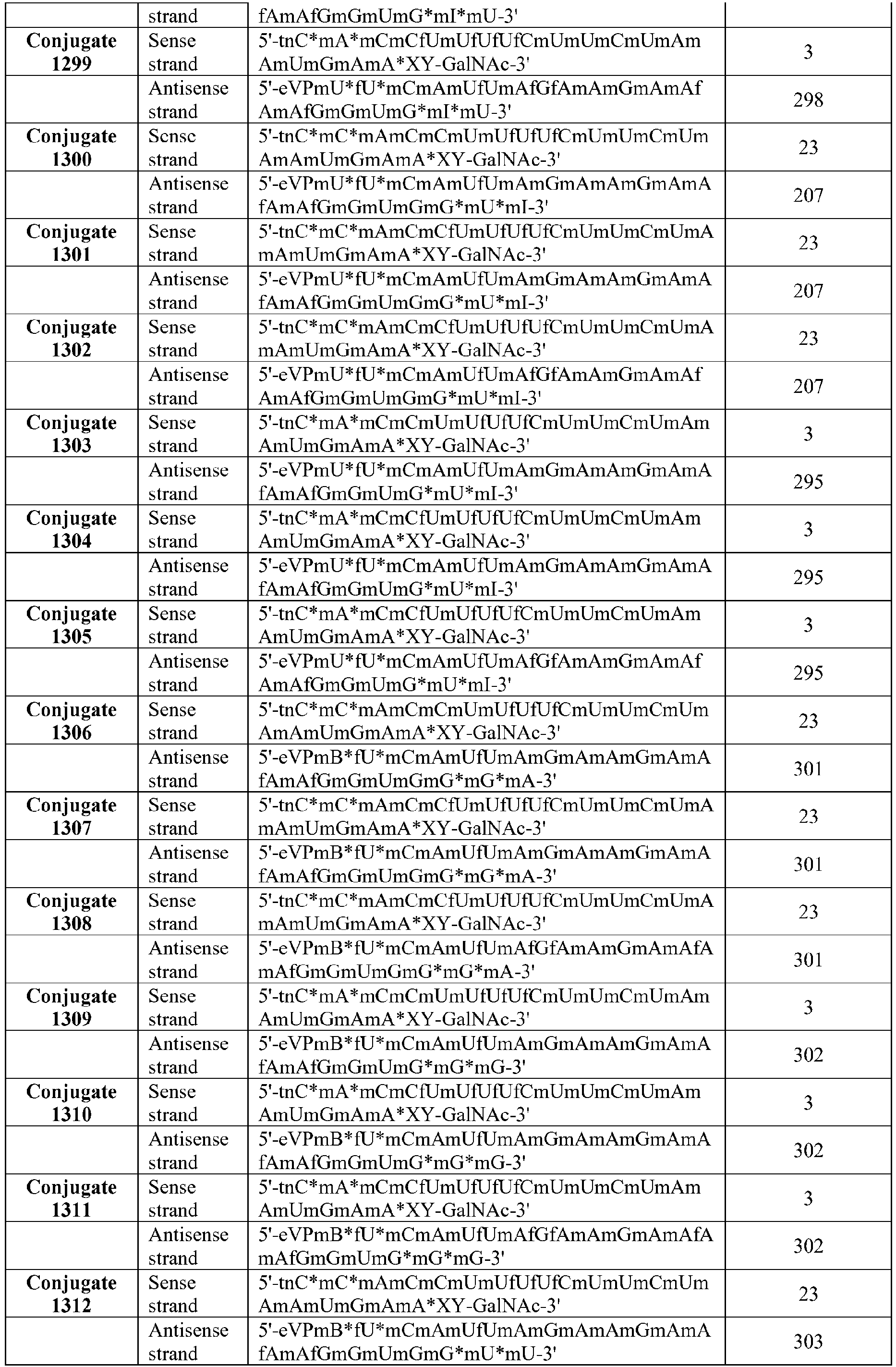

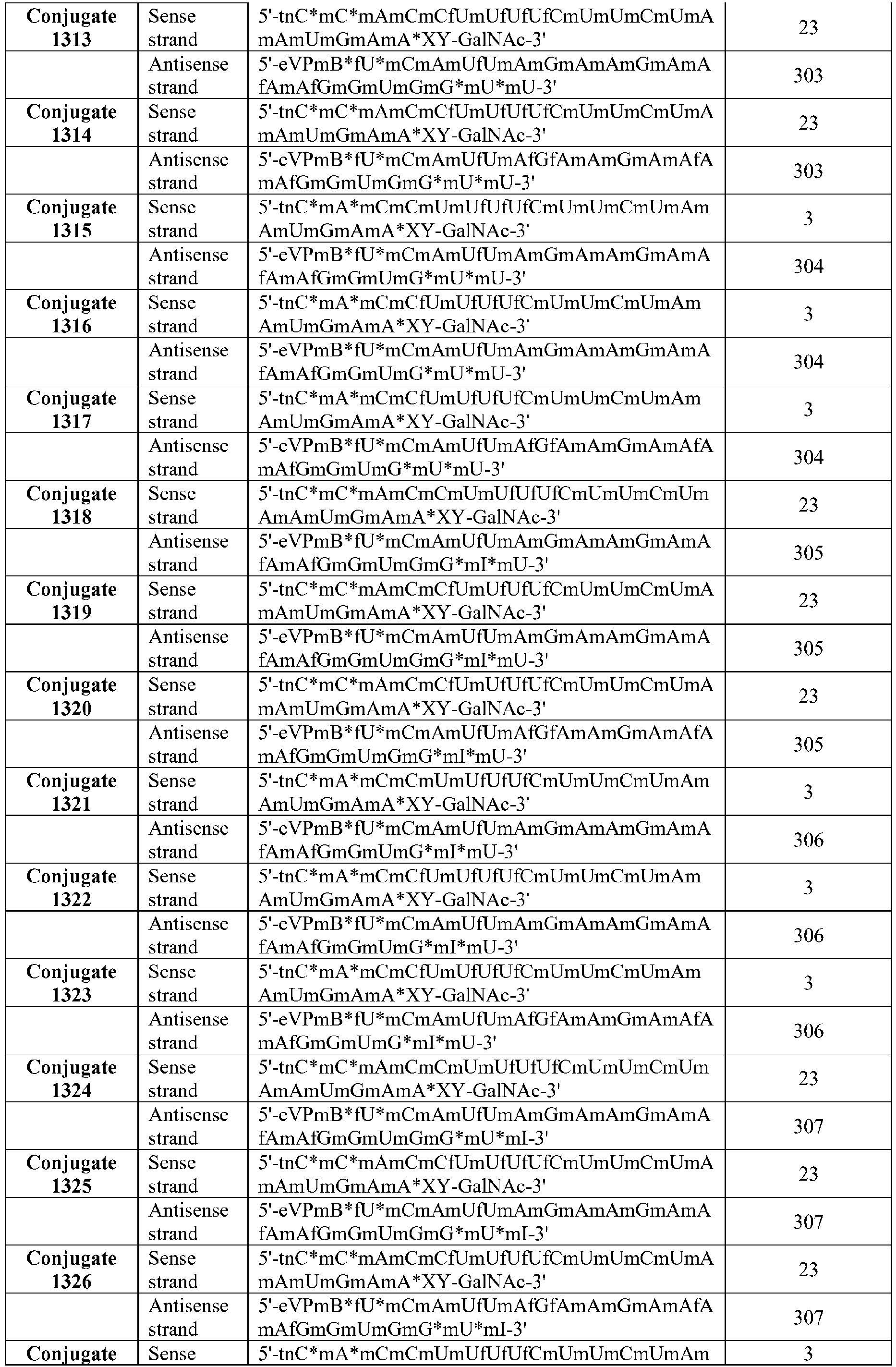

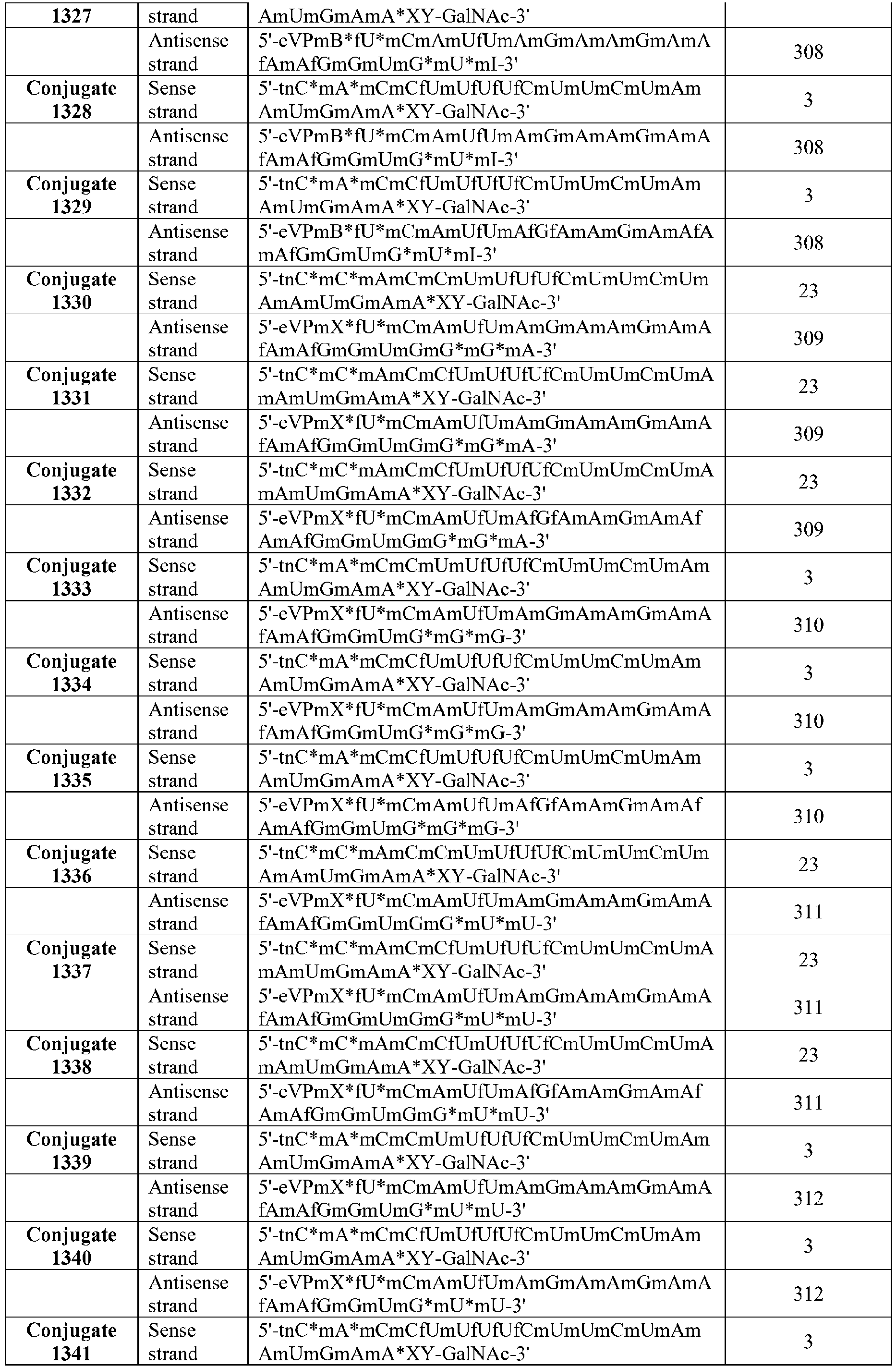

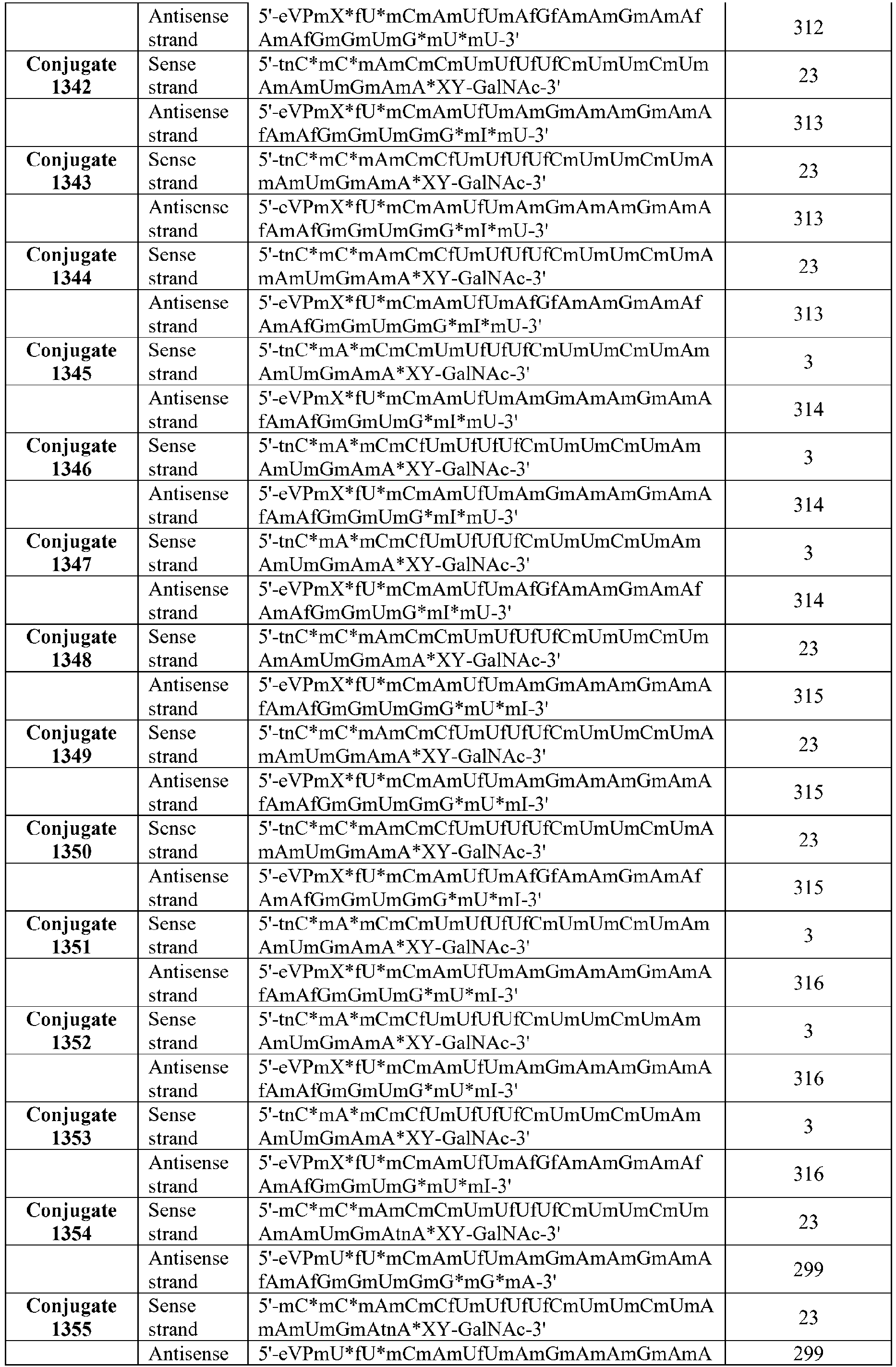

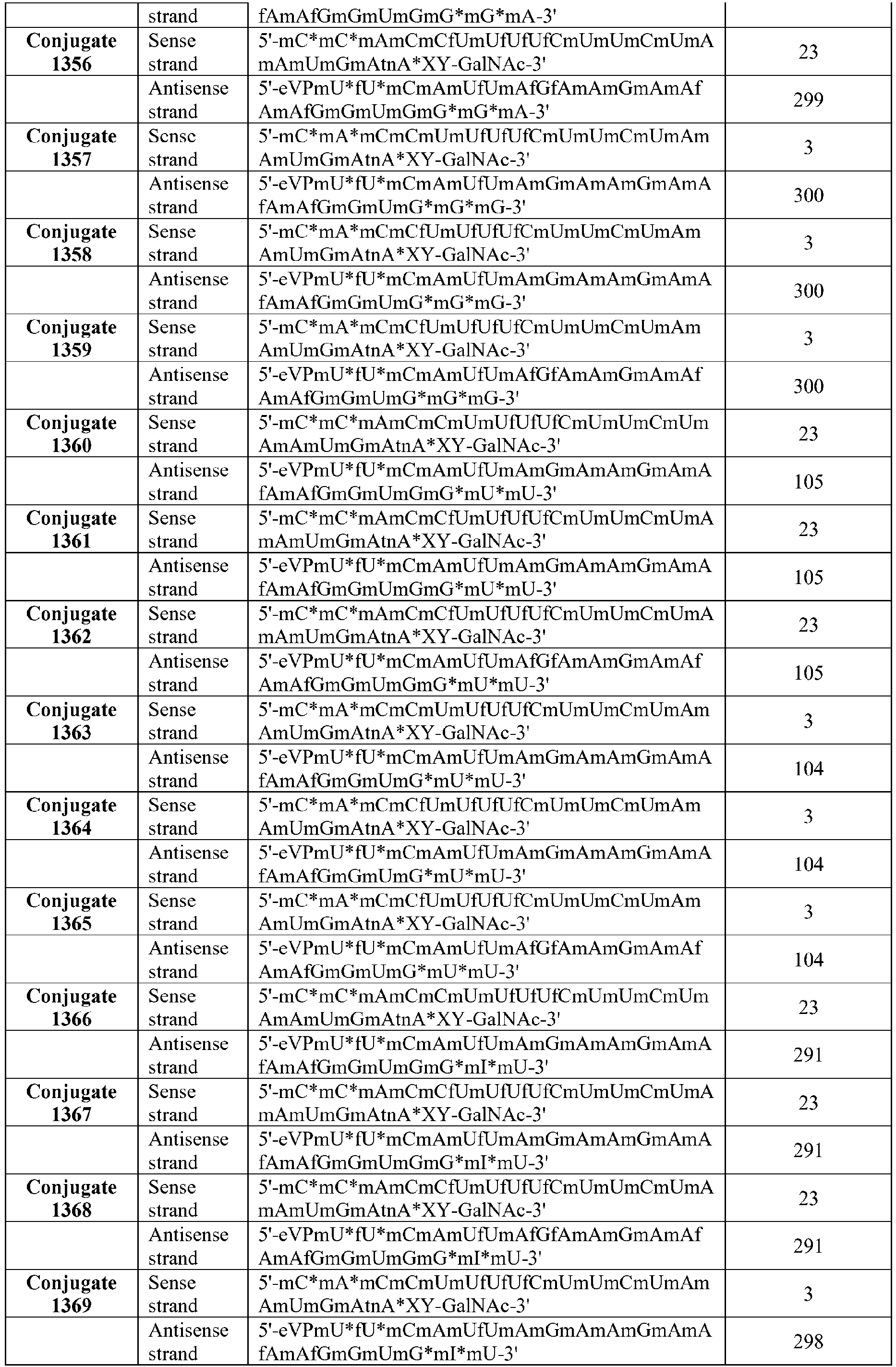

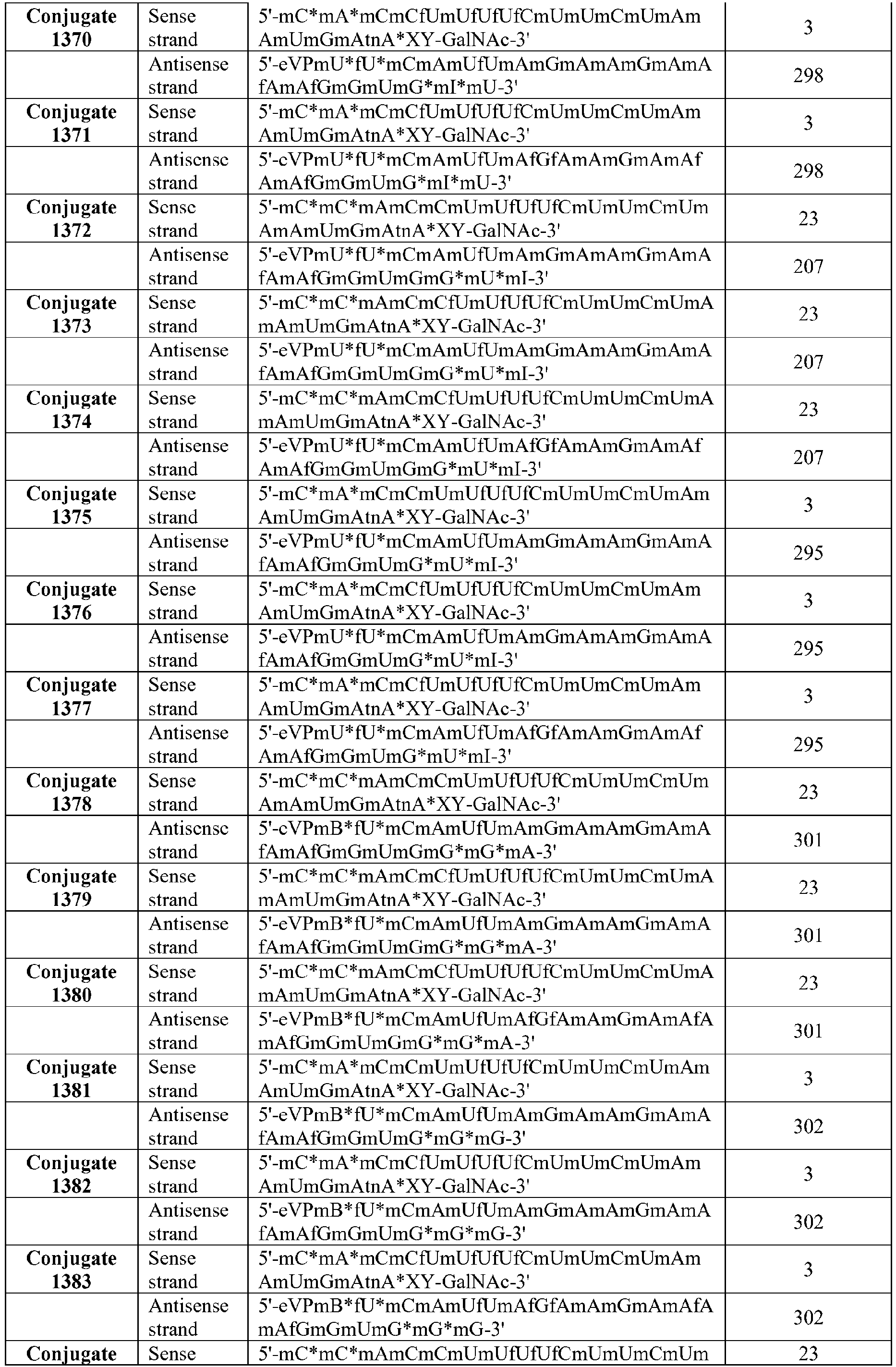

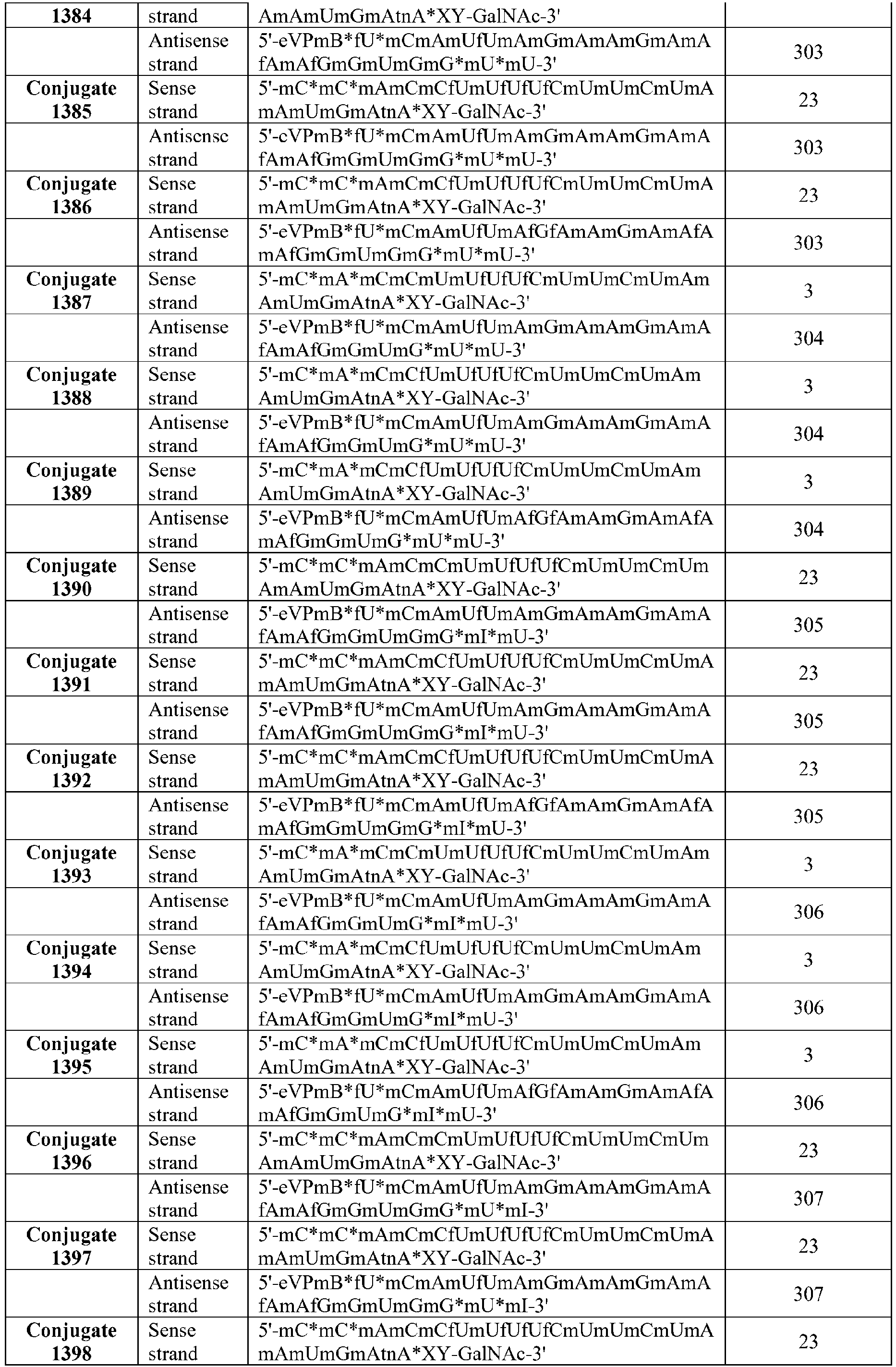

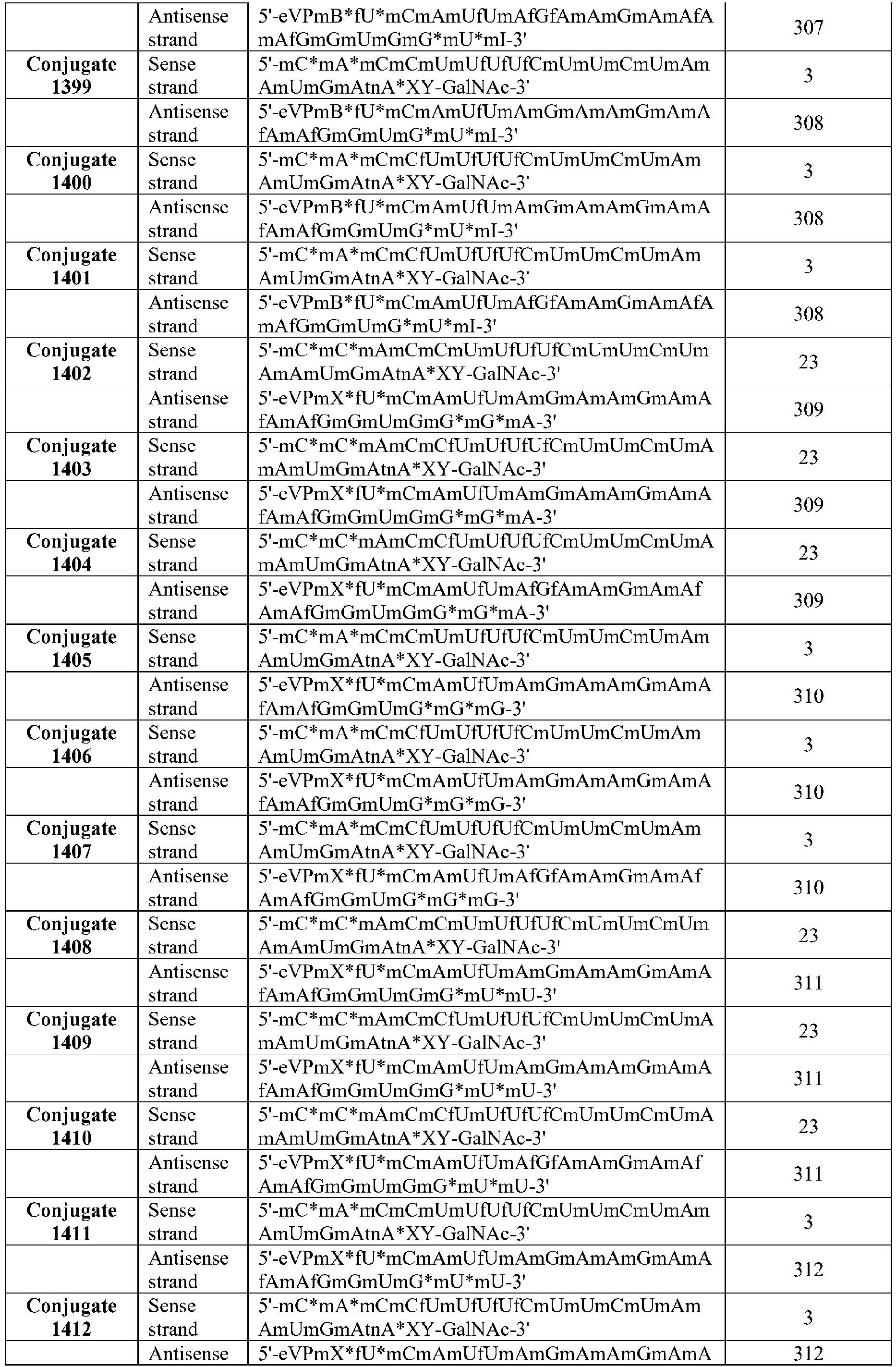

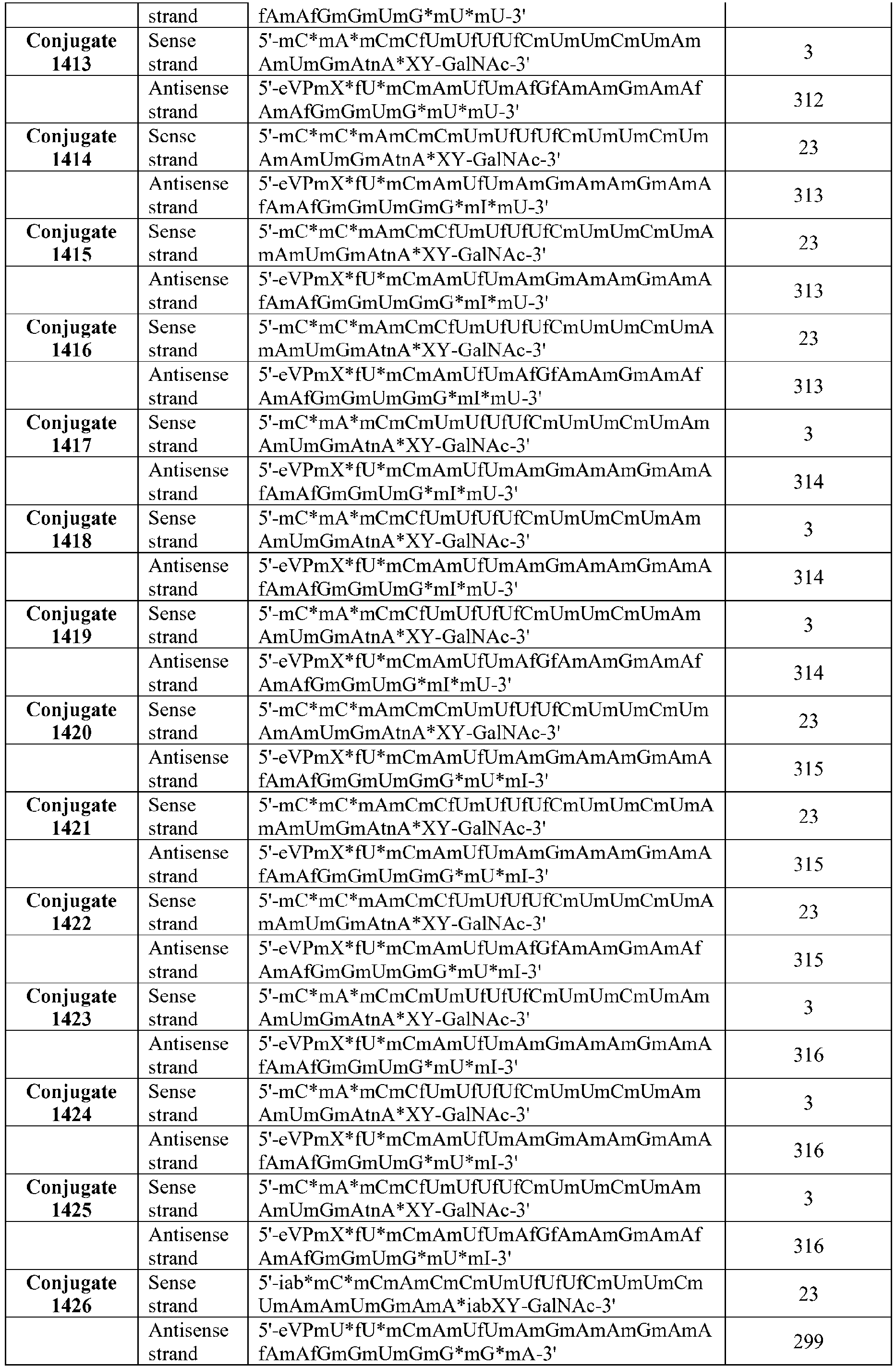

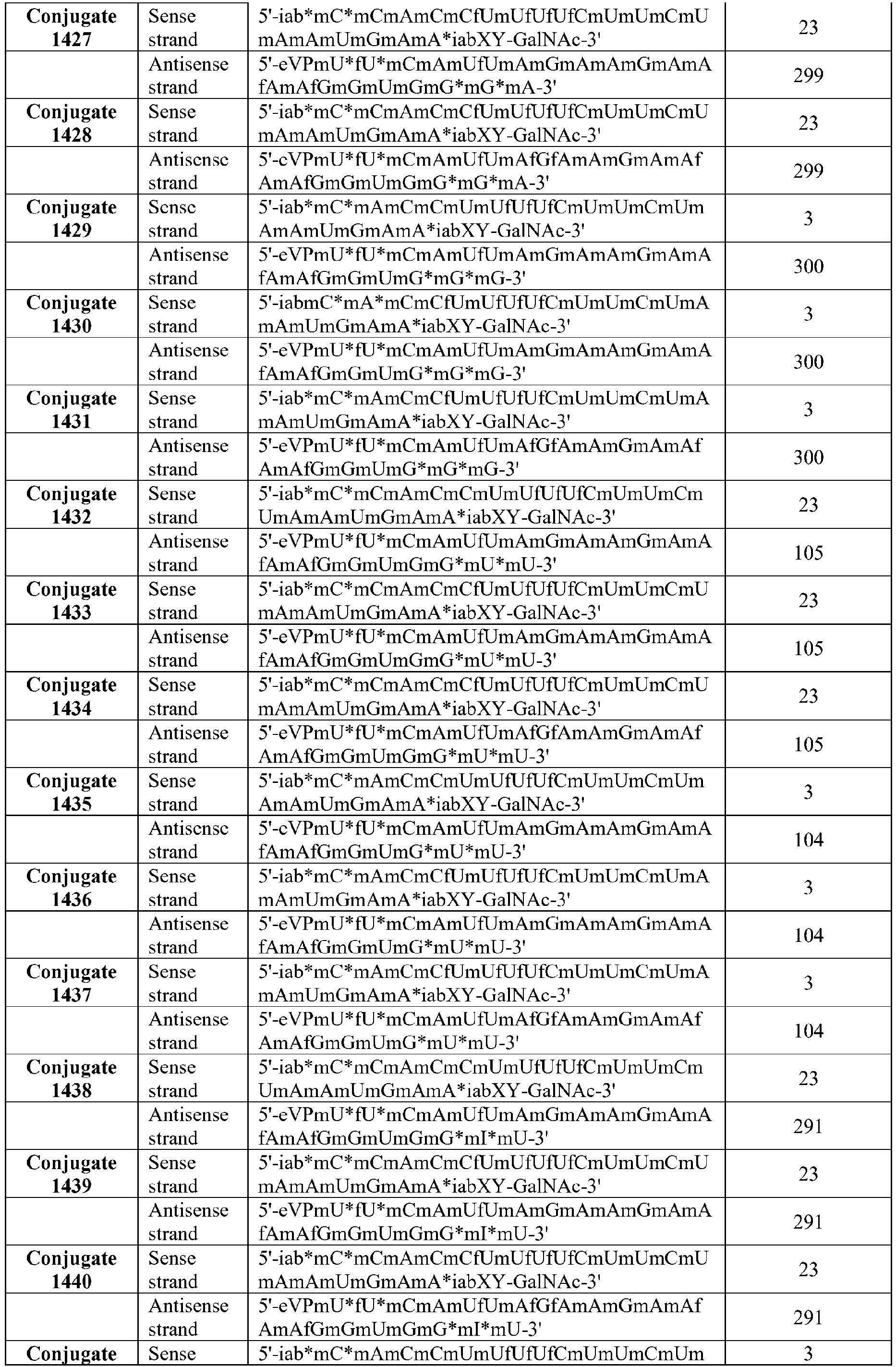

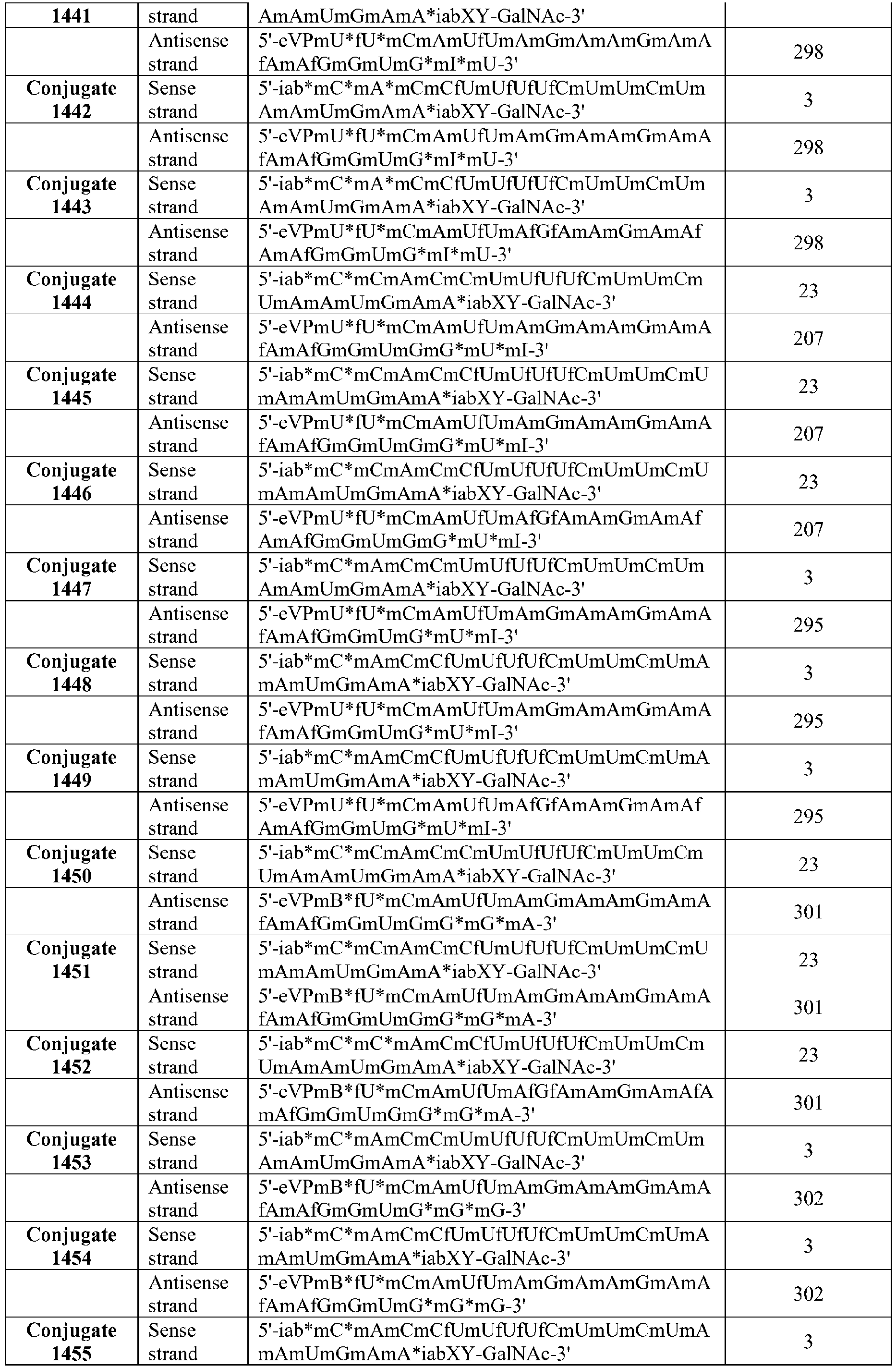

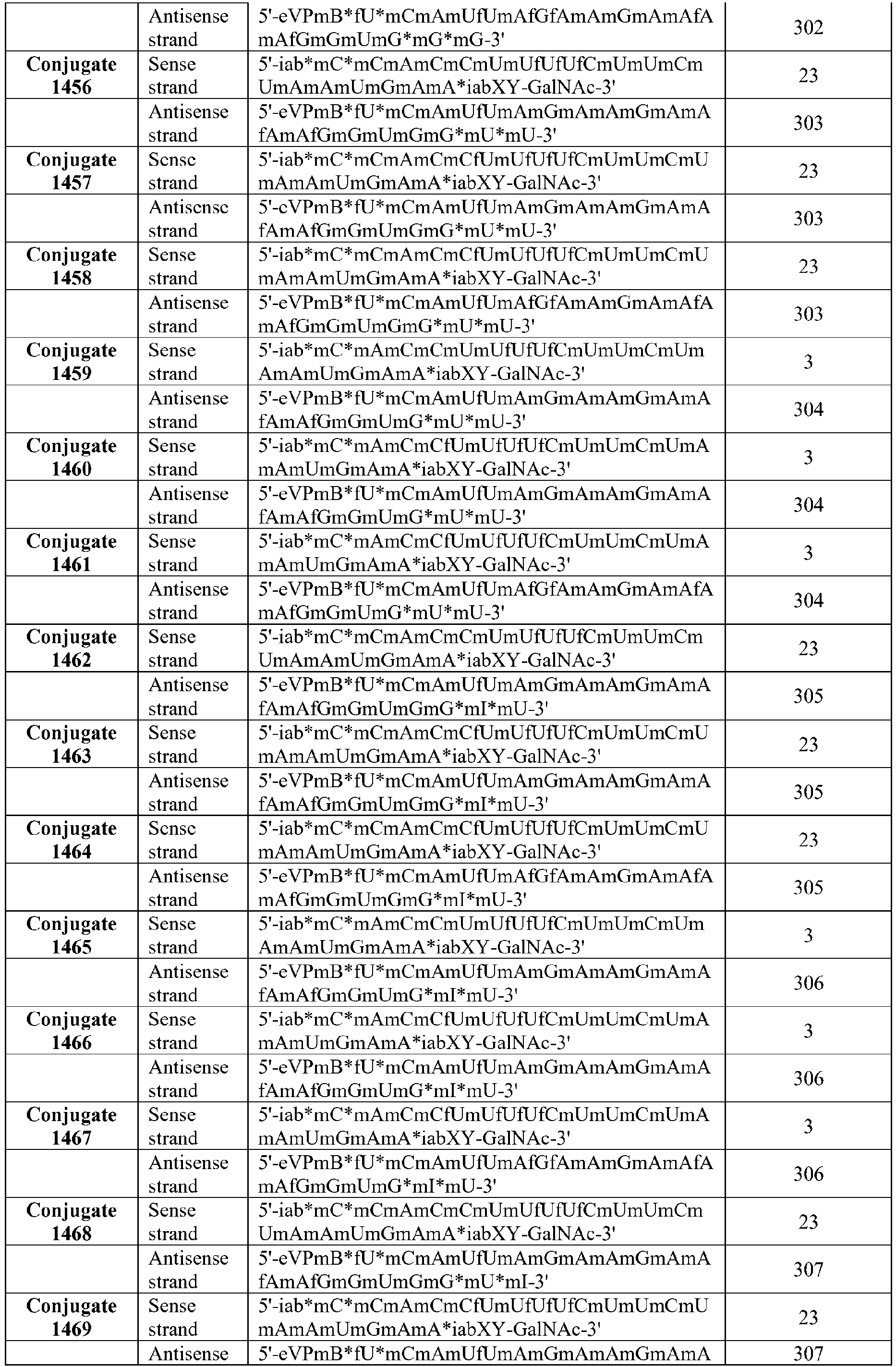

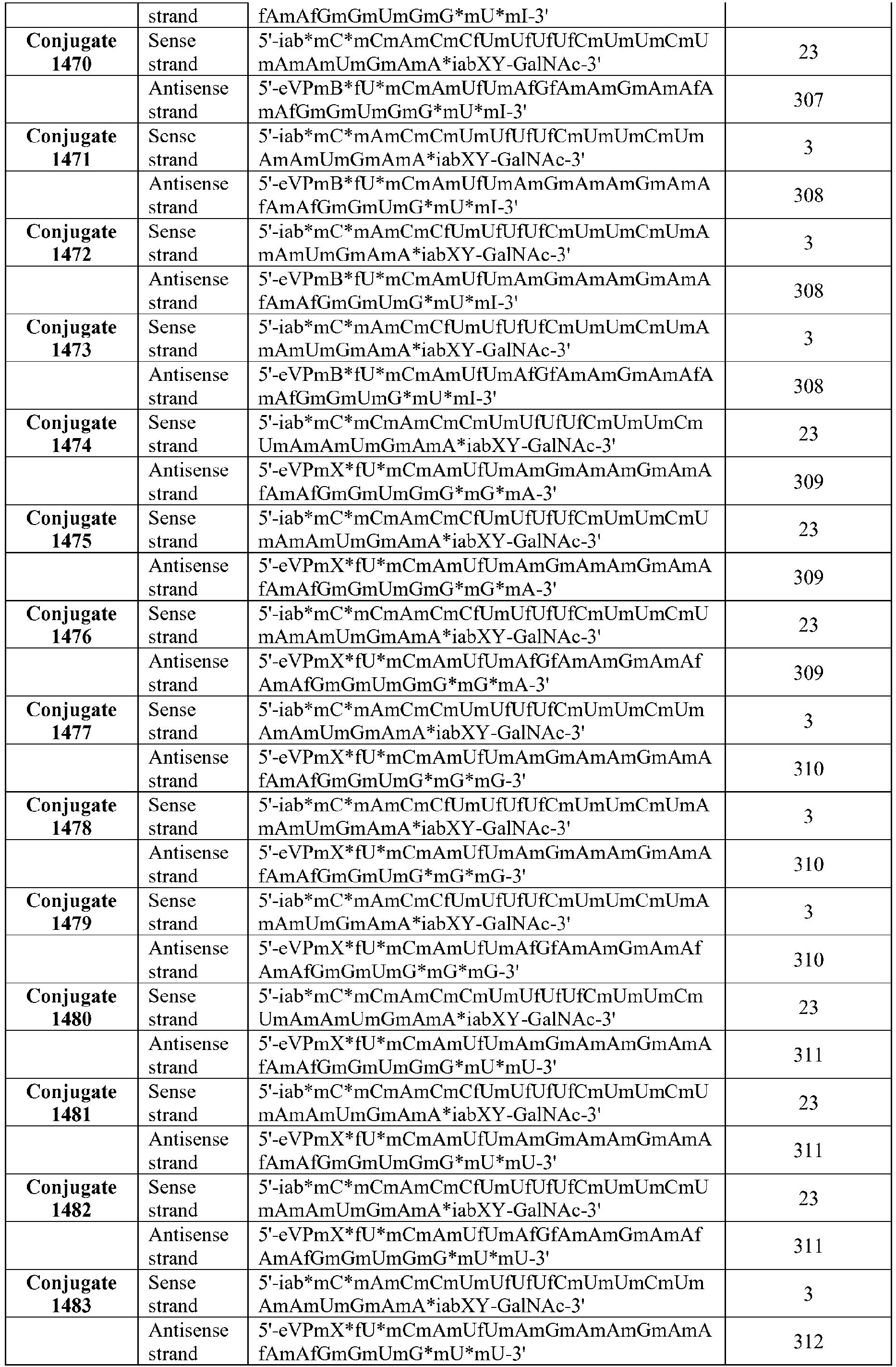

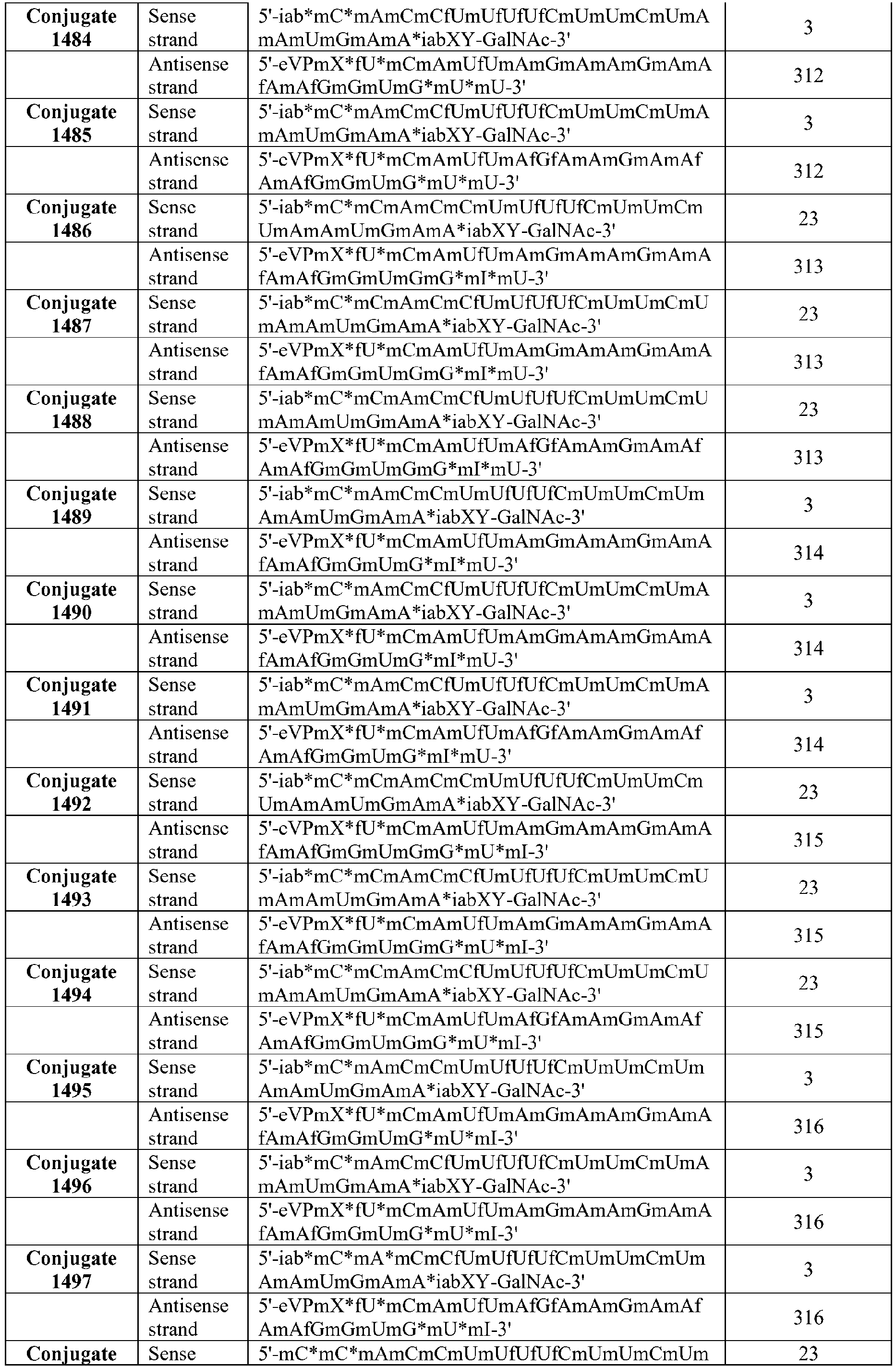

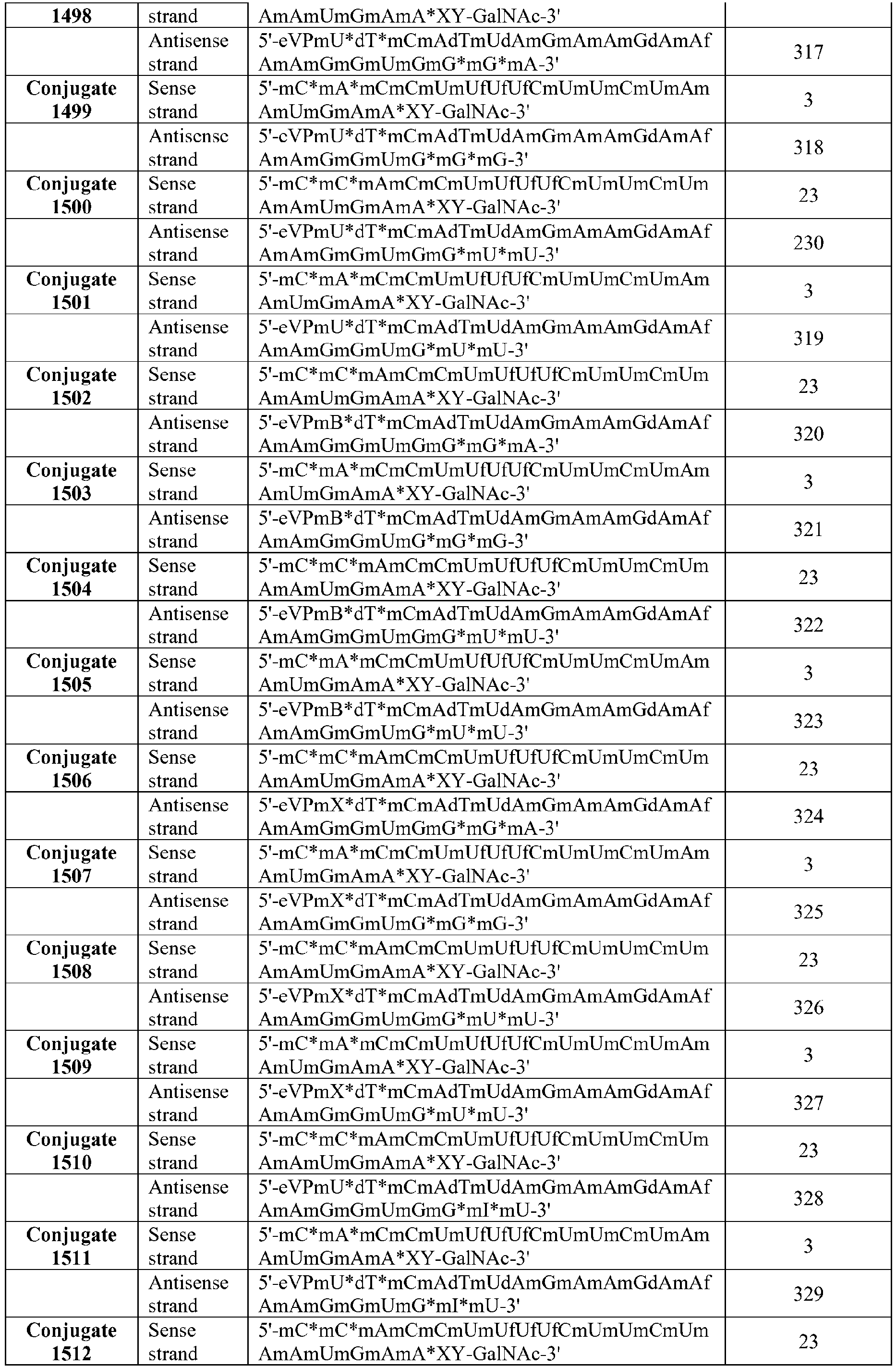

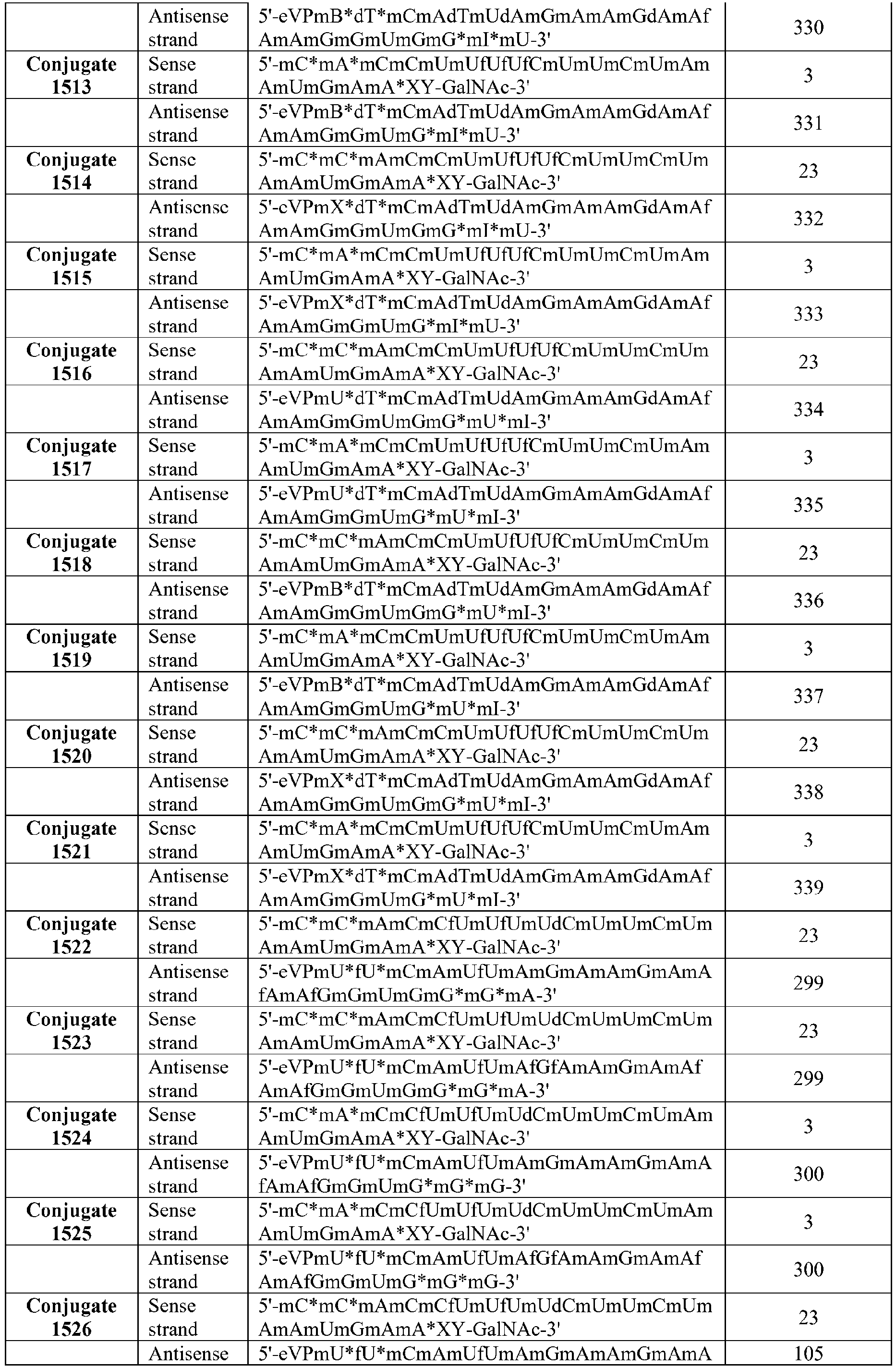

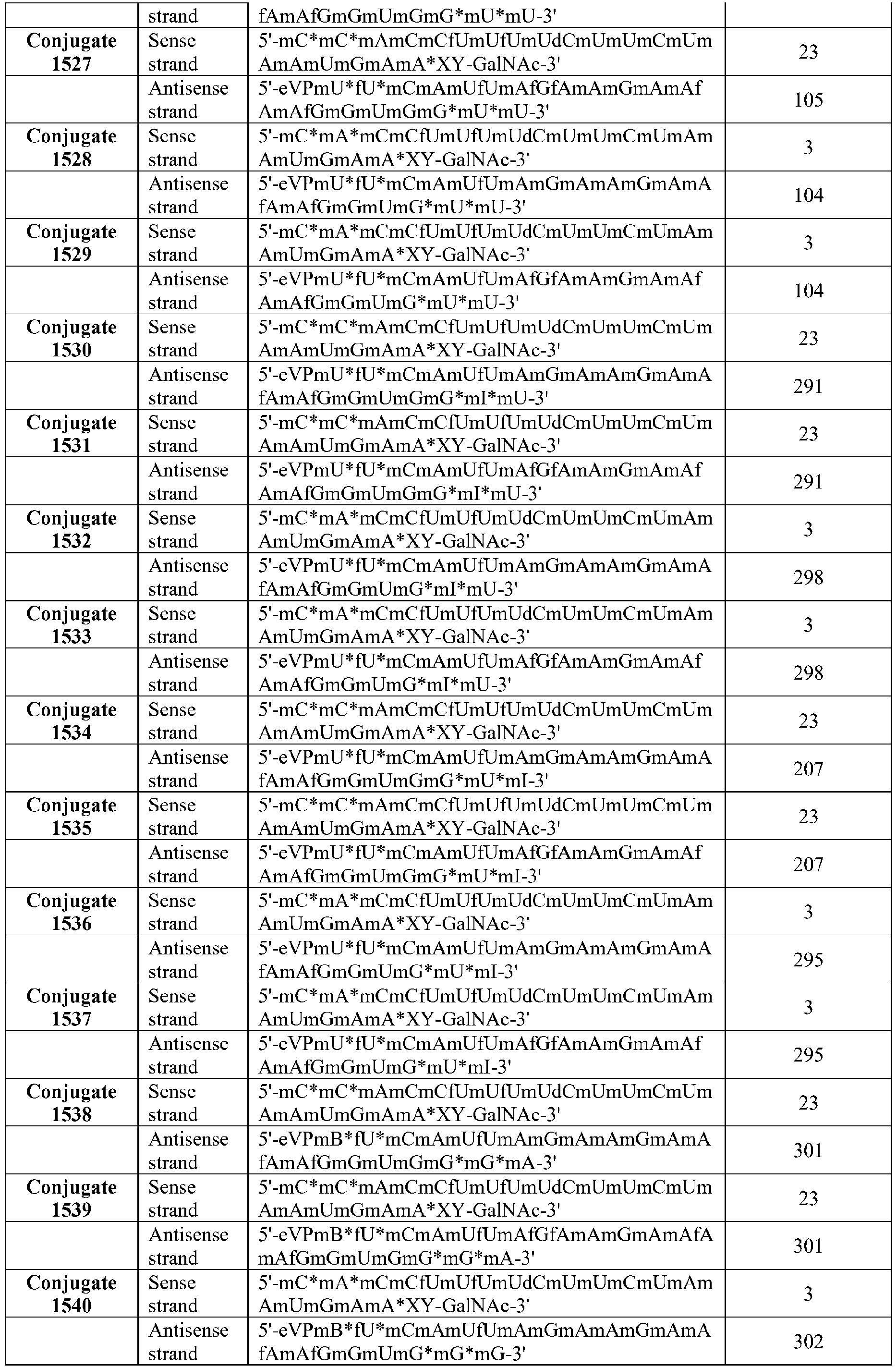

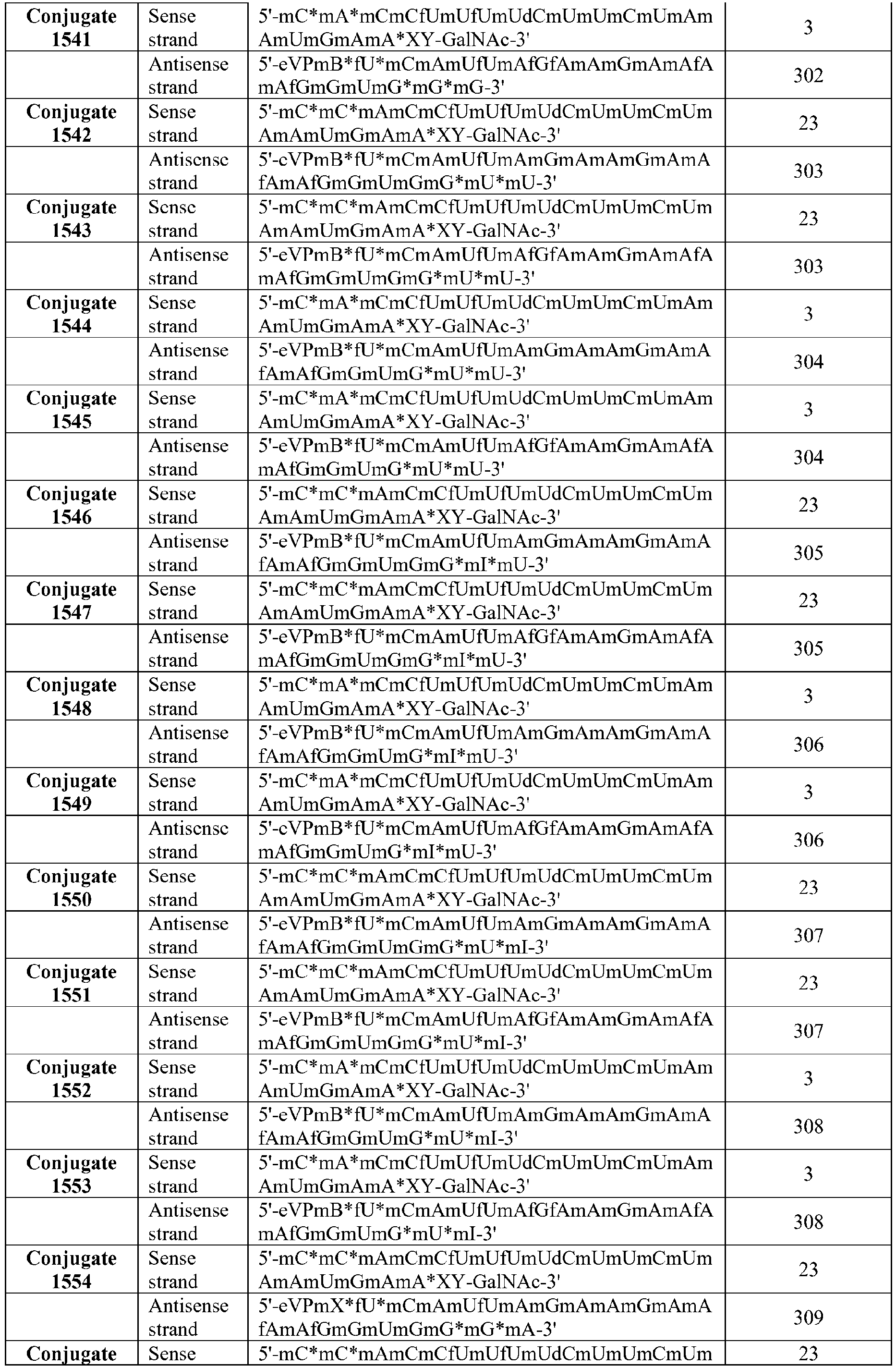

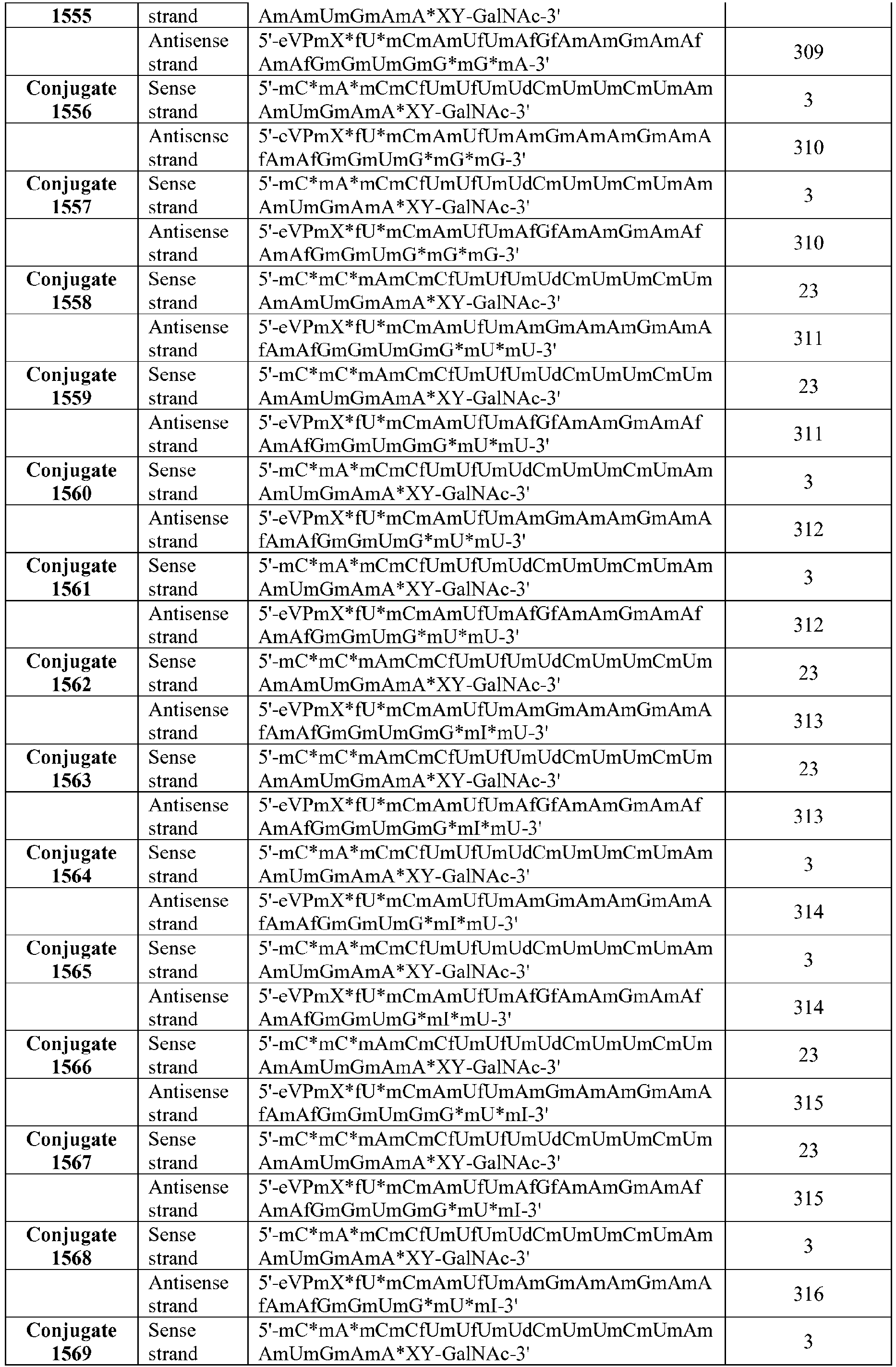

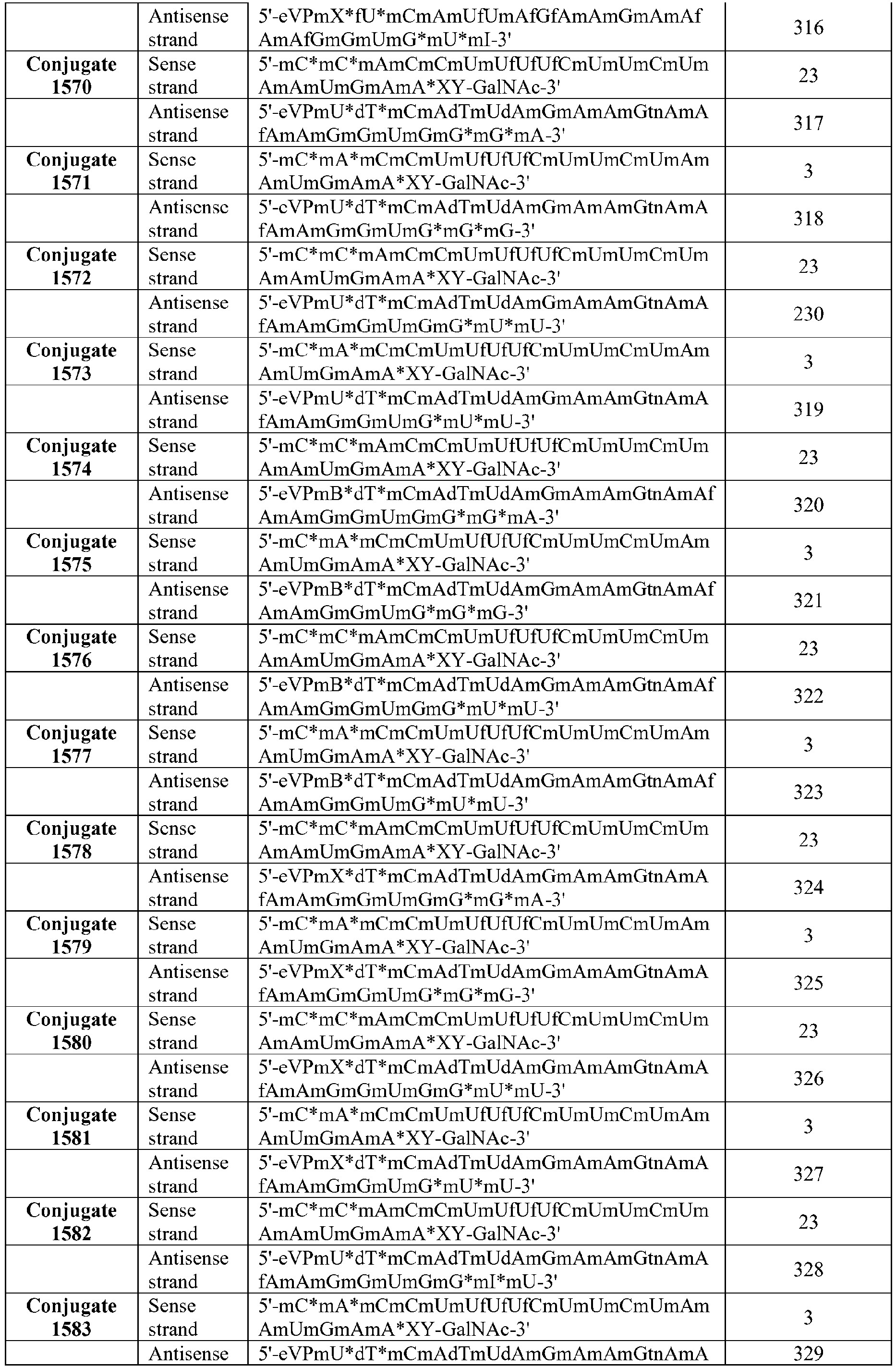

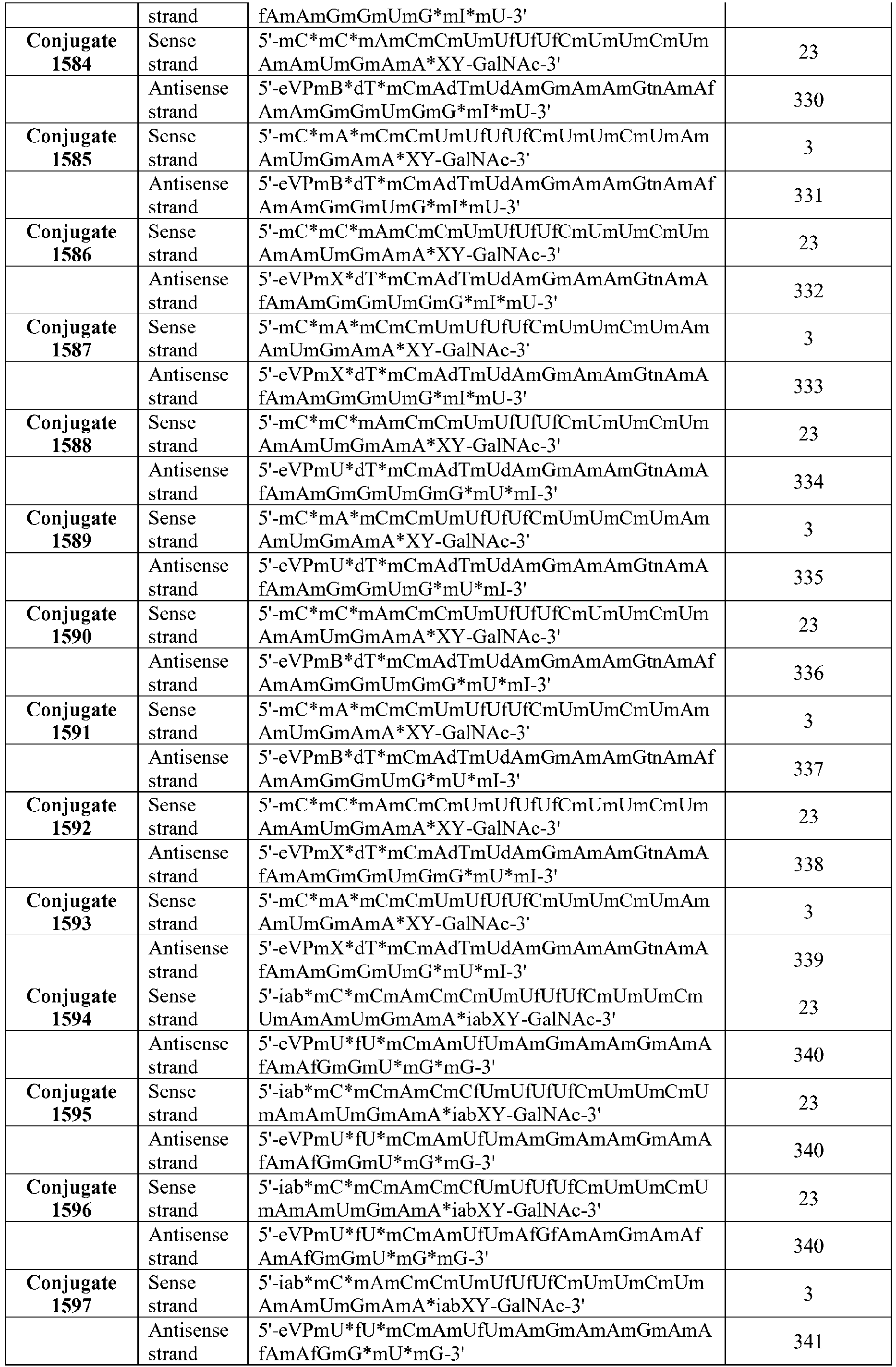

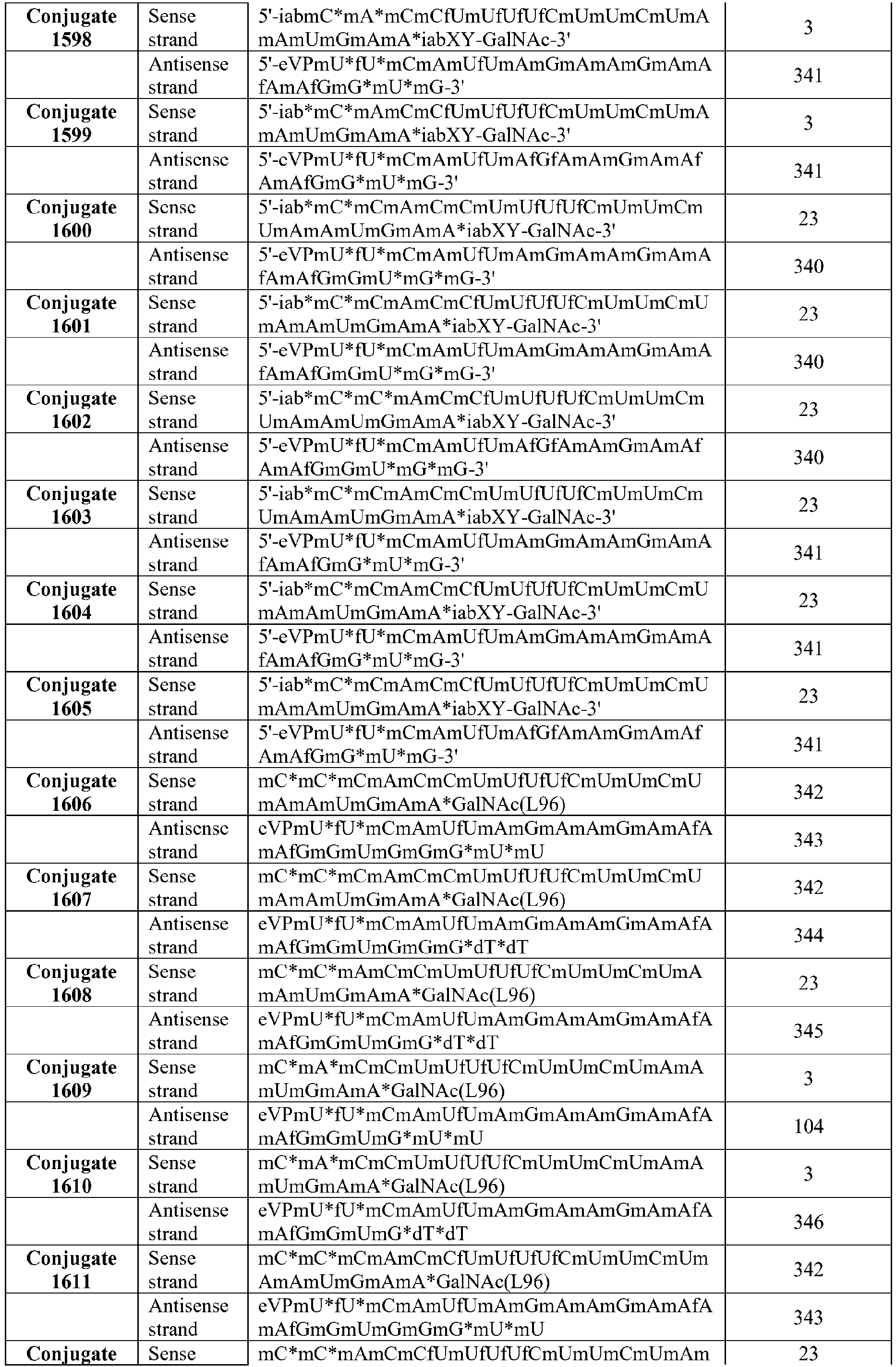

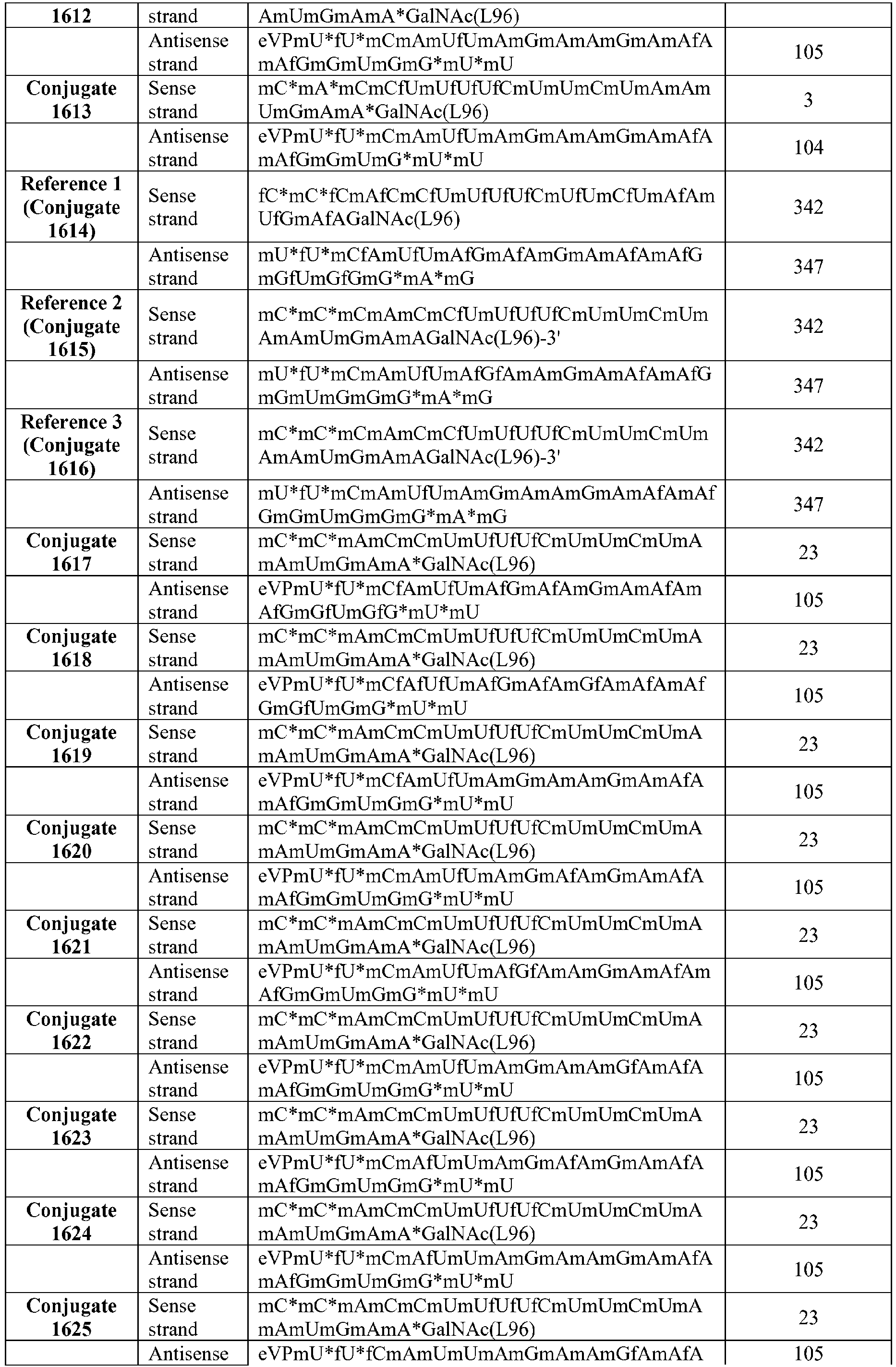

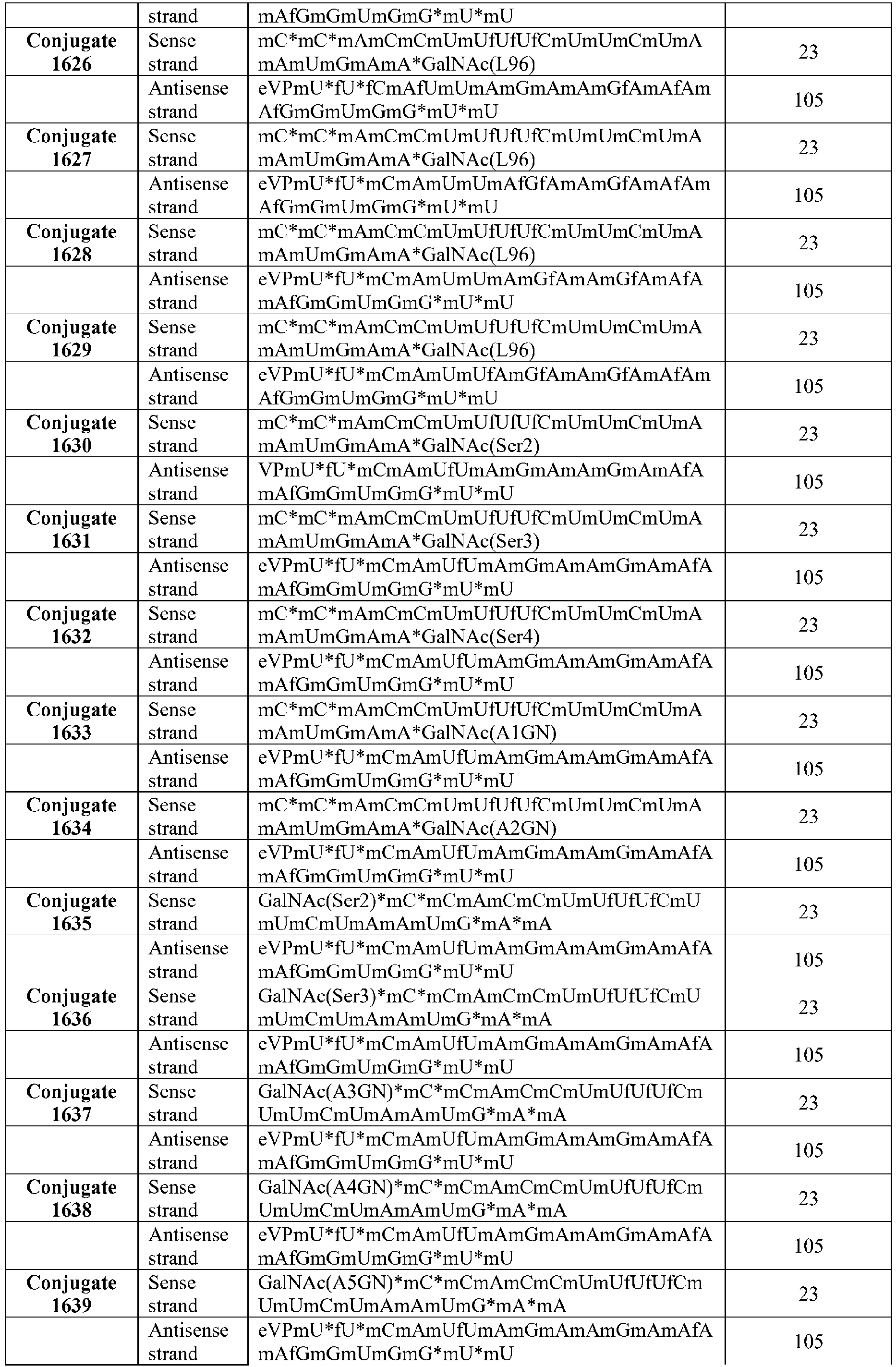

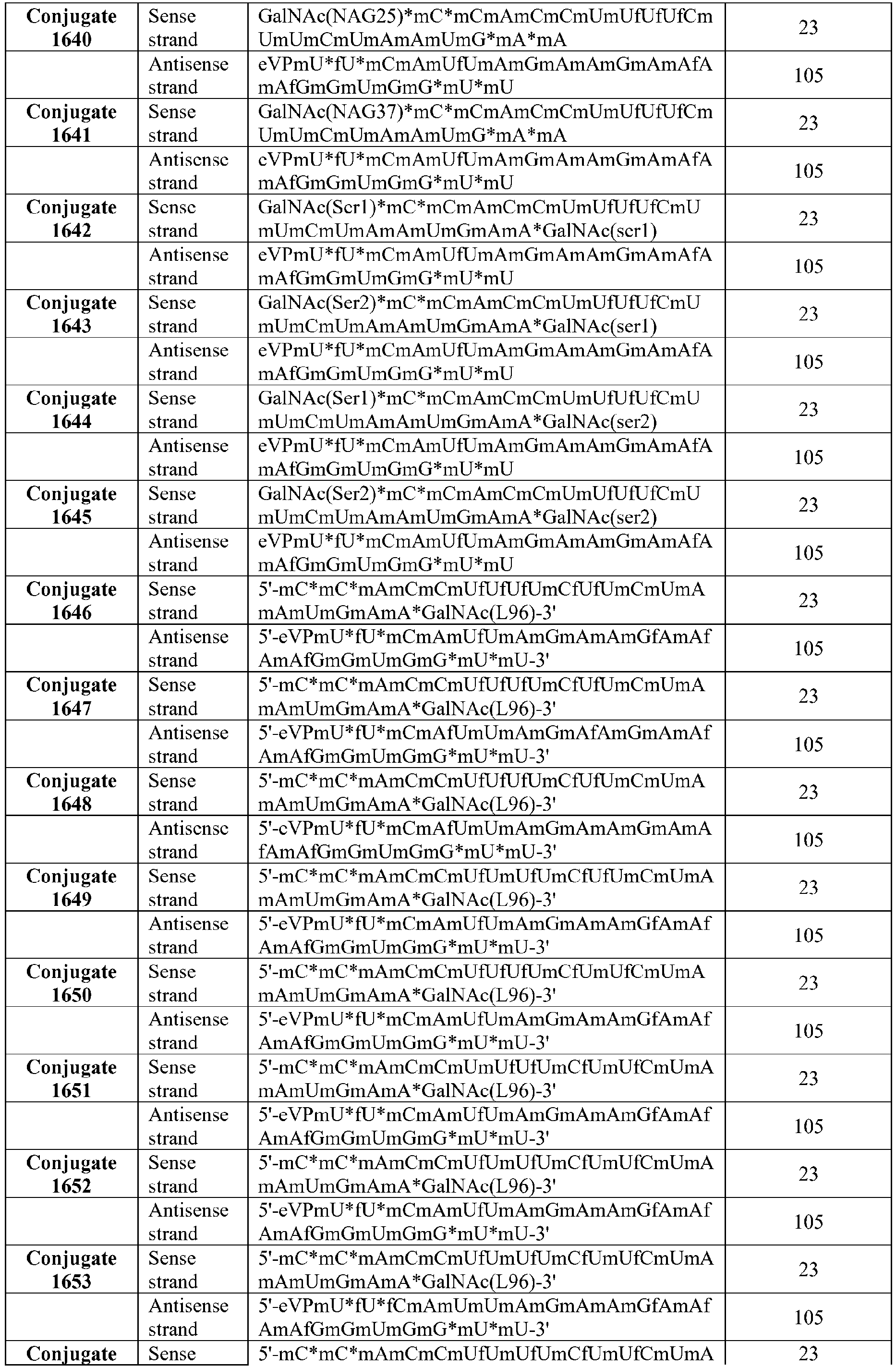

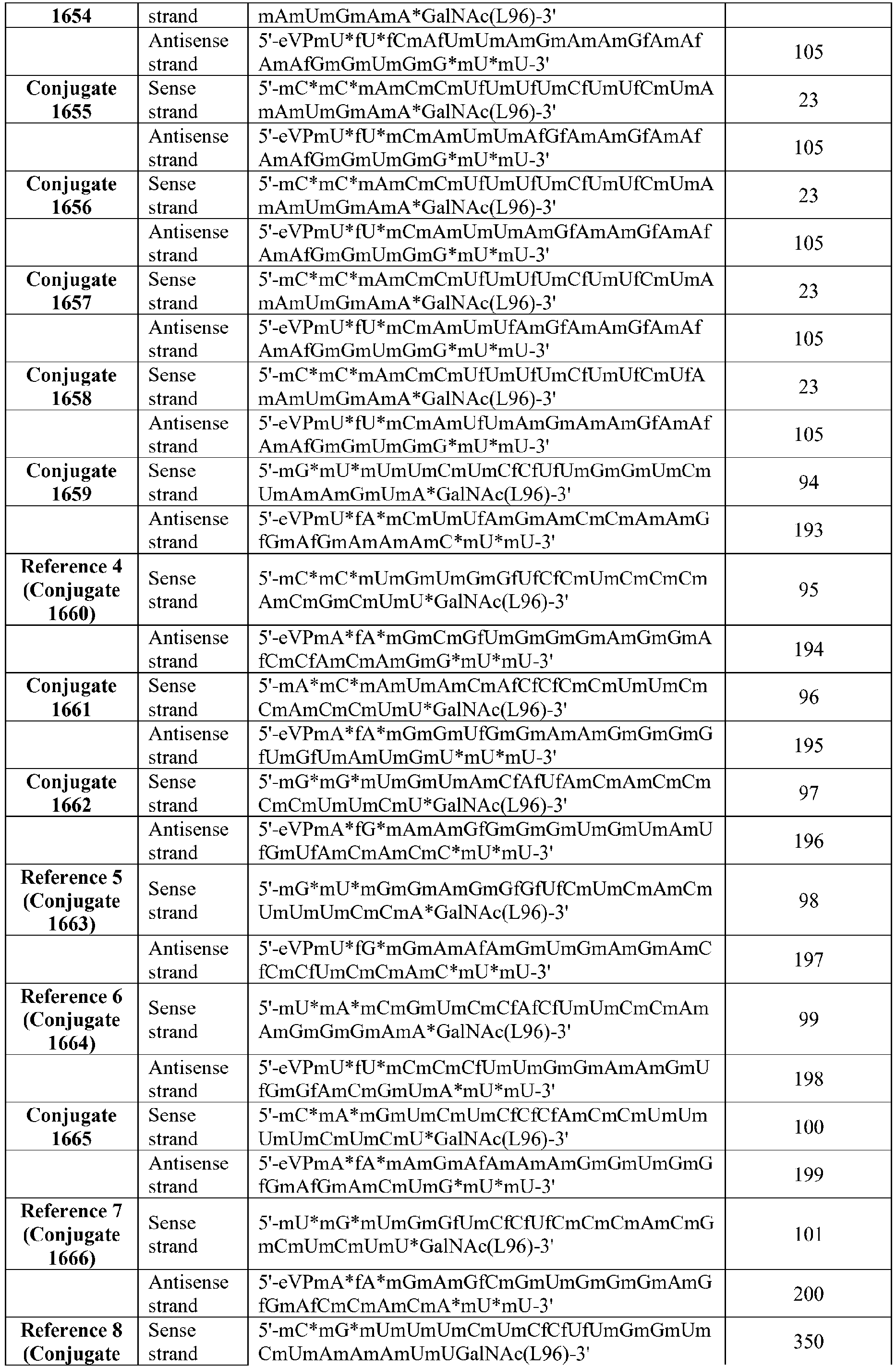

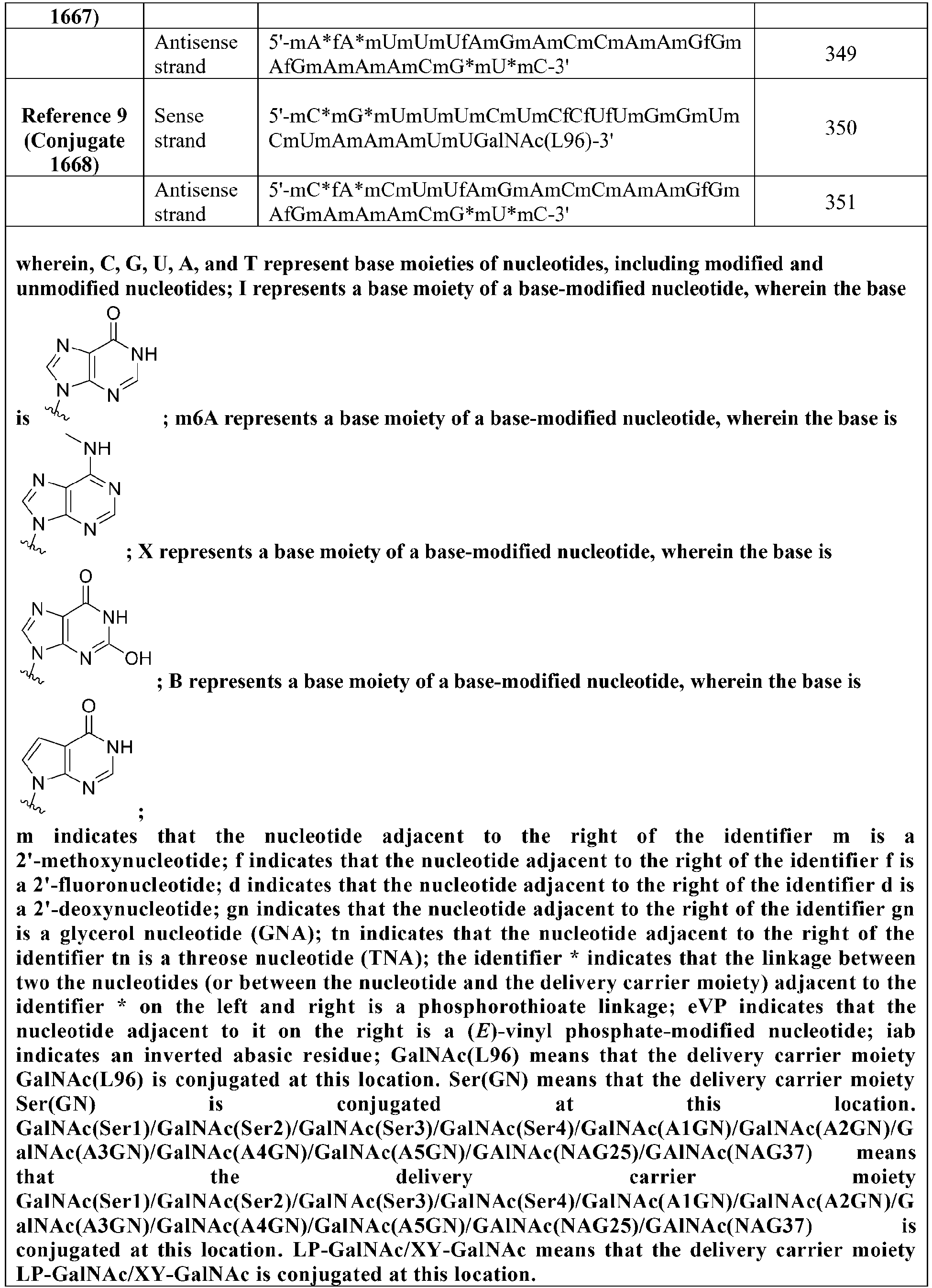

The present invention also provides a pharmaceutical composition, which comprises any one of the above-described siRNAs and/or any one of the above-described siRNA conjugates and a pharmaceutically acceptable carrier.

In some embodiments of the present invention, the pharmaceutical composition comprises one kind of siRNA as described in the first aspect. In other embodiments of the present disclosure, the pharmaceutical composition comprises at least two (for example, but not limited to, 2, 3, 4, 5, 6, 7, 8, 9, 10 or more) kinds of the siRNAs as described in the first aspect as active ingredients. Preferably, the at least two kinds of the siRNAs as described in the first aspect each target different target sequences in the AGT gene, thereby expecting a synergistic effect by acting simultaneously on different target sequences. Here, "different target sequences" means that there is no overlap between target sequences, or the number of overlapping consecutive nucleotides between target sequences is less than 5 (e.g., the number of overlapping consecutive nucleotides is 4, 3, 2, 1, or 0). In this case, the at least two kinds of the siRNAs as described in the first aspect can be present in any different proportions. Preferably, the at least two kinds of the siRNAs as described in the first aspect can be present in a molar ratio of 1:100 to 100:1; more preferably, the at least two kinds of the siRNAs as described in the first aspect can be present in a molar ratio of 1:10 to 10:1, 1:5 to 5:1, or 1:2 to 2:1. In some embodiments of the invention, the at least two kinds of the siRNAs as described in the first aspect are present in the same molar ratio.

The present invention also provides a use of any one of the above-described siRNAs and/or any one of the above-described siRNA conjugates and/or the above-described pharmaceutical composition in the manufacture of a medicament for treating and/or preventing a pathological condition or disease associated with AGT gene overexpression.

Furthermore, the pathological condition or disease is a disease associated with abnormal blood pressure.

The siRNAs, siRNA conjugates, and pharmaceutical compositions provided by the present invention exhibit good stability, excellent inhibitory activity against AGT gene, satisfactory cytotoxicity and immunostimulatory properties, and can significantly reduce blood pressure levels.

In the present invention, unless otherwise specified, uppercase letters C, G, U, A, and T represent base moieties of nucleotides, including modified and unmodified nucleotides; lowercase letter m indicates that the nucleotide adjacent to the right of the identifier m is a 2'-methoxynucleotide; lowercase letter f indicates that the nucleotide adjacent to the right of the identifier f is a 2'-fluoronucleotide; lowercase letter d indicates that the nucleotide adjacent to the right of the identifier d is a 2'-deoxynucleotide; gn indicates that the nucleotide adjacent to the right of the identifier gn is a glycerol nucleotide (GNA); tn indicates that the nucleotide adjacent to the right of the identifier tn is a threose nucleotide (TNA); the * identifier indicates that the linkage between two the nucleotides (or between the nucleotide and the delivery carrier moiety) adjacent to the identifier * on the left and right is a phosphorothioate linkage; eVP indicates that the nucleotide adjacent to its right is a (*E*)-vinyl phosphate-modified nucleotide, for example, when the base is U, the eVPmU structure is 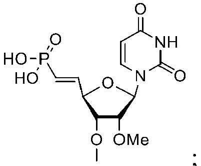 iab indicates an inverted abasic residue; GalNAc(L96) means that the delivery carrier moiety GalNAc(L96) is conjugated at this location. Ser(GN) means that that the delivery carrier moiety Ser(GN) is conjugated at this location.

In the present invention, unless otherwise specified, the uppercase latter I indicates a base moiety of a base-modified nucleotide, wherein the base is 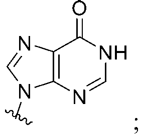 m6A indicates a base moiety of a base-modified nucleotide, wherein the base is 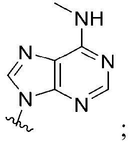 the uppercase latter X indicates a base moiety of a base-modified nucleotide, wherein the base is 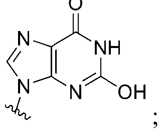 and the uppercase latter B indicates a base moiety of a base-modified nucleotide, wherein the base is

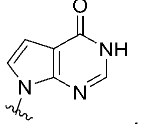

In the present invention, unless otherwise specified, the term "complementary" refers to the ability of an oligonucleotide of the first sequence to hybridize with an oligonucleotide of the second sequence under certain conditions and form a double-stranded structure. "At least partially complementary" means that the two sequences can be completely complementary, or have no more than 5, 4, 3, or 2 mismatched base pairs in total, while retaining the ability to hybridize under relevant conditions. Furthermore, when two oligonucleotides are designed to form one or more single-stranded overhangs during hybridization, such overhangs should not be considered mismatches for determining complementarity. In the present invention, to satisfy the above hybridization ability requirements, "complementary" sequences may also include or be entirely formed from non-Watson-Crick base pairs and/or from non-natural and modified nucleotides. Such non-Watson-Crick base pairs include, but are not limited to, G:U swing base pairing or Hoogstein base pairing. Correspondingly, in the present invention, unless otherwise specified, "mismatch" refers to a situation in the siRNA duplex molecule where the bases at corresponding positions are not paired in a complementary manner.

In the present invention, unless otherwise specified, "difference in nucleotide sequence" refers to a change in the type of bases at the same or corresponding positions of the nucleotide sequence compared to the original nucleotide sequence. For example, if a nucleotide base in the original nucleotide sequence is A, and the nucleotide base at the same or corresponding position is changed to U, C, G, or dT, dC, dG, etc., then a difference in nucleotide sequence is considered to exist at that position. It should be noted here that if, compared to the original nucleotide sequence, nucleotides at the same or corresponding positions differ only in the presence or type of modification, then it is not considered that there is a difference in nucleotide sequence at that position.

In the present invention, unless otherwise specified, the term "pharmaceutically acceptable" means that the carrier, delivery system, diluent, excipient, and/or the salt/ester/hydrate formed therefrom are generally chemically or physically compatible with other components constituting a drug dosage form and physiologically compatible with the receptor.

In the present invention, unless otherwise specified, the term "inhibition" refers to the down-regulation of target gene expression due to siRNA-mediated mRNA degradation. The "down-regulation" refers to a decrease in target gene expression level of 5%, 10%, 15%, 20%, 25%, 30%, 35%, 40%, 45%, 50%, 55%, 60%, 65%, 70%, 75%, 80%, 85%, 90%, 95%, or 99% or more, or even 100%, relative to the absence of siRNA treatment. A 100% decrease in target gene expression level means that there is no detectable level of target gene expression.

In the present invention, the siRNA may also comprise modified nucleotides as needed. These modified nucleotides do not significantly weaken or eliminate the siRNA's function of inhibiting AGT gene expression. Currently, various methods exist in the art for modifying siRNA, including, for example, backbone modification (such as phosphate group modification), ribose group modification, and base modification (Watts, J.K., G.F. Deleavey, and M.J. Damha, Chemically modified siRNA: tools and applications. Drug Discov Today, 2008. 13(19-20): p. 842-55).

Obviously, based on the above description of the present invention, and according to common technical knowledge and conventional methods in the art, various other modifications, substitutions, or alterations can be made without departing from the basic technical concept of the present invention.

The above contents of the present invention is further described by detailed embodiments in the form of examples. However, this should not be construed as limiting the scope of the above-described subject matter of the present invention to the following examples. All technologies implemented based on the above contents of the present invention fall within the scope of the present invention.

### Brief Description of the Drawings

Figure 1 shows a schematic diagram of the synthetic scheme of the conjugate of the present invention.
Figure 2 shows the process flow for the synthesis of the conjugate of the present invention.
Figures 3 and 4 show the MS spectra of the test sample Conjugate 100.
Figure 5 shows the ¹H-NMR of the test sample Conjugate 100.
Figure 6 shows the ¹³C-NMR of the test sample Conjugate 100.
Figure 7 shows the ³¹P-NMR of the test sample Conjugate 100.
Figure 8 shows the ¹⁹F-NMR of the test sample Conjugate 100.
Figure 9 shows the assay of melting temperature (Tₘ) of the test sample Conjugate 100.

### Specific Models for Carrying Out the present invention

Those skilled in the art will know that the siRNAs described in the present invention can be obtained by conventional siRNA preparation methods in the art (e.g., solid-phase synthesis and liquid-phase synthesis), wherein custom synthesis services for both solid-phase and liquid-phase methods are commercially available. Those skilled in the art will also understand that modified nucleotide groups can be introduced into the siRNAs described in the present invention by using nucleotide monomers with corresponding modifications. Methods for preparing nucleotide monomers with corresponding modifications are well known to those skilled in the art, and commercially available monomers are also available.

### Example 1: Synthesis of siRNA

For the sense and antisense strands of the siRNA sequence of the present invention, as well as the sense and antisense strands of the modified duplex, chain synthesis was initiated using a solid-phase support.

The preparation process of the conjugate of the present application is illustrated using Conjugate 100 as an example.

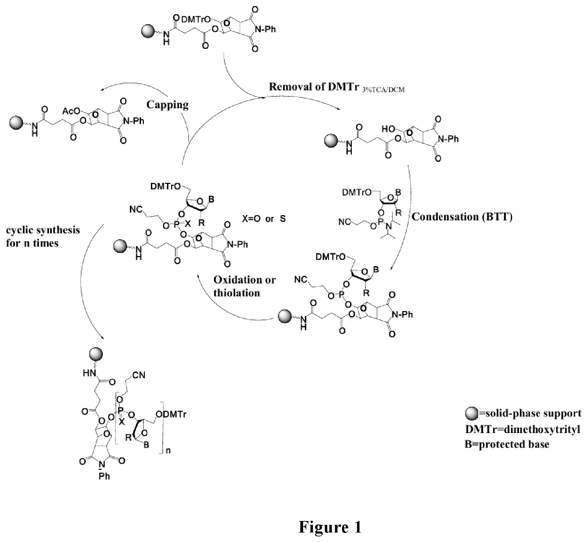

A solid-phase support modified with GalNAc (L96) was used for the starting cycle for sense strand synthesis, and a universal solid-phase support (Primer support 5G unylinker 350) was used for the starting cycle for antisense strand synthesis (Figure 1).

The structure of the GalNAc (L96) modified solid-phase support (L96-PS) is as follows:

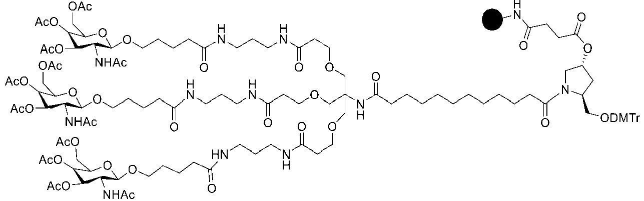

The structure of the Primer support 5G unylinker 350 support is as follows: 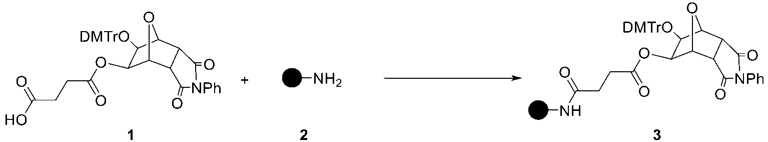
• represents PS (polystyrene) solid-phase support.

Using a YB-192S synthesizer, the phosphoramidite-triester solid-phase synthesis method was employed. Starting with the solid-phase support, nucleoside monomers were sequentially linked in the 3'-5' direction for a synthetic scale of 0.2 µmol.

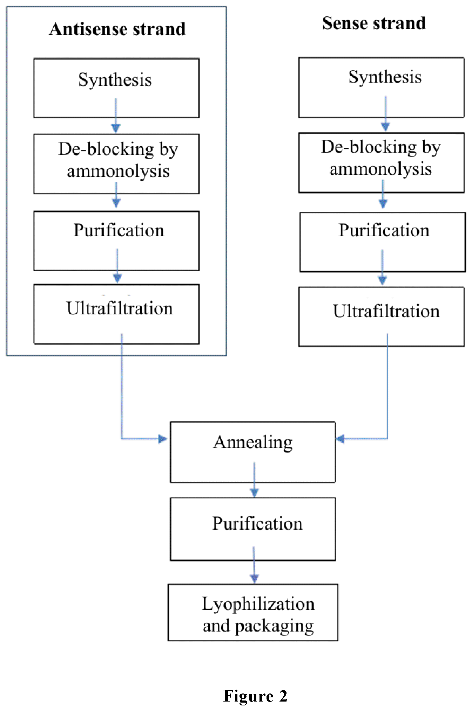

The process flow is shown in Figure 2.

Process description: Oligo synthesis began with a PS solid-phase support and employed a method using 3'-*O*-(2-cyanoethyl)phosphoramidite/4,4'-dimethoxytrityl (DMT) group protection to assemble an oligonucleotide chain on the PS solid-phase support. Each synthesis cycle involved de-blocking of 5'-hydroxy, coupling, capping, and oxidation (or thiolation). Each coupling reaction was performed by activating an appropriate phosphoramidite monomer and allowing it to react with the free 5'-hydroxy group of a protected nucleotide or oligonucleotide immobilized on the support. According to the sequence information, cyclic synthesis yielded the corresponding crude oligonucleotide single strand, PS-Oligo. The crude oligonucleotide strand was cleaved from the solid-phase support, and the relevant protecting groups were removed. Then, the final product was obtained through purification via preparative chromatography, desalting through ultrafiltration, annealing, and lyophilization.

### (1) Synthetic procedure

The following units were included:
1) De-blocking: For ribonucleotides with a DMT (di-p-methoxytrityl) group at the 5'-OH end, the DMT protecting group was removed from the solid support and ribonucleotide in the first step of the synthesis using trichloroacetic acid (TCA) to expose the 5'-OH of the ribonucleotide for coupling with a new base.
2) Coupling: The nucleotide monomer was mixed with an activating reagent and reacted with the PS-Oligo in the synthesis column. The activating reagent provided a proton to the nitrogen atom of the diisopropylamino group on the 3'-phosphate, forming a phosphoramidite tetrazole active intermediate. When the phosphoramidite tetrazole contacted the PS-Oligo, a nucleophilic reaction occurred with its 5'-hydroxyl group, leading to coupling and the release of tetrazole, thereby extending the oligo by one nucleotide.
3) Capping: Since the coupling efficiency could not reach 100%, a capping reagent was used to cap the 5'-hydroxyl group of the PS-Oligo in order to prevent any uncoupled PS-Oligo from proceeding to the next coupling step.
4) Oxidation or thiolation: After the coupling reaction, the nucleotide was linked to the oligonucleotide on the PS support via a phosphite bond (trivalent phosphorus). This phosphite bond was unstable and prone to hydrolysis by acids or bases. The trivalent phosphorus at this site was oxidized to pentavalent phosphorus using an oxidizing reagent. Thiolation referred to the reaction of the trivalent phosphorus in the phosphite bond under weakly alkaline conditions, using a thiolating reagent to form a phosphorothioate bond.
5) Removal of VP protecting group and ammonolysis

The synthesized PS-oligo was transferred from the synthesis column to a centrifuge tube. A deprotection reagent was prepared according to the volume ratio 3:2:100 = TMS-I:pyridine:DCM, and then added into the centrifuge tube to react for 30 min. A solution of TEA/acetonitrile = 1:1 was prepared, and 2-mercaptoethanol (final concentration 2 M) was added and mixed well, followed by addition to the reaction mixture to quench the reaction. The supernatant was removed, and the PS-oligo was washed twice for 5 min each time. After washing, an ammonolysis solution (2-mercaptoethanol (2 M)/28% Aqueous Ammonia) was added to carry out ammonolysis for 10 hours. After ammonolysis, the Aqueous Ammonia was removed by vacuum centrifugation, and purification was performed.

The sense and antisense strands of other siRNA sequences of the present invention, as well as the sense and antisense strands of modified duplexes, were prepared using similar methods.

### (2) Purification

The diluted sample after ammonolysis was purified by anion exchange chromatography using high-performance liquid chromatography (HPLC). The chromatographic conditions were as follows:
Column: PS-15Q10*250mm
Flow rate: 4ml/min
Detection wavelength: 260nm
Ion column preparation purification gradient program:
Mobile phase: Phase A: 10 mM NaOH solution (pH=10); Phase B: 10 mM NaOH solution (pH=10) + 2M NaCl

| Time (min) | Mobile phase A (%) | Mobile phase B (%) |
|---|---|---|
| 0 | 100 | 0 |
| 5 | 80 | 20 |
| 15 | 78 | 22 |
| 50 | 60 | 40 |
| 80 | 50 | 50 |

The prepared sample was then subjected to mass spectrometry confirmation and purity analysis.

### (3) Ultrafiltration

After purification, the sample was dissolved in 4 ml of PBS, transferred to a 1K ultrafiltration tube, centrifuged at 5000 r/min for 45 min, and subjected to detection for the presence of sample in the centrifuge tube using Nanodrop. If the presence of sample was detected at 260 nm, it was confirmed that the ultrafiltration membrane was damaged, the ultrafiltration tube needed to be replaced, and ultrafiltration was repeated. If the presence of sample was not detected at 260 nm, 4 ml of RNase water was added and ultrafiltration was repeated. Ultrafiltration was performed three times, 45 min each time.

### (4) Annealing

After ultrafiltration of the sense and antisense strands, the sample was diluted to a concentration of 3 mg/ml. The sense and antisense strands were mixed at a 1:1 molar ratio. The water bath was heated to 90°C, and the mixed sense and antisense strands were placed in the water bath and annealed for 30 min. The water bath was then turned off, and the mixture was allowed to cool naturally to room temperature for 16 hours. A 10 µL sample was taken for HPLC analysis.

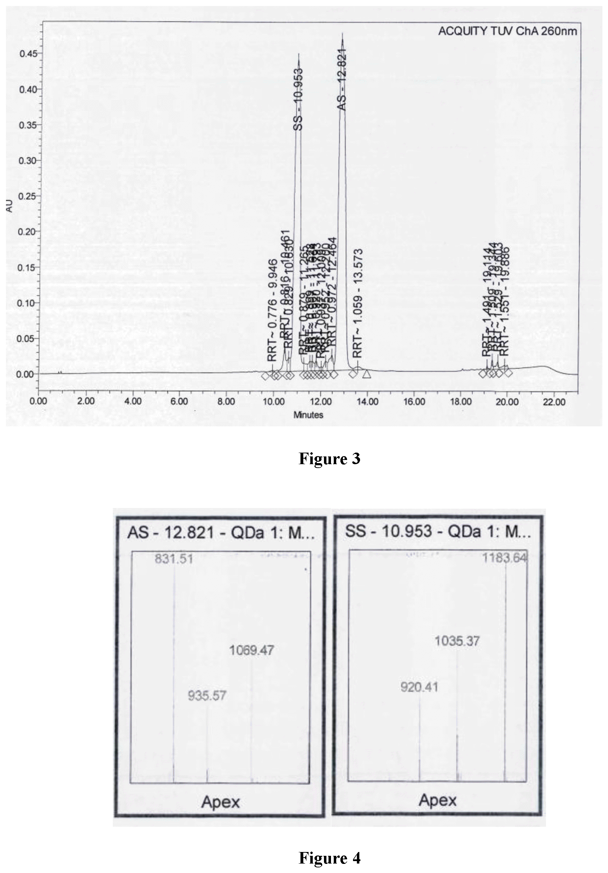

Representative detection method: LC-MS (Example: Conjugate 100)
1) Instrument model: Waters Xevo G2-XS Qtof
2) Testing conditions:
   Ion Source: ESI negative ion source
   Scanning range: 100-2000 Da
   Collision energies: 15-25 EV, 25-50 EV
3) The spectra of Conjugate 100 were shown in Figures 3 and 4.
4) Spectral analysis:
   When conjugate 100 was detected by denaturing IP·RP-LC, the complementary double strands were broken into single strands (sense and antisense strands). The sense/antisense precursor ions were then fragmented by tandem mass spectrometry. All detected fragment ions were analyzed and resolved using the CONFIRM Sequence software. In this experiment, the precursor ions were fragmented at energies of 15-25 eV and 25-50 eV, respectively, resulting in 100% coverage, that was, the sample sequence matched the theoretical sequence. The confirmed results are shown in the table below.

Sequence confirmation of the sense strand of Conjugate 100

| Collision energy | Parent ion molecular weight (Da) | Charge state | % Coverage |
|---|---|---|---|
| 25eV | 8286.9353 | 9- 8- 7- | 100.00 |
| 50eV | 8286.8611 | 9- 8- 7- | 100.00 |

Sequence confirmation of the antisense chain of Conjugate 100

| Collision energy | Parent ion molecular weight (Da) | Charge state | % Coverage |
|---|---|---|---|
| 25eV | 7488.1405 | 9- 8- 7- | 100.00 |
| 50eV | 7488.0876 | 9- 8- 7- | 100.00 |

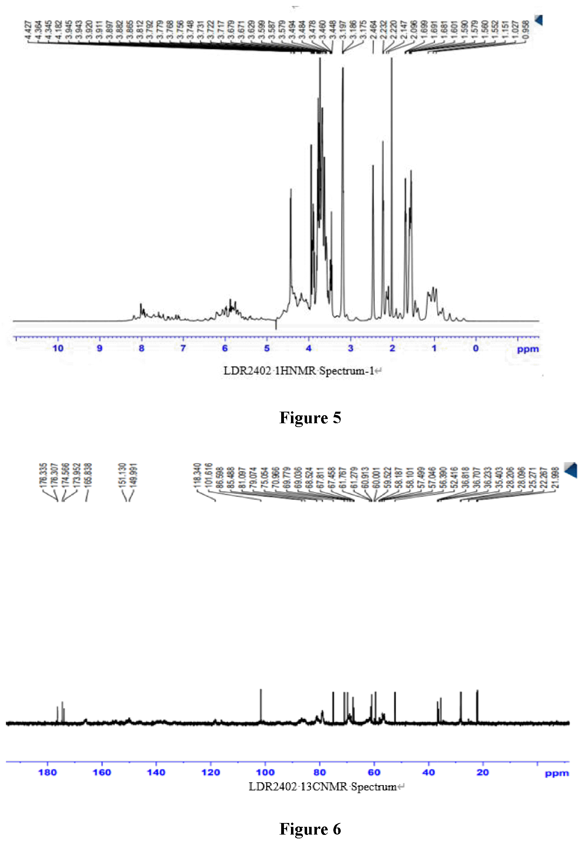

Nuclear magnetic resonance (NMR) detection of Conjugate 100
1) Instrument model: Bruker AVANCE III 600M NMR
2) Detection method: 95.0 mg of sample was weighed and dissolved in deuterium oxide (D₂O) and transferred to an NMR tube for detection.
3) Spectral analysis:
   The ¹H-NMR spectrum (Figure 5) showed the peaks corresponding to the hydrogens of aromatic heterocyclic rings, ribose, methyl groups, amino groups, methylene groups, sugar rings, and alcoholic hydroxyl groups. The series of peaks from 0.95 ppm to 2.5 ppm corresponded to methylene hydrogens not directly bonded to the amide. The peaks from 3.17 ppm to 3.20 ppm were attributed to the methylene hydrogens adjacent to the amide. The peaks from 3.4 ppm to 4.6 ppm were attributed to the methine hydrogens on the sugar ring, the methyl group at the 2'-position, and the ether-linked methylene hydrogens. The peaks from 5.1 ppm to 6.5 ppm were attributed to the methine hydrogen at the 1'-position of the sugar ring, the aromatic hydrogens of the pyrimidine base, and the vinyl hydrogens at the 5'-position. The peaks from 7.1 ppm to 8.2 ppm were attributed to the aromatic hydrogens of the purine base.

The ¹³C-NMR spectrum (Figure 6) showed the peaks corresponding to the carbons of carbonyl groups, aromatic heterocyclic rings, sugar rings, ether-linked methylenes, methylenes, and methyl groups in the molecule. The peaks from 170 ppm to 180 ppm were attributed to the carbonyl carbons, the peaks from 150 ppm to 165 ppm were attributed to the carbons of the aromatic heterocyclic rings (bases) and vinyl groups, the peaks from 95 ppm to 100 ppm were attributed to the methine at the 1'-position of ribose, the peaks from 70 ppm to 95 ppm were attributed to the carbons of methylene and methine of ribose, the peaks from 55 ppm to 70 ppm were attributed to the carbons of methoxy and ether-linked methylene, and the peaks from 20 ppm to 40 ppm were attributed to the methyl carbons at the α-position of acetyl groups.

These results indicated that aromatic heterocyclic rings (bases), vinyl groups, sugar rings, exocyclic methoxy groups, methylene groups, ether-linked methylene groups, and acetyl groups were observed in the ¹H-NMR and ¹³C-NMR, which were consistent with the structure of the target molecule.

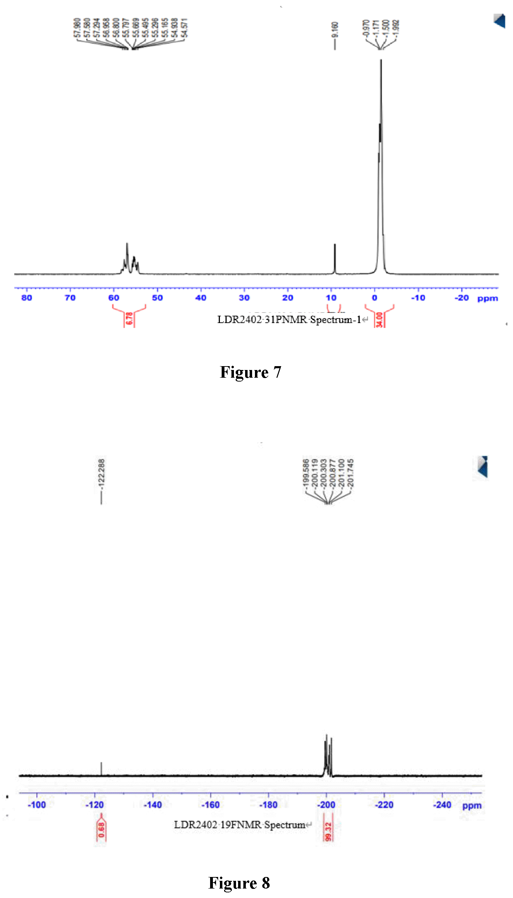

The ³¹P-NMR spectrum (Figure 7) showed signals for three types of phosphorus functional groups: thiophosphate diester (P=S) with chemical shift of 54.5 ppm to 57.5 ppm, phosphate diester (P=O) with chemical shift of -0.97 ppm to -1.5 ppm, and 5'-vinylphosphate with chemical shift of 9.16 ppm. The ratio of P=O bonds to P=S bonds was approximately 5:1, which was consistent with the information expected for the target molecule.

The ¹⁹F-NMR spectrum (Figure 8) showed the peak position of -199 ppm to -201 ppm, corresponding to the position of F at the 2'-position of the sugar ring.

These above analyses confirmed that the NMR signals were consistent with those of the target molecule.

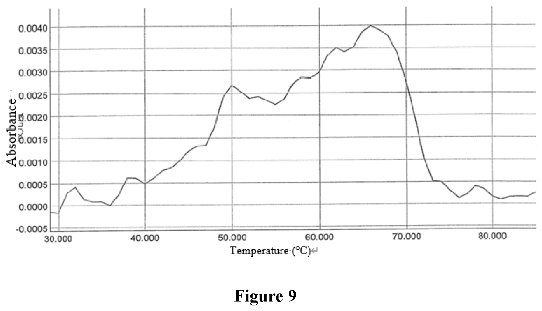

Detection of melting temperature (Tm) of Conjugate 100:
1) Instrument model: Cary 3500
2) Detection conditions:
   Scanning wavelength: 260 nm
   Temperature scan range: 25 °C~90 °C
   Heating rate: 3 °C/min
3) The spectrum of test sample Conjugate 100 was shown in Figure 9.
4) Spectrum analysis:
   Based on the heating curve of Conjugate 100, the software system calculated a Tm of 66.0 °C using the Savitzky-Golay algorithm.

LC-MS detection of the synthesized conjugates:

| **Conjugate** | **Sense strand** | | **Antisense strand** | |
|---|---|---|---|---|
| | **Theoretical molecular weight** | **Actual molecular weight** | **Theoretical molecular weight** | **Actual molecular weight** |
| **Duplex 23** | 6209.7 | 6208.6 | 7091.3 | 7089.5 |
| **Duplex 108** | 6282.7 | 6281.3 | 7033.3 | 7031.8 |
| **Duplex 109** | 6231.7 | 6231.4 | 7159.4 | 7158.8 |
| **Duplex 112** | 6359.8 | 6358.0 | 7001.3 | 6999.5 |
| **Duplex 113** | 6349.9 | 6348.6 | 6996.2 | 6996.5 |
| **Duplex 115** | 6232.7 | 6231.4 | 7143.4 | 7141.6 |
| **Conjugate 1** | 8824.7 | 8824.7 | 7505.2 | 7505.2 |
| **Conjugate 2** | 8719.6 | 8718.4 | 7650.0 | 7649.1 |
| **Conjugate 3** | 8719.6 | 8718.4 | 7604.1 | 7602.8 |
| **Conjugate 4** | 8719.6 | 8718.4 | 7686.1 | 7684.8 |
| **Conjugate 5** | 8719.6 | 8718.4 | 7662.1 | 7660.0 |
| **Conjugate 6** | 8719.6 | 8718.4 | 7646.2 | 7644.2 |
| **Conjugate 7** | 8800.7 | 8801.7 | 7554.0 | 7552.0 |
| **Conjugate 8** | 8800.7 | 8801.7 | 7508.0 | 7506.8 |
| **Conjugate 12** | 8242.2 | 8242.3 | 7569.1 | 7567.1 |
| **Conjugate 13** | 7971.1 | 7971.2 | 7132.8 | 7130.9 |
| **Conjugate 18** | 8415.4 | 8414.8 | 7436.9 | 7437.2 |
| **Conjugate 19** | 8415.4 | 8414.8 | 7436.9 | 7437.2 |
| **Conjugate 20** | 8415.4 | 8414.8 | 7436.9 | 7437.2 |
| **Conjugate 21** | 8385.3 | 8384.1 | 7569.1 | 7569.0 |
| **Conjugate 22** | 8385.3 | 8384.1 | 7569.1 | 7569.0 |
| **Conjugate 23** | 8385.3 | 8384.1 | 7569.1 | 7569.0 |
| **Conjugate 24** | 8433.4 | 8433.8 | 7436.9 | 7436.7 |
| **Conjugate 25** | 8450.4 | 8450.4 | 7436.9 | 7436.7 |
| **Conjugate 26** | 8450.4 | 8450.4 | 7436.9 | 7436.7 |
| **Conjugate 28** | 8433.4 | 8433.8 | 7515.0 | 7515.0 |
| **Conjugate 29** | 8450.4 | 8450.4 | 7515.0 | 7515.0 |
| **Conjugate 30** | 8450.4 | 8450.4 | 7294.9 | 7294.9 |
| **Conjugate 62** | 8173.2 | 8173.0 | 7594.1 | 7594.0 |
| **Conjugate 63** | 8203.2 | 8203.2 | 7564.1 | 7564.1 |
| **Conjugate 64** | 8203.2 | 8203.2 | 7510.1 | 7510.1 |
| **Conjugate 65** | 8182.2 | 8182.0 | 7571.1 | 7571.0 |
| **Conjugate 66** | 8226.2 | 8226.0 | 7541.1 | 7541.0 |
| **Conjugate 67** | 8226.2 | 8226.0 | 7487.1 | 7487.0 |
| **Conjugate 68** | 8246.2 | 8245.8 | 7571.1 | 75708 |
| **Conjugate 69** | 8290.2 | 8289.9 | 7461.1 | 74608 |
| **Conjugate 70** | 8290.2 | 8289.9 | 7422.1 | 7422.0 |
| **Conjugate 71** | 7927.0 | 7927.2 | 7571.1 | 7571.0 |
| **Conjugate 72** | 7971.0 | 7971.1 | 7541.1 | 7541.0 |
| **Conjugate 73** | 7971.0 | 7971.1 | 7487.1 | 7486.9 |
| **Conjugate 74** | 8719.6 | 8720.3 | 7681.1 | 7680.4 |
| **Conjugate 75** | 8675.6 | 8672.9 | 7725.1 | 7723.4 |
| **Conjugate 76** | 8384.4 | 8383.4 | 7442.0 | 7742.4 |
| **Conjugate 78** | 8025.1 | 8024.8 | 7122.8 | 7122.4 |
| **Conjugate 80** | 8800.7 | 8801.8 | 7585.0 | 7583.8 |
| **Conjugate 82** | 8449.4 | 8449.7 | 7345.9 | 7346.6 |
| **Conjugate 84** | 8106.2 | 8106.6 | 7026.7 | 7028.2 |
| **Conjugate 85** | 8106.2 | 8106.3 | 6950.7 | 6949.9 |
| **Conjugate 86** | 8729.4 | 8729.8 | 7760.0 | 7760.2 |
| **Conjugate 87** | 8725.7 | 8721.5 | 7730.0 | 7729.1 |
| **Conjugate 89** | 8414.4 | 8414.8 | 7436.9 | 7435.1 |
| **Conjugate 91** | 8071.2 | 8072.0 | 7116.7 | 7716.4 |
| **Conjugate 93** | 8129.2 | 8128.5 | 7003.7 | 7004.9 |
| **Conjugate 94** | 8203.2 | 8203.5 | 7593.1 | 7592.7 |
| **Conjugate 95** | 8203.2 | 8202.5 | 7594.1 | 7593.0 |
| **Conjugate 97** | 8226.2 | 8225.4 | 7593.1 | 7592.8 |
| **Conjugate 98** | 8226.2 | 8225.4 | 7571.1 | 7568.7 |
| **Conjugate 99** | 8273.8 | 8273.8 | 7881.8 | 7881.8 |
| **Conjugate 100** | 8290.3 | 8288.3 | 7492.0 | 7489.1 |
| **Conjugate 102** | 8290.3 | 8288.8 | 7504.0 | 7501.9 |
| **Conjugate 103** | 8290.3 | 8290.1 | 7515.0 | 7514.3 |
| **Conjugate 106** | 8274.3 | 8273.2 | 7492.0 | 7492.4 |
| **Conjugate 108** | 8291.3 | 8290.4 | 7492.0 | 7492.8 |
| **Conjugate 109** | 8719.6 | 8719.4 | 7637.0 | 7637.0 |
| **Conjugate 110** | 8719.6 | 8719.4 | 7713.0 | 7713.0 |
| **Conjugate 111** | 8719.6 | 8719.4 | 7591.1 | 7591.0 |
| **Conjugate 112** | 8719.6 | 8719.4 | 7667.1 | 7667.0 |
| **Conjugate 113** | 8800.7 | 8801.7 | 7541.0 | 7541.2 |
| **Conjugate 114** | 8800.7 | 8801.7 | 7617.0 | 7617.2 |
| **Conjugate 156** | 8769.7 | 8769.1 | 7689.1 | 7689.6 |
| **Conjugate 157** | 8769.7 | 8768.8 | 7700.2 | 7698.5 |
| **Conjugate 161** | 8586.2 | 8586.4 | 7571.1 | 7570.9 |
| **Conjugate 162** | 8651.7 | 8651.8 | 7492.3 | 7490.6 |
| **Conjugate 163** | 8650.9 | 8651.8 | 7503.8 | 7502.7 |
| **Conjugate 1606** | 8609.5 | 8607.4 | 7851.2 | 7849.3 |
| **Conjugate 1607** | 8609.5 | 8607.4 | 7819.2 | 7817.1 |
| **Conjugate 1608** | 8290.3 | 8288.3 | 7460.0 | 7458.1 |
| **Conjugate 1609** | 7971.1 | 7969.2 | 7132.8 | 7129.8 |
| **Conjugate 1610** | 7971.1 | 7969.2 | 7100.8 | 7099.1 |
| **Conjugate 1611** | 8597.4 | 8595.3 | 7851.2 | 7849.3 |
| **Conjugate 1612** | 8278.2 | 8277.3 | 7492.0 | 7490.1 |
| **Conjugate 1613** | 7959.0 | 7957.7 | 7132.8 | 7129.8 |
| **Conjugate 1614** | 8495.3 | 8493.3 | 7860.3 | 7858.8 |
| **Conjugate 1615** | 8581.4 | 8571.4 | 7813.2 | 7813.0 |
| **Conjugate 1616** | 8581.4 | 8571.4 | 7837.3 | 7837.2 |
| **Conjugate 1617** | 8290.3 | 8289.8 | 7431.8 | 7429.1 |
| **Conjugate 1618** | 8290.3 | 8289.8 | 7419.8 | 7417.1 |
| **Conjugate 1619** | 8290.3 | 8289.2 | 7480.0 | 7478.1 |
| **Conjugate 1620** | 8290.3 | 8289.2 | 7480.0 | 7477.4 |
| **Conjugate 1621** | 8290.3 | 8289.2 | 7467.9 | 7465.1 |
| **Conjugate 1622** | 8290.3 | 8288.4 | 7480.0 | 7477.8 |
| **Conjugate 1623** | 8290.3 | 8288.4 | 7480.0 | 7479.5 |
| **Conjugate 1624** | 8290.3 | 8288.4 | 7492.0 | 7491.5 |
| **Conjugate 1625** | 8290.3 | 8288.4 | 7480.0 | 7478.8 |
| **Conjugate 1626** | 8290.3 | 8288.4 | 7467.9 | 7465.4 |
| **Conjugate 1627** | 8290.3 | 8288.4 | 7467.9 | 7465.2 |
| **Conjugate 1628** | 8290.3 | 8288.4 | 7480.0 | 7477.9 |
| **Conjugate 1629** | 8290.3 | 8288.4 | 7467.9 | 7465.1 |
| **Conjugate 1633** | 8261.0 | 8261.9 | 7492.0 | 7489.1 |
| **Conjugate 1634** | 8247.0 | 8247.8 | 7492.0 | 7489.1 |
| **Conjugate 1637** | 8345.0 | 8345.9 | 7492.0 | 7489.1 |
| **Conjugate 1638** | 8331.0 | 8332.0 | 7492.0 | 7489.1 |
| **Conjugate 1642** | 7820.3 | 7821.0 | 7492.0 | 7489.1 |
| **Conjugate 1646** | 8266.2 | 8265.4 | 7480.0 | 7477.7 |
| **Conjugate 1647** | 8266.2 | 8265.4 | 7480.0 | 7477.7 |
| **Conjugate 1648** | 8266.2 | 8265.4 | 7492.0 | 7490.1 |
| **Conjugate 1649** | 8278.2 | 8277.7 | 7480.0 | 7477.7 |
| **Conjugate 1650** | 8266.2 | 8265.7 | 7480.0 | 7477.7 |
| **Conjugate 1651** | 8278.2 | 8277.7 | 7480.0 | 7477.7 |
| **Conjugate 1652** | 8278.2 | 8277.6 | 7480.0 | 7477.7 |
| **Conjugate 1653** | 8278.2 | 8277.6 | 7480.0 | 7477.6 |
| **Conjugate 1654** | 8278.2 | 8277.6 | 7467.9 | 7465.9 |
| **Conjugate 1655** | 8278.2 | 8277.6 | 7467.9 | 7465.8 |
| **Conjugate 1656** | 8278.2 | 8277.6 | 7480.0 | 7478.2 |
| **Conjugate 1657** | 8278.2 | 8277.6 | 7467.9 | 7466.0 |
| **Conjugate 1658** | 8266.2 | 8265.5 | 7480.0 | 7477.7 |
| **Conjugate 1659** | 8363.3 | 8364.7 | 7434.0 | 7431.6 |
| **Conjugate 1660** | 8312.3 | 8311.4 | 7560.1 | 7557.6 |
| **Conjugate 1663** | 8440.4 | 8441.5 | 7401.9 | 7400.1 |
| **Conjugate 1664** | 8430.4 | 8431.7 | 7396.9 | 7394.9 |
| **Conjugate 1666** | 8313.3 | 8312.2 | 7544.1 | 7541.8 |
| **PC c** | 8769.7 | 8769.0 | 7618.2 | 7618.4 |

wherein, PC c was a positive control, known to have an inhibitory activity against AGT gene, and its structure was as follows:
Sense strand: mG*mU*mCmAmUmCfCmAfCfAfAmUmGmAmGmAmGmUmAmCmA GalNAc(L96) (The unmodified base sequence corresponded to SEQ ID NO: 251);
Antisense strand: mU*fG*mUmAmCgnTmCmUmCmAmUmUmGfUmGfGmAmUmGmAmC*mG*mA (The unmodified base sequence corresponded to SEQ ID NO: 348).

### Example 2: In vitro activity assay

### Cell culture and transfection:

Cell culture: Hep3B cells (ATCC) were cultured at 37°C and 5% CO₂ in MEM complete medium (Gibco, with 10% FBS) until near confluence. The cells were then trypsinized and seeded in 12-well plates. Each well contained 2.0 × 10⁵ Hep3B cells and 1.0 mL of MEM complete medium (Gibco, with 10% FBS). After culturing at 37°C and 5% CO₂ for 16-24 hours, transfection was performed.

Cell transfection: 48.5 µL of opti-MEM per well was mixed with 1.5 µL of lipofectamine RNAiMax (Invitrogen), then 50 µL of siRNA was added and mixed. The mixture was transferred to a PCR tube and incubated at room temperature for 5 minutes. Finally, this siRNA mixture was added to the cells and cultured for 24 hours before RNA extraction. Single-dose experiments were performed using 10 nM and 0.1 nM or 0.1 nM and 0.01 nM siRNA duplex concentrations. IC₅₀ assays were performed using 10 nM, 1.0 nM, 0.1 nM, 0.01 nM, 0.001 nM, 0.0001 nM, and 0.00001 nM siRNA duplex concentrations. The IC₅₀ values of the compounds were calculated using the log(inhibitor) vs. response -- variable slope (four parameters) fitting method in the GraphPad Prism software.

### RNA extraction:

Using the Total RNA Isolation Kit (Omega, CAT: R6834-02): The cells were collected, washed with 1% PBS, and then lysed with 400 µL of lysis buffer (containing 2% β-mercaptoethanol). Subsequent steps were performed according to the kit's instructions. Finally, 30 µL of RNase-free water was added, and the cells were incubated for 2 minutes before centrifugation at 14000g for 2 minutes to collect RNA.

### cDNA Synthesis:

cDNA synthesis was performed using the TransGen gDNA Removal and cDNA Synthesis Kit (TransGen Biotechnology Co., Ltd., Beijing, China, Cat# AE311-03). 1 µg of total RNA was added to each sample, and cDNA synthesis was performed using a gradient thermal cycler (LongGene, A600) according to the manufacturer's instructions.

### Real-time fluorescence quantitative PCR

The synthesized cDNA and master mix (containing primers, qPCR premix, and ultrapure water) were added to a 384-well plate (Bokang Meihua, Cat# PC-0040-9U). The final real-time fluorescence quantitative PCR reaction mixture contained 0.25 µM each of upstream and downstream primers for the target gene (AGT) or the internal reference gene (GADPH), and 1× SYBR Green premix (Applied Biosystem Cat # A25742).

Real-time fluorescence PCR was performed on the ABI QuantStudio^{™} 6 Real-Time Fluorescence PCR System using the ΔΔCt method. Three to four independent transfection assays were carried out for each duplex, with each transfection assayed in triplicate to quadruplicate.

### Human/cynomolgus monkey primary liver cells:

Free Uptake: Cryopreserved primary hepatocytes from humans or cynomolgus monkeys were thawed and revived, subjected to viable cell counting, and adjusted to an appropriate density (6×10⁵ cells/mL). A 10 µL volume of 10× compound was added to each well of a collagen-coated cell culture plate. Then, 90 µL of the cell suspension was aliquoted into each well of the 96-well collagen-coated plate (5.4×10⁴ cells/well). The cell plate was placed and incubated in a cell culture incubator at 37°C and 5% CO₂ for 48 hours. Single-dose experiments were performed at siRNA duplex concentrations of 10 nM and 0.1 nM. IC₅₀ assays were performed at 10 nM, 3.33 nM, 1.11 nM, 0.37 nM, 0.12 nM, 0.041 nM, 0.014 nM, and 0.0046 nM siRNA duplex concentrations. The IC₅₀ value of the compound was calculated using the log(inhibitor) vs. response -- Variable slope (four parameters) fitting method in the GraphPad Prism software.

cDNA synthesis: After 48 hours of free uptake, the culture medium was removed and the cells were lysed for RNA extraction. Total RNA was extracted using the RNeasy^{®} 96 Kit (QIAGEN-74182) according to the kit's instructions. cDNA was then synthesized using the FastKing RT Kit (With gDNase) (Tiangen-KR116-02) according to the instructions.

Real-time fluorescence quantitative PCR: The synthesized cDNA and master mix (containing primers, qPCR premix, and ultrapure water) were added to a 384-well plate, so that the final real-time fluorescence quantitative PCR system contained 0.25 µM each of the upstream and downstream primers for the target gene (AGT) or the internal reference gene (GADPH), 0.125 µM probe, and 1× Universal Probe Master premix (Roche, catalog number 04914058001).

Real-time fluorescence PCR was performed on the ABI QuantStudio^{™} 6 Real-Time Fluorescence PCR System using the ΔΔCt method. Three to four independent transfection assays were carried out for each duplex, with each transfection assayed in triplicate to quadruplicate.

The in vitro activity assay results of some siRNA conjugates were shown in Tables 1 to 5, in which PC c, known to have an inhibitory activity against AGT gene, was used as a positive control.

**Table 1. Single-dose Hep3B cell activity**

| **Conjugate No.** | **0.1 nM siRNA** | | **0.01 nM siRNA** | |
|---|---|---|---|---|
| | **Mean*** | **SD** | **Mean*** | **SD** |
| **Duplex 23** | 0.056 | 0.008 | 0.097 | 0.012 |
| **Duplex 109** | 0.084 | 0.022 | 0.21 | 0.007 |
| **Duplex 112** | 0.099 | 0.023 | 0.376 | 0.023 |
| **Duplex 113** | 0.164 | 0.044 | 0.515 | 0.017 |
| **Duplex 115** | 0.216 | 0.033 | 0.689 | 0.018 |
| **Conjugate 1** | 0.050 | 0.005 | 0.224 | 0.012 |
| **Conjugate 2** | 0.074 | 0.009 | 0.368 | 0.032 |
| **Conjugate 3** | 0.065 | 0.007 | 0.267 | 0.009 |
| **Conjugate 4** | 0.097 | 0.001 | 0.473 | 0.007 |
| **Conjugate 5** | 0.074 | 0.002 | 0.343 | 0.033 |
| **Conjugate 6** | 0.081 | 0.005 | 0.343 | 0.020 |
| **Conjugate 7** | 0.055 | 0.005 | 0.202 | 0.003 |
| **Conjugate 8** | 0.091 | 0.006 | 0.307 | 0.017 |
| **Conjugate 20** | 0.062 | 0.000 | 0.291 | 0.018 |
| **Conjugate 27** | 0.049 | 0.002 | 0.200 | 0.014 |
| **Conjugate 29** | 0.072 | 0.012 | 0.388 | 0.021 |
| **Conjugate 30** | 0.068 | 0.014 | 0.262 | 0.024 |
| **Conjugate 35** | 0.061 | 0.005 | 0.283 | 0.023 |
| **Conjugate 36** | 0.058 | 0.009 | 0.258 | 0.026 |
| **Conjugate 41** | 0.057 | 0.002 | 0.314 | 0.028 |
| **Conjugate 42** | 0.073 | 0.006 | 0.305 | 0.007 |
| **Conjugate 45** | 0.088 | 0.013 | 0.346 | 0.008 |
| **Conjugate 72** | 0.048 | 0.006 | 0.181 | 0.021 |
| **Conjugate 74** | 0.097 | 0.005 | 0.371 | 0.019 |
| **Conjugate 75** | 0.063 | 0.004 | 0.242 | 0.004 |
| **Conjugate 77** | 0.131 | 0.015 | 0.502 | 0.017 |
| **Conjugate 79** | 0.115 | 0.011 | 0.480 | 0.010 |
| **Conjugate 80** | 0.063 | 0.011 | 0.247 | 0.005 |
| **Conjugate 81** | 0.091 | 0.007 | 0.419 | 0.007 |
| **Conjugate 83** | 0.057 | 0.007 | 0.240 | 0.025 |
| **Conjugate 85** | 0.064 | 0.013 | 0.287 | 0.010 |
| **Conjugate 86** | 0.046 | 0.003 | 0.252 | 0.024 |
| **Conjugate 87** | 0.055 | 0.009 | 0.270 | 0.014 |
| **Conjugate 88** | 0.064 | 0.004 | 0.396 | 0.021 |
| **Conjugate 90** | 0.079 | 0.006 | 0.511 | 0.009 |
| **Conjugate 96** | 0.056 | 0.002 | 0.162 | 0.009 |
| **Conjugate 100** | 0.044 | 0.004 | 0.222 | 0.012 |
| **Conjugate 102** | 0.049 | 0.003 | 0.190 | 0.017 |
| **Conjugate 105** | 0.062 | 0.002 | 0.117 | 0.006 |
| **Conjugate 106** | 0.044 | 0.002 | 0.137 | 0.005 |
| **Conjugate 109** | 0.068 | 0.019 | 0.256 | 0.008 |
| **Conjugate 110** | 0.039 | 0.000 | 0.174 | 0.008 |
| **Conjugate 111** | 0.065 | 0.002 | 0.286 | 0.013 |
| **Conjugate 112** | 0.053 | 0.002 | 0.219 | 0.013 |
| **Conjugate 113** | 0.040 | 0.002 | 0.203 | 0.017 |
| **Conjugate 114** | 0.034 | 0.002 | 0.153 | 0.007 |
| **Conjugate 62** | 0.067 | 0.006 | 0.342 | 0.018 |
| **Conjugate 64** | 0.051 | 0.001 | 0.164 | 0.009 |
| **Conjugate 65** | 0.066 | 0.006 | 0.230 | 0.009 |
| **Conjugate 66** | 0.082 | 0.005 | 0.280 | 0.007 |
| **Conjugate 67** | 0.066 | 0.002 | 0.303 | 0.010 |
| **Conjugate 68** | 0.058 | 0.001 | 0.249 | 0.013 |
| **Conjugate 69** | 0.094 | 0.013 | 0.375 | 0.029 |
| **Conjugate 70** | 0.096 | 0.005 | 0.448 | 0.024 |
| **Conjugate 71** | 0.065 | 0.011 | 0.274 | 0.034 |
| **Conjugate 73** | 0.082 | 0.005 | 0.291 | 0.021 |
| **Conjugate 120** | 0.234 | 0.016 | 0.767 | 0.033 |
| **Conjugate 122** | 0.294 | 0.036 | 0.729 | 0.036 |
| **Conjugate 123** | 0.083 | 0.010 | 0.221 | 0.035 |
| **Conjugate 124** | 0.096 | 0.025 | 0.344 | 0.097 |
| **Conjugate 125** | 0.067 | 0.007 | 0.348 | 0.044 |
| **Conjugate 127** | 0.066 | 0.004 | 0.267 | 0.031 |
| **Conjugate 128** | 0.089 | 0.009 | 0.545 | 0.019 |
| **Conjugate 129** | 0.077 | 0.027 | 0.249 | 0.057 |
| **Conjugate 130** | 0.194 | 0.057 | 1.070 | 0.119 |
| **Conjugate 131** | 0.138 | 0.009 | 0.686 | 0.161 |
| **Conjugate 132** | 0.168 | 0.031 | 0.723 | 0.040 |
| **Conjugate 133** | 0.106 | 0.030 | 0.321 | 0.027 |
| **Conjugate 134** | 0.229 | 0.052 | 0.506 | 0.017 |
| **Conjugate 135** | 0.165 | 0.031 | 0.486 | 0.014 |
| **Conjugate 136** | 0.106 | 0.024 | 0.384 | 0.114 |
| **Conjugate 137** | 0.107 | 0.037 | 0.375 | 0.129 |
| **Conjugate 138** | 1.284 | 0.089 | 2.277 | 0.018 |
| **Conjugate 139** | 0.228 | 0.085 | 0.640 | 0.023 |
| **Conjugate 140** | 0.152 | 0.032 | 0.490 | 0.014 |
| **Conjugate 141** | 0.102 | 0.036 | 0.388 | 0.065 |
| **Conjugate 142** | 0.171 | 0.037 | 0.526 | 0.021 |
| **Conjugate 143** | 0.117 | 0.038 | 0.380 | 0.019 |
| **Conjugate 144** | 0.171 | 0.013 | 0.572 | 0.016 |
| **Conjugate 145** | 0.080 | 0.014 | 0.333 | 0.102 |
| **Conjugate 146** | 0.360 | 0.104 | 0.708 | 0.017 |
| **Conjugate 147** | 0.083 | 0.015 | 0.340 | 0.011 |
| **Conjugate 148** | 0.059 | 0.009 | 0.189 | 0.009 |
| **Conjugate 149** | 0.132 | 0.074 | 0.333 | 0.013 |
| **Conjugate 150** | 0.120 | 0.006 | 0.413 | 0.009 |
| **Conjugate 151** | 0.068 | 0.011 | 0.319 | 0.033 |
| **Conjugate 153** | 0.077 | 0.035 | 0.176 | 0.021 |
| **Conjugate 154** | 0.087 | 0.006 | 0.320 | 0.041 |
| **Conjugate 155** | 0.071 | 0.007 | 0.268 | 0.029 |
| **Conjugate 156** | 0.071 | 0.003 | 0.219 | 0.007 |
| **Conjugate 157** | 0.066 | 0.004 | 0.253 | 0.006 |
| **Conjugate 158** | 0.113 | 0.009 | 0.488 | 0.007 |
| **Conjugate 159** | 0.086 | 0.001 | 0.378 | 0.012 |
| **Conjugate 160** | 0.053 | 0.001 | 0.129 | 0.005 |
| **Conjugate 161** | 0.078 | 0.006 | 0.221 | 0.005 |
| **Conjugate 163** | 0.066 | 0.005 | 0.204 | 0.006 |
| **Conjugate 1606** | 0.071 | 0.004 | 0.279 | 0.014 |
| **Conjugate 1607** | 0.093 | 0.009 | 0.491 | 0.022 |
| **Conjugate 1608** | 0.074 | 0.001 | 0.398 | 0.017 |
| **Conjugate 1609** | 0.055 | 0.007 | 0.300 | 0.021 |
| **Conjugate 1610** | 0.052 | 0.004 | 0.295 | 0.022 |
| **Conjugate 1611** | 0.077 | 0.008 | 0.427 | 0.024 |
| **Conjugate 1612** | 0.070 | 0.002 | 0.330 | 0.022 |
| **Conjugate 1613** | 0.066 | 0.008 | 0.306 | 0.026 |
| **Conjugate 1617** | 0.043 | 0.001 | 0.106 | 0.001 |
| **Conjugate 1618** | 0.038 | 0.001 | 0.112 | 0.002 |
| **Conjugate 1619** | 0.042 | 0.005 | 0.093 | 0.011 |
| **Conjugate 1620** | 0.043 | 0.001 | 0.105 | 0.005 |
| **Conjugate 1621** | 0.046 | 0.009 | 0.112 | 0.008 |
| **Conjugate 1622** | 0.049 | 0.002 | 0.105 | 0.005 |
| **Conjugate 1623** | 0.048 | 0.000 | 0.120 | 0.007 |
| **Conjugate 1624** | 0.050 | 0.004 | 0.123 | 0.002 |
| **Conjugate 1625** | 0.048 | 0.005 | 0.138 | 0.014 |
| **Conjugate 1626** | 0.054 | 0.008 | 0.152 | 0.005 |
| **Conjugate 1627** | 0.054 | 0.004 | 0.126 | 0.006 |
| **Conjugate 1628** | 0.059 | 0.011 | 0.122 | 0.006 |
| **Conjugate 1629** | 0.059 | 0.006 | 0.128 | 0.007 |
| **Reference 1 (Conjugate 1614)** | 0.148 | 0.007 | 0.616 | 0.024 |
| **Reference 2 (Conjugate 1615)** | 0.215 | 0.006 | 0.816 | 0.005 |
| **Reference 3 (Conjugate 1616)** | 0.229 | 0.010 | 0.768 | 0.010 |
| **Conjugate 1646** | 0.171 | 0.026 | 0.497 | 0.039 |
| **Conjugate 1647** | 0.156 | 0.012 | 0.457 | 0.037 |
| **Conjugate 1648** | 0.134 | 0.013 | 0.486 | 0.021 |
| **Conjugate 1649** | 0.192 | 0.018 | 0.609 | 0.083 |
| **Conjugate 1650** | 0.109 | 0.017 | 0.386 | 0.025 |
| **Conjugate 1651** | 0.098 | 0.012 | 0.353 | 0.012 |
| **Conjugate 1652** | 0.175 | 0.026 | 0.605 | 0.037 |
| **Conjugate 1653** | 0.188 | 0.036 | 0.609 | 0.047 |
| **Conjugate 1654** | 0.174 | 0.028 | 0.593 | 0.068 |
| **Conjugate 1655** | 0.248 | 0.014 | 0.684 | 0.077 |
| **Conjugate 1656** | 0.173 | 0.015 | 0.491 | 0.056 |
| **Conjugate 1657** | 0.106 | 0.008 | 0.350 | 0.028 |
| **Conjugate 1658** | 0.201 | 0.028 | 0.688 | 0.083 |
| **Reference 4 (Conjugate 1660)** | 0.715 | 0.03 | 0.893 | 0.027 |
| **Reference 5 (Conjugate 1663)** | 0.753 | 0.021 | 0.915 | 0.046 |
| **Reference 6 (Conjugate 1664)** | 0.627 | 0.05 | 0.862 | 0.032 |
| **Reference 7 (Conjugate 1666)** | 0.713 | 0.041 | 0.906 | 0.072 |
| **Reference 8 (Conjugate 1667)** | 0.529 | 0.076 | 0.823 | 0.088 |
| **Reference 9 (Conjugate 1668)** | 0.553 | 0.048 | 0.892 | 0.054 |
| **PC c** | 0.064 | 0.002 | 0.262 | 0.006 |
| *: This value is the relative expression level of AGT mRNA compared to the blank control. | | | | |

**Table 2. Single-dose human primary liver cell activity**

| **Conjugate No.** | 10 nM | | 1.0 nM | |
|---|---|---|---|---|
| | Mean* | SD | Mean* | SD |
| **Conjugate 1** | 0.072 | 0.012 | 0.275 | 0.012 |
| **Conjugate 74** | 0.120 | 0.005 | 0.468 | 0.021 |
| **Conjugate 75** | 0.068 | 0.002 | 0.308 | 0.022 |
| **Conjugate 80** | 0.074 | 0.004 | 0.334 | 0.006 |
| **Conjugate 81** | 0.104 | 0.006 | 0.409 | 0.017 |
| **Conjugate 83** | 0.072 | 0.006 | 0.320 | 0.023 |
| **Conjugate 85** | 0.094 | 0.003 | 0.352 | 0.033 |
| **Conjugate 86** | 0.049 | 0.001 | 0.199 | 0.008 |
| **Conjugate 87** | 0.089 | 0.004 | 0.282 | 0.021 |
| **Conjugate 96** | 0.029 | 0.001 | 0.107 | 0.013 |
| **Conjugate 100** | 0.034 | 0.001 | 0.126 | 0.004 |
| **Conjugate 102** | 0.037 | 0.004 | 0.136 | 0.010 |
| **Conjugate 105** | 0.037 | 0.001 | 0.160 | 0.007 |
| **Conjugate 106** | 0.036 | 0.001 | 0.174 | 0.011 |
| **PC c** | 0.078 | 0.002 | 0.205 | 0.028 |
| **Conjugate 72** | 0.034 | 0.001 | 0.363 | 0.048 |
| **Conjugate 120** | 0.131 | 0.003 | 0.317 | 0.020 |
| **Conjugate 122** | 0.111 | 0.018 | 0.169 | 0.001 |
| **Conjugate 123** | 0.084 | 0.005 | 0.681 | 0.052 |
| **Conjugate 125** | 0.230 | 0.023 | 0.160 | 0.004 |
| **Conjugate 127** | 0.101 | 0.008 | 0.380 | 0.036 |
| **Conjugate 129** | 0.052 | 0.004 | 0.197 | 0.011 |
| **Conjugate 147** | 0.137 | 0.018 | 0.294 | 0.023 |
| **Conjugate 148** | 0.076 | 0.015 | 0.221 | 0.008 |
| **Conjugate 151** | 0.060 | 0.007 | 0.120 | 0.005 |
| **Conjugate 153** | 0.071 | 0.002 | 0.680 | 0.047 |
| **Conjugate 154** | 0.061 | 0.001 | 0.301 | 0.014 |
| **Conjugate 155** | 0.066 | 0.003 | 0.332 | 0.023 |
| **Conjugate 160** | 0.037 | 0.001 | 0.267 | 0.004 |
| **Conjugate 161** | 0.037 | 0.004 | 0.504 | 0.052 |
| *: This value is the relative expression level of AGT mRNA compared to the blank control. | | | | |

**Table 3. IC₅₀ assay results of Hep3B cells in transfection**

| **Conjugate No.** | **IC₅₀ (pM)** | **Duplex No.** | **IC₅₀ (pM)** |
|---|---|---|---|
| **Conjugate 68** | 3.397 | **Conjugate 71** | 4.482 |
| **Conjugate 100** | 3.361 ± 2.238 | **Conjugate 102** | 3.921 |
| **Conjugate 161** | 1.324 | **Conjugate 162** | 2.576 |
| **Conjugate 163** | 1.317 | | |
| **PC c** | 2.645± 0.837 | | |

**Table 4. IC₅₀ assay results of PCH cells in free uptake**

| **Conjugate No.** | **IC₅₀ (nM)** | **Duplex No.** | **IC₅₀ (nM)** |
|---|---|---|---|
| **Conjugate 100** | 0.286 | **Conjugate 102** | 0.311 |
| **Conjugate 161** | 0.133 | **Conjugate 162** | 0.056 |
| **Conjugate 163** | 0.192 | **PC c** | 0.102 |

**Table 5. IC₅₀ assay results of PHH cells in free uptake**

| **Conjugate No.** | **IC₅₀ (nM)** | **Duplex No.** | **IC₅₀ (nM)** |
|---|---|---|---|
| **Conjugate 100** | 0.094 | **Conjugate 102** | 0.072 |
| **Conjugate 161** | 0.142 | **Conjugate 162** | 0.196 |
| **Conjugate 163** | 0.073 | **PC c** | 0.173 |

### Example 3: Tritosome stability

The siRNA to be tested was mixed in rat tritosome (5 µM) and incubated at 37°C for 24h and 48h, respectively. After incubation, phenol-chloroform (Beyotime, Cat: #p1011) in 1/3 the sample volume was added. The mixture was vortexed and mixed, then allowed to stand for 5 min, and centrifuged at 12,000 rpm for 30 minutes. The uppermost aqueous phase was taken and transferred to a new centrifuge tube. 3M NaAC (Solarbio, Cat: #A1070) in 1/10 the volume was added and mixed, then added with pre-cooled absolute ethanol in 2.5 times the volume and 1 µL of glycogen (Beyotime, Cat: #0812), mixed, allowed to stand at -20°C for 2 minutes, and centrifuged at 12,000 rpm for 30 minutes. The supernatant was discarded, and the pellet was air-dried, and dissolved in RNase-free water. The dissolved sample was mixed with formamide loading buffer and subjected to nucleic acid electrophoresis using a 10% PAGE gel. After electrophoresis, the PAGE gel was stained with GelRed (Beyotime, Cat: #D0140) for 30 minutes. Finally, the PAGE gel was imaged using a gel imaging system (Bio-Rad), and grayscale quantification analysis was performed using the Image Lab software. The grayscale quantification results indicated that Conjugate 100 exhibited better stability in rat tritosome compared to the clinical candidate PC c.

### Example 4: Cytotoxicity assay

Cell Culture: Hep3B cells (ATCC) were cultured at 37°C and 5% CO₂ in MEM complete medium (Gibco, supplemented with 10% FBS) until near confluence. The cells were then trypsinized and seeded into a 24-well plate, with 0.5×10⁵ Hep3B cells and 0.5 mL of MEM complete medium (Gibco, supplemented with 10% FBS) added to each well, cultured at 37°C and 5% CO₂ for 16-24 hour, and then subjected to transfection.

Cell transfection: 24.25 µL of opt-MEM per well was mixed with 0.75 µL of lipofectamine RNAiMax (Invitrogen), and then added with 25 µL of siRNA and mixed. This mixture was added to a PCR tube and incubated at room temperature for 5 minutes. Finally, this siRNA mixture was added to the cells, cultured for 48 or 96 hours, and then subjected to cytotoxicity assay. This experiment was performed at 10 nM and 1 nM siRNA duplex concentrations.

### Cytotoxicity assay:

Cytotoxicity assay was performed using a CCK-8 assay kit (Beyotime, CAT: #C0040). After 48/96 hours of cell transfection, the culture medium was removed, and 500 µL of the corresponding complete culture medium (containing 10% CCK-8) was added to each well, and incubated at 37°C for 30 to 60 minutes in the dark. OD values of the samples were measured using a microplate reader (Tecan CAT: #spark 20M), with a wavelength of 450 nm and a reference wavelength of 620 nm. The assay results showed that the cytotoxicity of Conjugates 100, 102, and 106 was similar to that of the clinical PC c molecule, i.e., none of them had significant toxicity.

### Example 5: siRNA Immunogenicity assay

hPBMC cells (Shanghai Saili Biotechnology Co., Ltd., XFB-HP050A) from three different donors were centrifuged and resuspended in RPMI-1640 cell culture medium (containing 10% FBS and 1% Penicillin-streptomycin solution). The mixture was then incubated overnight at 37°C and 5% CO₂. The compound to be tested was diluted to form a 20× working solution. Lipofectamine^{®} 3000 and Opti-Mem were formulated into a mixed solution at a ratio of 1.5:23.5 and vortexed before use. The prepared 20× working solution and the Lipofectamine^{®} 3000 mixed solution were mixed at a ratio of 1:1 in a 96-well V-bottom plate. The overnight culture medium was discarded, and the hPBMC cells were resuspended in RPMI-1640 cell culture medium (containing 10% FBS and 1% Penicillin-streptomycin solution). After cell counting, the cells were diluted to the required transfection density and added to the 96-well plate. The final cell count in the cell plate was 2.0×10⁵ cells/well, with a total volume of 200 µL. The cell plate was then incubated for 24 h in a 5% CO₂, 37°C incubator.

After the hPBMC cells were subjected to 24 hours of transfection, the cell supernatant was collected, and the levels of IFN alpha, IL-6, and TNF alpha in the hPBMC supernatant were detected using the ProcartaPlex Mix & Match 3-plex Kit. The fold change of each factor in the compound to be tested was calculated, wherein, fold change for the test siRNA, naked siRNA, and polyIC = detected factor concentration / factor concentration in the transfection reagent well; fold change for GS9688 = detected factor concentration / factor concentration in the DMSO well. The results showed that Conjugates 100, 102, 161, 162, and 163 did not exhibit significant immune activation in hPBMCs.

### Example 6: Evaluation of siRNA seed region off-target potential

The sequences for detecting on-target activity and seed region off-target activity were constructed separately for each siRNA sequence. The on-target detection sequence was a sequence that perfectly matched the siRNA to be tested, while the seed region off-target detection sequence was a sequence that perfectly matched the seed region (positions 2-8 at the 5' end) of the antisense strand of the siRNA to be tested, with complete mismatches at all other positions. Each psiCHECK plasmid was inserted with five copies of either the on-target or the seed region off-target detection sequence.

293T cells were seeded at 20,000 cells/well in a 96-well plate; Lipofectamine 2000, the siRNA to be tested were used to co-transfect with 25 ng of the constructed on-target or off-target psiCHECK plasmids. After culturing at 37°C and 5% CO₂ for 24 h, fluorescence values were measured using a Dual-Glo luciferase assay system (Promega, E2920). A control group was prepared by transfecting the psiCHECK plasmid without siRNA molecule. Each duplex was transfected 3-4 times independently, and each siRNA had transfection concentrations of 50 nM, 10 nM, 2.0 nM, 0.4 nM, 0.08 nM, 0.016 nM, and 0.0032 nM. The target inhibition effect was determined by measuring Renilla luciferase levels normalized to the constitutively expressed firefly luciferase levels, and the IC₅₀ of the on-target or seed-region off-target activity of the siRNA was calculated. The seed-region off-target risk of the siRNA was assessed by the ratio of the seed-region off-target activity IC₅₀ to the on-target activity IC₅₀. The test results showed that the off-target risks due to the seed region for Conjugate 100, Conjugate 102, and Conjugate 161 were lower than that of the clinical molecule PC c.

### Example 7: In vivo activity assay in hAGT mice

Male humanized hAGT mice aged 6-8 weeks (provided by Jiangsu Jicui, T054372) were used. Prior to grouping and dosing, the mice were not fasted. Blood was collected and serum was separated for hAGT detection by ELISA (abcam #). Based on hAGT levels, the mice were randomly divided into 4 groups, 6 mice in each group, designated as day 0. On day 1, PBS, PC c, Conjugate 100, Conjugate 102, and Conjugate 106 were subcutaneously injected at a dose of 1.0 mpk. On days 8, 15, 22, 29, 36, 43, and 50 after dosing, blood was collected and serum was separated for hAGT detection by ELISA. The serum protein concentration at each time point was compared with that of the PBS group at the corresponding time point. The detection results for the different conjugates were shown in Table 6. The candidate compounds showed superior in vitro inhibition of serum hAGT protein in hAGT mice compared to the clinical molecule PC c.

**Table 6. Changes in hAGT protein expression before and after administration of different modified siRNA duplexes.**

| **Conjugate** | **day 0** | | **day 8** | | **day 15** | | **day 22** | | **day 29** | | **day 36** | |
|---|---|---|---|---|---|---|---|---|---|---|---|---|
| | **Mea n*** | **SD** | **Mea n*** | **SD** | **Mea n*** | **SD** | **Mea n*** | **SD** | **Mea n*** | **SD** | **Mea n*** | **SD** |
| **PBS** | 1.00 0 | 0.0 64 | 1.00 0 | 0.1 34 | 1.00 0 | 0.0 73 | 1.00 0 | 0.0 76 | 1.00 0 | 0.1 35 | 1.00 0 | 0.1 75 |
| **PC c** | 1.00 0 | 0.1 05 | 0.27 9 | 0.0 44 | 0.36 3 | 0.1 91 | 0.28 2 | 0.0 26 | 0.40 5 | 0.0 32 | 0.54 5 | 0.0 49 |
| **Conjugate 100** | 0.99 5 | 0.1 42 | 0.11 6 | 0.0 32 | 0.23 5 | 0.0 16 | 0.24 7 | 0.0 29 | 0.28 8 | 0.0 45 | 0.33 5 | 0.0 67 |
| **Conjugate 102** | 0.99 8 | 0.0 86 | 0.09 4 | 0.0 20 | 0.18 0 | 0.1 04 | 0.23 9 | 0.1 04 | 0.26 3 | 0.0 46 | 0.35 1 | 0.0 97 |
| **Conjugate 106** | 1.00 1 | 0.0 54 | 0.22 4 | 0.0 12 | 0.34 4 | 0.0 44 | 0.27 7 | 0.0 16 | 0.42 3 | 0.0 13 | 0.52 7 | 0.1 39 |
| **Reference 4 (Conjugate 1660)** | 1.000 | 0.00 0 | 1.032 | 0.08 0 | 1.050 | 0.10 6 | 1.024 | 0.12 2 | | | | |
| **Reference 5 (Conjugate 1663)** | 1.000 | 0.00 0 | 1.065 | 0.24 9 | 1.034 | 0.25 5 | 1.024 | 0.20 7 | | | | |
| **Reference 6 (Conjugate 1664)** | 1.000 | 0.00 0 | 0.857 | 0.18 5 | 0.886 | 0.21 6 | 0.953 | 0.21 3 | | | | |
| **Reference 7 (Conjugate 1666)** | 1.000 | 0.00 0 | 1.127 | 0.13 5 | 1.190 | 0.08 2 | 0.984 | 0.09 1 | | | | |
| | | | | | | | | | | | | |

| **Conjugate** | **day 43** | | **day 50** | | | | | | | | | |
|---|---|---|---|---|---|---|---|---|---|---|---|---|
| | **Mea n*** | **SD** | **Mea n*** | **SD** | | | | | | | | |
| **PBS** | 1.00 0 | 0.1 21 | 1.00 0 | 0.0 65 | | | | | | | | |
| **PC c** | 0.53 2 | 0.0 56 | 0.52 3 | 0.0 59 | | | | | | | | |
| **Conjugate 100** | 0.37 0 | 0.0 34 | 0.39 2 | 0.0 43 | | | | | | | | |
| **Conjugate 102** | 0.32 1 | 0.0 74 | 0.33 7 | 0.0 52 | | | | | | | | |
| **Conjugate 106** | 0.59 2 | 0.0 42 | 0.37 4 | 0.0 80 | | | | | | | | |

| | | | | | | | | | | | | |
|---|---|---|---|---|---|---|---|---|---|---|---|---|
| * These values were relative expression levels of hAGT protein compared to that of the PBS control group. | | | | | | | | | | | | |

**Table 7. Changes in hAGT protein expression before and after administration of siRNA duplexes modified by conjugation with different delivery carriers**

| **Conjugate** | **day 0** | | **day 8** | | **day 15** | | **day 22** | | **day 29** | | **day 36** | |
|---|---|---|---|---|---|---|---|---|---|---|---|---|
| | **Mea n*** | **SD** | **Mea n*** | **SD** | **Mea n*** | **SD** | **Mea n*** | **SD** | **Mea n*** | **SD** | **Mea n*** | **SD** |
| **PBS** | 1.000 0 | 0.000 0 | 0.904 6 | 0.091 8 | 1.018 6 | 0.146 1 | 0.970 3 | 0.130 5 | 0.886 8 | 0.104 8 | 1.000 3 | 0.161 4 |
| **Conjugate 100** | 1.000 0 | 0.000 0 | 0.218 6 | 0.038 6 | 0.210 1 | 0.042 1 | 0.214 0 | 0.045 7 | 0.211 8 | 0.034 5 | 0.258 1 | 0.076 4 |
| **Conjugate 1633** | 1.000 0 | 0.000 0 | 0.220 3 | 0.042 1 | 0.195 4 | 0.030 7 | 0.196 9 | 0.037 1 | 0.214 2 | 0.044 4 | 0.246 0 | 0.049 8 |
| **Conjugate 1634** | 1.000 0 | 0.000 0 | 0.245 4 | 0.046 4 | 0.213 0 | 0.030 1 | 0.209 7 | 0.034 1 | 0.233 5 | 0.044 8 | 0.257 3 | 0.054 0 |
| **Conjugate 1637** | 1.000 0 | 0.000 0 | 0.276 4 | 0.070 7 | 0.253 8 | 0.084 2 | 0.282 1 | 0.109 7 | 0.270 8 | 0.087 5 | 0.306 7 | 0.112 4 |
| **Conjugate 1638** | 1.000 0 | 0.000 0 | 0.240 8 | 0.053 5 | 0.233 4 | 0.100 6 | 0.232 7 | 0.092 0 | 0.262 7 | 0.107 9 | 0.267 3 | 0.069 1 |
| **Conjugate 1642** | 1.000 0 | 0.000 0 | 0.213 8 | 0.034 3 | 0.214 9 | 0.036 1 | 0.206 8 | 0.044 4 | 0.236 1 | 0.049 6 | 0.293 9 | 0.031 3 |

| | | | | | | | | | | | | |
|---|---|---|---|---|---|---|---|---|---|---|---|---|
| * These values were relative expression levels of hAGT protein compared to that of the PBS control group. | | | | | | | | | | | | |

### Example 8: In vivo activity assay in cynomolgus monkeys

In this experiment, 12 male cynomolgus monkeys aged 3-6 years (weighing 2.5-6.0 kg) were selected based on body weight, body temperature, blood pressure, electrocardiogram, serum AGT level, hematology, and blood biochemistry. They were divided into 4 groups with 3 monkeys per group. Each animal was subcutaneously injected with 3.0 mpk of the test conjugate molecule.

After a 12-hour fast, blood was collected from the cynomolgus monkeys at a fixed time weekly before administration (day 0) and after administration. Whole blood samples were placed in centrifuge tubes without anticoagulant, kept in an ice box to coagulate for 30 minutes, and then centrifuged at approximately 1800× g for 10 minutes at 2-8 °C. The serum was separated and stored below -80 °C.

Serum AGT protein levels were measured using an AGT ELISA detection kit (IBL, 27412). The AGT protein content at each detection time point was compared with that in the same monkey's serum before administration (day 0) to calculate the inhibition of serum AGT content by the test conjugate at different time points. As shown in Table 8, the candidate compound at a 3.0 mpk dose achieved over 90% inhibition of serum AGT protein in cynomolgus monkeys, showing no significant difference from the results reported for the clinical molecule PC c in the patent (WO_2019222166_A1).

**Table 8. Inhibition of serum AGT protein in cynomolgus monkeys after administration of different conjugates**

| **Conjugate** | **Dose** | **day 8** | **day 15** | **day 22** | **day 29** |
|---|---|---|---|---|---|
| **Conjugate 100** | 3.0 mpk | 70.33% | 89.87% | 93.08% | 93.96% |
| **Conjugate 161** | 3.0 mpk | 68.04% | 88.61% | 92.55% | 92.46% |
| **Conjugate 162** | 3.0 mpk | 64.72% | 87.38% | 87.19% | 84.30% |
| **Conjugate 163** | 3.0 mpk | 66.35% | 87.17% | 86.84% | 80.79% |

## Claims

1. A siRNA for inhibiting AGT gene expression, wherein the siRNA comprises a sense strand and an antisense strand; wherein, the antisense strand comprises at least 17 consecutive nucleotides differing by no more than 4 nucleotides from any one of the nucleotide sequences as set forth in SEQ ID NO: 102 to SEQ ID NO: 192, SEQ ID NO: 201 to SEQ ID NO: 207, SEQ ID NO: 209 to SEQ ID NO: 220, SEQ ID NO: 222 to SEQ ID NO: 223, SEQ ID NO: 225 to SEQ ID NO: 233, SEQ ID NO: 235 to SEQ ID NO: 250, SEQ ID NO: 252 to SEQ ID NO: 291, SEQ ID NO: 293 to SEQ ID NO: 341, SEQ ID NO: 343 to SEQ ID NO: 346, the antisense strand is 17-30 nucleotides in length; and the sense strand is 17-30 nucleotides in length and is at least partially complementary to the antisense strand.

2. The siRNA according to claim 1, wherein the antisense strand is 19-27 nucleotides in length; and the sense strand is 19-25 nucleotides in length.

3. The siRNA according to claim 1, wherein the antisense strand differs by no more than 4 nucleotides from any one of the nucleotide sequences as set forth in SEQ ID NO: 102 to SEQ ID NO: 192, SEQ ID NO: 201 to SEQ ID NO: 207, SEQ ID NO: 209 to SEQ ID NO: 220, SEQ ID NO: 222 to SEQ ID NO: 223, SEQ ID NO: 225 to SEQ ID NO: 233, SEQ ID NO: 235 to SEQ ID NO: 250, SEQ ID NO: 252 to SEQ ID NO: 291, SEQ ID NO: 293 to SEQ ID NO: 341, and SEQ ID NO: 343 to SEQ ID NO: 346.

4. The siRNA according to claim 1, wherein the sense strand and the antisense strand have a mismatch of no more than 3 nucleotides.

5. The siRNA according to claims 1-4, wherein the sequence of the siRNA is selected from duplex 1 to duplex 107.

6. The siRNA according to any one of claims 1-5, wherein the siRNA comprises at least one modified nucleotide.

7. The siRNA according to claim 6, wherein all nucleotides in the sense strand and/or the antisense strand of the siRNA are modified nucleotides or nucleotide analogs.

8. The siRNA according to claim 7, wherein the modified nucleotide is selected from the group consisting of 2'-methoxynucleotides, 2'-fluoronucleotides, 2'-deoxynucleotides, 2',3'-seco-nucleotide analogs, 2'-fluoroarabinonucleotides, 2'-methoxyethylnucleotides, 2'-amino-modified nucleotides, 2'-alkyl-modified nucleotides, 3'-methoxynucleotides, 2'-allyl-modified nucleotides, phosphorothioate group-containing nucleotides, methylphosphonate group-containing nucleotides, 5'-phosphate group-containing nucleotides, 5'-phosphate mimic-containing nucleotides, diol-modified nucleotides, abasic nucleotides, morpholinonucleotides, locked nucleotides, unlocked nucleotides, threose nucleotides, and glycerol nucleotides.

9. The siRNA according to any one of claims 1 to 8, wherein the 5' end and 3' end of the sense strand each independently comprise 0, 1, or 2 phosphorothioate linkages; and/or the 5' end and 3' end of the antisense strand each independently comprise1 or 2 phosphorothioate linkages.

10. The siRNA according to any one of claims 1 to 9, wherein the first nucleotide at the 5' end of the antisense strand is a (E)-vinylphosphate-modified nucleotide.

11. The siRNA according to any one of claims 1 to 10, wherein the siRNA comprises at least one base-modified nucleotide.

12. The siRNA according to any one of claims 1 to 11, wherein
the antisense strand of the siRNA is 22 nucleotides in length, wherein 3-10 positions (e.g., positions 2, 14, 16; positions 2, 5, 14, 16; positions 2, 6, 14, 16; positions 2, 4, 6, 14, 16; positions 2, 6, 10, 14, 16; positions 2, 6, 12, 14, 16; positions 2, 5, 10, 14, 16; positions 2, 3, 12, 14, 16; positions 2, 9, 12, 14, 16; positions 2, 6, 8, 9, 14, 16; positions 2, 3, 5, 12, 14, 16; positions 2, 8, 9, 12, 14, 16; positions 2, 7, 9, 12, 14, 16; positions 2, 4, 6, 8, 10, 14, 16, 18, 20; positions 2, 4, 5, 6, 8, 10, 12, 14, 16, 18 at the 5' end) of the antisense strand are2'-fluoronucleotides, and the remaining positions are 2'-methoxynucleotides,
the sense strand of the siRNA is 20 nucleotides in length, wherein positions 8, 9, and 10 at the 5' end of the sense strand are 2'-fluoronucleotides, optionally one position (preferably position 1 at the 5' end) is a threose nucleotide, optionally one position (preferably position 6 at the 5' end) is a 2'-deoxynucleotide, and the remaining positions are 2'-methoxynucleotide conjugates;
preferably, the antisense strand of the siRNA is 22 nucleotides in length, wherein the following positions: positions 2, 14, 16; or positions 2, 5, 14, 16; or positions 2, 6, 14, 16; or positions 2, 4, 6, 14, 16; or positions 2, 6, 10, 14, 16; or positions 2, 6, 12, 14, 16; or positions 2, 5, 10, 14, 16; or positions 2, 3, 12, 14, 16; or positions 2, 9, 12, 14, 16; or positions 2, 6, 8, 9, 14, 16; or positions 2, 3, 5, 12, 14, 16; or positions 2, 8, 9, 12, 14, 16; or positions 2, 7, 9, 12, 14, 16; or positions 2, 4, 6, 8, 10, 14, 16, 18, 20; or positions 2, 4, 5, 6, 8, 10, 12, 14, 16, 18, at the 5' end of the antisense strand are 2'-fluoronucleotides, and the remaining positions are 2'-methoxynucleotides,
the sense strand of the siRNA is 20 nucleotides in length, wherein positions 8, 9, and 10 at the 5' end of the sense strand are 2'-fluoronucleotides, optionally, position 1 at the 5' end is a threose nucleotide, or optionally, position 6 at the 5' end is a 2'-deoxynucleotide, and the remaining positions are 2'-methoxynucleotides;
preferably, the 2'-deoxynucleotide at position 6 is independently selected from the group consisting of deoxyadenosine monophosphate (dA), deoxyguanosine monophosphate (dG), deoxycytidine monophosphate (dC), deoxythymidine monophosphate (dT), deoxyuridine monophosphate (dU); more preferably, the 2'-deoxynucleotide at position 6 is deoxythymidine monophosphate (dT);
more preferably, the antisense strand of the siRNA is 22 nucleotides in length, wherein the following positions: positions 2, 14, 16; or positions 2, 5, 14, 16; or positions 2, 6, 14, 16; or positions 2, 4, 6, 14, 16; or positions 2, 6, 10, 14, 16; or positions 2, 6, 12, 14, 16; or positions 2, 5, 10, 14, 16; or positions 2, 3, 12, 14, 16; or positions 2, 9, 12, 14, 16; or positions 2, 6, 8, 9, 14, 16; or positions 2, 3, 5, 12, 14, 16; or positions 2, 8, 9, 12, 14, 16; or positions 2, 7, 9, 12, 14, 16; or positions 2, 4, 6, 8, 10, 14, 16, 18, 20; or positions 2, 4, 5, 6, 8, 10, 12, 14, 16, 18, at the 5' end of the antisense strand are 2'-fluoronucleotides, and the remaining positions are 2'-methoxynucleotides; the sense strand of the siRNA is 20 nucleotides in length, wherein positions 8, 9, 10 at the 5' end of the sense strand are 2'-fluoronucleotides, and the remaining positions are 2'-methoxynucleotides; or
the antisense strand of the siRNA is 22 nucleotides in length, wherein positions 2, 6, 14, 16 at the 5' end of the antisense strand are 2'-fluoronucleotides, and the remaining positions are 2'-methoxynucleotides; the sense strand of the siRNA is 20 nucleotides in length, wherein positions 8, 9, and 10 at the 5' end of the sense strand are 2'-fluoronucleotides, position 1 is a threose nucleotide, and the remaining positions are 2'-methoxynucleotides; or
the antisense strand of the siRNA is 22 nucleotides in length, wherein positions 2, 6, 14, 16 at the 5' end of the antisense strand are 2'-fluoronucleotides, and the remaining positions are 2'-methoxynucleotides; the sense strand of the siRNA is 20 nucleotides in length, wherein positions 8, 9, 10 at the 5' end of the sense strand are 2'-fluoronucleotides, position 6 is a 2'-deoxynucleotide, and the remaining positions are 2'-methoxynucleotides; preferably, the 2'-deoxynucleotide at position 6 is independently selected from the group consisting of deoxyadenosine monophosphate (dA), deoxyguanosine monophosphate (dG), deoxycytidine monophosphate (dC), deoxythymidine monophosphate (dT), deoxyuridine monophosphate (dU); more preferably, the 2'-deoxynucleotide at position 6 is deoxythymidine monophosphate (dT);
more preferably, the antisense strand of the siRNA is the nucleotide sequence as set forth in SEQ ID NO: 105, and the sense strand of the siRNA is the nucleotide sequence as set forth in SEQ ID NO: 23.

13. The siRNA according to any one of claims 1 to 11, wherein the antisense strand of the siRNA is 22 nucleotides in length, wherein positions 2, 6, 14, 16 at the 5' end of the antisense strand are 2'-fluoronucleotides, and the remaining positions are 2'-methoxynucleotides; the sense strand of the siRNA is 20 nucleotides in length, wherein positions 8, 9, 10 at the 5' end of the sense strand are 2'-fluoronucleotides, and the remaining positions are 2'-methoxynucleotides;
preferably, the antisense strand of the siRNA is the nucleotide sequence as set forth in SEQ ID NO: 116, and the sense strand of the siRNA is the nucleotide sequence as set forth in SEQ ID NO: 22.

14. The siRNA according to any one of claims 1 to 11, wherein
the antisense strand of the siRNA is 22 nucleotides in length, wherein position 14 at the 5' end of the antisense strand is a 2'-fluoronucleotide, positions 2, 5, 7 are 2'-deoxynucleotides, position 12 is a threose nucleotide, and the remaining positions are 2'-methoxynucleotides; the sense strand of the siRNA is 20 nucleotides in length, wherein positions 8, 9, 10 at the 5' end of the sense strand are 2'-fluoronucleotides, and the remaining positions are 2'-methoxynucleotides;
preferably, the 2'-deoxynucleotides at positions 2, 5, 7 are independently selected from the group consisting of deoxyadenosine monophosphate (dA), deoxyguanosine monophosphate (dG), deoxycytidine monophosphate (dC), deoxythymidine monophosphate (dT), and deoxyuridine monophosphate (dU);
further preferably, the 2'-deoxynucleotides at positions 2, 5, 7 are, respectively, deoxythymidine monophosphate (dT), deoxythymidine monophosphate (dT), and deoxyadenosine monophosphate (dA);
more preferably, the antisense strand of the siRNA is the nucleotide sequence as set forth in SEQ ID NO: 105, and the sense strand of the siRNA is the nucleotide sequence as set forth in SEQ ID NO: 23.

15. The siRNA according to any one of claims 1 to 11, wherein
the antisense strand of the siRNA is 21 nucleotides in length, wherein positions 2, 6, 14, 16 at the 5' end of the antisense strand are 2'-fluoronucleotides, and the remaining positions are 2'-methoxynucleotides; the sense strand of the siRNA is 19 nucleotides in length, wherein positions 7, 8, 9 at the 5' end of the sense strand are 2'-fluoronucleotides, position 1 is a threose nucleotide, and the remaining positions are 2'-methoxynucleotides; or
the antisense strand of the siRNA is 21 nucleotides in length, wherein position 14 at the 5' end of the antisense strand is a 2'-fluoronucleotide, positions 2, 5, 7 are 2'-deoxynucleotides, position 12 is a threose nucleotide, and the remaining positions are 2'-methoxynucleotides; the sense strand of the siRNA is 19 nucleotides in length, wherein positions 7, 8, 9 at the 5' end of the sense strand are 2'-fluoronucleotides, and the remaining positions are 2'-methoxynucleotides;
preferably, the 2'-deoxynucleotides at positions 2, 5, and 7 are independently selected from the group consisting of deoxyadenosine monophosphate (dA), deoxyguanosine monophosphate (dG), deoxycytidine monophosphate (dC), deoxythymidine monophosphate (dT), and deoxyuridine monophosphate (dU);
further preferably, the 2'-deoxynucleotides at positions 2, 5, and 7 are respectively: deoxythymidine monophosphate (dT), deoxyuridine monophosphate (dU), and deoxyadenosine monophosphate (dA);
more preferably, the antisense strand of the siRNA is the nucleotide sequence as set forth in SEQ ID NO: 104, and the sense strand of the siRNA is the nucleotide sequence as set forth in SEQ ID NO: 3.

16. The siRNA according to any one of claims 12 to 15, wherein the 5' end and 3' end of the sense strand each independently comprise 0, 1, or 2 phosphorothioate linkages; and/or, the 5' end and 3' end of the antisense strand each independently comprise 1 or 2 phosphorothioate linkages.

17. The siRNA according to any one of claims 12 to 16, wherein the first nucleotide at the 5' end of the antisense strand is a (E)-vinylphosphate-modified nucleotide.

18. The siRNA according to any one of claims 12 to 17, wherein the 3' end and/or 5' end of the sense strand each are attached to an inverted abasic residue (iab), preferably the 3' end and 5' end of the sense strand each are attached to an inverted abasic residue (iab).

19. An siRNA conjugate, which is obtained by conjugating the siRNA according to any one of claims 1 to 18 with a conjugating molecule.

20. The siRNA conjugate according to claim 19, wherein the conjugating molecule is derived from a delivery carrier moiety via a covalent bond, and is independently or simultaneously linked to the 3' or 5' end of the sense strand of the siRNA.

21. The siRNA conjugate according to claim 20, wherein the delivery carrier moiety is selected from the group consisting of: GalNAc(L96), Ser(GN), GalNAc(Serl), GalNAc(Ser2), GalNAc(Ser3), GalNAc(Ser4), LP-GalNAc, XY-GalNAc, GalNAc(A1GN), GalNAc(A2GN), GalNAc(A3GN), GalNAc(A4GN), GalNAc(A5GN), GAlNAc(NAG25), and GAINAc(NAG37),
wherein,
GalNAc(L96) has the following structure: 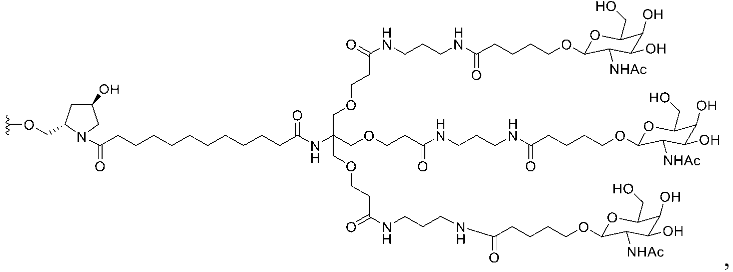
Ser(GN) has the following structure: 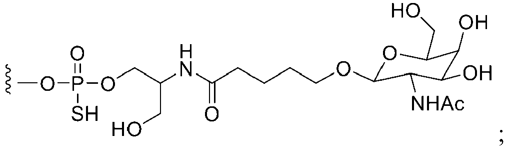
GalNAc(Ser1), GalNAc(Ser2), GalNAc(Ser3), GalNAc(Ser4) have the following structures, respectively: 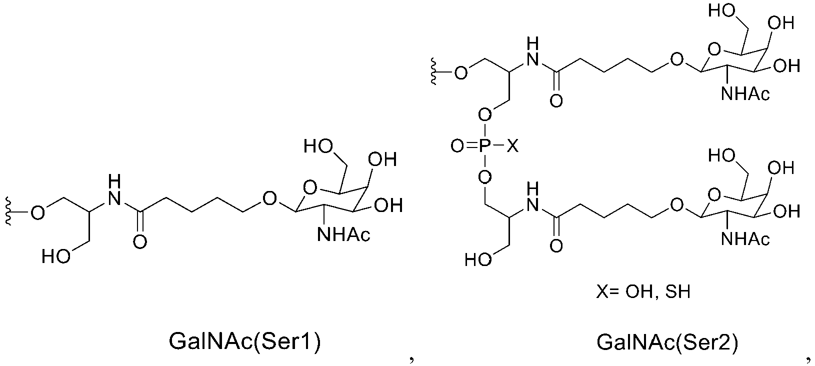 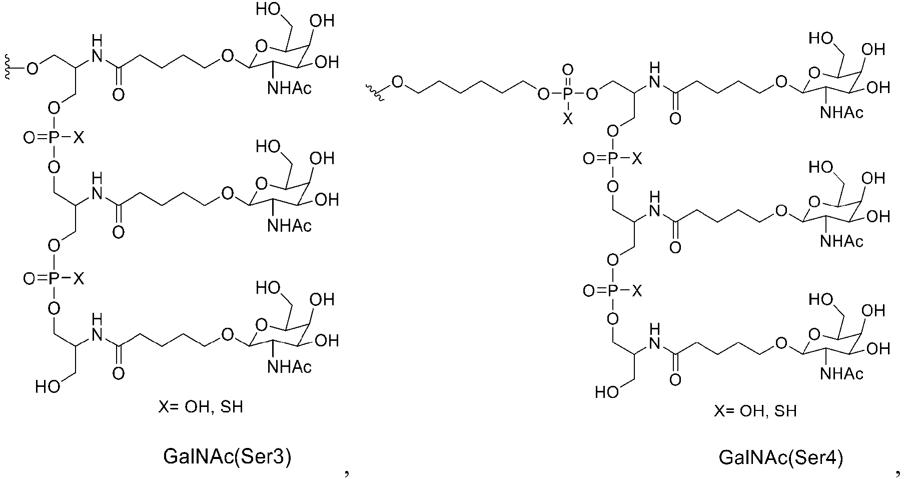
LP-GalNAc has the following structure: 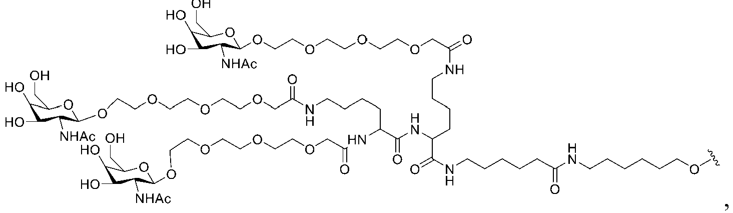
XY-GalNAc has the following structure: 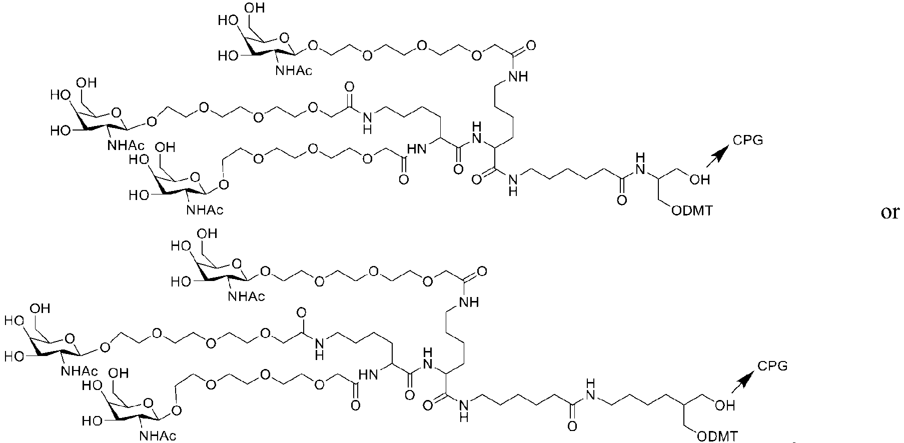
GalNAc(A1GN), GalNAc(A2GN), GalNAc(A3GN), GalNAc(A4GN), GalNAc(A5GN), GAlNAc(NAG25), and GAINAc(NAG37) have the following structures, respectively: 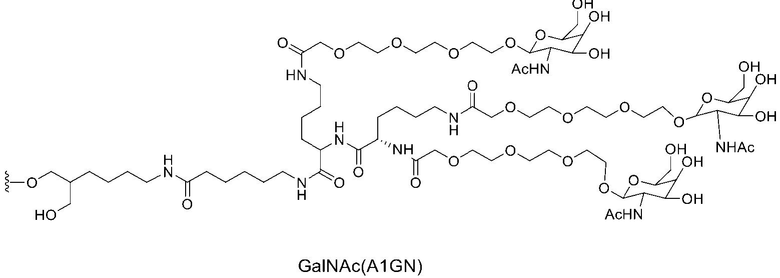 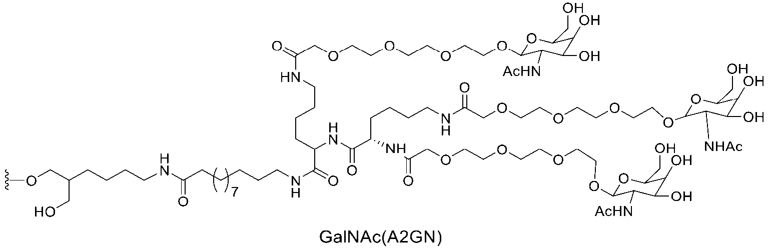 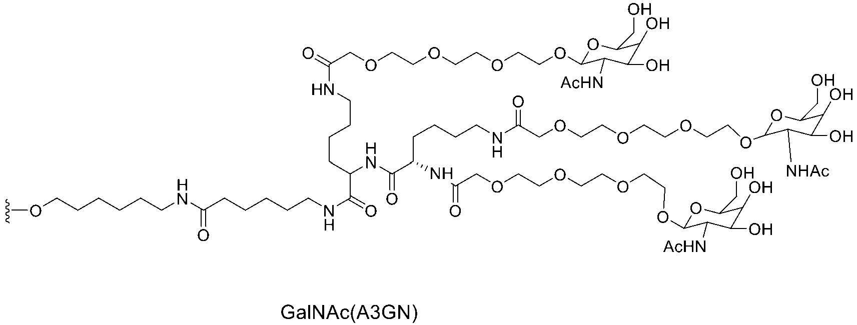 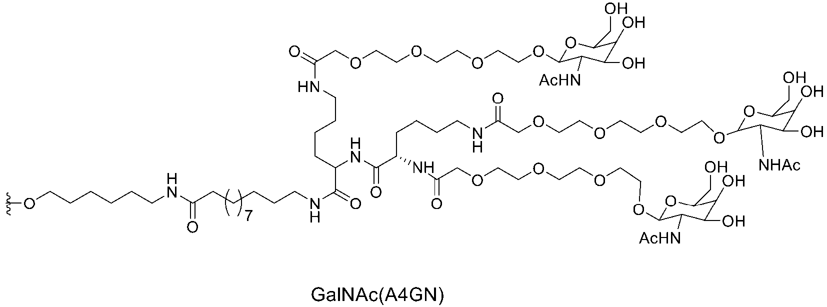 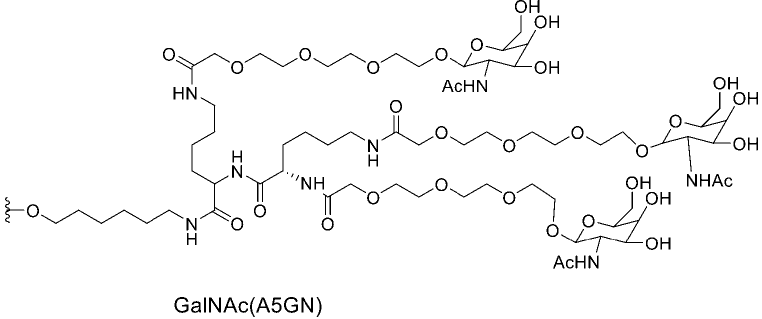 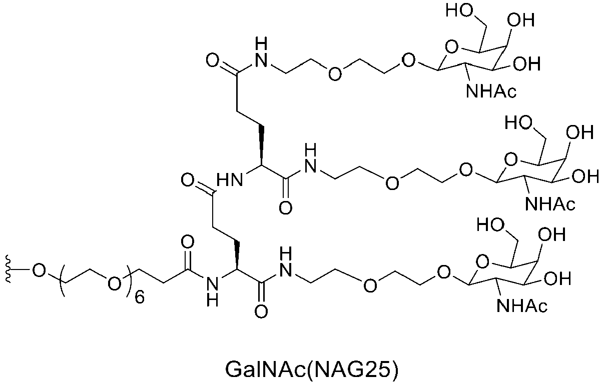 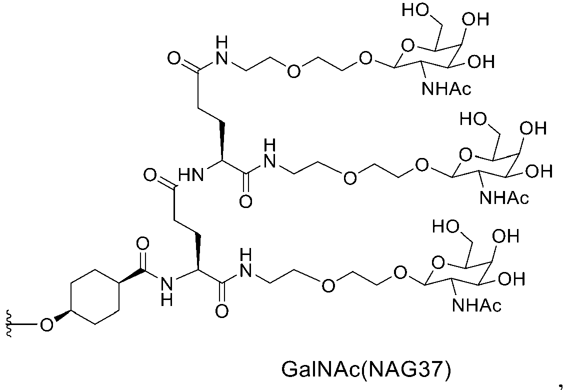
optionally, the above delivery carrier moiety is attached to the 3' end of the siRNA's sense strand, or to the 5' end of the siRNA's sense strand, or to an inverted abasic residue (iab) at the 3' end of the siRNA's sense strand, or to an inverted abasic residue (iab) at the 5' end of the siRNA's sense strand, or one or more of the above delivery carrier moieties are simultaneously attached to the 3' end of the siRNA's sense strand, the 5' end of the siRNA's sense strand, the inverted abasic residue (iab) at the 3' end of the siRNA's sense strand, or the inverted abasic residue (iab) at the 5' end of the siRNA's sense strand; preferably, the delivery carrier moiety GalNAc(L96) is attached to the 3' end of the siRNA's sense strand or to the inverted abasic residue (iab) at the 3' end of the siRNA's sense strand, the delivery carrier moiety Ser (GN) is simultaneously attached to the 3' end and the 5' end of the siRNA's sense strand, the delivery carrier moiety LP-GalNAc is attached to the 5' end of the siRNA's sense strand, the delivery carrier moiety XY-GalNAc is attached to the 3' end of the siRNA's sense strand, the delivery carrier moiety GalNAc(Ser2) is attached to the 3' end of the siRNA's sense strand or to the 5' end of the siRNA's sense strand or simultaneously to the 3' end and the 5' end of the siRNA's sense strand, the delivery carrier moiety GalNAc(Ser3) is attached to the 3' end of the siRNA's sense strand or to the 5' end of the siRNA's sense strand, the delivery carrier moiety GalNAc(Ser4) is attached to the 3' end of the siRNA's sense strand, the delivery carrier moiety GalNAc(A1GN) is attached to the 3' end of the siRNA's sense strand, the delivery carrier moiety GalNAc(A2GN) is attached to the 3' end of the siRNA's sense strand, the delivery carrier moiety GalNAc(A3GN) is attached to the 5' end of the siRNA's sense strand, the delivery carrier moiety GalNAc(A4GN) is attached to the 5' end of the siRNA's sense strand, the delivery carrier moiety GalNAc(A5GN) is attached to the 5' end of the siRNA's sense strand, the delivery carrier moiety GalNAc(NAG25) is attached to the 5' end of the siRNA's sense strand, the delivery carrier moiety GalNAc(NAG37) is attached to the 5' end of the siRNA's sense strand, and the delivery carrier moieties GalNAc(Ser1) and GalNAc(Ser2) are simultaneously attached to the 3' end and 5' end of the siRNA's sense strand, respectively, or simultaneously attached to the 5' end and 3' end of the siRNA's sense strand, respectively;
preferably, the delivery carrier moiety is selected from the group consisting of: GalNAc(L96) attached to the 3' end of the siRNA's sense strand, GalNAc(A1GN) attached to the 3' end of the siRNA's sense strand, GalNAc(A2GN) attached to the 3' end of the siRNA's sense strand, GalNAc(A3GN) attached to the 5' end of the siRNA's sense strand, GalNAc(A4GN) attached to the 5' end of the siRNA's sense strand, and GalNAc(Ser2) simultaneously attached to both the 3' and 5' ends of the siRNA's antisense strand.

22. The siRNA conjugate according to any one of claims 19 to 21, wherein the siRNA conjugate is selected from Conjugates 1 to 1613 and Conjugates 1617 to 1658.

23. A pharmaceutical composition, wherein the pharmaceutical composition comprises the siRNA according to any one of claims 1 to 18 and/or the siRNA conjugate according to any one of claims 19 to 22, and a pharmaceutically acceptable carrier.

24. Use of the siRNA according to any one of claims 1 to 18 and/or the siRNA conjugate according to any one of claims 19 to 22 and/or the pharmaceutical composition according to claim 23 in the manufacture of a medicament for treating and/or preventing a pathological condition or disease associated with AGT gene overexpression.

25. The use according to claim 24, wherein the pathological condition or disease is a disease associated with abnormal blood pressure.
